# EUROPEAN PATENT APPLICATION

(11) **EP 2 275 575 A2**
(43) Date of publication of application: **19.01.2011**
(21) Application number: 10175418.2
(22) Date of filing: 14.08.1998
(51) Int. Cl.: C12Q 1/68, C07K 14/47

(54) **Coding sequence haplotypes of the human BRCA2 gene**

(30) Priority: 15.08.1997 US 55784 P; 07.11.1997 US 64926 P; 12.11.1997 US 65367 P; 01.05.1998 US 71715; 22.05.1998 US 84471
(62) Divisional of application: 98945756.9
(71) Applicant: Gene Logic Inc., Gaithersburg, MD 20879 (US)
(72) Inventor: The designation of the inventor has not yet been filed
(74) Representative: Vossius & Partner

(57) **Abstract**

Five DNA and protein sequences have been determined for the BRCA2 gene, as have been ten polymorphic sites and their rates of occurrence in the normal alleles of BRCA2. The sequences BRCA^{(omil-5)} and the ten polymorphic sites will provide accuracy and reliability for genetic testing. One skilled in the art will be able to avoid misinterpretations of changes in the gene and/or protein sequence, determine the presence of a normal sequence, and of mutations of BRCA2. This invention is also related to a method of performing gene therapy with BRCA2^{(omil-5)} coding sequences or fragments thereof. This invention is further related to protein therapy with BRCA2^{(omil-5)} proteins or their functional equivalents.

## Description

This is an U.S. utility patent application based on U.S. Provisional Application Serial Nos. 60/055,784 filed on August 15, 1997, 60/064,926 filed on November 7, 1997, and 60/065,367 filed on November 12, 1997.

### FIELD OF THE INVENTION

This invention relates to a gene which has been associated with breast cancer where the gene is found to be mutated. More specifically, this invention relates to five unique coding sequences of BRCA2 gene BRCA2^{(omi1)}, BRCA2^{(omi2)}, BRCA2^{(omi3)}, BRCA2^{(omi4)}, and BRCA2^{(omi5)} identified in human subjects which define five novel haplotype.

### BACKGROUND OF THE INVENTION

It has been estimated that about 5-10% of breast cancer is inherited (Rowell, S., et al., American Journal of Human Genetics 55:861-865 (1994)). The first gene associated with both breast and ovarian cancer was cloned in 1994 from chromosome 17 by Miki, Y., et a/., Science 266:66-71 (1994). A second high-risk breast cancer conferring gene was located on chromosome 13 in 1994 (Wooster, R., et al., Science 265:2088-2090) and subsequently cloned in 1995 (Wooster, R., et al., Nature 378:789-792). Mutations in this "tumor suppressor" gene are thought to account for roughly 35% of inherited breast cancer and 80-90% of families with male breast cancer.

Locating one or more mutations in the BRCA2 region of chromosome 13 provides a promising approach to reducing the high incidence and mortality associated with breast cancer through the early detection of women and men at high risk. These individuals, once identified, can be targeted for more aggressive prevention programs. Screening is carried out by a variety of methods which include karyotyping, probe binding and DNA sequencing.

In DNA sequencing technology, genomic DNA is extracted from whole blood and the coding regions of the BRCA2 gene are amplified. Each of the coding regions may be sequenced completely and the results are compared to the normal DNA sequence of the gene. Alternatively, the coding sequence of the sample gene may be compared to a panel of known mutations or other screening procedure before completely sequencing the gene and comparing it to a normal sequence of the gene.

The BRCA2 gene is divided into 27 separate exons. Exon 1 is noncoding, in that it is not part of the final functional BRCA2 protein product. The BRCA2 coding region spans roughly 10433 base pairs (bp) over 70 kb. Each exon consists of 100-600 bp, except for exons 10, 11 and 27. The full length mRNA is 11-12 kb. To sequence the coding region of the BRCA2 gene, each exon is amplified separately and the resulting PCR products are sequenced in the forward and reverse directions. Because exons 10, 11, and 27 are so large, we have divided them into three, twenty-one, and two overlapping PCR fragments (respectively) of approximately 250-625 bp each (segments "A" through "C" of exon 10, "A" through "U" of exon 11, and "A" through "B" of exon 27).

Many mutations and normal polymorphisms have already been reported in the BRCA2 gene. A world wide web site has been built to facilitate the detection and characterization of alterations in breast cancer susceptibility genes. Such mutations in BRCA2 can be accessed through the Breast Cancer Information Core (BIC) at http://www.nhgri.nih.gov/Intramural_research/Lab_transfer/Bic. This data site became publicly available on November 1, 1995. Friend, S. et al. Nature Genetics 11:238, (1995). The information on BRCA2 was added in February, 1996.

The genetics of Breast Cancer Syndrome is autosomal dominant with reduced penetrance. In simple terms, this means that the syndrome runs through families: (1) both sexes can be carriers (mostly women get the disease but men can both pass it on and occasionally get breast cancer); (2) most generations will likely have breast cancer; (3) occasionally women carriers either die young before they have the time to manifest disease (and yet have offspring who get it) or they never develop breast or ovarian cancer and die of old age (the latter people are said to have "reduced penetrance" because they never develop cancer). Pedigree analysis and genetic counseling is absolutely essential to the proper workup of a family prior to any lab work.

Until now, the only sources of genomic sequence information for BRCA2 were GenBank (Accession Number U43746), or through the Breast Information Core (BIC) database on the Internet which requires membership in the BIC consortium. However, based upon the disclosure of this patent application, in neither GenBank nor BIC were the sequences identified and listed entirely accurate. There is a need in the art to correct these mistakes which otherwise may lead to misinterpretation of the sequence data from the patient as abnormal when it was not, or vice versa.

In addition, there is a need in the art to have available a functional allele profile which represents the most likely BRCA2 sequences to be found in the majority of the normal population. This functional allele profile is based upon frequent polymorphisms and the correct backbone sequence. The knowledge of several common normal haplotypes will make it possible for true mutations to be easily identified or differentiated from polymorphisms. Identification of mutations of the BRCA2 gene and protein would allow more widespread diagnostic screening for hereditary breast cancer than is currently possible.

The use of these common normal haplotypes, in addition to the previously published BRCA2 sequence, will reduce the likelihood of misinterpreting a "sequence variation" found in the normal population with a pathologic "mutation" (i.e. causes disease in the individual or puts the individual at a high risk of developing the disease). With large interest in breast cancer predisposition testing, misinterpretation is particularly worrisome. People who already have breast cancer are asking the clinical question: "is my disease caused by a heritable genetic mutation?" The relatives of the those with breast cancer are asking the question: "Am I also a carrier of the mutation my relative has? Thus, is my risk increased, and should I undergo a more aggressive surveillance program?"

### SUMMARY OF THE INVENTION

The present invention is based on the discovery of the correct genomic BRCA2 sequence and five novel sequence haplotypes found in normal human subjects of the BRCA2 gene.

It is an object of this invention to provide the correct intronic/exonic sequence of the BRCA2 gene.

It is another object of this invention to provide five unique haplotype sequences of the BRCA2 gene in normal individuals which do not correspond to increased cancer susceptibility.

It is another object of this invention to sequence a BRCA2 gene or a portion thereof and compare it to the five haplotype sequences to determine whether a sequence variation noted represents a polymorphism or a potentially harmful mutation.

It is another object of this invention to provide a list of the pairs which occur at each of ten polymorphic points in the BRCA2 gene.

It is another object of this invention to provide the rates of occurrence for the polymorphisms at codons 289, 372, 455, 743, 894, 991,1132, 1269, 2414, and 2951 in the BRCA2 gene.

It is another object of this invention to provide a method wherein all exons of BRCA2 gene or parts thereof, are amplified with one or more oligonucleotide primers.

It is another object of this invention to provide a method of identifying a individual who carries no mutation(s) of the BRCA2 gene and is therefore at no increased risk or susceptibility to breast or ovarian cancer based on a finding that the individual does not carry an abnormal BRCA2 genes.

It is another object of this invention to provide a method of identifying a mutation in BRCA2 gene leading to predisposition or higher susceptibility to breast or ovarian cancer.

It is another object of this invention to provide five novel BRCA2 protein sequences derived from five BRCA2 haplotype sequences.

It is another object of the invention to encompass prokaryotic or eukaryotic host cells comprising an expression vector having a DNA sequence that encodes for all or a fragment of the five novel BRCA2 protein sequences, a BRCA2 polypeptide thereof, or a functional equivalent thereof.

It is another object of the invention to encompass an anti-BRCA2 protein antibody using all of fragments of the five novel BRCA2 protein sequences, a BRCA2 polypeptide thereof or a functional equivalent thereof as an immunogen.

There is a need in the art for cDNA sequences of the BRCA2 gene and for the protein sequences of BRCA2 gene from normal individuals who are not at risk for increased susceptibility for cancer. In order to determine whether a sample from a patient suspected of containing a BRCA2 mutation actually has the mutation, the patient's BRCA2 DNA and/or amino acid sequence need to be compared to all known normal BRCA2 sequences. Failure to compare the sequence obtained to all naturally occurring normal sequences may result in reporting a sample as containing a potentially harmful mutation when it is a polymorphism without clinical significance.

A person skilled in the art of genetic susceptibility testing will find the present invention useful for:
a) identifying individuals having a normal BRCA2 gene with no coding sequence mutations, who therefore cannot be said to have an increased genetic susceptibility to breast or ovarian cancer from their BRCA2 genes;
b) avoiding misinterpretation of normal polymorphisms found in the BRCA2 gene;
c) determining the presence of a previously unknown mutation in the BRCA2 gene;
d) identifying a mutation in exon 11 of BRCA2 which indicates a
   predisposition or higher susceptibility to ovarian cancer than breast
   cancer (i.e., resides in the putative "ovarian cancer cluster" region);
e) probing a human sample of the BRCA2 gene by allele to determine the presence or absence of either polymorphic alleles or mutations;
f) performing gene therapy with the correct BRCA2 gene sequence.
g) performing protein replacement therapy with the correct BRCA 2 protein sequence or a functional equivalent thereof.

### BRIEF DESCRIPTION OF THE FIGURES

FIGURE 1 shows the GenBank genomic sequence of BRCA2 (Accession Number U43746). The lower case letters denote intronic sequences and the upper case letters denote exonic sequences. Incorrect exonic sequences at exons 5 and 16 are shown with boldface type.
FIGURE 2 shows the corrected genomic sequence of BRCA2. The lower case letters denote intronic sequences and the upper case letters denote exonic sequences. Corrected intronic and exonic sequences at exons 5, 11 and 15 are shown with boldface type.

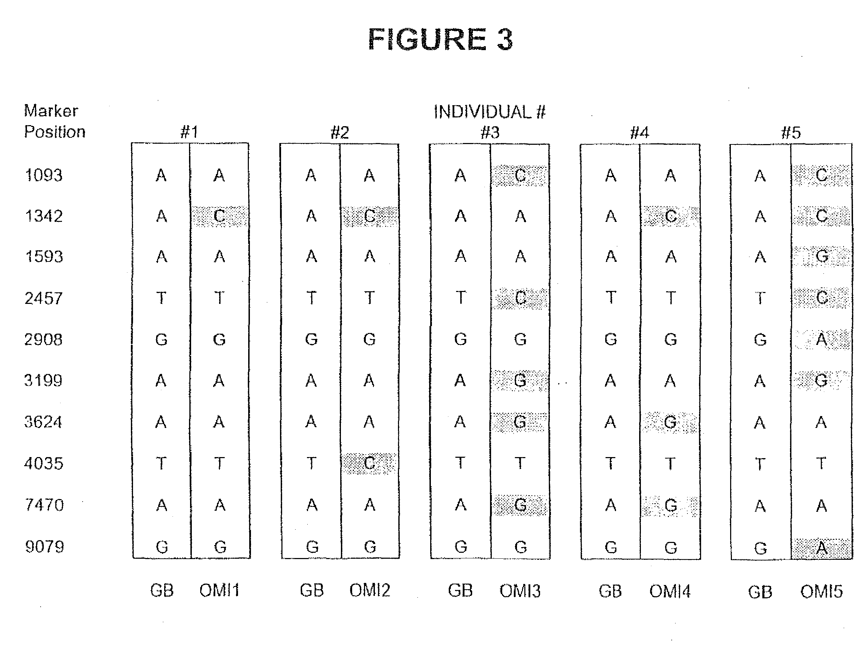

FIGURE 3 shows the alternative alleles at polymorphic sites along a chromosome which can be represented as a unit or "haplotype" within a gene such as BRCA2. The haplotype that is in GenBank (GB) is shown with light shading. Five additional haplotypes are shown in FIGURE 3 (encompassing the alternative alleles found at nucleotide sites 1093, 1342, 1593, 2457, 2908, 3199, 3624, 4035, 7470 and 9079). BRCA2 ^{(omi-1)}, BRCA2 ^{(omi-2)}, BRCA2 ^{(omi-3)}, BRCA2 ^{(omi-4)}, and BRCA2 ^{(omi-5)} are represented with mixed dark and light shading (numbers 2, 4, 6, 8 and 10 from left to right). In total, 5 of 10 haplotypes along the BRCA2 gene are unique.

### DETAILED DESCRIPTION OF THE INVENTION

### DEFINITIONS

The following definitions are provided for the purpose of understanding this invention.

"Breast and Ovarian cancer" is understood by those skilled in the art to include breast, ovarian and pancreatic cancer in women and also breast, prostate and pancreatic cancer in men. BRCA2 is associated with genetic susceptibility to breast, ovarian and pancreatic cancer. Therefore, claims in this document which recite breast and/or ovarian cancer refer to breast, ovarian, prostate, and pancreatic cancers in men and women.

"Coding sequence" refers to those portions of a gene which, taken together, code for a peptide (protein), or which nucleic acid itself has function.

"Protein" or "peptide" refers to a sequence of amino acids which has function.

"BRCA2^{(omi)}" refers to the genomic BRCA2 sequence disclosed in Genbank (Accession Number U43746) wherein,
(1) a 10 bp stretch (5'-TTTATTTTAG-3') is intronic at 3' end of intron 4, rather than at the 5' end of exon 5; and
(2) a 16 bp stretch (5'-GTGTTCTCATAAACAG-3') is exonic at the 3' end of exon 15, rather than at the 5' end of exon.

"BRCA2^{(omi 1-5)}" refers to five unique DNA sequences of the BRCA2 gene and their introns (particularly the slice sites adjacent to the exons). These sequences were found by end to end sequencing of the BRCA2 gene from 5 individuals randomly drawn from the population and who were documented to have no family history of breast or ovarian cancer. The sequenced exons were found not to contain any truncating mutations. In all cases the change of a nucleic acid at a polymorphic site lead to a codon change and a change of amino acid from the previously published standard in GenBank (see TABLE III). In some cases the frequency of occurrence of a nucleic acid change was found to differ from the published frequency or was newly determined. These sequence variations are believed to be alleles whose haplotypes do not indicate an increased risk for cancer.

"Normal DNA sequence" also called " normal gene sequence" refers to a nucleic acid sequence, the nucleic acid of which are known to occur at their respective positions with high frequency in a population of individuals who carry the gene which codes for a normally functioning protein, or which itself has normal function.

"Normal Protein Sequence" refers to the protein sequence, the amino acids of which are known to occur with high frequency in a population of individuals who carry the gene which codes for a normally functioning protein.

"Normal Sequence" refers to the nucleic acid or protein sequence, the nucleic or amino acids of which are known to occur with high frequency in a population of individuals who carry the gene which codes for a normally functioning protein, or which nucleic acid itself has a normal function.

"Haplotype" refers to a series of specific alleles within a gene along a chromosome.

"Functional allele profile" refers a list of those alleles in the normal population which have the funll function.

"Mutation" refers to a base change or a gain or loss of base pair(s) in a DNA sequence, which results in a DNA sequence coding for a non-functional protein or a protein with substantially reduced or altered function.

"Polymorphism" refers to a base change in a DNA sequence which is not associated with known pathology.

"Primer" refers to a sequence comprising about 15 or more nucleotides having a sequence complementary to the BRCA2 gene. Other primers which can be used for primer hybridization will be known or readily ascertainable to those skilled in the art.

"Substantially complementary to" refers to primer sequences which hybridize to the sequences provided under stringent conditions and/or sequences having sufficient homology with BRCA2 sequences, such that the allele specific oligonucleotide primers hybridize to the BRCA2 sequences to which they are complimentary.

"Isolated nucleic acids" refers to nucleic acids substantially free of other nucleic acids, proteins, lipids, carbohydrates or other materials with which they may be associated. Such association is typically either in cellular material or in a synthesis medium.

"Biological sample" or "body sample" refers to a sample containing DNA oatained from a biological source. The sample may be from a living, dead or even archeological source from a variety of tissues and cells. Examples include body fluid (e.g. blood (leukocytes), urine (epithelial cells), saliva, breast milk, menstrual flow, cervical and vaginal secretions, etc.), skin, hair roots/follicle, mucus membrane (e.g. buccal or tongue cell scrapings), cervicovaginal cells (from PAP smear, etc.), lymphatic tissue, internal tissue (normal or tumor).

"Vector" refers to any polynucleotide which is capable of self replication or inducing integration into a self-replicating polynucleotide. Examples include polynucleotides containing an origin or replication or an integration site. Vectors may be intergrated into the host cell's chromosome or form an autonomously replicating unit.

"A tumor growth inhibitor" refers to a molecule such as, all or a fragment of BRCA2 protein, a BRCA2 polypeptide, or a functional equivalent thereof that is effective for preventing the formation of, reducing, or eliminating a transformed or malignant phenotype of breast or ovarian cancer cells.

"A BRCA2 polypeptide" refers to a BRCA2 polypeptide either directly derived from the BRCA2 protein, or homologous to the BRCA2 protein, or a fusion protein consisting of all or fragments of the BRCA2 protein and polypeptides.

"A functional equivalent" refers to a molecule including an unnatural BRCA2 polypeptide, a drug or a natural product which retains substantial biological activity as the native BRCA2 protein. The activity and function of BRCA2 protein may include transactivation, granin, DNA repair, among others.

"A target polynucleotide" refers to the nucleic acid sequence of interest, for example, the BRCA2 encoding polynucleotide. Other primers which can be used for primer hybridization will be known or readily ascertainable to those of skill in the art.

The invention in several of its embodiments includes: an isolated DNA sequence of the BRCA2 coding sequence as set forth in SEQ ID NO:4, 6, 8, 10, and 12, a protein sequence of the BRCA2 protein as set forth in SEQ ID NO:5, 7, 9, 11, 13, a method of identifying individuals having a normal BRCA2 gene with no increased risk for breast and ovarian cancer, a method of detecting an increased genetic susceptibility to breast and ovarian cancer in an individual resulting from the presence of a mutation in the BRCA2 coding sequence, a method of performing gene therapy to prevent or treat a tumor, a method of protein replacement therapy to prevent or treat a tumor, a diagnostic reagent comprising all or fragments of the disclosed BRCA2 cDNA and protein sequences.

### SEQUENCING

Any nucleic acid specimen, in purified or non-purified form, can be utilized as the starting nucleic acid, providing it contains, or is suspected of containing, the specific nucleic acid sequence containing a polymorphic or a mutant allele. Thus, the process may amplify, for example, DNA or RNA, including mRNA and cDNA, wherein DNA or RNA may be single stranded or double stranded. In the event that RNA is to be used as a template, enzymes and/or conditions optimal for reverse transcribing the template to DNA would be utilized. In addition, a DNA-RNA hybrid which contains one strand of each may be utilized. A mixture of nucleic acids may also be employed, or the nucleic acids produced in a previous method such as an amplification reaction using the same or different primers may be so utilized. The specific nucleic acid sequence to be amplified, i.e., the polymorphic and/or the mutant allele, may be a fraction of a larger molecule or can be present initially as a discrete molecule, so that the specific sequence constitutes the entire nucleic acid. A variety of amplification techniques may be used such as ligating the DNA sample or fragments thereof to a vector capable of replication or incorporation into a replicating system thereby increasing the number of copies of DNA suspected of containing at least a portion of the BRCA2 gene. Amplification techniques include so called "shot gun cloning". It is not necessary that the sequence to be amplified be present initially in a pure form; it may be a minor fraction of a complex mixture, such as contained in whole human DNA.

It should be noted that one need not sequence the entire coding region or even an entire DNA fragment in order to determine whether or not a mutation is present. For example, when a mutation is known in one family member, it is sufficient to determine the sequence at only the mutation site by sequencing or by other mutation detection systems such as ASO when testing other family members.

DNA utilized herein may be extracted from a body sample, such as blood, tissue material and other biological sample by a variety of techniques such as that described by Maniatis, et al. in Molecular Cloning: A Laboratory Manual, Cold Spring Harbor, NY, p 280-281, 1982). If the extracted sample is impure, it may be treated before amplification with an amount of a reagent effective to open the cells, and to expose and/or separate the strand(s) of the nucleic acid(s). This lysing and nucleic acid denaturing step to expose and separate the strands will allow amplification to occur much more readily.

For amplification by cloning, the isolated DNA may be cleaved into fragments by a restriction endonuclease or by shearing by passing the DNA containing mixture through a 25 gauge needle from a syringe to prepare 1-1.5 kb fragments. The fragments are then ligated to a cleaved vector (virus, plasmid, transposon, cosmid etc.) and then the recombinant vector so formed is then replicated in a manner typical for that vector.

For a PCR amplification, the deoxyribonucleotide triphosphates dATP, dCTP, dGTP, and dTTP are added to the synthesis mixture, either separately or together with the primers, in adequate amounts and the resulting solution is heated to about 90°-100°C from about 1 to 10 minutes, preferably from 1 to 4 minutes. After this heating period, the solution is allowed to cool, which is preferable for the primer hybridization. To the cooled mixture is added an appropriate agent for effecting the primer extension reaction (called herein "agent for polymerization"), and the reaction is allowed to occur under conditions known in the art. The agent for polymerization may also be added together with the other reagents if it is heat stable. This synthesis (or amplification) reaction may occur at room temperature up to a temperature above which the agent for polymerization no longer functions. Thus, for example, if DNA polymerase is used as the agent, the temperature is generally no greater than about 40°C. Most conveniently the reaction occurs at room temperature. When using thermostable DNA polymerase such as Taq, higher temperature may be used.

The allele specific oligonucleotide primers are useful in determining whether a subject is at risk of having breast or ovarian cancer, and also useful for characterizing a tumor. Primers direct amplification of a target polynucleotide prior to sequencing. These unique BRCA2 oligonucleotide primers for exons 2-27 shown in TABLE II were designed and produced specifically to optimize amplification of portions of BRCA2 which are to be sequenced.

The primers used to carry out this invention embrace oligonucleotides of sufficient length and appropriate sequence to provide initiation of polymerization. Environmental conditions conducive to synthesis include the presence of nucleoside triphosphates and an agent for polymerization, such as DNA polymerase, and a suitable temperature and pH. The primer is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on many factors, including temperature, buffer, and nucleotide composition. The oligonucleotide primer typically contains 18-28 bp plus in some cases an M13 "tail" for convenience.

Primers used to carry out this invention are designed to be substantially complementary to each strand of the genomic locus to be amplified. This means that the primers must be sufficiently complementary to hybridize with their respective strands under conditions which allow the agent for polymerization to perform. In other words, the primers should have sufficient complementarity with the 5' and 3' sequences flanking the mutation to hybridize therewith and permit amplification of the genomic locus.

Oligonucleotide primers of the invention are employed in the amplification process which is an enzymatic chain reaction that produces exponential quantities of polymorphic locus relative to the number of reaction steps involved. Typically, one primer is complementary to the negative (-) strand of the polymorphic locus and the other is complementary to the positive (+) strand. Annealing the primers to denatured nucleic acid followed by extension with an enzyme, such as the large fragment of DNA polymerase I (Klenow) and nucleotides, results in newly synthesized + and - strands containing the target polymorphic locus sequence. Because these newly synthesized sequences are also templates, repeated cycles of denaturing, primer annealing, and extension results in exponential production of the region (*i*.*e*., the target polymorphic locus sequence) defined by the primers. The product of the chain reaction is a discreet nucleic acid duplex with termini corresponding to the ends of the specific primers employed.

The oligonucleotide primers of the invention may be prepared using any suitable method, such as conventional phosphotriester and phosphodiester methods or automated embodiments thereof. In one such automated embodiment, diethylphosphoramidites are used as starting materials and may be synthesized as described by Beaucage, et al., Tetrahedron Letters, 22:1859-1862, 1981. One method for synthesizing oligonucleotides on a modified solid support is described in U.S. Patent No. 4,458,066.

The agent for polymerization may be any compound or system which will function to accomplish the synthesis of primer extension products, including enzymes. Suitable enzymes for this purpose include, for example, *E. coli* DNA polymerase I, Klenow fragment of *E. coli* DNA polymerase, polymerase muteins, reverse transcriptase, other enzymes, including heat-stable enzymes (*i*.*e*., those enzymes which perform primer extension after being subjected to temperatures sufficiently elevated to cause denaturation), such as *Taq* polymerase. Suitable enzymes will facilitate combination of the nucleotides in the proper manner to form the primer extension products which are complementary to each polymorphic locus nucleic acid strand. Generally, the synthesis will be initiated at the 3' end of each primer and proceed in the 5' direction along the template strand, until synthesis terminates, producing molecules of different lengths.

The newly synthesized strand and its complementary nucleic acid strand will form a double-stranded molecule under hybridizing conditions described above and this hybrid is used in subsequent steps of the process. In the next step, the newly synthesized double-stranded molecule is subjected to denaturing conditions using any of the procedures described above to provide single-stranded molecules.

The steps of denaturing, annealing, and extension product synthesis can be repeated as often as needed to amplify the target polymorphic locus nucleic acid sequence to the extent necessary for detection. The amount of the specific nucleic acid sequence produced will accumulate in an exponential fashion. Amplification is described in PCR. A Practical Approach, ILR Press, Eds. M. J. McPherson, P. Quirke, and G. R. Taylor, 1992.

The amplification products may be detected by Southern blots analysis, without using radioactive probes. In such a process, for example, a small sample of DNA containing a very low level of the nucleic acid sequence of the polymorphic locus is amplified, and analyzed via a Southern blotting technique or similarly, using dot blot analysis. The use of nonradioactive probes or labels is facilitated by the high level of the amplified signal. Alternatively, probes used to detect the amplified products can be directly or indirectly detectably labeled, for example, with a radioisotope, a fluorescent compound, a bioluminescent compound, a chemiluminescent compound, a metal chelator or an enzyme. Those of ordinary skill in the art will know of other suitable labels for binding to the probe, or will be able to ascertain such, using routine experimentation.

Sequences amplified by the methods of the invention can be further evaluated, detected, donned, sequenced, and the like, either in solution or after binding to a solid support, by any method usually applied to the detection of a specific DNA sequence such as PCR, oligomer restriction (Saiki, et. al., Bio/Technology, 3:1008-1012, 1985), allele-specific oligonucleotide (ASO) probe analysis (Conner, et al., Proc, Natl. Acad. Sci. U.S.A., 80:278, 1983), oligonucleotide ligation assays (OLAs) (Landgren, et al., Science, 241:1007, 1988), and the like. Molecular techniques for DNA analysis have been reviewed (Landgren, et al., Science, 242:229-237, 1988).

Preferably, the method of amplifying is by PCR, as described herein and as is commonly used by those of ordinary skill in the art. Alternative methods of amplification have been described and can also be employed as long as the BRCA2 locus amplified by PCR using primers of the invention is similarly amplified by the alternative means. Such alternative amplification systems include but are not limited to self-sustained sequence replication, which begins with a short sequence of RNA of interest and a T7 promoter. Reverse transcriptase copies the RNA into cDNA and degrades the RNA, followed by reverse transcriptase polymerizing a second strand of DNA. Another nucleic acid amplification technique is nucleic acid sequence-based amplification (NASBA) which uses reverse transcription and T7 RNA polymerase and incorporates two primers to target its cycling scheme. NASBA can begin with either DNA or RNA and finish with either, and amplifies to 10⁸ copies within 60 to 90 minutes. Alternatively, nucleic acid can be amplified by ligation activated transcription (LAT). LAT works from a single-stranded template with a single primer that is partially single-stranded and partially double-stranded. Amplification is initiated by ligating a cDNA to the promoter oligonucleotide and within a few hours, and amplification is 10⁸ to 10⁹ fold. Another amplification system useful in the method of the invention is the Qβ Replicase System. The Qβ replicase system can be utilized by attaching an RNA sequence called MDV-1 to RNA complementary to a DNA sequence of interest. Upon mixing with a sample, the hybrid RNA finds its complement among the specimen's mRNAs and binds, activating the replicase to copy the tag-along sequence of interest. Another nucleic acid amplification technique, ligase chain reaction (LCR), works by using two differently labeled halves of a sequence of interest which are covalently bonded by ligase in the presence of the contiguous sequence in a sample, forming a new target. The repair chain reaction (RCR) nucleic acid amplification technique uses two complementary and target-specific oligonucleotide probe pairs, thermostable polymerase and ligase, and DNA nucleotides to geometrically amplify targeted sequences. A 2-base gap separates the oligonucleotide probe pairs, and the RCR fills and joins the gap, mimicking normal DNA repair. Nucleic acid amplification by strand displacement activation (SDA) utilizes a short primer containing a recognition site for *hincil* with short overhang on the 5' end which binds to target DNA. A DNA polymerase fills in the part of the primer opposite the overhang with sulfur-containing adenine analogs. *Hincil* is added but only cuts the unmodified DNA strand. A DNA polymerase that lacks 5' exonuclease activity enters at the site of the nick and begins to polymerize, displacing the initial primer strand downstream and building a new one which serves as more primer. SDA produces greater than 10⁷-fold amplification in 2 hours at 37°C. Unlike PCR and LCR, SDA does not require instrumented Temperature cycling.

Another method is a process for amplifying nucleic acid sequences from a DNA or RNA template which may be purified or may exist in a mixture of nucleic acids. The resulting nucleic acid sequences may be exact copies of the template, or may be modified. The process has advantages over PCR in that it increases the fidelity of copying a specific nucleic acid sequence, and it allows one to more efficiently detect a particular point mutation in a single assay. A target nucleic acid is amplified enzymatically while avoiding strand displacement. Three primers are used. A first primer is complementary to the first end of the target. A second primer is complementary to the second end of the target. A third primer which is similar to the first end of the target and which is substantially complementary to at least a portion of the first primer such that when the third primer is hybridized to the first primer, the position of the third primer complementary to the base at the 5' end of the first primer contains a modification which substantially avoids strand displacement. This method is detailed in U.S. Patent 5,593,840 to Bhatnagar et al. 1997, incorporated herein by reference.

Finally, recent application of DNA chips or microarray technology where DNA or oligonucleotides are immobilized on small solid support may also be used to rapidly sequence sample BRCA2 gene and analyze its expression. Typically, high density arrays of DNA fragment are fabricated on glass or nylon substrates by *in situ* light-directed combinatorial synthesis or by conventional synthesis followed by immobilization (Fodor et al. U.S. patent No. 5,445,934). Sample DNA or RNA may be amplified by PCR, labeled with a fluorescent tag, and hybridized to the microarray. Examples of this technology are provided in U.S. Patents 5,510, 270, U.S. 5,547,839, incorporated herein by reference.

All exonic and adjacent intronic sequences of the BRCA2 gene were obtained by end to end sequencing of five normal subjects in the manner described above followed by analysis of the data obtained. The data obtained provided us with the opportunity to establish the correct intronic/exonic structure of the BRCA2 gene. In addition, we evaluated six previously published normal polymorphisms (1342, 2457, 3199, 3624, 4035, and 7470) for correctness and frequency in the population, and to identify four additional polymorphisms not previously characterized (1093, 1593, 2908, and 9079).

### GENE THERAPY

The polynucleotide(s) which result from either sense or antisense transcription of any exon or the entire coding sequence or fragments of BRCA2 gene may be used for gene therapy. A variety of methods are known for gene transfer, any of which might be available for use.

Direct injection of Recombinant DNA in vivo:
1. Direct injection of "naked" DNA directly with a syringe and needle into a specific tissue, infused through a vascular bed, or transferred through a catheter into endothelial cells.
2. Direct injection of DNA that is contained in artificially generated lipid vesicles or other encapsulating vehicles.
3. Direct injection of DNA conjugated to a target receptor structure, such as a diptheria toxin, an antibody or other suitable receptor.
4. Direct injection by particle bombardment. For example, the DNA may be coated onto gold particles and shot into the cells.

### Human Artificial Chromosomes

The gene delivery approach involves the use of human chromosomes that have been stripped down to contain only the essential components for replication and the genes desired for transfer.

### Receptor-Mediated Gene Transfer

DNA is linked to a targeting molecule that will bind to specific cell-surface receptors, inducing endocytosis and transfer of the DNA into mammalian cells. One such technique uses poly-L-lysine to link asialoglycoprotein to DNA. An adenovirus is also added to the complex to disrupt the lysosomes and thus allow the DNA to avoid degradation and move to the nucleus. Infusion of these particles intravenously has resulted in gene transfer into hepatocytes.

### RECOMBiNANT VIRUS VECTORS

Several vectors may be used in gene therapy. Among them are the Moloney Murine Leukemia Virus (MoMLV) Vectors, the adenovirus vectors, the Adeno-Associated Virus (AAV) vectors, the herpes simplex virus (HSV) vectors, the poxvirus vectors, the retrovirus vectors, and human immunodeficiency virus (HIV) vectors.

### GENE REPLACEMENT AND REPAIR

The ideal genetic manipulation for treatment of a genetic disease would be the actual replacement of the defective gene with a normal copy of the gene. Homologous recombination is the term used for switching out a section of DNA and replacing it with a new piece. By this technique, the defective gene may be replaced with a normal gene which expresses a functioning BRCA2 tumor growth inhibitor protein.

A complete description of gene therapy can also be found in "Gene Therapy A Primer For Physicians" 2d Ed. by Kenneth W. Culver, M.D. Publ. Mary Ann Liebert Inc. (1996). Two Gene Therapy Protocols for BRCA1 gene have been approved by the Recombinant DNA Advisory Committee for Jeffrey T. Holt et al. They are listed as 9602-148, and 9603-149 and are available from the NIH. Protocols for BRCA2 gene therapy may be similarly employed. The isolated BRCA2 gene may be synthesized or constructed from amplification products and inserted into a vector such as the LXSN vector.

### A BRCA2 POLYPEPTIDE OR ITS FUNCTIONAL EQUIVALENT

The growth of breast and ovarian cancer may be arrested or prevented by directly increasing the BRCA2 protein level where inadequate functional BRCA2 activity is responsible for breast and ovarian cancer. The cDNA and amino acid sequences of five novel BRCA2 haplotypes are disclosed herein (SEQ. ID No:4-13). All or a fragment of BRCA2 protein may be used in therapeutic or prophylactic treatment of breast and ovarian cancer. Such a fragment may have a similar biological function as the native BRCA2 protein or may have a desired biological function as specified below. BRCA2 polypeptides or their functional equivalents including homologous and modified polypeptide sequences are also within the scope of the present invention. Changes in the native sequence may be advantageous in producing or using the BRCA2 derived polypeptides or functional equivalents suitable for therapeutic or prophylactic treatment of breast and ovarian cancer. For example, these changes may be desirable for producing resistance against *in vivo* proteolytic cleavage, for facilitating transportation and delivery of therapeutic reagents, for localizing and compartmentalizing tumor suppressing agents, or for expression, isolating and purifying the target species.

There are a variety of methods to produce an active BRCA2 polypeptide or a functional equivalent as a tumor growth inhibitor. For example, one or more amino acids may be substituted, deleted, or inserted using methods well known in the art (Maniatis *et al*., 1982). Considerations of polarity, charge, solubility, hydrophobicity, hydrophilicity and/or the amphiphathic nature of the amino acids play an important role in designing homologous polypeptide changes suitable for the intended treatment. In particular, conservative amino acid substitution using amino acids that are related in side-chain structure and charge may be employed to preserve the chemical and biological property. A homologous polyeptide typically contains at least 70% homology to the native sequence. Unnatural forms of the polypeptide may also be incorporated so long as the modification retains substantial biological activity. These unnatural polypeptides typically include structural mimics and chemical medications, which have similar three-dimensional structures as the active regions of the native BRCA2 protein. For example, these modifications may include terminal D-amino acids, cyclic peptides, unnatural amino acids side chains, pseudopeptide bonds, N-terminal acetylation, glycosylation, and biotinylation, etc. These unnatural forms of polypeptide may have a desired biological function, for example, they may be particularly robust in the presence of cellular or serum proteases and exopeptidase. An effective BRCA2 polypeptide or a functional equivalent may also be recognized by the reduction of the native BRCA2 protein. Regions of the BRCA2 protein may be systematically deleted to identify which regions are essential for tumor growth inhibitor activity. These smaller fragments of BRCA2 protein may then be subjected to structural and functional modification to derive therapeutically or prophylactically effective regiments. Finally, drugs, natural products or small molecules may be screened or synthesized to mimic the function of the BRCA2 protein. Typically, the active species retain the essential three-dimensional shape and chemical reactivity, and therefore retain the desired aspects of the biological activity of the native BRCA2 protein. The activity and function of BRCA2 may include transactivation, granin, DNA repair among others. Functions of BRCA2 protein are also reviewed in Bertwistle and Ashworth, Curr. Opin. Genet. Dev. 8(1): 14-20 (1998) and Zhang et al., Cell 92:433-436 (1998). It will be apparent to one skilled in the art that a BRCA2 polypeptide or a functional equivalent may be selected because such polypeptide or functional equivalent possesses similar biological activity as the native BRCA2 protein.

### EXPRESSION OF THE BRCA2 PROTEIN AND POLYPEPTIDE IN HOST CELLS

All or fragments of the BRCA2 protein and polypeptide may be produced by host cells that are capable of directing the replication and the expression of foreign genes. Suitable host cells include prokaryotes, yeast cells, or higher eukaryotic cells, which contain an expression vector comprising all or a fragment of the BRCA2 cDNA sequence (SEQ. ID No: 4, 6, 8, 10, or 12) operatively linked to one or more regulatory sequences to produce the intended BRCA2 protein or polypeptide. Prokaryotes may include gram negative or gram positive organisms, for example *E. coli* or *Bacillus* strains. Suitable eukaryotic host cells may include yeast, virus, and mamalian systems. For example, Sf9 insect cells and human cell lines, such as COS, MCF7, HeLa, 293T, HBL100, SW480, and HCT116 cells.

A broad variety of suitable expression vectors are available in the art. An expression vector typically contains an origin of replication, a promoter, a phenotypic selection gene (antibiotic resistance or autotrophic requirement), and a DNA sequence coding for all or fragments of the BRCA2 protein. The expression vectors may also include other operatively linked regulatory DNA sequences known in the art, for example, stability leader sequences, secretory leader sequences, restriction enzyme cleavage sequences, polyadenylation sequences, and termination sequences, among others. The essential and regulatory elements of the expression vector must be compatible with the intended host cell. Suitable expression vectors containing the desired coding and control regions may be constructed using standard recombinant DNA techniques known in the art, many of which are described in Sambrook, et al., Molecular Cloning: A Laboratory Manual, Second Edition, Cold Spring Harbor Laboratory, Cold Spring Harbor, N.Y. (1989). For example, suitable origins of replication may include Col E1, SV4O viral and M13 origins of replication. Suitable promoters may be constitutive or inducible, for example, tac promoter, lac Z promoter, SV40 promoter, MMTV promoter, and LXSN promoter. Examples of selectable markers include neomycin, ampicillin, and hygromycin resistance and the like. Many suitable prokaryotic, viral and mammalian expression vectors may be obtained commercially, for example, from Invitrogen Corp., San Diego, CA or from Clontech, Palo Alto, CA. It may be desirable that the BRCA2 protein or polypeptide is produced as a fusion protein to enhance the expression in selected host cells, to detect the expression in transfected cells, or to simplify the purification process. Suitable fusion partners for the BRCA2 protein or polypeptide are well known in the art and may include β-galactosidase, glutathione-S-transferase, and poly-histidine tag.

Expression vectors may be introduced into host cells by various methods known in the art. The transformation procedure used depends upon the host to be transformed. Methods for introduction of vectors into host cells may include calcium phosphate precipitation, electrosporation, dextranmediated transfection, liposome encapsulation, nucleus microinjection, and viral or phage infection, among others.

Once an expression vector has been introduced into a suitable host cell, the host cell may be cultured under conditions permitting expression of large amounts of the BRCA2 protein or polypeptide. The expression product may be identified by many approaches well known in the art, for example, sequencing after PCR-based amplification, hybridization using probes complementary to the desired DNA sequence, the presence or absence of marker gene functions such as enzyme activity or antibiotic resistance, the level of mRNA production encoding the intended sequence, immunological detection of a gene product using monoclonal and polyclonal antibodies, such as Western blotting or ELISA. The BRCA2 protein or polypeptides produced in this manner may then be isolated following cell lysis and purified using various protein purification techniques known in the art, for example, ion exchange chromatography, gel filtration chromatography and immunoaffinity chromatography.

It is generally preferred that whenever possible, longer fragments of BRCA2 protein or polypeptide are used, particularly to include the desired functional domains of BRCA2 protein. Expression of shorter fragments of DNA may be useful in generating BRCA2 derived immunogen for the production of anti-BRCA2 antibodies. It should, of course, be understood that not all expression vectors, DNA regulatory sequences or host cells will function equally well to express the BRCA2 protein or polypeptides of the present invention. However, one of ordinary skill in the art may make a selection among expression vectors, DNA regulatory sequences, host cells, and codon usage in order to optimize expression using known technology in the art without undue experimentation. Studies of BRCA2 protein function and examples of genetic manipulation of BRCA2 protein are summarized in two recent review articles, Bertwistle and Ashworth, Curr. Opin. Genet. Dev. 8(1): 14-20 (1998) and Zhang et al., Cell 92:433-436 (1998).

### IN VITRO SYNTHESIS AND CHEMICAL SYNTHESIS

Although it is preferred that fragments of the BRCA2 protein or polypeptides be obtained by overexpression in prokaryotic or eukaryotic host cells, the BRCA2 polypeptides or their functional equivalents may also be obtained by *in vitro* translation or synthetic means by methods known to those of ordinary skill in the art. For example, *in vitro* translation may employ an mRNA encoded by a DNA sequence coding for fragments of the BRCA2 protein or polypeptides. Chemical synthesis methodology such as solid phase synthesis may be used to synthesize a BRCA2 polypeptide structural mimic and chemically modified analogs thereof. The polypeptides or the modifications and mimic thereof produced in this manner may then be isolated and purified using various purification techniques, such as chromatographic procedures including ion exchange chromatography, gel filtration chromatography and immunoaffinity chromatography.

### PROTEIN REPLACEMENT THERAPY

The tumor suppressing function of BRCA2 suggests that various BRCA2 protein targeted therapies may be utilized in treating and preventing tumors in breast and ovarian cancer. The present invention therefore includes therapeutic and prophylactic treatment of breast and ovarian cancer using therapeutic pharmaceutical compositions containing the BRCA2 protein, polypeptides, or their functional equivalents. For example, protein replacement therapy may involve directly administering the BRCA2 protein, a BRCA2 polypeptide, or a functional equivalent in a pharmaceutically effective carrier. Alternatively, protein replacement therapy may utilize tumor antigen specific antibody fused to fragments of the BRCA2 protein, a polypeptide, or a functional equivalent to deliver anti-cancer regiments specifically to the tumor cells.

To prepare the pharmaceutical compositions of the present invention, an active BRCA2 protein, a BRCA2 polypeptide, or its functional equivalent is combined with a pharmaceutical carrier selected and prepared according to conventional pharmaceutical compounding techniques. A suitable amount of the composition may be administered locally to the site of a tumor or systemically to arrest the proliferation of tumor cells. The methods for administration, may include parenteral, oral, or intravenous, among others according to established protocols in the art.

Pharmaceutically acceptable solid or liquid carriers or components which may be added to enhance or stabilize the composition, or to facilitate preparation of the composition include, without limitation, syrup, water, isotonic solution, 5 % glucose in water or buffered sodium or ammonium acetate solution, oils, glycerin, alcohols, flavoring agents, preservatives, coloring agents, starches, sugars, diluents, granulating agents, lubricants, binders, and sustained release materials. The dosage at which the therapeutic compositions are administered may vary within a wide range and depends on various factors, such as the stage of cancer progression, the age and condition of the patient, and may be individually adjusted.

### DIAGNOSTIC REAGENTS

The BRCA2 protein, polypeptides, their functional equivalents, antibodies, and polynucleotides may be used in a wide variety of ways in addition to gene therapy and protein replacement therapy. They may be useful as diagnostic reagents to measure normal or abnormal activity of BRCA2 at the DNA, RNA, and protein level. The present invention therefore encompasses the diagnostic reagents derived from the BRCA2 cDNA and protein sequences as set forth in SEQ. ID. Nos: 4-13. These reagents may be utilized in methods for monitoring disease progression, for determining patients suited for gene and protein replacement therapy, or for detecting the presence or quantifying the amount of a tumor growth inhibitor following such therapy. Such methods may involve conventional histochemical techniques, such as obtaining a tumor tissue from the patient, preparing an extract and testing this extract for tumor growth or metabolism. For example, the test for tumor growth may involve measuring abnormal BRCA2 activity using conventional diagnostic assays, such as Southern, Northern, and Western blotting, PCR, RT-PCR, and immunoprecipitation. In biopsies of tumor tissues, the loss of BRCA2 expression in tumor tissue may be verified by RT-PCR and Northern blotting at the RNA level. A Southern blot analysis, genomic PCR, or fluorescence in situ hybridization (FISH) may also be performed to examine the mutations of BRCA2 at the DNA level. And, a Western blotting, protein truncation assay, or immunoprecipitation may be utilized to analysis the effect at the protein level.

These diagnostic reagents are typically either covalently or non convalently attached to a detectable label. Such a label includes a radioactive label, a colorimetric enzyme label, a fluorescence label, or an epitope label. Frequently, a reporter gene downstream of the regulatory sequences is fused with the BRCA2 protein or polypeptide to facilitate the detection and purification of the target species. Commonly used reporter genes in BRCA2 fusion proteins include β-galactosidase and luciferase gene.

The BRCA2 protein, polypeptides, their functional equivalents, antibodies, and polynucleotides may also be useful in the study of the characteristics of BRCA2 proteins, such as structure and function of BRCA2 in oncogenesis or subcellular localization of BRCA2 protein in normal and cancerous cell. For example, yeast two-hybrid system has been used in the study of cellular function of BRCA2 to identify the regulator and effector of BRCA2 tumor suppressing function (Sharan et al., Nature 386:804-810 (1991) and Katagiri et al., Genes, Chromosomes & Cancer 21:217-222 (1988)). In addition, the BRCA2 protein, polypeptides, their functional equivalents, antibodies, and polynucleotides may also be used in *in vivo* cell based and *in vitro* cell free assays to screen natural products and synthetic compounds which may mimic, regulate or stimulate BRCA2 protein function.

### ANTISENSE INHIBITION

Antisense suppression of endogenous BRCA2 expression may assess the effect of BRCA2 protein on cell growth inhibition using known method in the art (Crooke, Annu. Rev. Pharmacol. Toxicol. 32:329-376 (1992) and Robinson-Benion and Holt, Methods Enzymol. 254:363-375 (1995)). Given the cDNA sequence as set forth in SEQ ID. NO: 4, 6, 8, 10, and 12, one of skill in the art can readily obtain anti-sense strand of DNA and RNA sequences to interfere with the production of wild-type BRCA2 protein or the mutated form of BRCA2 protein. Alternatively, antisense oligonucleotide may be designed to target the control sequences of BRCA2 gene to reduce or prevent the expression of the endogenous BRCA2 gene.

### ANTIBODIES

The BRCA2 protein, polypeptides, or their functional equivalents may be used as immunogens to prepare polyclonal or monoclonal antibodies capable of binding the BRCA2 derived antigens in a known manner (Harlow & Lane, Antibodies: A Laboratory Manual, Cold Spring Harbor Laboratory, Cold Spring Harbor, NY, 1988). These antibodies may be used for the detection of the BRCA2 protein, polypeptides, or a functional equivalent in an immunoassay, such as ELISA, Western blot, radioimmunoassay, enzyme immunoassay, and immunocytochemistry. Typically, an anti-BRCA2 antibody is in solution or is attached to a solid surface such as a plate, a particle, a bead, or a tube. The antibody is allowed to contact a biological sample or a blot suspected of containing the BRCA2 protein or polypeptide to form a primary immunocomplex. After sufficient incubation period, the primary immunocomplex is washed to remove any non-specifically bound species. The amount of specifically bound BRCA2 protein or polypeptide may be determined using the detection of an attached label or a marker, such as a radioactive, a fluorescent, or an enzymatic label. Alternatively, the detection of BRCA2 derived antigen is allowed by forming a secondary immunocomplex using a second antibody which is attached with a such label or marker. The antibodies may also be used in affinity chromatography for isolating or purifying the BRCA2 protein, polypeptides or their functional equivalents.

### EXAMPLE 1

### Determination of the Coding Sequence Haplotypes of the BRCA2 Gene From Normal Individuals

Approximately 150 volunteers were screened in order to identify individuals with no cancer history in their immediate family (i.e. first and second degree relatives). Each person was asked to fill out a hereditary cancer prescreening questionnaire (See TABLE I). Five of these were randomly chosen for end-to-end sequencing of their BRCA2 gene. A first degree relative is a parent, sibling, or offspring. A second degree relative is an aunt, uncle, grandparent, grandchild, niece, nephew, or half-sibling.

Genomic DNA was isolated from white blood cells of five normal subjects selected from analysis of their answers to the questions above. Dideoxy sequence analysis was performed following polymerase chain reaction amplification.

All exons of the BRCA2 gene were subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye was attached for automated sequencing using the Taq Dye Terminator Kit (Perkin-Elmer^{®} cat# 401628). DNA sequencing was performed in both forward and reverse directions on an Applied Biosystems, Inc. (ABI) automated sequencer (Model 377). The software used for analysis of the resulting data was "Sequence Navigator" purchased through ABI,

### 1. Polymerase Chain Reaction (PCR) Amplification

Genomic DNA (100 nanograms) extracted from white blood cells of five normal subjects. Each of the five samples was sequenced end to end. Each sample was amplified in a final volume of 25 microliters containing 1 microliter (100 nanograms) genomic DNA, 2.5 microliters 10X PCR buffer (100 mM Tris, pH 8.3, 500 mM KCI, 1.2 mM MgCl₂), 2.5 microliters 10X dNTP mix (2 mM each nucleotide), 2.5 microliters forward primer, 2.5 microliters reverse primer, and 1 microliter Taq polymerase (5 units), and 13 microliters of water.

The primers in TABLE II below were used to carry out amplification of the various sections of the BRCA2 gene samples. The primers were synthesized on an DNA/RNA Synthesizer Model 394^{®}.

Thirty-five cycles were performed, each consisting of denaturing (95°C; 30 seconds), annealing (55°C; 1 minute), and extension (72°C; 90 seconds), except during the first cycle in which the denaturing time was increased to 5 minutes, and during the last cycle in which the extension time was increased to 5 minutes.

PCR products were purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye was attached to PCR products for automated sequencing using the Taq Dye Terminator Kit (Perkin-Elmer^{®} cat # 401628). DNA sequencing was performed in both forward and reverse directions on an Applied Biosystems, Inc. (ABI) Foster City, CA., automated sequencer (Model 377). The software used for analysis of the resulting data was "Sequence Navigator^{®}" purchased through ABI.

### 3. RESULTS

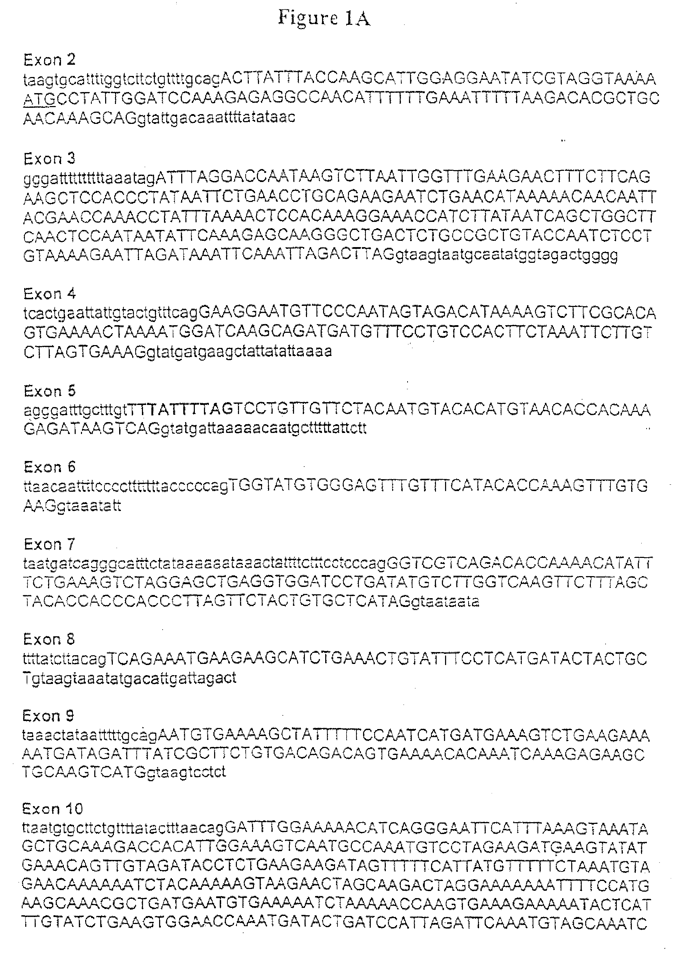

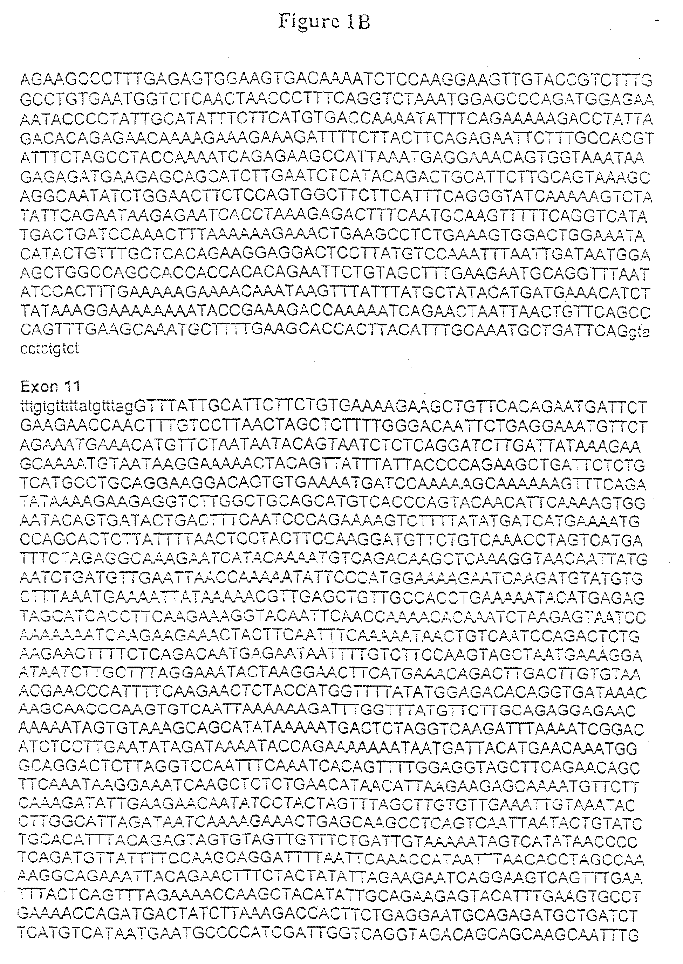

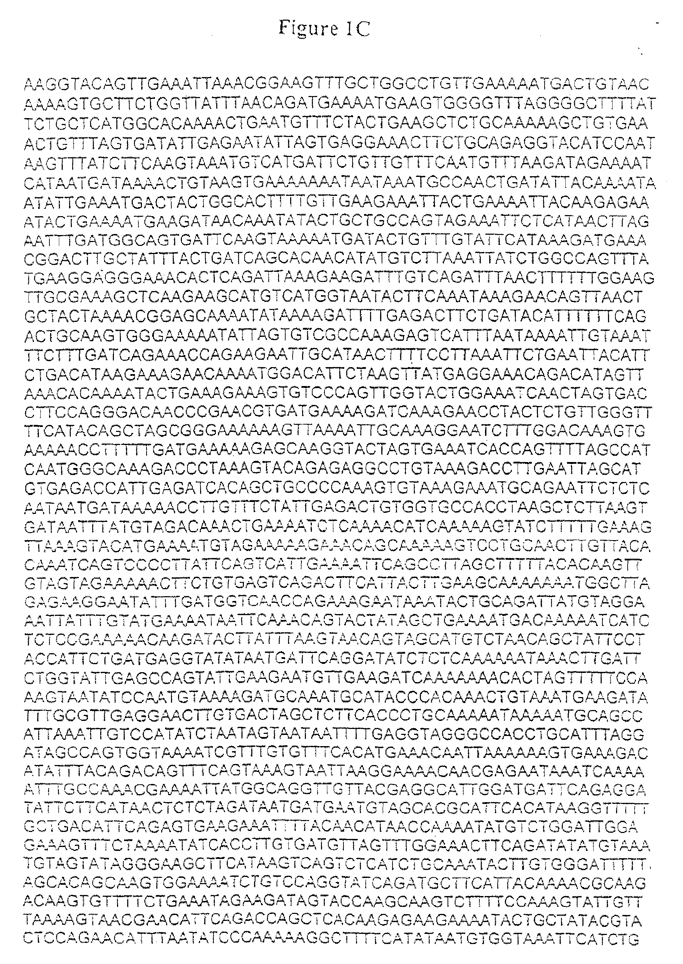

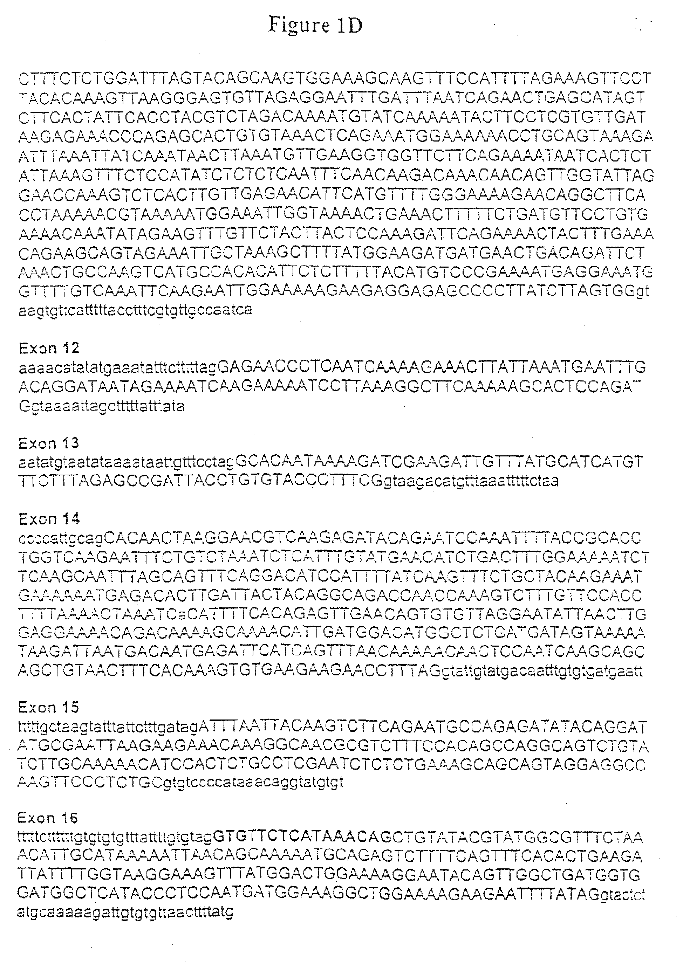

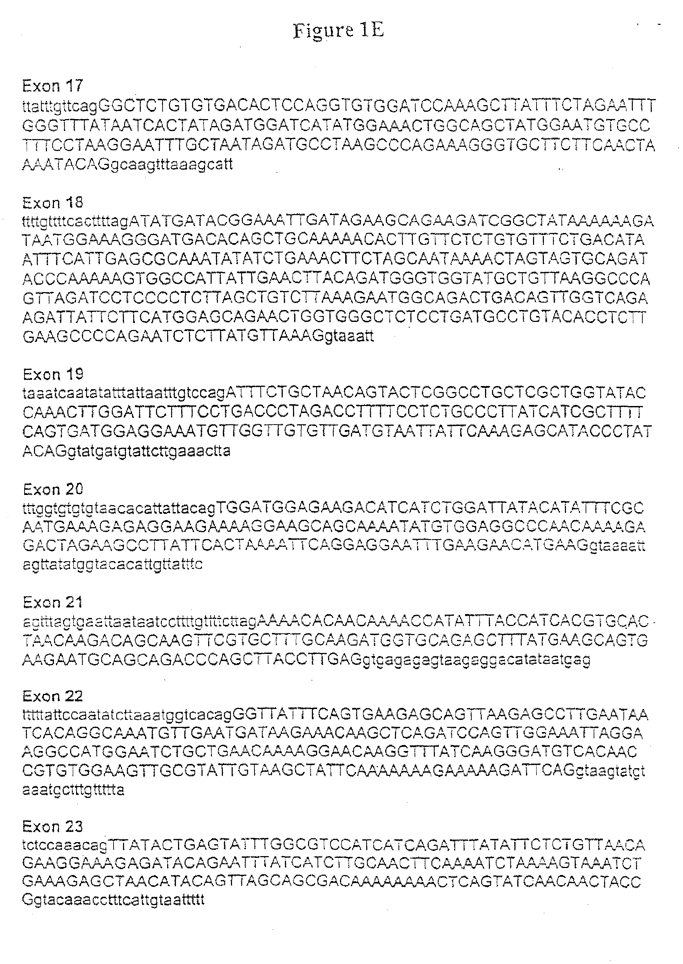

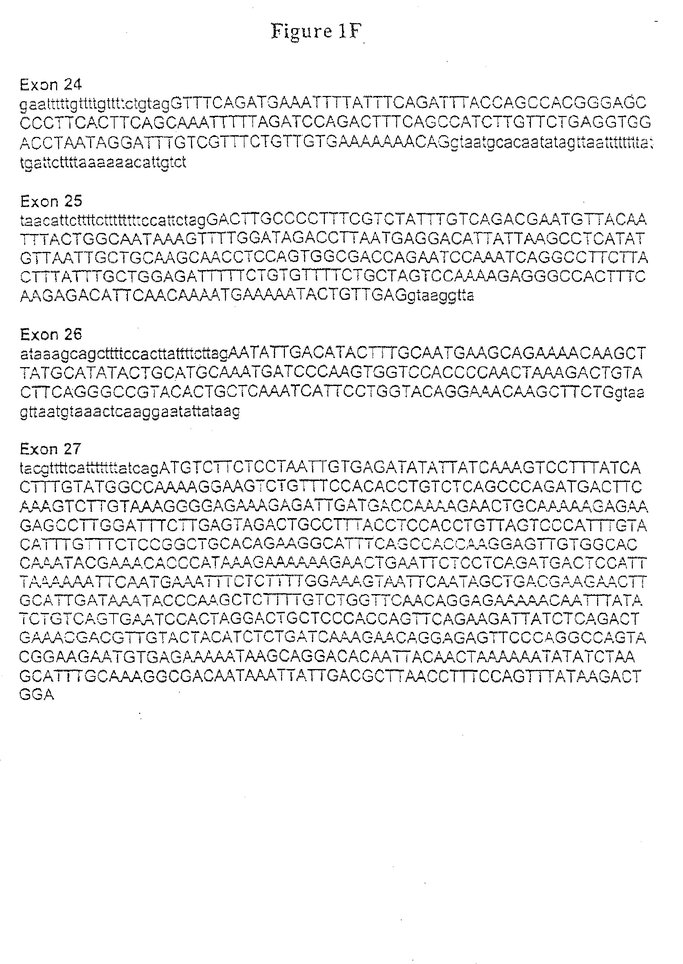

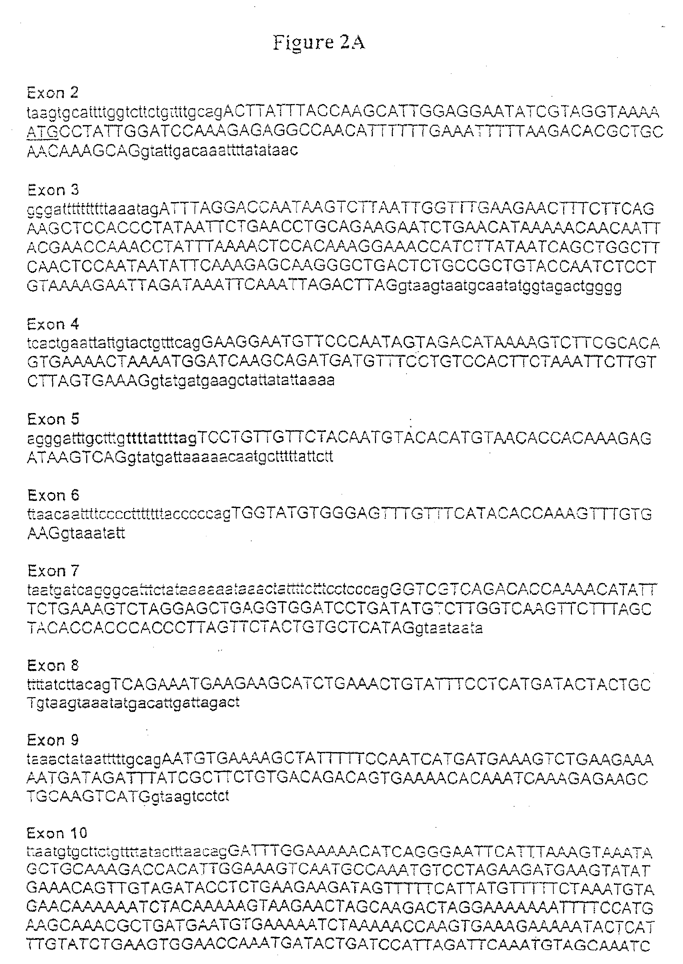

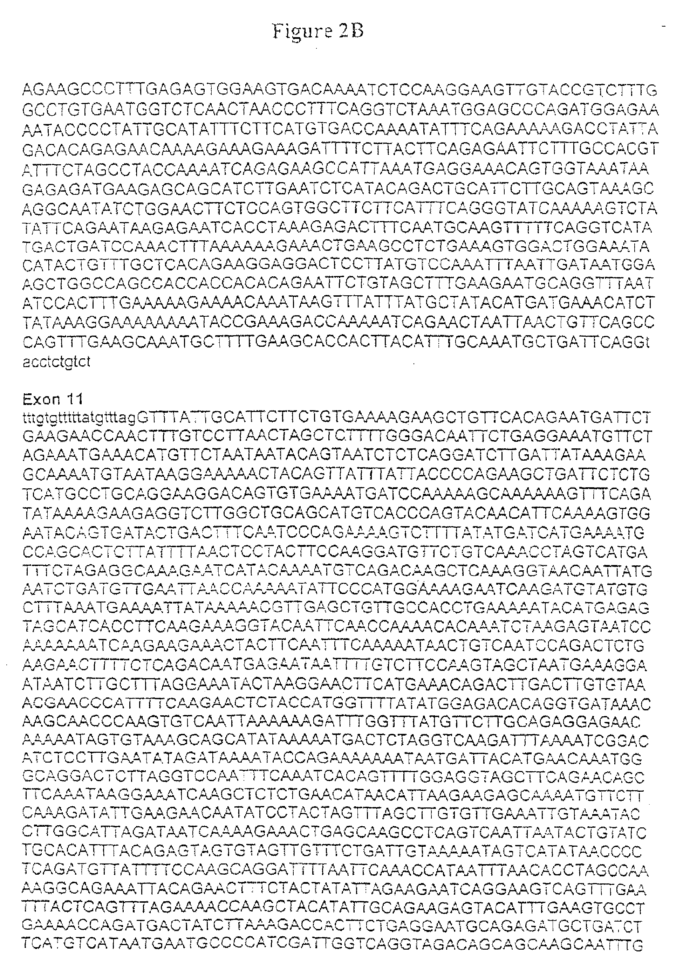

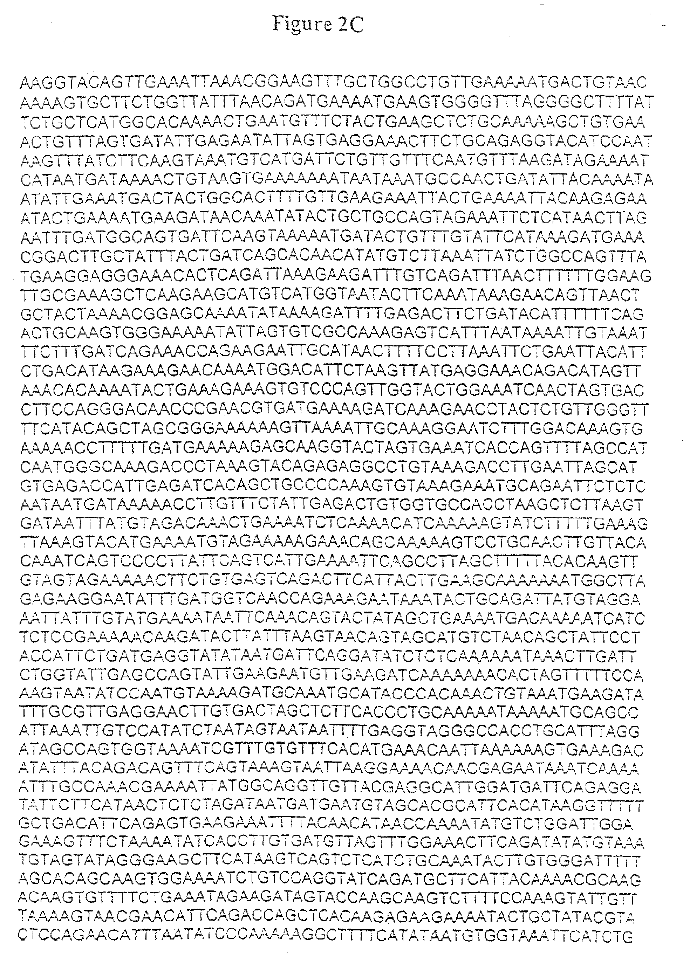

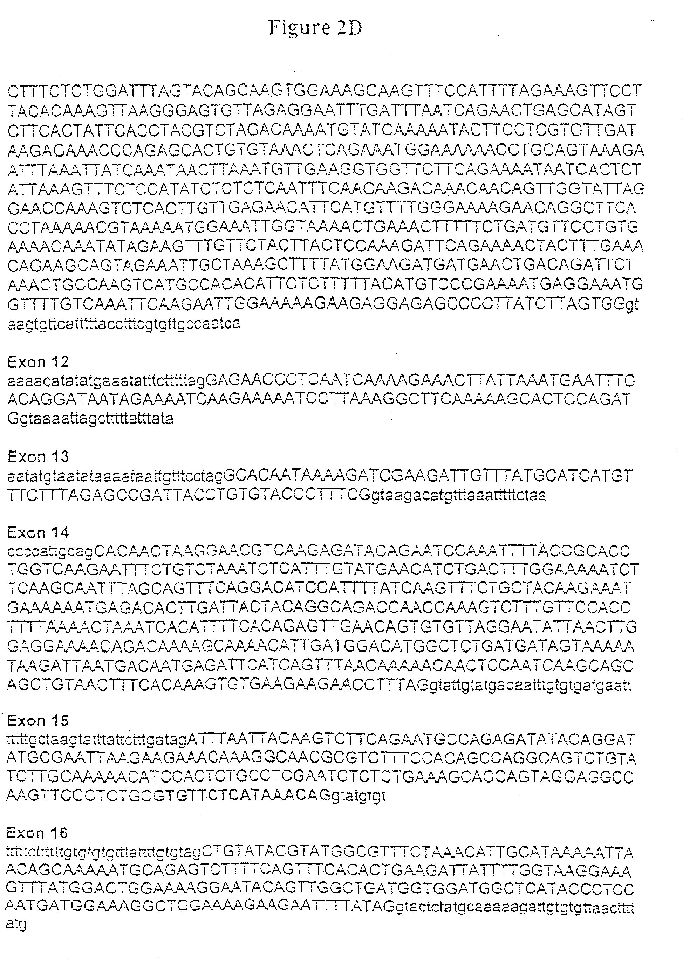

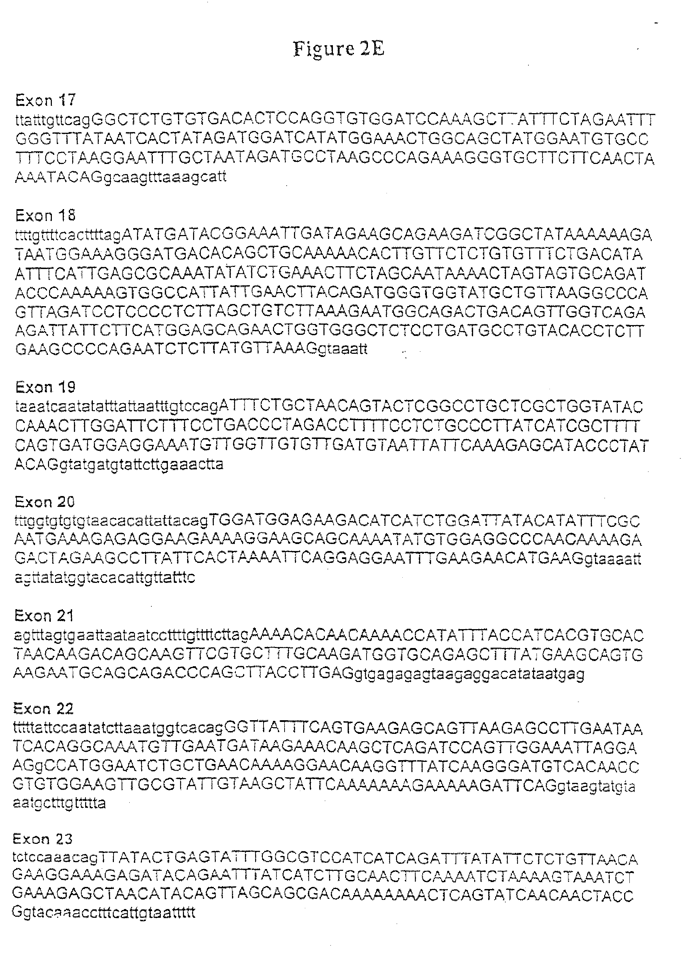

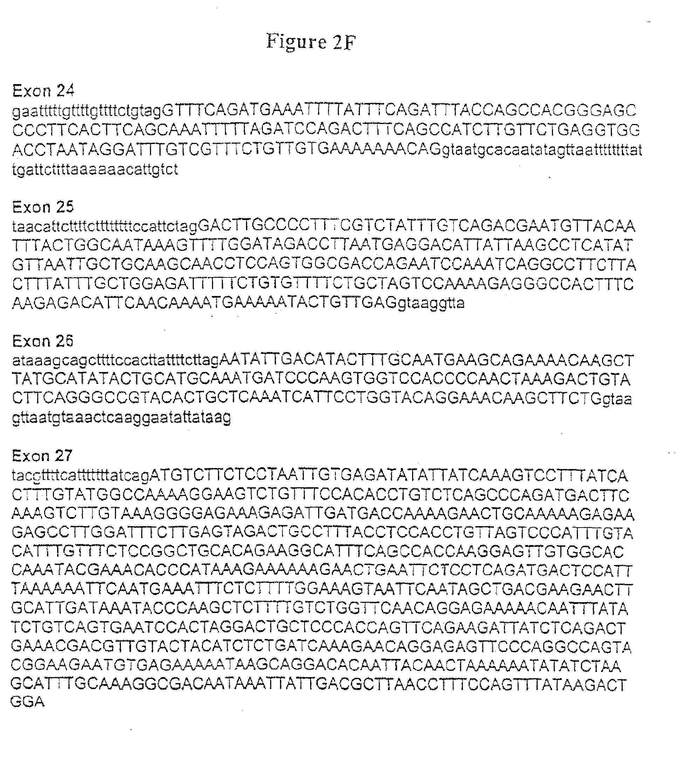

Based upon the sequencing of the five normal individuals, it was determined that the standard sequence found in both GenBank and BIC were inaccurate. In Genbank, a 10 bp stretch (5'-TTTATTTTAG-3') was mistakenly listed as exonic at the 5' end of exon 5 while it should be intronic which would not be included in the cDNA and resultant protein. In addition, a more detrimental error that has the significant potential to lead to an incorrect diagnosis of breast cancer propensity exists in both Genbank and BIC: a sequence of 16 bp (5'-GTGTTCTCATAAACAG-3') should be at the end of exon 15, but instead is listed at the beginning of exon 16 in the database. The disclosure and listing of GenBank is shown in Figure 1. The correct intron/exon sequence of BRCA2 is presented in Figure 2, wherein,
(1) a 10 bp stretch (5'-TTTATTTTAG-3') is intronic at 3' end of intron 4, rather than at the 5' end of exon 5 (corrected exon 5 is listed as SEQ. ID. NO: 1) and
(2) a 16 bp stretch (5'-GTGTTCTCATAAACAG-3') is exonic at the 3' end of exon 15, rather than at the 5' end of exon 16 (corrected exons 15 and 16 are listed as SEQ. ID. No: 2 and 3 respectively)

The BIC BRCA2 sequence also contains sequence errors in which a strech of nine nucleotides at positions 5554-5460 is listed as CGTTTGTGT (amino acids: Arg-Leu-Cys). The correct sequence at these positions is GTTTGTGTT (amino acids: Val-Cys-Val). In addition, the BIC BRCA2 nucleotides at positions 2024 (codon 599), 4553 (codon 1442), 4815 (codon 1529), 5841 (codon 1871), and 5972 (codon 1915) are T, T, A, C, and T respectively, wherein the correct nucleotides at these positions are C, C, G, T, and C respectively. Among them, the nuclotide errors at codon 599, 1442, 1915 result in amino acids changes.

Additional differences in the nucleic acids of the five normal individuals were found in ten polymorphic locations. The changes and their positions are found in TABLE III. The individual haplotypes of each chromosome of BRCA2 are displayed in FIGURE 3. In each case, the initial haplotype reported in Genbank (accession number U43746) was subtracted to determine the new haplotypes OMI 1-5. Thus, the Genbank sequence only represents 50% of the haplotypes found; the five new BRCA2 ^{(omi 1-5)} DNA sequences are shown as SEQ. ID. NO: 4, 6, 8, 10, and 12, respectively (See FIGURE 3), and the corresponding polypeptides are listed as SEQ. ID. NO: 5, 7, 9, 11, and 13 respectively. In combination, these seven haplotypes represent a functional allele profile for the BRCA2 gene.

The data show that for each of the samples, all exons of BRCA2 were identical except in the region of ten polymorphisms. Six of these polymorphisms were previously identified (Tartigan et al., Nature Genetics 12: 333-337 (1996); Phelan et al., Nature Genetics 13: 120-122 (1996); Couch et al., Nature Genetics 13: 123-125 (1996); Teng, et al., Nature Genetics 13: 241-244 (1996); Schubert et al 60: 1031-1040 (1997)), but four were unique to this work. Even though the individual polymorphisms may have been identified, none of these complete haplotypes has been previously determined.

**TABLE I**

| **Hereditary Cancer Pro-Screening Questionnaire** | | | |
|---|---|---|---|
| **Part A**: Answer the following questions about your family | | | |
| | | | |
| 1. | To your knowledge, has anyone in your family been diagnosed with a very specific hereditary colon disease called Familial Adenomatous Polyposis (FAP)? | | |
| | | | |
| 2. | To your knowledge, have you or any aunt had breast cancer diagnosed before the age 35? | | |
| | | | |
| 3. | Have you had Inflammatory Bowel Disease, also called Crohn's Disease or Ulcerative Colitis, for more than 7 years? | | |
| | | | |
| **Part B:** Refer to the list of cancers below for your responses only to questions in Part B | | | |
| | | | |
| | Bladder Cancer | Lung Cancer | Pancreatic Cancer |
| | Breast Cancer | Gastric Cancer | Prostate Cancer |
| | Colon Cancer | Malignant Melanoma | Renal Cancer |
| | Endometrial Cancer | Ovarian Cancer | Thyroid Cancer |
| | | | |
| 4. | Have your mother or father, your sisters or brothers or your children had any of the listed cancers? | | |
| | | | |
| 5 | Have there been diagnosed in your mother's brothers or sisters, or your mother's parents more than one of the cancers in the above list? | | |
| | | | |
| 6. | Have there been diagnosed in your father's brothers or sisters, or your father's parents more than one of the cancers in the above list? | | |
| | | | |
| **Part C:** Refer to the list of relatives below for responses only to questions in Part C | | | |
| | | | |
| | You | Your mother | |
| | Your sisters or brothers | Your mother's sisters or brothers (maternal aunts & uncles) | |
| | Your children | Your mother's parents (maternal grandparents) | |
| | | | |
| 7. | Have there been diagnosed in Do not count "simple" skin cancer, | these relatives 2 or more identical also called basal cell or squamous | types of cancer? cell skin cancer. |
| | | | |
| 8. | Is there a total of 4 or more of any cancers in the list of relatives above other than "simple" skin cancers? | | |
| | | | |
| **Part D:** Refer to the list of relatives below for responses only to questions in Part D. | | | |
| | | | |
| | You | Your father | |
| | Your sisters or brothers | Your father's sisters or brothers (paternal aunts and uncles) | |
| | Your children | Your father's parents (paternal grandparents) | |
| | | | |
| 9. | Have there been diagnosed in these relatives 2 or more identical types of cancer? Do not count "simple" skin cancer, also called basal cell or squamous cell skin cancer. | | |
| | | | |
| 10. | Is there a total of 4 or more of any cancers in the list of relatives above other than "simple" skin cancers? | | |
| | | | |
| © Copyright 1996, OncorMed, Inc. | | | |

**TABLE II**

| **BRCA2 PRIMER SEQUENCES** | | | | | |
|---|---|---|---|---|---|
| **Exon** | **Label** | **SEQUENCE (5' TO 3') NOTE: M13 TAIL INCLUDED** | **Oligo Length** | **PCR Product Length** | **SEQ. ID. Number** |
| | | M13 FORWARD = TGT AAA ACG ACG GCC AGT M13 REVERSE = CAG GAA ACA GCT ATG ACC | | | |
| 2 | BRCA2-2F | 5'-TGA GTT TTA CCT CAG TCA CA-3' | 20 | 263 | 14 |
| 2 | BRCA2-2R/M 13R | 5'-CAG GAA ACA GCT ATG ACC CTG TGA CGT ACT GGG TTT TTA GC-3' | 41 | | 15 |
| 3 | BRCA2-3FII | 5'-GAT CTT TAA CTG TTC TGG GTC ACA-3' | 24 | 364 | 16 |
| 3 | BRCA2-3RII | 5'-CCC AGC ATG ACA CAA TTA ATG A-3' | 22 | | 17 |
| 4 | BRCA2-4F/M 13F | 5'-TGT AAA ACG ACG GCC AGT AGA ATG CAA ATT TAT AAT CCA GAG TA-3' | 44 | 268 | 18 |
| 4 | BRCA2-4R-1A | 5'-ATC AGA TTC ATC TTT ATA GAA C-3' | 22 | | 19 |
| 5&6 | BRCA2-5+6F/M13F | 5'-TGT AAA ACG ACG GCC AGT TGT GTT GGC ATT TTA AAC ATC A-3' | 40 | 453 | 20 |
| 5&6 | BRCA2-5+6R/M13R | 5'-CAG GAA ACA GCT ATG ACC CAG GGC AAA GGT ATA ACG CT-3' | 38 | | 21 |
| 7 | BRCA2-7F/M13F | 5'-TGT AAA ACG ACG GCC AGT TAA GTG AAA TAA AGA GTG AA-3' | 38 | 248 | 22 |
| 7 | BRCA2-7R/M13R | 5'-CAG GAA ACA GCT ATG ACC AGA AGT ATT AGA GAT GAC-3' | 36 | | 23 |
| 8 | BRCA2-8F/M13F | 5'-TGT AAA ACG ACG GCC AGT GCC ATA TCT TAC CAC CTT GTG A-3' | 40 | 319 | 24 |
| | BRCA2-8FIA | 5'-TTG CAT TCT AGT GAT AAT ATA C-3' | 22 | 143 | 25 |
| 8 | BRCA2-8RIA | 5'-AAT TGT TAG CAA TTT CAA C-3' | 19 | | 26 |
| 9 | BRCA2-9F/M13F | 5'-TGT AAA ACG ACG GCC AGT TGG ACC TAG GTT GAT TGC AGA T-3' | 40 | 338 | 27 |
| 9 | BRCA2-9R/M13R | 5'-CAG GAA ACA GCT ATG ACC TAA ACT GAG ATC ACG GGT GAC A-3' | 40 | | 28 |
| 10A | BRCA2-10AF | 5'-GAA TAA TAT AAA TTA TAT GGC TTA-3' | 24 | 255 | 29 |
| 10A | BRCA2-10AR/M13R | 5'-CAG GAA ACA GCT ATG ACC CCT AGT CTT GCT AGT TCT T-3' | 37 | | 30 |
| 10B | BRCA2-10BF/M13F | 5'- TGT AAA ACG ACG GCC AGT ARC TGA AGT GGA ACC AAA TGA TAC-3' | 42 | 621 | 31 |
| 10B | BRCA2-10BR/M13R | 5'- CAG GAA ACA GCT ATG ACC ACG TGG CAA AGA ATT CTC TGA AGT AA- 3' | 44 | | 32 |
| 10C | BRCA2-10CF/M13F | 5'-TGT AAA ACG ACG GCC AGT CAG CAT CTT GAA TCT CAT ACA G-3' | 40 | 508 | 33 |
| 10C | BRCA2-10CRII | 5'-AGA CAG AGG TAC CTG AAT C-3' | 19 | | 34 |
| 11 | BRCA2-11 AF-M13 | 5'- TGT AAA ACG ACG GCC AGT TGG TAC TTT AAT TTT GTC ACT T-3' | 40 | 304 | 35 |
| 11 | BRCA2-11AR-M13 | 5'-CAG GAA ACA GCT ATG ACC TGC AGG CAT GAC AGA GAA T-3' | 37 | | 36 |
| 11 | BRCA2-11BF | 5'-AAG AAG CAA AAT GTA ATA AGG A-3' | 22 | 411 | 37 |
| 11 | BRCA2-11BR | 5'-CAT TTA AAG CAC ATA CAT CTT G-3' | 22 | | 38 |
| 11 | BRCA2-11CF | 5'-TCT AGA GGC AAA GAA TCA TAC-3' | 21 | 349 | 39 |
| 11 | BRCA2-11CR | 5'-CAA GAT TAT TCC TTT CAT TAG C-3' | 22 | | 40 |
| 11 | BRCA2-11DF | 5'-AAC CAA AAC ACA AAT CTA AGA G-3' | 22 | 344 | 41 |
| 11 | BRCA2-11 DR | 5'-GTC ATT TTT ATA TGC TGC TTT AC-3' | 23 | | 42 |
| 11 | BRCA2-11EF | 5'-GGT TTT ATA TGG AGA CAC AGG-3' | 21 | 369 | 43 |
| 11 | BRCA2-11ER | 5'-GTA TTT ACA ATT TCA ACA CAA GC-3' | 23 | | 44 |
| 11 | BRCA2-11FF | 5'-ATC ACA GTT TTG GAG GTA GC-3' | 20 | 368 | 45 |
| 11 | BRCA2-11FR | 5'-CTG ACT TCC TGA TTC TTC TAA-3' | 21 | | 46 |
| 11 | BRCA2-11GF | 5'-CTC AGA TGT TAT TTT CCA AGC-3' | 21 | 366 | 47 |
| 11 | BRCA2-11GR | 5'-CTG TTA AAT AAC CAG AAG CAC-3' | 21 | | 48 |
| 11 | BRCA2-11HF | 5'-AGG TAG ACA GCA GCA AGC-3' | 18 | 360 | 49 |
| 11 | BRCA2-11HR | 5'-GTA ATA TCA GTT GGC ATT TAT T-3' | 22 | | 50 |
| 11 | BRCA2-11IF | 5'-TGC AGA GGT ACA TCC AAT AAG-3' | 21 | 326 | 51 |
| 11 | BRCA2-11IR | 5'-GAT CAG TAA ATA GCA AGT CCG-3' | 21 | | 52 |
| 11 | BRCA2-11JF | 5'-TAC TGA AAA TGA AGA TAA CAA AT-3' | 23 | 477 | 53 |
| 11 | BRCA2-11JR | 5'-ATT TTG TTC TTT CTT ATG TCA G-3' | 22 | | 54 |
| 11 | BRCA2-11KF-M13 | 5'-TGT AAA ACG ACG GCC AGT CTA CTA AAA CGG AGC AA-3' | 35 | 382 | 55 |
| 11 | BRCA2-11KR-M13 | 5'-CAG GAA ACA GCT ATG ACC GTA TGA AAA CCC AAC AG-3' | 35 | | 56 |
| 11 | BRCA2-11LF | 5'-CAC AAA ATA CTG AAA GAA AGT G-3' | 22 | 374 | 57 |
| 11 | BRCA2-11LR | 5'-GGG ACC ACA GTC TCA ATA G-3' | 19 | | 58 |
| 11 | BRCA2-11MF | 5'-GCA AAG ACC CTA AAG TAC AG-3' | 20 | 409 | 59 |
| 11 | BRCA2-11MR | 5'-CAT CAA ATA TTC CTT CTC TAA G-3' | 22 | | 60 |
| 11 | BRCA2-11NF-M13 | 5'-TGT AAA ACG ACG GCC AGT GAA AAT TCA GCC TTA GC-3' | 35 | 306 | 61 |
| 11 | BRCA2-11NR-M13 | 5'- CAG GAA ACA GCT ATG ACC ATC AGA ATG GTA GGA AT-3' | 35 | | 62 |
| 11 | BRCA2-11OF | 5'-GTA CTA TAG CTG AAA ATG ACA A-3' | 22 | 383 | 63 |
| 11 | BRCA2-11OR | 5'-ACC ACT GGC TAT CCT AAA TG-3' | 20 | | 64 |
| 11 | BRCA2-11PF | 5'-TGA AGA TAT TTG CGT TGA GG-3' | 20 | 355 | 65 |
| 11 | BRCA2-11PR | 5'-GTC AGC AAA AAC CTT ATG TG-3' | 20 | | 66 |
| 11 | BRCA2-11QF | 5'-AGG AAA ATT ATG GCA GGT TGT-3' | 21 | 337 | 67 |
| 11 | SRCA2-11QR | 5'-CTT GTC TTG CGT TTT GTA ATG-3' | 21 | | 68 |
| 11 | BRCA2-11RF | 5'-GCT TCA TAA GTC AGT CTC AT-3' | 20 | 360 | 69 |
| 11 | BRCA2-11RR | 5'-TCA AAT TCC TCT AAC ACT CC-3' | 20 | | 70 |
| 11 | BRCA2-11SF-M13 | 5'-TGT AAA ACG ACG GCC AGT TAC AGC AAG TGG AAA GC-3' | 35 | 458 | 71 |
| 11 | BRCA2-11SR-M13 | 5'-CAG GAA ACA GCT ATG ACC AAG TTT CAG TTT TAC CAA T-3' | 37 | | 72 |
| 11 | BRCA2-11TF | 5'-GTT CTT CAG AAA ATA ATC ACT C-3' | 22 | 344 | 73 |
| 11 | BRCA2-11TR | 5'-TGT AAA AAG AGA ATG TGT GGC-3' | 21 | | 74 |
| 11 | BRCA2-11UF-M13 | 5'-TGT AAA ACG ACG GGC AGT ACT TTT TCT GAT GTT CCT GTG-3' | 39 | 328 | 75 |
| 11 | BRCA2-11UR-M13 | 5'-CAG GAA ACA GCT ATG ACC TAA AAA TAG TGA TTG GCA ACA-3' | 39 | | 76 |
| 12 | BRCA2-12F/M13F | 5'-TGT AAA ACG ACG GCC AGT AGT GGT GTT TTA AAG TGG TCA AAA-3' | 42 | 391 | 77 |
| 12 | BRCA2-12R/M13R | 5'-CAG GAA ACA GCT ATG ACC GGA TCC ACC TGA GGT CAG AAT A-3' | 40 | | 78 |
| 13 | BRCA2/13-2F | 5'-TAA CAT TTA AGC ATC CGT TAC-3' | 21 | 310 | 79 |
| 13 | BRCA2/13-2R | 5'-AAA CGA GAC TTT TCT CAT ACT GTA TTA G-3' | 28 | | 80 |
| 14 | BRCA2-14F | 5'-ACC ATG TAG CAA ATG AGG GTC T-3' | 22 | 391 | 81 |
| 14 | BRCA2-14AR | 5'-GCT TTT GTC TGT TTT CCT CCA A-3' | 22 | | 82 |
| 15 | BRCA2-15-2F | 5'-CCA GGG GTT GTG CTT TTT AAA-3' | 21 | 284 | 83 |
| 15 | BRCA2-15FUT/M13-R | 5'-CAG GAA ACA GCT ATG ACC ACT CTG TCA TAA AAG CCA TC-3' | 38 | | 84 |
| 16 | BRCA2-16AF | 5'-TTT GGT TTG TTA TAA TTG TTT TTA-3' | 24 | 394 | 85 |
| 16 | BRCA2-18AR | 5'-CCA ACT TTT TAG TTC GAG AG-3' | 20 | | 86 |
| 17 | BRGA2-17F | 5'-TTC AGT ATC ATC CTA TGT G-3' | 19 | 282 | 87 |
| 17 | BRCA2-17AR | 5'-AGA AAC CTT AAC CCA TAC TG-3' | 20 | | 88 |
| 18 | BRCA2-18FUT/M13-AF | 5'-TGT AAA ACG ACG GCC AGT GAA TTC TAG AGT CAC ACT TCC-3' | 39 | 275 | 89 |
| 18 | BRCA2-18R/M13R | 5'-CAG GAA ACA GCT ATG ACC TTT AAC TGA ATC AAT GAC TG-3' | 38 | | 90 |
| 19 | BRCA2-19F/M13F | 5'-TGT AAA ACG ACG GCC AGT AAG TGA ATA TTT TTA AGG CAG TT-3' | 41 | 355 | 91 |
| 19 | BRCA2-19FUT/M13-R | 5'-CAG GAA ACA GCT ATG ACC AAG AGA CCG AAA CTC CAT CTC-3' | 39 | | 92 |
| 20 | BRCA2-20F/M13F | 5'-TGT AAA ACG ACG GCC AGT CAC TGT GCC TGG CCT GAT AC-3' | 38 | 296 | 93 |
| 20 | BRCA2-20R/M13R | 5'-CAG GAA ACA GCT ATG ACC ATG TTA AAT TCA AAG TCT CTA-3' | 39 | | 94 |
| 21 | BRCA2-21F/M13F | 5'-TGT AAA ACG ACG GCC AGT GGG TGT TTT ATG CTT GGT TCT-3' | 39 | 304 | 95 |
| 21 | BRCA2-21 R/M13R | 5'-CAG GAA ACA GCT ATG ACC CAT TTC AAC ATA TTC CTT CCT G-3' | 40 | | 96 |
| 22 | BRCA2-22F-1A | 5'-AAC CAC ACC CTT AAG ATG A-3' | 19 | 453 | 97 |
| 22 | BRCA2-22R-1A | 5'-GCA TTA GTA GTG GAT TTT GC-3' | 20 | | 98 |
| 23 | BRCA2-23FII | 5'-TCA CTT CCA TTG CAT C-3' | 16 | 290 | 99 |
| 23 | BRCA2-23RII | 5'-TGC CAA CTG GTA GCT CC-3' | 17 | | 100 |
| 24 | BRCA2-24 2F | 5'-TAC AGT TAG CAG CGA CAA AA-3' | 20 | 373 | 101 |
| 24 | BRCA2-24R/M13R | 5'-CAG GAA ACA GCT ATG ACC ATT TGC CAA CTG GTA GCT CC-3' | 38 | | 102 |
| 25 | BRCA2-25F-7/23 | 5'-GCT TTC GCC AAA TTC AGC TA-3' | 20 | 427 | 103 |
| 25 | BRCA2-25R-7/23 | 5'-TAC CAA AAT GTG TGG TGA TG-3' | 20 | | 104 |
| 26 | BRCA2/26-2F | 5'-AAT CAC TGA TAC TGG TTT TG-3' | 20 | 530 | 105 |
| 26 | BRCA2/26-2R | 5'-TAT ACT TAC AGG AGC CAC AT-3' | 20 | | 106 |
| 27A | BRCA2-27AF-1A | 5'-CTG TGT GTA ATA TTT GCG-3' | 18 | 495 | 107 |
| 27A | BRCA2-27AR/M13R | 5'-CAG GAA ACA GCT ATG ACG GCA AGT TCT TCG TCA GCT ATT G-3' | 40 | | 108 |
| 27B | BRCA2-27BF/M13F | 5'-TGT AAA ACG ACG GCC AGT GAA TTC TCC TCA GAT GAC TCC A-3' | 40 | 417 | 109 |
| 27B | BRCA2-27BR/M13R | 5'-CAG GAA ACA GCT ATG ACC TCT TTG CTC ATT GTG CAA CA-3' | 38 | | 110 |

**TABLE III**

| **NORMAL PANEL TYPING** | | | | | | | | |
|---|---|---|---|---|---|---|---|---|
| **Position nt/codon** | **Nucleotide Change** | **Amino Acid Change** | **1** | **2** | **3** | **4** | **5** | **Frequency** |
| 1093/289 | AAT → CAT | Asn → His | A/A | A/C | A/A | A/A | A/C | A = 8 |
| | | | | | | | | C = 2 |
| | | | | | | | | |
| 1342/372 | AAT → CAT | Asn → His | A/C | A/A | A/C | A/C | A/C | A = 0.6 |
| | | | | | | | | C = 0.4 |
| | | | | | | | | |
| 1593/455 | TCA → TCG | Ser → Ser | A/A | A/A | A/A | A/A | A/G | A = 0.9 |
| | | | | | | | | G = 0.1 |
| | | | | | | | | |
| 2457/743 | CAT → CAC | His → His | T/T | C/T | T/T | T/T | C/T | T = 0.8 |
| | | | | | | | | C = 0.2 |
| | | | | | | | | |
| 2908/894 | GTA → ATA | Val → Ile | G/G | G/G | G/G | G/G | A/G | G = 0.9 |
| | | | | | | | | A = 0.1 |
| | | | | | | | | |
| 3199/991 | AAC → GAC | Asn → Asp | A/A | A/G | A/A | A/A | A/G | A = 0.8 |
| | | | | | | | | G = 0.2 |
| | | | | | | | | |
| 3624/1132 | AAA → AAG | Lys → Lys | A/A | A/G | A/A | A/G | A/A | A = 0.8 |
| | | | | | | | | G = 0.2 |
| | | | | | | | | |
| 4035/1269 | GTT → GTC | Val → Val | C/T | T/T | T/T | T/T | T/T | T = 0.9 |
| | | | | | | | | C = 0.1 |
| | | | | | | | | |
| 7470/2414 | TCA → TCG | Ser → Ser | A/A | A/G | A/A | A/G | A/A | A = 0.8 |
| | | | | | | | | G = 0.2 |
| | | | | | | | | |
| 9079/2951 | GCC → ACC | Ala → Thr | GIG | G/G | G/G | G/G | A/G | G = 0.9 |
| | | | | | | | | A = 0.1 |
| | | | | | | | | |

### EXAMPLE 2

### Determination Of A Normal Individual Using BRCA2^{(OMI 1-5)} and The Ten Polymorphisms For Reference

A person skilled in the art of genetic susceptibility testing will find the present invention useful for:
a) identifying individuals having a normal BRCA2 gene;
b) avoiding misinterpretation of normal polymorphisms found in the normal population.

Sequencing was carried out as in EXAMPLE 1 using a blood sample from the patient in question. However, the BRCA2^{(omi1-5)} sequences were used for reference and any polymorphic sites seen in the patient were compared to the nucleic acid sequences listed above for normal codons at each polymorphic site. A normal sample is one which is comparable to the BRCA2^{(omi 1-5)} sequences and contains only minor variations which occur at minor polymorphic sites. The allelic variations which occur at each of the polymorphic sites are paired here for reference.
- AAT (Asn) and CAT (His) at position 1093 (codon 289)
- CAT (His) and AAT (Asn) at position 1342 (codon 372)
- TCA (Ser) and TCG (Ser) at position 1593 (codon 455)
- CAT (His) and CAC (His) at position 2457 (codon 743)
- GTA (Val) and ATA (Ile) at position 2908 (codon 894)
- AAC (Asn) and GAC (Asp) at position 3199 (codon 991)
- AAA (Lys) and AAG (Lys) at position 3624 (codon 1132)
- GTT (Val) and GTC (Val) at position 4035 (codon 1269)
- TCA (Ser) and TCG (Ser) at position 7470 (codon 2414)
- GCC (Ala) and ACC (Thr) at position 9079 (codon 2951)

The availability of these polymorphic pairs provides added assurance that one skilled in the art can correctly interpret the polymorphic variations without mistaking a normal variation for a mutation.

All exons of the BRCA2 gene are subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye is attached for automated sequencing using the Taq Dye Terminator Kit (Perkin-Eimer^{®} cat# 401628). DNA sequencing is performed in both forward and reverse directions on an Applied Biosystems, Inc. (ABI) automated sequencer (Model 377). The software used for analysis of the resulting data is "Sequence Navigator" purchased through ABI.

### 1. Polymerase Chain Reaction PCR Amplification

The PCR primers used to amplify a patient's sample BRCA2 gene are listed in TABLE II. The primers were synthesized on a DNA/RNA Synthesizer Model 394^{®}. Thirty-five cycles are of amplification are performed, each consisting of denaturing (95°C; 30 seconds), annealing (55°C; 1 minute), and extension (72°C; 90 seconds), except during the first cycle in which the denaturing time is increased to 5 minutes and during the last cycle in which the extension time is increased to 5 minutes.

PCR products are purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye is attached to PCR products for automated sequencing using the Taq Dye Terminator Kit (Perkin-Elmer^{®} cat# 401628). DNA sequencing is performed in both forward and reverse directions on an Applied Biosystems, Inc. (ABI) Foster City, CA., automated sequencer (Model 377). The software used for analysis of the resulting data is "Sequence Navigator^{®}" purchased through ABI. The BRCA2^{(omi 1-5)} sequences were entered sequentially into the Sequence Navigator software as the standards for comparison. The Sequence Navigator software compares the patient sample sequence to each BRCA2 ^{(omi 1-5)} standard, base by base. The Sequence Navigator highlights all differences between the standards (omi 1-5) and the patient's sample sequence.

A first technologist checks the computerized results by comparing visually the BRCA2 ^{(omi 1-5)} standards against the patient's sample, and again highlights any differences between the standard and the sample. The first primary technologist then interprets the sequence variations at each position along the sequence. Chromatograms from each sequence variation are generated by the Sequence Navigator and printed on a color printer. The peaks are interpreted by the first primary technologist and a second primary technologist. A secondary technologist then reviews the chromatograms. The results are finally interpreted by a geneticist. In each instance, a variation is compared to known normal polymorphism for position and base change.

### 3. Results

The patient's BRCA2 sequence was found to be heterozygous at seven nucleotide positions: 1093 (A/C), 1342 (A/C), 1593 (A/G), 2457 (C/T), 2908 (A/G), 3199 (A/G) and 9079 (A/G). In addition, this changes five amino acids in the polypeptide product: Asn to His at codon 289, Asn to His at codon 372, Val to Ile at codon 894, Asn to Asp at codon 991, and Ala to Thr at codon 2951. The question arises whether any or all of these changes have significance to the patient. Comparison of the patient's results to the BRCA ^{(omi 1-5)} haplotypes demonstrates that it matches one of the BRCA2 omi standards (#5), and thus the patient sample is interpreted as carrying a normal gene sequence without causing any elevation in their risk status for breast cancer.

### EXAMPLE 3

### DETERMINING THE PRESENCE OF A MUTATION IN EXON 11 OF THE BRCA2 GENE USING BRCA2^{(omi1-5)}

A person skilled in the art of genetic susceptibility testing will find the present invention useful for determining the presence of a known or previously unknown mutation in the BRCA2 gene. A list of mutations of BRCA2 is publicly available in the Breast Cancer Information Core at http://www.nchgr.nih.gov/dir/lab_transfer/bic. This data site became publicly available on November 1, 1995. Friend, S. et al. Nature Genetics 11:238, (1995).

In this example, a mutation in exon 11 is characterized by amplifying the region of the mutation with a primer set which amplifies the region of the mutation. Sequencing was carried out as in Example 1 using a blood sample from the patient in question. Specifically, exon 11 of the BRCA2 gene is subjected to direct dideoxy sequence analysis by asymmetric amplification using the polymerase chain reaction (PCR) to generate a single stranded product amplified from this DNA sample. Shuldiner, et al., Handbook of Techniques in Endocrine Research, p. 457-486, DePablo, F., Scanes, C., eds., Academic Press, Inc., 1993. Fluorescent dye is attached for automated sequencing using the Taq Dye Terminator Kit (Perkin-Elmer^{®} cat# 401628). DNA sequencing is performed in both forward and reverse directions on an Applied Biosystems, Inc. (ABI) automated sequencer (Model 377). The software used for analysis of the resulting data is "Sequence Navigator" purchased through ABI.

### 1. Polymerase Chain Reaction PCR Amplification

Genomic DNA (100 nanograms) extracted from white blood cells of the subject is amplified in a final volume of 25 microliters containing 1 microliter (100 nanograms) genomic DNA, 2.5 microliters 10X PCR buffer (100 mM Tris, pH 8.3, 500 mM KCI, 1.2 mM MgCl₂), 2.5 microliters 10X dNTP mix (2 mM each nucleotide), 2.5 microliters forward primer (BRCA2-11Q-F, 10 micromolar solution), 2.5 microliters reverse primer (BRCA2-11Q-R, 10 micromolar solution),and 1 microliter Taq polymerase (5 units), and 13 microliters of water.

The PCR primers used to amplify segment Q of exon 11 (where the mutation 6174delT is found) are as follows:
BRCA2-11Q-F: 5'- ACG' AAA' ATT' ATG' GCA' GGT' TGT-3'
BRCA2-11Q-R: 5'- CTT' GTC' TTG' CGT' TTT' GTA' ATG-3'

The primers are synthesized on an DNA/RNA Synthesizer Model 394^{®}. Thirty-five cycles are performed, each consisting of denaturing (95°C; 30 seconds), annealing (55°C; 1 minute), and extension (72°C; 90 seconds), except during the first cycle in which the denaturing time is increased to 5 minutes, and during the last cycle in which the extension time is increased to 5 minutes.

PCR products are purified using Qia-quick^{®} PCR purification kits (Qiagen^{®}, cat# 28104; Chatsworth, CA). Yield and purity of the PCR product are determined spectrophotometrically at OD₂₆₀ on a Beckman DU 650 spectrophotometer.

### 2. Dideoxy Sequence Analysis

Fluorescent dye is attached to PCR products for automated sequencing using the Taq Dye Terminator Kit (Perkin-Elmer^{®} cat# 401628). DNA sequencing is performed in both forward and reverse directions on an Applied Biosystems, Inc. (ABI) Foster City, CA., automated sequencer (Model 377). The software used for analysis of the resulting data is "Sequence Navigator^{®}" purchased through ABI. The BRCA2^{(omi 1-5)} sequence is entered into the Sequence Navigator software as the Standard for comparison. The Sequence Navigator software compares the sample sequence to the BRCA2^{(omi)} standard, base by base. The Sequence Navigator highlights all differences between the BRCA2^{(omi)} normal DNA sequence and the patient's sample sequence.

A first technologist checks the computerized results by comparing visually the BRCA2^{(omi 1-5)} standard against the patient's sample, and again highlights any differences between the standard and the sample. The first primary technologist then interprets the sequence variations at each position along the sequence. Chromatograms from each sequence variation are generated by the Sequence Navigator and printed on a color printer. The peaks are interpreted by the first primary technologist and a second primary technologist. A secondary technologist then reviews the chromatograms. The results are finally interpreted by a geneticist. In each instance, a sequence variation is compared to known normal polymorphisms for position and base change. The ten frequent polymorphisms which occur in BRCA2 are:
- AAT (Asn) and CAT (His) at position 1093 (codon 289)
- CAT (His) and AAT (Asn) at position 1342 (codon 372)
- TCA (Ser) and TCG (Ser) at position 1593 (codon 455)
- CAT (His) and CAC (His) at position 2457 (codon 743)
- GTA (Val) and ATA (Ile) at position 2908 (codon 894)
- AAC (Asn) and GAC (Asp) at position 3199 (codon 991)
- AAA (Lys) and AAG (Lys) at position 3624 (codon 1132)
- GTT (Val) and GTC (Val) at position 4035 (codon 1269)
- TCA (Ser) and TCG (Ser) at position 7470 (codon 2414)
- GCC (Ala) and ACC (Thr) at position 9079 (codon 2951)

### 3. Results

Using the above PCR amplification and standard fluorescent sequencing technology, the 6174delT mutation may be found. Mutations are noted by the length of non-matching sequence variation. Such a lengthy mismatch pattern occurs with deletions and insertions. This mutation is named in accordance with the suggested nomenclature for naming mutations, Beaudet, A et a/., Human Mutation 2:245-248, (1993). The 6174delT mutation at codon 1982 of the BRCA2 gene lies in segment "Q" of exon 11. The DNA sequence results demonstrate the presence of a one base pair deletion of a T at nucleotide 6174 of the BRCA2^{(omi 1-5)} sequences. This mutation interrupts the normal reading frame of the BRCA2 transcript, resulting in the appearance of an in-frame terminator (TAG) at codon position 2003. This mutation is, therefore, predicted to result in a truncated, and most likely, non-functional protein.

### EXAMPLE 4

### GENERATION OF MONOCLONAL AND POLYCLONAL ANTIBODIES USING GST-BRCA2 FUSION PROTEIN AS AN IMMUNOGEN

DNA primers are used to amplify a fragment of BRCA2 using PCR technology. The product is then digested with suitable restriction enzymes and fused in frame with the gene encoding glutathione S-transferase (GST) in *Escherichia coli* using GST expression vector pGEX (Pharmacia Biotech Inc.) The expression of the fusion protein is induced by the addition of isopropyl-β-thiogalactopyranoside. The bacteria are then lysed and the overexpressed fusion protein is purified with glutathione-sepharose beads. The fusion protein is then verified by SDS/PAGE gel and N-terminus protein sequencing. The purified protein is used to immunize rabbits according to standard procedures described in Harlow & Lane (1988). Polycolonal antibody is collected from the serum several weeks after and purified using known methods in the art. Monoclonal antibodies against all or fragments of BRCA2 protein, polypeptides, or functional equivalents are obtained using hybridoma technology, see also Harlow & Lane (1988). The BRCA2 protein or polypeptide is coupled to the carrier keyhole limpet hemocyanin in the presence of glutaraldehyde. The conjugated immunogen is mixed with an adjuvant and injected into rabbits. Spleens from antibody-containing rabbits are removed. The B-cells isolated from spleen are fused to myeloma cells using polyethylene glycol (PEG) to promote fusion. The hybrids between the myeloma and B-cells are selected and screened for the production of antibodies to immunogen BRCA2 protein or polypeptide. Positive cells are recloned to generate monoclonal antibodies.

### EXAMPLE 5

### DETECTION OF BRGA2 EXPRESSION IN HUMAN TISSUES AND CELL LINES

The expression of BRCA2 in human tissues is determined using Northern blot analysis. Human tissues include those from pancreas, testis, prostate, ovary, breast, small intestine, and colon are obtained from Clontech Laboratories, Inc., Palo Alto, CA. The poly(A)+ mRNA Northern blots from different human tissues is hybridized to BRCA2 cDNA probes according to manufacture protocol. The expression level is further conformed by RT-PCR using oligo-d(T) as a primer and other suitable primers.

For Northern Blot analysis of cancer cell lines, the human ovarian cancer cell line SKOV-3 and the human breast cancer cell line MCF-7 are obtained from the American Type Culture Collection. Total RNA is prepared by lysing cell in the presence of guanidinium isocyanate. Poly(A)⁺ mRNA is isolated using the PolyATract mRNA isolation system from Promega, Madison, WI. The isolated RNA is then electrophoresed under denaturing conditions and transferred to Nylon membrane. The probe used for Northern blot is a fragment of BRCA2 sequence obtained by PCR amplification. The probes are labeled with [α-³²P] dCTP using a random-primed labeling kit (Amersham Life Science, Arlington Heights, IL).

### EXAMPLE 6

### EXPRESSION OF THE BRCA2 PROTEIN

The whole-cell extracts of BRCA2 transfected cells are subjected to immunoprecipitation and immunoblotting to determine the BRCA2 protein level. The BRCA2 protein or polypeptide is immunoprecipitated using anti-BRCA2 antibodies prepared according to Example 4. Samples are then fractionated using SDS/PAGE gel and transferred to nitrocellulose. Western blot of the BRCA2 protein or polypeptide is performed with the indicated antibodies. Antibody reaction is revealed using enhanced chemiluminescence reagents (Dupont New England Nuclear, Boston, MA).

### EXAMPLE 7

### USE OF THE BRCA2^{(omi1-5)} GENE THERAPY

The growth of ovarian or breast cancer may be arrested by increasing the expression of the BRCA2 gene where inadequate expression of that gene is responsible for hereditary ovarian or breast cancer. Gene therapy may be performed on a patient to reduce the size of a tumor. The LXSN vector may be transformed with a BRCA2^{(omil-5)} coding sequence as presented SEQ ID NO:4, 6, 8, 10, or 12 or a fragment thereof.

### Vector

The LXSN vector is transformed with a fragment of the wildtype BRCA2^{(omi1-5)} coding sequence as set forth in SEQ ID NO:4, 6, 8, 10, or 12. The LXSN-BRCA2^{(omi1-5)} retroviral expression vector is constructed by cloning a Sa/ I linkered BRCA2^{(omil-5)} cDNA or fragments thereof into the Xho I site of the vector LXSN. Constructs are confirmed by DNA sequencing. See Holt et al., Nature Genetics 12: 298-302 (1996). Retroviral vectors are manufactured from viral producer cells using serum free and phenol-red free conditions and tested for sterility, absence of specific pathogens, and absence of replication-competent retrovirus by standard assays. Retrovirus is stored frozen in aliquots which have been tested.

Patients receive a complete physical exam, blood, and urine tests to determine overall health. They may also have a chest X-ray, electrocardiogram, and appropriate radiologic procedures to assess tumor stage.

Patients with metastatic ovarian cancer are treated with retroviral gene therapy by infusion of recombinant LXSN-BRCA2^{(omi1-5)} retroviral vectors into peritoneal sites containing tumor, between 10⁹ and 10¹⁰ viral particles per dose. Blood samples are drawn each day and tested for the presence of retroviral vector by sensitive polymerase chain reaction (PCR)-based assays. The fluid which is removed is analyzed to determine:
1. The percentage of cancer cells which are taking up the recombinant LXSN-BRCA2^{(omi1-5)} retroviral vector combination. Successful transfer of BRCA1 gene into cancer cells has been shown by both RT-PCR analysis and *in situ* hybridization. RT-PCR is performed with by the method of Thompson et al., Nature Genetics 9: 444-450 (1995), using primers derived from a BRCA2^{(omi1-5)} coding sequence as in SEQ ID NO:4, 6, 8,10, or 12 or fragments thereof. Cell lysates are prepared and immunoblotting is performed by the method of Jensen et al., Nature Genetics 12: 303-308 (1996) and Jensen et al., Biochemistry 31: 10887-10892 (1992).
2. Presence of programmed cell death using APOTAG^{®} *in situ* apoptosis detection kit (ONCOR, INC., Gaithersburg, Maryland) and DNA analysis.
3. Measurement of BRCA2 gene expression by slide immunofluorescence or Western blot.

Patients with measurable disease are also evaluated for a clinical response to LXSN-BRCA2^{(omi1-5)} especially those that do not undergo a palliative intervention immediately after retroviral vector therapy. Fluid cytology, abdominal girth, CT scans of the abdomen, and local symptoms are followed.

For other sites of disease, conventional response criteria are used as follows:
1. Complete Response (CR), complete disappearance of all measurable lesions and of all signs and symptoms of disease for at least 4 weeks.
2. Partial Response (PR), decrease of at least 50% of the sum of the products of the 2 largest perpendicular diameters of all measurable lesions as determined by 2 observations not less than 4 weeks apart. To be considered a PR, no new lesions should have appeared during this period and none should have increased in size.
3. Stable Disease, less than 25% change in tumor volume from previous evaluations.
4. Progressive Disease, greater than 25% increase in tumor measurements from prior evaluations. The number of doses depends upon the response to treatment.

### EXAMPLE 8

### PROTEIN REPLACEMENT THERAPY

Therapeutically elevated level of functional BRCA2 protein may alleviate the absence or reduced endogenous BRCA2 tumor suppressing activity. Breast or ovarian cancer is treated by the administration of a therapeutically effective amount of the BRCA2 protein, a polypeptide, or its functional equivalent in a pharmaceutically acceptable carrier. Clinically effective delivery method is applied either locally at the site of the tumor or systemically to reach other metastasized locations with known protocols in the art. These protocols may employ the methods of direct injection into a tumor or diffusion using time release capsule. A therapeutically effective dosage is determined by one of skill in the art.

Breast or ovarian cancer may be prevented by the administration of a prophylactically effective amount of the BRCA2 protein, polypeptide, or its functional equivalent in a pharmaceutically acceptable carrier. Individuals with known risk for breast or ovarian cancer are subjected to protein replacement therapy to prevent tumorigenesis or to decrease the risk of cancer. Elevated risk for breast and ovarian cancer includes factors such as carriers of one or more known BRCA1 and BRCA2 mutations, late child bearing, early onset of menstrual period, late occurrence of menopause, and certain high risk dietary habits. Clinically effective delivery method is used with known protocols in the art, such as administration into peritoneal cavity, or using an implantable time release capsule. A prophylactically effective dosage is determined by one of skill in the art.

### TABLE OF REFERENCE

1. Sanger, F., et al., J. Mol. Biol. 42:1617, (1980).
2. Beaucage, et al., Tetrahedron Letters 22:1859-1862, (1981).
3. Maniatis, et al. in Molecular Cloning: A Laboratory Manual, Cold Spring Harbor, NY, p 280-281, (1982).
4. Conner, et al., Proc. Natl. Acad. Sci. U.S.A. 80:278, (1983)
5. Saiki, et.al.., Bio/Technology 3:1008-1012, (1985)
6. Landgren, et al., Science 241:1007,(1988)
7. Landgren, et al., Science 242 :229-237, (1988).
8. PCR. A Practical Approach, ILR Press, Eds. M. J. McPherson, P. Quirke, and G. R. Taylor, (1992).
9. Easton et al., American Journal of Human Genetics 52:678-701, (1993).
10.Patent No. 4.458,066.
11.Rowell, S., et al., American Journal of Human Genetics 55:861-865, (1994)
12.Miki, Y. et al., Science 266:66-71, (1994).
13.Wooster, R. et al., Science 265:2088-2090, (1994).
14.Wooster, R. et al., Nature 378:789-792, (1995).
15.Beaudet, A et al., Human Mutation 2:245-248, (1993).
16.Friend, S. et al. Nature Genetics 11:238, (1995).
17.Teng et al, Nature Genetics 13: 241-244 (1996).
18.Couch et al, Nature Genetics 13: 123-125 (1996).
19.Tartigan et al, Nature Genetics 12: 333-337 (1996).
20. Phelan et al, Nature Genetics 13: 120-122 (1996).
21.Schubert et al, American Journal of Human Genetics 60: 1031-1040 (1996).
22. Sambrook, et al., Molecular Cloning: A Laboratory Manual, Second Edition, Cold Spring Harbor Laboratory, Cold Spring Harbor, N.Y. (1989).
23. Bertwistle and Ashworth, Curr. Opin. Genet. Dev. 8(1): 14-20 (1998).
24.Zhang et al., Cell 92:433-436 (1998).
25. Sharan et al., Nature 386:804-810 (1997).
26. Katagiri et al., Genes, Chromosomes & Cancer 21:217-222 (1988).
27. Crooke, Annu. Rev. Pharmacol. Toxical. 32:329-376 (1992)
28. Robinson-Benion and Holt, Methods Enzymol. 254:363-375 (1995).
29.Harlow & Lane, Antibodies: A Laboratory Manual, Cold Spring Harbor Laboratory, Cold Spring Harbor, NY, 1988.
30. Shuldiner, et al., Handbook of Techniques in Endocrine Research, p. 457-486, DePablo, F., Scanes, C., eds., Academic Press, Inc., 1993.
31.Holt et al., Nature Genetics 12: 298-302 (1996).
32.Thompson et al., Nature Genetics 9: 444-450 (1995).
33. Jensen et al., Nature Genetics 12: 303-308 (1996)
34.Jensen at al., Biochemistry 31: 10887-10892 (1992).

Although the invention has been described with reference to the presently preferred embodiments, it should be understood that various modifications can be made without departing from the spirit of the invention. Accordingly, the invention is limited only by the following claims.

The present invention also relates to the following items:
1. A genomic DNA containing a BRCA2 gene,
   wherein the first twelve nucleotides beginning exon 5 are 5'-TCCTGTTGTTCT-3' as set forth in SEQ. ID. NO: 1,
   wherein nucleotides numbers 5782-5790 are GTTTGTGTT as set forth in SEQ. ID. NO: 4, and
   wherein the last 20 nucleotides ending exon 15 are 5'-CTGCGTGTTCTCATAAACAG-3' as set forth in SEQ. ID. NO: 2 and the first 20 nucleotides beginning exon 16 are 5'-CTGTATACGTATGGCGTTTC-3' as set forth in SEQ. ID. NO: 3.
2. The genomic DNA according to item 1 wherein the coding sequence nucleotides are as follows:
   1093 A
   1342 A
   1593 A
   2457 T
   2908 G
   3199 A
   3624 A
   4035 T
   7470 A
   9079 G.
3. The genomic DNA according to item 1 wherein the coding sequence nucleotides are as follows:
   1093 A
   1342 C
   1593 A
   2457 T
   2908 G
   3199 A
   3624 A
   4035 T
   7470 A
   9079 G
4. The genomic DNA according to item 1 wherein the coding sequence nucleotides are as follows:
   1093 A
   1342 C
   1593 A
   2457 T
   2908 G
   3199 A
   3624 A
   4035 C
   7470 A
   9079 G.
5. The genomic DNA according to item 1 wherein the coding sequence nucleotides are as follows:
   1093 C
   1342 A
   1593 A
   2457 C
   2908 G
   3199 G
   3624 G
   4035 T
   7470 G
   9079 G.
6. The genomic DNA according to item 1 wherein the coding sequence nucleotides are as follows:
   1093 A
   1342 C
   1593 A
   2457 T
   2908 G
   3199 A
   3624 G
   4035 T
   7470 G
   9079 G.
7. The genomic DNA according to item 1 wherein the coding sequence nucleotides are as follows:
   1093 C
   1342 C
   1593 G
   2457 C
   2908 A
   3199 G
   3624 A
   4035 T
   7470 A
   9079 A.
8. The genomic DNA according to item 1 wherein the coding sequence nucleotides are as follows:
   2024 C
   4553 C
   4815 G
   5841 T
   5972 C.
9. A DNA comprising a BRCA2 coding sequence,
   wherein nucleotide numbers 643-666 are CTTAGTGAAAGTCCTGTTGTTCTA and
   wherein nucleotides numbers 5782-5790 are GTTTGTGTT.
10. The DNA according to item 9 wherein the coding sequence nucleotides are as follows:
   1093 A
   1342 A
   1593 A
   2457 T
   2908 G
   3199 A
   3624 A
   4035 T
   7470 A
   9079 G.
11. The DNA according to item 9 wherein the coding sequence nucleotides are as follows:
   1093 A
   1342 C
   1593 A
   2457 T
   2908 G
   3199 A
   3624 A
   4035 T
   7470 A
   9079 G
   as set forth in SEQ. ID. NO: 4.
12. The DNA according to item 9 wherein the coding sequence nucleotides are as follows:
   1093 A
   1342 C
   1593 A
   2457 T
   2908 G
   3199 A
   3624 A
   4035 C
   7470 A
   9079 G
   as set forth in SEQ. ID. NO: 6.
13. The DNA according to item 9 wherein the coding sequence nucleotides are as follows:
   1093 C
   1342 A
   1593 A
   2457 C
   2908 G
   3199 G
   3624 G
   4035 T
   7470 G
   9079 G
   as set forth in SEQ. ID. NO: 8.
14. The DNA according to item 9 wherein the coding sequence nucleotides are as follows:
   1093 A
   1342 C
   1593 A
   2457 T
   2908 G
   3199 A
   3624 G
   4035 T
   7470 G
   9079 G
   as set forth in SEQ. ID. NO: 10.
15. The DNA according to item 9 wherein the coding sequence nucleotides are as follows:
   1093 C
   1342 C
   1593 G
   2457 C
   2908 A
   3199 G
   3624 A
   4035 T
   7470 A
   9079 A
   as set forth in SEQ. ID. NO: 12.
16. The DNA according to item 9 wherein the coding sequence nucleotides are as follows:
   2024 C
   4553 C
   4815 G
   5841 T
   5972 C.
17. A BRCA2 protein having the following amino acids at the following peptide numbers:
   289 asparagine
   372 histidine
   894 valine
   991 asparagine
   1852 valine
   1853 cysteine
   1854 valine
   2951 alanine
   as set forth in SEQ. ID. NO: 5.
18. The BRCA2 protein having the following amino acids at the following peptide numbers:
   289 asparagine
   372 asparagine
   599 serine
   894 valine
   991 asparagine
   2951 alanine.
19. The BRCA2 protein having the following amino acids at the following peptide numbers:
   289 histidine
   372 histidine
   894 valine
   991 asparatic acid
   2951 alanine
   as set forth in SEQ. ID. NO: 9.
20. The BRCA2 protein having the following amino acids at the following peptide numbers:
   289 histidine
   372 asparagine
   894 isoleucine
   991 aspartic acid
   2951 threonine
   as set forth in SEQ. ID. NO: 13.
21. The BRCA2 protein according to items 17-20 having the following amino acids at the following peptide numbers:
   599 serine
   1442 serine
   1915 threonine.
22. A haplotype of BRCA2 coding sequence (BRCA2^{omi 1}) as set forth in SEQ. ID. NO: 4 or a sequence complementary thereto.
23. A BRCA2 protein comprising an amino acid sequence derived from BRCA2^{omi 1} as set forth in SEQ. ID. NO: 5.
24. A haplotype of BRCA2 coding sequence (BRCA2^{omi 2}) as set forth in SEQ. ID. NO: 6 or a sequence complementary thereto.
25. A BRCA2 protein comprising an amino acid sequence derived from BRCA2^{omi 2} as set forth in SEQ. ID. NO: 7.
26. A haplotype of BRCA2 coding sequence (BRCA2^{omi 3}) as set forth in SEQ. ID. NO: 8 or a sequence complementary thereto.
27. A BRCA2 protein comprising an amino acid sequence derived from BRCA2^{omi 3} as set forth in SEQ. ID. NO: 9.
28. A haplotype of BRCA2 coding sequence (BRCA2^{omi 4}) as set forth in SEQ. ID. NO: 10 or a sequence complementary thereto.
29. A BRCA2 protein comprising an amino acid sequence derived from BRCA2^{omi 4} as set forth in SEQ. ID. NO: 11.
30. A haplotype of BRCA2 coding sequence (BRCA2^{omi 5}) as set forth in SEQ. ID. NO: 12 or a sequence complementary thereto.
31. A BRCA2 protein comprising an amino acid sequence derived from BRCA2^{omi 5} as set forth in SEQ. ID. NO: 13.
32. A method of identifying individuals having a BRCA2 gene with a BRCA2 coding sequence not associated with disease, comprising:
   (a) amplifying a DNA or a fragment thereof of an individual's BRCA2 coding sequence;
   (b) sequencing said amplified DNA fragment;
   (c) if necessary, repeating steps (a) and (b) until said individual's BRCA2 coding sequence is sufficiently sequenced to determine whether a mutation is present;
   (d) comparing the sequence of said amplified DNA fragment to a BRCA2^{(omi)} DNA sequence selecting from the group consisting of SEQ. ID. NO: 4, SEQ. ID. NO: 6, SEQ. ID. NO: 8, SEQ. ID. NO: 10, SEQ. ID. NO: 12, and their respective complementary sequences;
   (e) determining the presence of absence of each of the following polymorphic variations in said individual's BRCA2 coding sequence:
      (i) AAT and CAT at position 1093,
      (ii) CAT and AAT at position 1342,
      (iii) TCA and TCG at position 1593,
      (iv) CAT and CAC at position 2457,
      (v) GTA and ATA at position 2908,
      (vi) AAC and GAC at position 3199,
      (vii) AAA and AAG at position 3624,
      (viii) GTT and GTC at position 4035,
      (ix) TCA and TCG at position 7470, and
      (x) GCC and ACC at position 9079; and
   (f) determining any sequence differences between said individual's BRCA2 coding sequences and a BRCA2(^{omi}) DNA sequence selected from the group consisting of SEQ. ID. NO: 4, SEQ. ID. NO: 6, SEQ. ID. NO: 8, SEQ. ID. NO: 10, SEQ. ID. NO: 12, and their respective complementary sequences, wherein the presence of said polymorphic variations and the absence of a variation outside of positions 1093, 1342, 1593, 2457, 2908, 3199, 3624, 4035, 7470, and 9079 is correlated with an absence of increased genetic susceptibility to breast or ovarian cancer resulting from a BRCA2 mutation in the BRCA2 coding sequence.
33. A method of identifying individuals having a BRCA2 gene with a BRCA2 coding sequence not associated with disease, comprising:
   (a) amplifying a DNA or a fragment thereof of an individual's BRCA2 coding sequence;
   (b) sequencing said amplified DNA fragment;
   (c) if necessary, repeating steps (a) and (b) until said individual's BRCA2 coding sequence is sufficiently sequenced to determine whether a mutation is present;
   (d) comparing the sequence of said amplified DNA fragment to a BRCA2^{(omi)} DNA sequence selecting from the group consisting of SEQ. ID. NO: 4, SEQ. ID. NO: 6, SEQ. ID. NO: 8, SEQ. ID. NO: 10, SEQ. ID. NO: 12, and their respective complementary sequences;
   (e) determining the presence of absence of each of the following polymorphic variations in said individual's BRCA2 coding sequence:
      (i) AAT and CAT at position 1093,
      (ii) CAT and AAT at position 1342,
      (iii) TCA and TCG at position 1593,
      (iv) CAT and CAC at position 2457,
      (v) GTA and ATA at position 2908,
      (vi) AAC and GAC at position 3199,
      (vii) AAA and AAG at position 3624,
      (viii) GTT and GTC at position 4035,
      (ix) TCA and TCG at position 7470, and
      (x) GCC and ACC at position 9079; and
   (f) determining any sequence differences between said individual's BRCA2 coding sequences and a BRCA2^{(omi)} DNA sequence selected from the group consisting of SEQ. ID. NO: 4, SEQ. ID. NO: 6, SEQ. ID. NO: 8, SEQ. ID. NO: 10, SEQ. ID. NO: 12, and their respective complementary sequences, wherein the presence of said polymorphic variations and the absence of a variation outside of positions 1093, 1342, 1593, 2457, 2908, 3199, 3624, 4035, 7470, and 9079 is correlated with an absence of increased genetic susceptibility to breast or ovarian cancer resulting from a BRCA2 mutation in the BRCA2 coding sequence; wherein, codon variations occur at the following frequencies, respectively, in a Caucasian population of individuals free of disease:
      (i) at position 1093, AAT and CAT occur at frequencies from about 75-85%, and from about 15-25%, respectively,
      (ii) at position 1342, CAT and AAT occur at frequencies from about 35-45%, and from about 55-65%, respectively,
      (iii) at position 1593, TCA and TCG occur at frequencies from about 85-95%, and from about 5-15%, respectively,
      (iv) at position 2457, CAT and CAC occur at frequencies from about 75-85%, and from about 15-25%, respectively,
      (v) at position 2908, GTA and ATA occur at frequencies from about 85-95%, and from about 5-15%, respectively,
      (vi) at position 3199, AAC and GAC occur at frequencies from about 75-85%, and from about 15-25%, respectively,
      (vii) at position 3624, AAA and AAG occur at frequencies from about 75-85%, and from about 15-25%, respectively,
      (viii) at position 4035, GTT and GTC occur at frequencies from about 85-95%, and from about 5-15%, respectively,
      (ix) at position 7470, TCA and TCG occur at frequencies from about 75-85%, and from about 15-25%, respectively, and
      (x) at position 9079, GCC and ACC occur at frequencies from about 85-95%, and from about 5-15%, respectively.
34. A method of detecting an increased genetic susceptibility to breast and ovarian cancer in an individual resulting from the presence of a mutation in the BRCA2 coding sequence, comprising:
   (a) amplifying a DNA or a fragment thereof of an individual's BRCA2 coding sequence;
   (b) sequencing said amplified DNA fragment;
   (c) if necessary, repeating steps (a) and (b) until said individual's BRCA2 coding sequence is sufficiently sequenced to determine whether a mutation is present;
   (d) comparing the sequence of said amplified DNA fragment to a BRCA2^{(omi)} DNA sequence selected from the group consisting of SEQ. ID. NO: 4, SEQ. ID. NO: 6, SEQ. ID. NO: 8, SEQ. ID. NO: 10, SEQ. ID. NO: 12, and their respective complementary sequences;
   (e) determining any sequence differences between said individual's BRCA2 coding sequences and a BRCA2^{(omi)} DNA sequence selected from the group consisting of SEQ. ID. NO: 4, SEQ. ID. NO: 6, SEQ. ID. NO: 8, SEQ. ID. NO: 10, SEQ. ID. NO: 12, and their respective complementary sequences in order to determine the presence or absence of base changes in said individual's BRCA2 coding sequence wherein a base change which is not any one of the following:
      (i) AAT and CAT at position 1093,
      (ii) CAT and AAT at position 1342,
      (iii) TCA and TCG at position 1593,
      (iv) CAT and CAC at position 2457,
      (v) GTA and ATA at position 2908,
      (vi) AAC and GAC at position 3199,
      (vii) AAA and AAG at position 3624,
      (viii) GTT and GTC at position 4035,
      (ix) TCA and TCG at position 7470, and
      (x) GCC and ACC at position 9079, is correlated with the potential of increased genetic susceptibility to breast or ovarian cancer resulting from a BRCA2 mutation in the BRCA2 coding sequence.
35. A method of detecting an increased genetic susceptibility to breast and ovarian cancer in an individual resulting from the presence of a mutation in the BRCA2 coding sequence, comprising:
   (a) amplifying a DNA or a fragment thereof of an individual's BRCA2 coding sequence;
   (b) sequencing said amplified DNA fragment;
   (c) if necessary, repeating steps (a) and (b) until said individual's BRCA2 coding sequence is sufficiently sequenced to determine whether a mutation is present;
   (d) comparing the sequence of said amplified DNA fragment to a BRCA2^{(omi)} DNA sequence selected from the group consisting of: SEQ. ID. NO: 4, SEQ. ID. NO: 6, SEQ. ID. NO: 8, SEQ. ID. NO: 10, SEQ. ID. NO: 12, and their respective complementary sequences;
   (e) determining any sequence differences between said individual's BRCA2 coding sequences and a BRCA2^{(omi)} DNA sequence selected from the group consisting of: SEQ. ID. NO: 4, SEQ. ID. NO: 6, SEQ. ID. NO: 8, SEQ. ID. NO: 10, SEQ. ID. NO: 12, and their respective complementary sequences in order to determine the presence or absence of base changes in said individual's BRCA2 coding sequence wherein a base change which is not any one of the following:
      (i) AAT and CAT at position 1093,
      (ii) CAT and AAT at position 1342,
      (iii) TCA and TCG at position 1593,
      (iv) CAT and CAC at position 2457,
      (v) GTA and ATA at position 2908,
      (vi) AAC and GAC at position 3199,
      (vii) AAA and AAG at position 3624,
      (viii) GTT and GTC at position 4035,
      (ix) TCA and TCG at position 7470, and
      (x) GCC and ACC at position 9079, is correlated with the potential of increased genetic susceptibility to breast or ovarian cancer resulting from a BRCA2 mutation in the BRCA2 coding sequence, wherein, codon variations occur at the following frequencies, respectively, in a Caucasian population of individuals free of disease:
         (i) at position 1093, AAT and CAT occur at frequencies from about 75-85%, and from about 15-25%, respectively,
         (ii) at position 1342, CAT and AAT occur at frequencies from about 35-45%, and from about 55-65%, respectively,
         (iii) at position 1593, TCA and TCG occur at frequencies from about 85-95%, and from about 5-15%, respectively,
         (iv) at position 2457, CAT and CAC occur at frequencies from about 75-85%, and from about 15-25%, respectively,
         (v) at position 2908, GTA and ATA occur at frequencies from about 85-95%, and from about 5-15%, respectively,
         (vi) at position 3199, AAC and GAC occur at frequencies from about 75-85%, and from about 15-25%,
            respectively,
         (vii) at position 3624, AAA and AAG occur at frequencies from about 75-85%, and from about 15-25%,
            respectively,
         (viii) at position 4035, GTT and GTC occur at frequencies from about 85-95%, and from about 5-15%, respectively,
         (ix) at position 7470, TCA and TCG occur at frequencies from about 75-85%, and from about 15-25%, respectively, and
         (x) at position 9079, GCC and ACC occur at frequencies from about 85-95%, and from about 5-15%, respectively.
36. A method according to any of the items 32-35 wherein the said amplifying is performed by annealing at least one oligonucleotide primer to said DNA fragment and extending the oligonucleotide primer by an agent for polymerization.
37. A method according to item 36 wherein said oligonucleotide primer is directly or indirectly labeled with a radioactive label, a fluorescent label, a bioluminescent label, a chemiluminescent label, a metal chelator, or an enzyme label.
38. A BRCA2 coding sequence according to item 32, wherein the codon pairs occur at the following frequencies:
   (i) at position 1093, AAT and CAT occur at frequencies from about 75-85%, and from about 15-25%, respectively,
   (ii) at position 1342, CAT and AAT occur at frequencies from about 35-45%, and from about 55-65%, respectively,
   (iii) at position 1593, TCA and TCG occur at frequencies from about 85-95%, and from about 5-15%, respectively,
   (iv) at position 2457, CAT and CAC occur at frequencies from about 75-85%, and from about 15-25%, respectively,
   (v) at position 2908, GTA and ATA occur at frequencies from about 85-95%, and from about 5-15%, respectively,
   (vi) at position 3199, AAC and GAC occur at frequencies from about 75-85%, and from about 15-25%,
      respectively,
   (vii) at position 3624, AAA and AAG occur at frequencies from about 75-85%, and from about 15-25%, respectively,
   (viii) at position 4035, GTT and GTC occur at frequencies from about 85-95%, and from about 5-15%, respectively,
   (ix) at position 7470, TCA and TCG occur at frequencies from about 75-85%, and from about 15-25%, respectively, and
   (x) at position 9079, GCC and ACC occur at frequencies from about 85-95%, and from about 5-15%, respectively.
39. An oligonucleotide primer capable of hybridizing to a sample of BRCA2 gene, or its respective complementary sequences selected from the group consisting of SEQ. ID. NO: 14, 19, 22, 23, 25, 26, 29-76, 83, 85-88, 90, 91, 97, 98, 101, and 104-107.
40. A chip array having "n" elements for performing allele specific sequence-based techniques comprising a solid phase chip and oligonucleotides having "n" different nucleotide sequences,
   wherein "n" is an interger greater than or equal to ten,
   wherein said oligonucleotides are bound to said solid phase chip in a manner which permits said oligonucleotides to effectively hybridize to complementary oligonucleotides or polynucleotides,
   wherein oligonucleotides having different nucleotide sequence are bound to said solid phase chip at different locations so that a particular location on said solid phase chip exclusively binds oligonucleotides having a specific nucleotide sequence, and
   wherein at least ten oligonucleotides are capable of specifically hybridizing to the BRCA2 DNA having the sequence as set forth in SEQ. ID. NO: 4, SEQ. ID. NO: 6, SEQ. ID. NO: 8, SEQ. ID. NO: 10, SEQ. ID. NO: 12 or their respective complementary sequences, at least one oligonucleotide being capable of specifically hybridizing at each of the nucleotide positions 1093, 1342, 1593, 2457, 2908, 3199, 3624, 4035, 7470, 9079, or complementary thereto.
41. A method of performing gene therapy on a patient, comprising:
   a) contacting cancer cells *in vivo* with an effective amount of a vector comprising DNA containing at least a portion of BRCA2 sequence selected from the group consisting of SEQ. ID. NO: 4, SEQ. ID. NO: 6, SEQ. ID. NO: 8, SEQ. ID. NO: 10, SEQ. ID. NO: 12, or their respective complementary sequences
   b) allowing the vector to enter the cancer cells, and
   c) measuring a reduction in tumor growth.
42. The method according to item 41 wherein said cancer cells have a mutation in the BRCA2 gene.
43. The method according to item 41 wherein said patient has a mutation in the BRCA2 gene of non-cancer cells.
44. A method of performing gene therapy on a patient or a sample, comprising:
   a) contacting cells *in vivo* or *in vitro* with an effective amount of a vector comprising DNA containing at least a portion of BRCA2 sequence selected from the group consisting of SEQ. ID. NO: 4, SEQ. ID. NO: 6, SEQ. ID. NO: 8, SEQ. ID. NO: 10, SEQ. ID. NO: 12, or their respective complementary sequences, and
   b) allowing the vector to enter the cells,
   wherein said patient has a reduced susceptibility for developing a cancer associated with a mutation in the BRCA2 gene.
45. A method according to item 44 wherein said cells include healthy breast, ovarian or pancreatic tissues.
46. A method according to item 44 wherein a patient has an inherited mutation in the BRCA2 gene.
47. A method of treating a patient suspected of having a tumor, comprising:
   a) administering to a patient an effective amount of BRCA2 tumor growth inhibitor having an amino acid sequence selected from the group consisting of SEQ. ID. NO: 5, SEQ. ID. NO: 7, SEQ. ID. NO: 9, SEQ. ID. NO: 1, SEQ. ID. NO: 13, any fragments thereto, and any functional equivalent thereof;
   b) allowing the patient's cells to take up the protein, and
   c) measuring a reduction in tumor growth.
48. The method according to item 47 wherein said tumor is a breast cancer, an ovarian cancer or a pancreatic cancer.
49. The method according to item 47 wherein said patient has an inherited mutation in the BRCA2 gene.
50. A method of preventing the formation or growth of a tumor, comprising:
   a) adminstering to a patient an effective amount of BRCA2 tumor growth inhibiting protein having an amino acid sequence selected from the group consisting of SEQ. ID. NO: 5, SEQ. ID. NO: 7, SEQ. ID. NO: 9, SEQ. ID. NO: 11, SEQ. ID. NO: 13, any fragments thereto, and any functional equivalent thereof; and
   b) allowing the patient cells to take up the protein.
51. The method according to item 31 wherein the protein is administered parenternally, by buccal adsorption or inhalation.
52. A cloning vector comprising:
   (a) a DNA sequence as set forth in SEQ. ID. NO: 4, SEQ. ID. NO: 6, SEQ. ID. NO: 8, SEQ. ID. NO: 10, SEQ. ID. NO: 12, or any fragments thereof; and
   (b) one or more suitable regulatory sequences to induce replication and/or integration in a host cell.
53. An expression vector comprising a DNA sequence as set forth in SEQ. ID. NO: 4, SEQ. ID. NO: 6, SEQ. ID. NO: 8, SEQ. ID. NO: 10, SEQ. ID. NO: 12, or any fragments thereof operatively linked to one or more promoter sequences capable of directing expression of said sequence in a host cell.
54. A host cell transformed with the vector according to item 52 or 53.
55. A BRCA2 polypeptide which is selected from the group consisting of:
   (a) a fragment of BRCA2 protein sequence as set forth in SEQ. ID. NO: 5, SEQ. ID. NO: 7, SEQ. ID. NO: 9, SEQ. ID. NO: 11, or SEQ. ID. NO:13;
   (b) an amino acid sequence which is substantially homologous to the BRCA2 protein sequence as set forth in SEQ. ID. NO: 5, SEQ. ID. NO: 7, SEQ. ID. NO: 9, SEQ. ID. NO: 11, or SEQ. ID. NO: 13;
   (c) a molecule which has similar function to the BRCA2 protein; and
   (d) a fusion protein of (a), (b), or (c).
56. An anti-BRCA2 antibody wherein a molecule according to items 17-21, 23, 25, 27, 29, 31, or 55 is used as an immunogen.
57. A diagnostic reagent comprising a molecule selected from the group consisting of:
   (a) a DNA sequence as set forth in SEQ. ID. NO: 4, SEQ. ID. NO: 6, SEQ. ID. NO: 8, SEQ. ID. NO: 10, SEQ. ID. NO: 12, or their complementary sequences;
   (b) a nucleic acid fragment of (a) comprising at least 10 nucleotide in length;
   (c) a sequence which hybridizes to (a) or (b);
   (d) a polypeptide according to items 17-21, 23, 25, 27, 29, 31, or 55; and
   (e) an antibody which specifically binds to the polypeptide of (d).
58. A pharmaceutical composition comprising a molecule according to any one of the items 17-21, 23, 25, 27, 29, 31, 55 in a pharmaceutically acceptable carrier.
59. A pharmaceutical composition comprising a molecule according item 56 in a pharmaceutically acceptable carrier.
60. A pharmaceutical composition comprising a molecule according to item 57 in a pharmaceutically acceptable carrier.

## Claims

1. A nucleic acid comprising:
(a) at least 18 contiguous nucleotides of the following nucleotide sequence: 
tttttgctaagtatttattctttgatagATTTAATTACAAGTCTTCAGAATGC
CAGAGATATACAGGATATGCGAATTAAGAAGAAACAAAGG
CAACGCGTCTTTCCACAGCCAGGCAGTCTGTATCTTGCAAA
AACATCCACTCTGCCTCGAATCTCTCTGAAAGCAGCAGTAG
GAGGCCAAGTTCCCTCTGCGTGTTCTCATAAACAGgtatgtgt
 and comprising the 3' exon/intron junction of exon 15 as shown in said nucleotide sequence;
(b) at least 18 contiguous nucleotides of the following nucleotide sequence: 
tttttctttttttgtgtgtgtttattttgtgtagCTGTATACGTATGGCGTTTCTAA
ACATTGCATAAAAATTAACAGCAAAAATGCAGAGTCTTTTC
AGTTTCACACTGAAGATTATTTTGGTAAGGAAAGTTTATGG
ACTGGAAAAGGAATACAGTTGGCTGATGGTGGATGGCTCA
TACCCTCCAATGATGGAAAGGCTGGAAAAGAAGAATTTTA
TAGgtactctatgcaaaaagattgtgtgttaacttttatg
 and comprising the 5' exon/intron junction of exon 16 as shown in said nucleotide sequence;
(c) the nucleotide sequence 5'-GTGTTCTCATAAACAG-3' of exon 15 of the BRCA2 gene and the adjacent 3' splice site according to Figure 2D;
(d) the nucleotide sequence 5'-CTGTATACGTATGGCGTTTC-3' of exon 16 of the BRCA2 gene and the adjacent 5' splice site according to Figure 2D;
(e) the nucleotide sequence 5'-GTGTTCTCATAAACAGgtatgtgt-3'; or
(f) the nucleotide sequence
5'-tttttcttttttgtgtgtgtttattttgtgtagCTGTATACGTATGGCGTTTC-3'.

2. A method of synthesizing a nucleic acid comprising exon 15 of a BRCA2 gene, wherein the sequence of the last 20 nucleotides of exon 15 is 5'-CTGCGTGTTCTCATAAACAG-3', the method comprising:
obtaining a sample containing genomic DNA from a human subject; and
synthesizing a nucleic acid comprising exon 15.

3. The method of claim 2, wherein said synthesizing step comprises amplification using (1) a first nucleic acid comprising at least 18 contiguous nucleotides of the 5' exon/intron junction of the exon 15 sequence shown in claim 1(a) and (2) a second nucleic acid being complementary to the nucleic acid as defined in claim 1(a).

4. The method of claim 2 or 3, wherein said synthesizing step comprises synthesizing a nucleic acid comprising the last 20 nucleotides of SEQ ID NO:2.

5. The method of claim 3 or 4, wherein said second nucleic acid comprises the last 20 nucleotides of SEQ ID NO:2.

6. A method of synthesizing a nucleic acid comprising the 3' exon/intron junction of exon 15 of the BRCA2 gene comprising:
synthesizing a nucleic acid comprising the last 20 nucleotides of SEQ ID NO:2.

7. A method of synthesizing a nucleic acid comprising exon 16 of a BRCA2 gene, wherein the sequence of the first 20 nucleotides of exon 16 is 5'-CTGTATACGTATGGCGTTTC-3', the method comprising:
obtaining a sample containing genomic DNA from a human subject; and
synthesizing a nucleic acid comprising exon 16.

8. The method of claim 7, wherein said synthesizing step comprises amplification using (1) a first nucleic acid as defined in claim 1(b) and (2) a second nucleic acid comprising a nucleotide sequence complementary to at least 18 contiguous nucleotides of the 3' exon/intron junction of the exon 16 sequence shown in claim 1(b).

9. The method of claim 7 or 8, wherein said synthesizing step comprises synthesizing a nucleic acid comprising the first 20 nucleotides of SEQ ID NO:3.

10. The method of claim 8 or 9, wherein said first nucleic acid comprises the first 20 nucleotides of SEQ ID NO:3.

11. A method of synthesizing a nucleic acid comprising the 5' exon/intron junction of exon 16 of the BRCA2 gene comprising:
synthesizing a nucleic acid comprising the first 20 consecutive nucleotides of SEQ ID NO:3.

12. The method of any one of claims 2 to 11 further comprising sequencing the product of said synthesizing step.

13. The method of any one of claims 2 to 12, wherein said nucleic acid is synthesized by PCR amplification of said genomic DNA.


SEQUENCE LISTING

(1) GENERAL INFORMATION

(i) APPLICANT: Murphy, Patricia
White, Marga
Rabin, Mark
Olson, Sheri
Yoshikawa, Matthew
Jackson, Geoffrey
Eskanderi, Tara
Schryer, Brenda
Park, Michael

(ii) TITLE OF THE INVENTION: NOVEL CODING SEQUENCE HAPLOTYPES
OF THE HUMAN BRCA2 GENE

(iii) NUMBER OF SEQUENCES: 111

(iv) CORRESPONDENCE ADDRESS:
(A) ADDRESSEE: Howrey & Simon
(B) STREET: 1299 Pennsylvania Avenue N.W.
(C) CITY: Washington
(D) STATE: DC
(E) COUNTRY: USA
(F) ZIP: 20004

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: Halluin, Albert P
(B) REGISTRATION NUMBER: 25,227
(C) REFERENCE/DOCKET NUMBER: 5371.31.US02

(ix) TELECOMMUNICATION INFORMATION:
(A) TELEPHONE: 650-463-8109
(B) TELEFAX: 650-463-8400
(C) TELEX:

(2) INFORMATION FOR SEQ ID NO:1:

(i) SEQUENCE CHARACTERISTICS:
(A) LENGTH: 50 base pairs

(B) TYPE: nucleic acid
(C) STRANDEDNESS: double
(D) TOPOLOGY: linear

(ii) MOLECULE TYPE: Genomic DNA
(ix) FEATURE:

(A) NAME/KEY: exon
(B) LOCATION: 1...50
(D) OTHER INFORMATION: Exon 5

(xi) SEQUENCE DESCRIPTION: SEQ ID NO:1:

TCCTGTTGTT CTACAATGTA CACATGTAAC ACCACAAAGA GATAAGTCAG 50

(2) INFORMATION FOR SEQ ID NO:2:

(i) SEQUENCE CHARACTERISTICS:
(A) LENGTH: 182 base pairs
(B) TYPE: nucleic acid
(C) STRANDEDNESS: double
(D) TOPOLOGY: linear

(ii) MOLECULE TYPE: Genomic DNA
(ix) FEATURE:

(A) NAME/KEY: exon
(B) LOCATION: 1...182
(D) OTHER INFORMATION: Exon 15

(xi) SEQUENCE DESCRIPTION: SEQ ID NO:2:

ATTTAATTAC AAGTCTTCAG AATGCCAGAG ATATACAGGA TATGCGAATT AAGAAGAAAC 60
AAAGGCAACG CGTCTTTCCA CAGCCAGGCA GTCTGTATCT TGCAAAAACA TCCACTCTGC 120
CTCGAATCTC TCTGAAAGCA GCAGTAGGAG GCCAAGTTCC CTCTGCGTGT TCTCATAAAC 180
AG 182

(2) INFORMATION FOR SEQ ID NO:3:

(i) SEQUENCE CHARACTERISTICS:
(A) LENGTH: 188 base pairs
(B) TYPE: nucleic acid
(C) STRANDEDNESS: double
(D) TOPOLOGY: linear

(ii) MOLECULE TYPE: Genomic DNA
(ix) FEATURE:

(A) NAME/KEY: exon
(B) LOCATION: 1...188
(D) OTHER INFORMATION: Exon 16

(xi) SEQUENCE DESCRIPTION: SEQ ID NO:3:

CTGTATACGT ATGGCGTTTC TAAACATTGC ATAAAAATTA ACAGCAAAAA TGCAGAGTCT 60
TTTCAGTTTC ACACTGAAGA TTATTTTGGT AAGGAAAGTT TATGGACTGG AAAAGGAATA 120
CAGTTGGCTG ATGGTGGATG GCTCATACCC TCCAATGATG GAAAGGCTGG AAAAGAAGAA 180
TTTTATAG 188

(2) INFORMATION FOR SEQ ID NO:4:

(i) SEQUENCE CHARACTERISTICS:
(A) LENGTH: 10485 base pairs
(B) TYPE: nucleic acid
(C) STRANDEDNESS: single
(D) TOPOLOGY: linear

(ii) MOLECULE TYPE: cDNA
(ix) FEATURE:

(A) NAME/KEY: Coding Sequence
(B) LOCATION: 229...10482
(D) OTHER INFORMATION: BRCA2 (OMI1)

(xi) SEQUENCE DESCRIPTION: SEQ ID NO:4:
GGTGGCGCGA GCTTCTGAAA CTAGGCGGCA GAGGCGGAGC CGCTGTGGCA CTGCTGCGCC 60
TCTGCTGCGC CTCGGGTGTC TTTTGCGGCG GTGGGTCGCC GCCGGGAGAA GCGTGAGGGG 120
ACAGATTTGT GACCGGCGCG GTTTTTGTCA GCTTACTCCG GCCAAAAAAG AACTGCACCT 180
CTGGAGCGGA CTTATTTACC AAGCATTGGA GGAATATCGT AGGTAAAA ATG CCT ATT 237
GGA TCC AAA GAG AGG CCA ACA TTT TTT GAA ATT TTT AAG ACA CGC TGC 285
AAC AAA GCA GAT TTA GGA CCA ATA AGT CTT AAT TGG TTT GAA GAA CTT 333
TCT TCA GAA GCT CCA CCC TAT AAT TCT GAA CCT GCA GAA GAA TCT GAA 381
CAT AAA AAC AAC AAT TAC GAA CCA AAC CTA TTT AAA ACT CCA CAA AGG 429
AAA CCA TCT TAT AAT CAG CTG GCT TCA ACT CCA ATA ATA TTC AAA GAG 477
CAA GGG CTG ACT CTG CCG CTG TAC CAA TCT CCT GTA AAA GAA TTA GAT 525
AAA TTC AAA TTA GAC TTA GGA AGG AAT GTT CCC AAT AGT AGA CAT AAA 573
AGT CTT CGC ACA GTG AAA ACT AAA ATG GAT CAA GCA GAT GAT GTT TCC 621
TGT CCA CTT CTA AAT TCT TGT CTT AGT GAA AGT CCT GTT GTT CTA CAA 669
TGT ACA CAT GTA ACA CCA CAA AGA GAT AAG TCA GTG GTA TGT GGG AGT 717
TTG TTT CAT ACA CCA AAG TTT GTG AAG GGT CGT CAG ACA CCA AAA CAT 765
ATT TCT GAA AGT CTA GGA GCT GAG GTG GAT CCT GAT ATG TCT TGG TCA 813
AGT TCT TTA GCT ACA CCA CCC ACC CTT AGT TCT ACT GTG CTC ATA GTC 861
AGA AAT GAA GAA GCA TCT GAA ACT GTA TTT CCT CAT GAT ACT ACT GCT 909
AAT GTG AAA AGC TAT TTT TCC AAT CAT GAT GAA AGT CTG AAG AAA AAT 957
GAT AGA TTT ATC GCT TCT GTG ACA GAC AGT GAA AAC ACA AAT CAA AGA 1005
GAA GCT GCA AGT CAT GGA TTT GGA AAA ACA TCA GGG AAT TCA TTT AAA 1053
GTA AAT AGC TGC AAA GAC CAC ATT GGA AAG TCA ATG CCA AAT GTC CTA 1101
GAA GAT GAA GTA TAT GAA ACA GTT GTA GAT ACC TCT GAA GAA GAT AGT 1149
TTT TCA TTA TGT TTT TCT AAA TGT AGA ACA AAA AAT CTA CAA AAA GTA 1197
AGA ACT AGC AAG ACT AGG AAA AAA ATT TTC CAT GAA GCA AAC GCT GAT 1245
GAA TGT GAA AAA TCT AAA AAC CAA GTG AAA GAA AAA TAC TCA TTT GTA 1293
TCT GAA GTG GAA CCA AAT GAT ACT GAT CCA TTA GAT TCA AAT GTA GCA 1341
CAT CAG AAG CCC TTT GAG AGT GGA AGT GAC AAA ATC TCC AAG GAA GTT 1389
GTA CCG TCT TTG GCC TGT GAA TGG TCT CAA CTA ACC CTT TCA GGT CTA 1437
AAT GGA GCC CAG ATG GAG AAA ATA CCC CTA TTG CAT ATT TCT TCA TGT     1485
GAC CAA AAT ATT TCA GAA AAA GAC CTA TTA GAC ACA GAG AAC AAA AGA     1533
AAG AAA GAT TTT CTT ACT TCA GAG AAT TCT TTG CCA CGT ATT TCT AGC     1581
CTA CCA AAA TCA GAG AAG CCA TTA AAT GAG GAA ACA GTG GTA AAT AAG     1629
AGA GAT GAA GAG CAG CAT CTT GAA TCT CAT ACA GAC TGC ATT CTT GCA     1677
GTA AAG CAG GCA ATA TCT GGA ACT TCT CCA GTG GCT TCT TCA TTT CAG     1725
GGT ATC AAA AAG TCT ATA TTC AGA ATA AGA GAA TCA CCT AAA GAG ACT     1773
TTC AAT GCA AGT TTT TCA GGT CAT ATG ACT GAT CCA AAC TTT AAA AAA     1821
GAA ACT GAA GCC TCT GAA AGT GGA CTG GAA ATA CAT ACT GTT TGC TCA     1869
CAG AAG GAG GAC TCC TTA TGT CCA AAT TTA ATT GAT AAT GGA AGC TGG     1917
CCA GCC ACC ACC ACA CAG AAT TCT GTA GCT TTG AAG AAT GCA GGT TTA     1965
ATA TCC ACT TTG AAA AAG AAA ACA AAT AAG TTT ATT TAT GCT ATA CAT     2013
GAT GAA ACA TCT TAT AAA GGA AAA AAA ATA CCG AAA GAC CAA AAA TCA     2061
GAA CTA ATT AAC TGT TCA GCC CAG TTT GAA GCA AAT GCT TTT GAA GCA     2109
CCA CTT ACA TTT GCA AAT GCT GAT TCA GGT TTA TTG CAT TCT TCT GTG     2157
AAA AGA AGC TGT TCA CAG AAT GAT TCT GAA GAA CCA ACT TTG TCC TTA     2205
ACT AGC TCT TTT GGG ACA ATT CTG AGG AAA TGT TCT AGA AAT GAA ACA 2253
TGT TCT AAT AAT ACA GTA ATC TCT CAG GAT CTT GAT TAT AAA GAA GCA 2301
AAA TGT AAT AAG GAA AAA CTA CAG TTA TTT ATT ACC CCA GAA GCT GAT 2349
TCT CTG TCA TGC CTG CAG GAA GGA CAG TGT GAA AAT GAT CCA AAA AGC 2397
AAA AAA GTT TCA GAT ATA AAA GAA GAG GTC TTG GCT GCA GCA TGT CAC 2445
CCA GTA CAA CAT TCA AAA GTG GAA TAC AGT GAT ACT GAC TTT CAA TCC 2493
CAG AAA AGT CTT TTA TAT GAT CAT GAA AAT GCC AGC ACT CTT ATT TTA 2541
ACT CCT ACT TCC AAG GAT GTT CTG TCA AAC CTA GTC ATG ATT TCT AGA 2589
GGC AAA GAA TCA TAC AAA ATG TCA GAC AAG CTC AAA GGT AAC AAT TAT 2637
GAA TCT GAT GTT GAA TTA ACC AAA AAT ATT CCC ATG GAA AAG AAT CAA 2685
GAT GTA TGT GCT TTA AAT GAA AAT TAT AAA AAC GTT GAG CTG TTG CCA 2733
CCT GAA AAA TAC ATG AGA GTA GCA TCA CCT TCA AGA AAG GTA CAA TTC 2781
AAC CAA AAC ACA AAT CTA AGA GTA ATC CAA AAA AAT CAA GAA GAA ACT 2829
ACT TCA ATT TCA AAA ATA ACT GTC AAT CCA GAC TCT GAA GAA CTT TTC 2877
TCA GAC AAT GAG AAT AAT TTT GTC TTC CAA GTA GCT AAT GAA AGG AAT 2925
AAT CTT GCT TTA GGA AAT ACT AAG GAA CTT CAT GAA ACA GAC TTG ACT     2973
TGT GTA AAC GAA CCC ATT TTC AAG AAC TCT ACC ATG GTT TTA TAT GGA     3021
GAC ACA GGT GAT AAA CAA GCA ACC CAA GTG TCA ATT AAA AAA GAT TTG     3069
GTT TAT GTT CTT GCA GAG GAG AAC AAA AAT AGT GTA AAG CAG CAT ATA     3117
AAA ATG ACT CTA GGT CAA GAT TTA AAA TCG GAC ATC TCC TTG AAT ATA     3165
GAT AAA ATA CCA GAA AAA AAT AAT GAT TAC ATG AAC AAA TGG GCA GGA     3213
CTC TTA GGT CCA ATT TCA AAT CAC AGT TTT GGA GGT AGC TTC AGA ACA     3261
GCT TCA AAT AAG GAA ATC AAG CTC TCT GAA CAT AAC ATT AAG AAG AGC     3309
AAA ATG TTC TTC AAA GAT ATT GAA GAA CAA TAT CCT ACT AGT TTA GCT     3357
TGT GTT GAA ATT GTA AAT ACC TTG GCA TTA GAT AAT CAA AAG AAA CTG     3405
AGC AAG CCT CAG TCA ATT AAT ACT GTA TCT GCA CAT TTA CAG AGT AGT     3453
GTA GTT GTT TCT GAT TGT AAA AAT AGT CAT ATA ACC CCT CAG ATG TTA     3501
TTT TCC AAG CAG GAT TTT AAT TCA AAC CAT AAT TTA ACA CCT AGC CAA     3549
AAG GCA GAA ATT ACA GAA CTT TCT ACT ATA TTA GAA GAA TCA GGA AGT     3597
CAG TTT GAA TTT ACT CAG TTT AGA AAA CCA AGC TAC ATA TTG CAG AAG     3645
AGT ACA TTT GAA GTG CCT GAA AAC CAG ATG ACT ATC TTA AAG ACC ACT 3693
TCT GAG GAA TGC AGA GAT GCT GAT CTT CAT GTC ATA ATG AAT GCC CCA 3741
TCG ATT GGT CAG GTA GAC AGC AGC AAG CAA TTT GAA GGT ACA GTT GAA 3789
ATT AAA CGG AAG TTT GCT GGC CTG TTG AAA AAT GAC TGT AAC AAA AGT 3837
GCT TCT GGT TAT TTA ACA GAT GAA AAT GAA GTG GGG TTT AGG GGC TTT 3885
TAT TCT GCT CAT GGC ACA AAA CTG AAT GTT TCT ACT GAA GCT CTG CAA 3933
AAA GCT GTG AAA CTG TTT AGT GAT ATT GAG AAT ATT AGT GAG GAA ACT 3981
TCT GCA GAG GTA CAT CCA ATA AGT TTA TCT TCA AGT AAA TGT CAT GAT 4029
TCT GTT GTT TCA ATG TTT AAG ATA GAA AAT CAT AAT GAT AAA ACT GTA 4077
AGT GAA AAA AAT AAT AAA TGC CAA CTG ATA TTA CAA AAT AAT ATT GAA 4125
ATG ACT ACT GGC ACT TTT GTT GAA GAA ATT ACT GAA AAT TAC AAG AGA 4173
AAT ACT GAA AAT GAA GAT AAC AAA TAT ACT GCT GCC AGT AGA AAT TCT 4221
CAT AAC TTA GAA TTT GAT GGC AGT GAT TCA AGT AAA AAT GAT ACT GTT 4269
TGT ATT CAT AAA GAT GAA ACG GAC TTG CTA TTT ACT GAT CAG CAC AAC 4317
ATA TGT CTT AAA TTA TCT GGC CAG TTT ATG AAG GAG GGA AAC ACT CAG 4365
ATT AAA GAA GAT TTG TCA GAT TTA ACT TTT TTG GAA GTT GCG AAA GCT 4413
CAA GAA GCA TGT CAT GGT AAT ACT TCA AAT AAA GAA CAG TTA ACT GCT 4461
ACT AAA ACG GAG CAA AAT ATA AAA GAT TTT GAG ACT TCT GAT ACA TTT 4509
TTT CAG ACT GCA AGT GGG AAA AAT ATT AGT GTC GCC AAA GAG TCA TTT 4557
AAT AAA ATT GTA AAT TTC TTT GAT CAG AAA CCA GAA GAA TTG CAT AAC 4605
TTT TCC TTA AAT TCT GAA TTA CAT TCT GAC ATA AGA AAG AAC AAA ATG 4653
GAC ATT CTA AGT TAT GAG GAA ACA GAC ATA GTT AAA CAC AAA ATA CTG 4701
AAA GAA AGT GTC CCA GTT GGT ACT GGA AAT CAA CTA GTG ACC TTC CAG 4749
GGA CAA CCC GAA CGT GAT GAA AAG ATC AAA GAA CCT ACT CTG TTG GGT 4797
TTT CAT ACA GCT AGC GGG AAA AAA GTT AAA ATT GCA AAG GAA TCT TTG 4845
GAC AAA GTG AAA AAC CTT TTT GAT GAA AAA GAG CAA GGT ACT AGT GAA 4893
ATC ACC AGT TTT AGC CAT CAA TGG GCA AAG ACC CTA AAG TAC AGA GAG 4941
GCC TGT AAA GAC CTT GAA TTA GCA TGT GAG ACC ATT GAG ATC ACA GCT 4989
GCC CCA AAG TGT AAA GAA ATG CAG AAT TCT CTC AAT AAT GAT AAA AAC 5037
CTT GTT TCT ATT GAG ACT GTG GTG CCA CCT AAG CTC TTA AGT GAT AAT 5085
TTA TGT AGA CAA ACT GAA AAT CTC AAA ACA TCA AAA AGT ATC TTT TTG 5133
AAA GTT AAA GTA CAT GAA AAT GTA GAA AAA GAA ACA GCA AAA AGT CCT     5181
GCA ACT TGT TAC ACA AAT CAG TCC CCT TAT TCA GTC ATT GAA AAT TCA     5229
GCC TTA GCT TTT TAC ACA AGT TGT AGT AGA AAA ACT TCT GTG AGT CAG     5277
ACT TCA TTA CTT GAA GCA AAA AAA TGG CTT AGA GAA GGA ATA TTT GAT     5325
GGT CAA CCA GAA AGA ATA AAT ACT GCA GAT TAT GTA GGA AAT TAT TTG     5373
TAT GAA AAT AAT TCA AAC AGT ACT ATA GCT GAA AAT GAC AAA AAT CAT     5421
CTC TCC GAA AAA CAA GAT ACT TAT TTA AGT AAC AGT AGC ATG TCT AAC     5469
AGC TAT TCC TAC CAT TCT GAT GAG GTA TAT AAT GAT TCA GGA TAT CTC     5517
TCA AAA AAT AAA CTT GAT TCT GGT ATT GAG CCA GTA TTG AAG AAT GTT     5565
GAA GAT CAA AAA AAC ACT AGT TTT TCC AAA GTA ATA TCC AAT GTA AAA     5613
GAT GCA AAT GCA TAC CCA CAA ACT GTA AAT GAA GAT ATT TGC GTT GAG     5661
GAA CTT GTG ACT AGC TCT TCA CCC TGC AAA AAT AAA AAT GCA GCC ATT     5709
AAA TTG TCC ATA TCT AAT AGT AAT AAT TTT GAG GTA GGG CCA CCT GCA     5757
TTT AGG ATA GCC AGT GGT AAA ATC GTT TGT GTT TCA CAT GAA ACA ATT     5805
AAA AAA GTG AAA GAC ATA TTT ACA GAC AGT TTC AGT AAA GTA ATT AAG     5853
GAA AAC AAC GAG AAT AAA TCA AAA ATT TGC CAA ACG AAA ATT ATG GCA     5901
GGT TGT TAC GAG GCA TTG GAT GAT TCA GAG GAT ATT CTT CAT AAC TCT     5949
CTA GAT AAT GAT GAA TGT AGC ACG CAT TCA CAT AAG GTT TTT GCT GAC     5997
ATT CAG AGT GAA GAA ATT TTA CAA CAT AAC CAA AAT ATG TCT GGA TTG     6045
GAG AAA GTT TCT AAA ATA TCA CCT TGT GAT GTT AGT TTG GAA ACT TCA     6093
GAT ATA TGT AAA TGT AGT ATA GGG AAG CTT CAT AAG TCA GTC TCA TCT     6141
GCA AAT ACT TGT GGG ATT TTT AGC ACA GCA AGT GGA AAA TCT GTC CAG     6189
GTA TCA GAT GCT TCA TTA CAA AAC GCA AGA CAA GTG TTT TCT GAA ATA     6237
GAA GAT AGT ACC AAG CAA GTC TTT TCC AAA GTA TTG TTT AAA AGT AAC     6285
GAA CAT TCA GAC CAG CTC ACA AGA GAA GAA AAT ACT GCT ATA CGT ACT     6333
CCA GAA CAT TTA ATA TCC CAA AAA GGC TTT TCA TAT AAT GTG GTA AAT     6381
TCA TCT GCT TTC TCT GGA TTT AGT ACA GCA AGT GGA AAG CAA GTT TCC     6429
ATT TTA GAA AGT TCC TTA CAC AAA GTT AAG GGA GTG TTA GAG GAA TTT     6477
GAT TTA ATC AGA ACT GAG CAT AGT CTT CAC TAT TCA CCT ACG TCT AGA     6525
CAA AAT GTA TCA AAA ATA CTT CCT CGT GTT GAT AAG AGA AAC CCA GAG     6573
CAC TGT GTA AAC TCA GAA ATG GAA AAA ACC TGC AGT AAA GAA TTT AAA 6621
TTA TCA AAT AAC TTA AAT GTT GAA GGT GGT TCT TCA GAA AAT AAT CAC 6669
TCT ATT AAA GTT TCT CCA TAT CTC TCT CAA TTT CAA CAA GAC AAA CAA 6717
CAG TTG GTA TTA GGA ACC AAA GTC TCA CTT GTT GAG AAC ATT CAT GTT 6765
TTG GGA AAA GAA CAG GCT TCA CCT AAA AAC GTA AAA ATG GAA ATT GGT 6813
AAA ACT GAA ACT TTT TCT GAT GTT CCT GTG AAA ACA AAT ATA GAA GTT 6861
TGT TCT ACT TAC TCC AAA GAT TCA GAA AAC TAC TTT GAA ACA GAA GCA 6909
GTA GAA ATT GCT AAA GCT TTT ATG GAA GAT GAT GAA CTG ACA GAT TCT 6957
AAA CTG CCA AGT CAT GCC ACA CAT TCT CTT TTT ACA TGT CCC GAA AAT 7005
GAG GAA ATG GTT TTG TCA AAT TCA AGA ATT GGA AAA AGA AGA GGA GAG 7053
CCC CTT ATC TTA GTG GGA GAA CCC TCA ATC AAA AGA AAC TTA TTA AAT 7101
GAA TTT GAC AGG ATA ATA GAA AAT CAA GAA AAA TCC TTA AAG GCT TCA 7149
AAA AGC ACT CCA GAT GGC ACA ATA AAA GAT CGA AGA TTG TTT ATG CAT 7197
CAT GTT TCT TTA GAG CCG ATT ACC TGT GTA CCC TTT CGC ACA ACT AAG 7245
GAA CGT CAA GAG ATA CAG AAT CCA AAT TTT ACC GCA CCT GGT CAA GAA 7293
TTT CTG TCT AAA TCT CAT TTG TAT GAA CAT CTG ACT TTG GAA AAA TCT     7341
TCA AGC AAT TTA GCA GTT TCA GGA CAT CCA TTT TAT CAA GTT TCT GCT     7389
ACA AGA AAT GAA AAA ATG AGA CAC TTG ATT ACT ACA GGC AGA CCA ACC     7437
AAA GTC TTT GTT CCA CCT TTT AAA ACT AAA TCA CAT TTT CAC AGA GTT     7485
GAA CAG TGT GTT AGG AAT ATT AAC TTG GAG GAA AAC AGA CAA AAG CAA     7533
AAC ATT GAT GGA CAT GGC TCT GAT GAT AGT AAA AAT AAG ATT AAT GAC     7581
AAT GAG ATT CAT CAG TTT AAC AAA AAC AAC TCC AAT CAA GCA GCA GCT     7629
GTA ACT TTC ACA AAG TGT GAA GAA GAA CCT TTA GAT TTA ATT ACA AGT     7677
CTT CAG AAT GCC AGA GAT ATA CAG GAT ATG CGA ATT AAG AAG AAA CAA     7725
AGG CAA CGC GTC TTT CCA CAG CCA GGC AGT CTG TAT CTT GCA AAA ACA     7773
TCC ACT CTG CCT CGA ATC TCT CTG AAA GCA GCA GTA GGA GGC CAA GTT     7821
CCC TCT GCG TGT TCT CAT AAA CAG CTG TAT ACG TAT GGC GTT TCT AAA     7869
CAT TGC ATA AAA ATT AAC AGC AAA AAT GCA GAG TCT TTT CAG TTT CAC     7917
ACT GAA GAT TAT TTT GGT AAG GAA AGT TTA TGG ACT GGA AAA GGA ATA     7965
CAG TTG GCT GAT GGT GGA TGG CTC ATA CCC TCC AAT GAT GGA AAG GCT     8013
GGA AAA GAA GAA TTT TAT AGG GCT CTG TGT GAC ACT CCA GGT GTG GAT     8061
CCA AAG CTT ATT TCT AGA ATT TGG GTT TAT AAT CAC TAT AGA TGG ATC 8109
ATA TGG AAA CTG GCA GCT ATG GAA TGT GCC TTT CCT AAG GAA TTT GCT 8157
AAT AGA TGC CTA AGC CCA GAA AGG GTG CTT CTT CAA CTA AAA TAC AGA 8205
TAT GAT ACG GAA ATT GAT AGA AGC AGA AGA TCG GCT ATA AAA AAG ATA 8253
ATG GAA AGG GAT GAC ACA GCT GCA AAA ACA CTT GTT CTC TGT GTT TCT 8301
GAC ATA ATT TCA TTG AGC GCA AAT ATA TCT GAA ACT TCT AGC AAT AAA 8349
ACT AGT AGT GCA GAT ACC CAA AAA GTG GCC ATT ATT GAA CTT ACA GAT 8397
GGG TGG TAT GCT GTT AAG GCC CAG TTA GAT CCT CCC CTC TTA GCT GTC 8445
TTA AAG AAT GGC AGA CTG ACA GTT GGT CAG AAG ATT ATT CTT CAT GGA 8493
GCA GAA CTG GTG GGC TCT CCT GAT GCC TGT ACA CCT CTT GAA GCC CCA 8541
GAA TCT CTT ATG TTA AAG ATT TCT GCT AAC AGT ACT CGG CCT GCT CGC 8589
TGG TAT ACC AAA CTT GGA TTC TTT CCT GAC CCT AGA CCT TTT CCT CTG 8637
CCC TTA TCA TCG CTT TTC AGT GAT GGA GGA AAT GTT GGT TGT GTT GAT 8685
GTA ATT ATT CAA AGA GCA TAC CCT ATA CAG TGG ATG GAG AAG ACA TCA 8733
TCT GGA TTA TAC ATA TTT CGC AAT GAA AGA GAG GAA GAA AAG GAA GCA 8781
GCA AAA TAT GTG GAG GCC CAA CAA AAG AGA CTA GAA GCC TTA TTC ACT     8829
AAA ATT CAG GAG GAA TTT GAA GAA CAT GAA GAA AAC ACA ACA AAA CCA     8877
TAT TTA CCA TCA CGT GCA CTA ACA AGA CAG CAA GTT CGT GCT TTG CAA     8925
GAT GGT GCA GAG CTT TAT GAA GCA GTG AAG AAT GCA GCA GAC CCA GCT     8973
TAC CTT GAG GGT TAT TTC AGT GAA GAG CAG TTA AGA GCC TTG AAT AAT     9021
CAC AGG CAA ATG TTG AAT GAT AAG AAA CAA GCT CAG ATC CAG TTG GAA     9069
ATT AGG AAG GCC ATG GAA TCT GCT GAA CAA AAG GAA CAA GGT TTA TCA     9117
AGG GAT GTC ACA ACC GTG TGG AAG TTG CGT ATT GTA AGC TAT TCA AAA     9165
AAA GAA AAA GAT TCA GTT ATA CTG AGT ATT TGG CGT CCA TCA TCA GAT     9213
TTA TAT TCT CTG TTA ACA GAA GGA AAG AGA TAC AGA ATT TAT CAT CTT     9261
GCA ACT TCA AAA TCT AAA AGT AAA TCT GAA AGA GCT AAC ATA CAG TTA     9309
GCA GCG ACA AAA AAA ACT CAG TAT CAA CAA CTA CCG GTT TCA GAT GAA     9357
ATT TTA TTT CAG ATT TAC CAG CCA CGG GAG CCC CTT CAC TTC AGC AAA     9405
TTT TTA GAT CCA GAC TTT CAG CCA TCT TGT TCT GAG GTG GAC CTA ATA     9453
GGA TTT GTC GTT TCT GTT GTG AAA AAA ACA GGA CTT GCC CCT TTC GTC     9501
TAT TTG TCA GAC GAA TGT TAC AAT TTA CTG GCA ATA AAG TTT TGG ATA     9549
GAC CTT AAT GAG GAC ATT ATT AAG CCT CAT ATG TTA ATT GCT GCA AGC     9597
AAC CTC CAG TGG CGA CCA GAA TCC AAA TCA GGC CTT CTT ACT TTA TTT     9645
GCT GGA GAT TTT TCT GTG TTT TCT GCT AGT CCA AAA GAG GGC CAC TTT     9693
CAA GAG ACA TTC AAC AAA ATG AAA AAT ACT GTT GAG AAT ATT GAC ATA     9741
CTT TGC AAT GAA GCA GAA AAC AAG CTT ATG CAT ATA CTG CAT GCA AAT     9789
GAT CCC AAG TGG TCC ACC CCA ACT AAA GAC TGT ACT TCA GGG CCG TAC     9837
ACT GCT CAA ATC ATT CCT GGT ACA GGA AAC AAG CTT CTG ATG TCT TCT     9885
CCT AAT TGT GAG ATA TAT TAT CAA AGT CCT TTA TCA CTT TGT ATG GCC     9933
AAA AGG AAG TCT GTT TCC ACA CCT GTC TCA GCC CAG ATG ACT TCA AAG     9981
TCT TGT AAA GGG GAG AAA GAG ATT GAT GAC CAA AAG AAC TGC AAA AAG     10029
AGA AGA GCC TTG GAT TTC TTG AGT AGA CTG CCT TTA CCT CCA CCT GTT     10077
AGT CCC ATT TGT ACA TTT GTT TCT CCG GCT GCA CAG AAG GCA TTT CAG     10125
CCA CCA AGG AGT TGT GGC ACC AAA TAC GAA ACA CCC ATA AAG AAA AAA     10173
GAA CTG AAT TCT CCT CAG ATG ACT CCA TTT AAA AAA TTC AAT GAA ATT     10221
TCT CTT TTG GAA AGT AAT TCA ATA GCT GAC GAA GAA CTT GCA TTG ATA    10269
AAT ACC CAA GCT CTT TTG TCT GGT TCA ACA GGA GAA AAA CAA TTT ATA    10317
TCT GTC AGT GAA TCC ACT AGG ACT GCT CCC ACC AGT TCA GAA GAT TAT    10365
CTC AGA CTG AAA CGA CGT TGT ACT ACA TCT CTG ATC AAA GAA CAG GAG    10413
AGT TCC CAG GCC AGT ACG GAA GAA TGT GAG AAA AAT AAG CAG GAC ACA    10461
ATT ACA ACT AAA AAA TAT ATC TAA                                    10485

(2) INFORMATION FOR SEQ ID NO:5:

(i) SEQUENCE CHARACTERISTICS:
(A) LENGTH: 3418 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:5:

(2) INFORMATION FOR SEQ ID NO:6:

(i) SEQUENCE CHARACTERISTICS:
(A) LENGTH: 10485 base pairs
(B) TYPE: nucleic acid
(C) STRANDEDNESS: double
(D) TOPOLOGY: linear

(ii) MOLECULE TYPE: cDNA
(ix) FEATURE:

(A) NAME/KEY: Coding Sequence
(B) LOCATION: 229...10482
(D) OTHER INFORMATION: BRCA2 (OMI2)

(xi) SEQUENCE DESCRIPTION: SEQ ID NO:6:

GGTGGCGCGA GCTTCTGAAA CTAGGCGGCA GAGGCGGAGC CGCTGTGGCA CTGCTGCGCC     60
TCTGCTGCGC CTCGGGTGTC TTTTGCGGCG GTGGGTCGCC GCCGGGAGAA GCGTGAGGGG    120
ACAGATTTGT GACCGGCGCG GTTTTTGTCA GCTTACTCCG GCCAAAAAAG AACTGCACCT    180
CTGGAGCGGA CTTATTTACC AAGCATTGGA GGAATATCGT AGGTAAAA ATG CCT ATT    237

Met Pro Ile
1

GGA TCC AAA GAG AGG CCA ACA TTT TTT GAA ATT TTT AAG ACA CGC TGC 285
Gly Ser Lys Glu Arg Pro Thr Phe Phe Glu Ile Phe Lys Thr Arg Cys
    5                   10                  15

AAC AAA GCA GAT TTA GGA CCA ATA AGT CTT AAT TGG TTT GAA GAA CTT 333
Asn Lys Ala Asp Leu Gly Pro Ile Ser Leu Asn Trp Phe Glu Glu Leu
20                  25                  30                  35

TCT TCA GAA GCT CCA CCC TAT AAT TCT GAA CCT GCA GAA GAA TCT GAA 381
Ser Ser Glu Ala Pro Pro Tyr Asn Ser Glu Pro Ala Glu Glu Ser Glu
                40                  45                  50

CAT AAA AAC AAC AAT TAC GAA CCA AAC CTA TTT AAA ACT CCA CAA AGG 429
His Lys Asn Asn Asn Tyr Glu Pro Asn Leu Phe Lys Thr Pro Gln Arg
            55                  60                  65

AAA CCA TCT TAT AAT CAG CTG GCT TCA ACT CCA ATA ATA TTC AAA GAG 477
Lys Pro Ser Tyr Asn Gln Leu Ala Ser Thr Pro Ile Ile Phe Lys Glu
        70                  75                  80

CAA GGG CTG ACT CTG CCG CTG TAC CAA TCT CCT GTA AAA GAA TTA GAT 525
Gln Gly Leu Thr Leu Pro Leu Tyr Gln Ser Pro Val Lys Glu Leu Asp
    85                  90                  95

AAA TTC AAA TTA GAC TTA GGA AGG AAT GTT CCC AAT AGT AGA CAT AAA 573
Lys Phe Lys Leu Asp Leu Gly Arg Asn Val Pro Asn Ser Arg His Lys
100                 105                 110                 115

AGT CTT CGC ACA GTG AAA ACT AAA ATG GAT CAA GCA GAT GAT GTT TCC 621
Ser Leu Arg Thr Val Lys Thr Lys Met Asp Gln Ala Asp Asp Val Ser
                120                 125                 130

TGT CCA CTT CTA AAT TCT TGT CTT AGT GAA AGT CCT GTT GTT CTA CAA 669
Cys Pro Leu Leu Asn Ser Cys Leu Ser Glu Ser Pro Val Val Leu Gln
            135                 140                 145

TGT ACA CAT GTA ACA CCA CAA AGA GAT AAG TCA GTG GTA TGT GGG AGT 717
Cys Thr His Val Thr Pro Gln Arg Asp Lys Ser Val Val Cys Gly Ser
        150                 155                 160

TTG TTT CAT ACA CCA AAG TTT GTG AAG GGT CGT CAG ACA CCA AAA CAT 765
Leu Phe His Thr Pro Lys Phe Val Lys Gly Arg Gln Thr Pro Lys His
    165                 170                 175

ATT TCT GAA AGT CTA GGA GCT GAG GTG GAT CCT GAT ATG TCT TGG TCA 813
Ile Ser Glu Ser Leu Gly Ala Glu Val Asp Pro Asp Met Ser Trp Ser
180                 185                 190                 195

AGT TCT TTA GCT ACA CCA CCC ACC CTT AGT TCT ACT GTG CTC ATA GTC 861
Ser Ser Leu Ala Thr Pro Pro Thr Leu Ser Ser Thr Val Leu Ile Val
                200                 205                 210

AGA AAT GAA GAA GCA TCT GAA ACT GTA TTT CCT CAT GAT ACT ACT GCT 909
Arg Asn Glu Glu Ala Ser Glu Thr Val Phe Pro His Asp Thr Thr Ala
            215                 220                 225

AAT GTG AAA AGC TAT TTT TCC AAT CAT GAT GAA AGT CTG AAG AAA AAT 957
Asn Val Lys Ser Tyr Phe Ser Asn His Asp Glu Ser Leu Lys Lys Asn

        230                 235                 240

GAT AGA TTT ATC GCT TCT GTG ACA GAC AGT GAA AAC ACA AAT CAA AGA 1005
Asp Arg Phe Ile Ala Ser Val Thr Asp Ser Glu Asn Thr Asn Gln Arg
    245                 250                 255

GAA GCT GCA AGT CAT GGA TTT GGA AAA ACA TCA GGG AAT TCA TTT AAA 1053
Glu Ala Ala Ser His Gly Phe Gly Lys Thr Ser Gly Asn Ser Phe Lys
260                 265                 270                 275

GTA AAT AGC TGC AAA GAC CAC ATT GGA AAG TCA ATG CCA AAT GTC CTA 1101
Val Asn Ser Cys Lys Asp His Ile Gly Lys Ser Met Pro Asn Val Leu
                280                 285                 290

GAA GAT GAA GTA TAT GAA ACA GTT GTA GAT ACC TCT GAA GAA GAT AGT 1149
Glu Asp Glu Val Tyr Glu Thr Val Val Asp Thr Ser Glu Glu Asp Ser
            295                 300                 305

TTT TCA TTA TGT TTT TCT AAA TGT AGA ACA AAA AAT CTA CAA AAA GTA 1197
Phe Ser Leu Cys Phe Ser Lys Cys Arg Thr Lys Asn Leu Gln Lys Val
        310                 315                 320

AGA ACT AGC AAG ACT AGG AAA AAA ATT TTC CAT GAA GCA AAC GCT GAT 1245
Arg Thr Ser Lys Thr Arg Lys Lys Ile Phe His Glu Ala Asn Ala Asp
    325                 330                 335

GAA TGT GAA AAA TCT AAA AAC CAA GTG AAA GAA AAA TAC TCA TTT GTA 1293
Glu Cys Glu Lys Ser Lys Asn Gln Val Lys Glu Lys Tyr Ser Phe Val
340                 345                 350                 355

TCT GAA GTG GAA CCA AAT GAT ACT GAT CCA TTA GAT TCA AAT GTA GCA 1341
Ser Glu Val Glu Pro Asn Asp Thr Asp Pro Leu Asp Ser Asn Val Ala
                360                 365                 370

CAT CAG AAG CCC TTT GAG AGT GGA AGT GAC AAA ATC TCC AAG GAA GTT 1389
His Gln Lys Pro Phe Glu Ser Gly Ser Asp Lys Ile Ser Lys Glu Val
            375                 380                 385

GTA CCG TCT TTG GCC TGT GAA TGG TCT CAA CTA ACC CTT TCA GGT CTA 1437
Val Pro Ser Leu Ala Cys Glu Trp Ser Gln Leu Thr Leu Ser Gly Leu
        390                 395                 400

AAT GGA GCC CAG ATG GAG AAA ATA CCC CTA TTG CAT ATT TCT TCA TGT 1485
Asn Gly Ala Gln Met Glu Lys Ile Pro Leu Leu His Ile Ser Ser Cys
    405                 410                 415

GAC CAA AAT ATT TCA GAA AAA GAC CTA TTA GAC ACA GAG AAC AAA AGA 1533
Asp Gln Asn Ile Ser Glu Lys Asp Leu Leu Asp Thr Glu Asn Lys Arg
420                 425                 430                 435

AAG AAA GAT TTT CTT ACT TCA GAG AAT TCT TTG CCA CGT ATT TCT AGC 1581
Lys Lys Asp Phe Leu Thr Ser Glu Asn Ser Leu Pro Arg Ile Ser Ser
                440                 445                 450

CTA CCA AAA TCA GAG AAG CCA TTA AAT GAG GAA ACA GTG GTA AAT AAG 1629
Leu Pro Lys Ser Glu Lys Pro Leu Asn Glu Glu Thr Val Val Asn Lys
            455                 460                 465

AGA GAT GAA GAG CAG CAT CTT GAA TCT CAT ACA GAC TGC ATT CTT GCA 1677
Arg Asp Glu Glu Gln His Leu Glu Ser His Thr Asp Cys Ile Leu Ala
        470                 475                 480

GTA AAG CAG GCA ATA TCT GGA ACT TCT CCA GTG GCT TCT TCA TTT CAG 1725
Val Lys Gln Ala Ile Ser Gly Thr Ser Pro Val Ala Ser Ser Phe Gln
    485                 490                 495

GGT ATC AAA AAG TCT ATA TTC AGA ATA AGA GAA TCA CCT AAA GAG ACT 1773
Gly Ile Lys Lys Ser Ile Phe Arg Ile Arg Glu Ser Pro Lys Glu Thr
500                 505                 510                 515

TTC AAT GCA AGT TTT TCA GGT CAT ATG ACT GAT CCA AAC TTT AAA AAA 1821
Phe Asn Ala Ser Phe Ser Gly His Met Thr Asp Pro Asn Phe Lys Lys
                520                 525                 530

GAA ACT GAA GCC TCT GAA AGT GGA CTG GAA ATA CAT ACT GTT TGC TCA 1869
Glu Thr Glu Ala Ser Glu Ser Gly Leu Glu Ile His Thr Val Cys Ser
            535                 540                 545

CAG AAG GAG GAC TCC TTA TGT CCA AAT TTA ATT GAT AAT GGA AGC TGG 1917
Gln Lys Glu Asp Ser Leu Cys Pro Asn Leu Ile Asp Asn Gly Ser Trp
        550                 555                 560

CCA GCC ACC ACC ACA CAG AAT TCT GTA GCT TTG AAG AAT GCA GGT TTA 1965
Pro Ala Thr Thr Thr Gln Asn Ser Val Ala Leu Lys Asn Ala Gly Leu
    565                 570                 575

ATA TCC ACT TTG AAA AAG AAA ACA AAT AAG TTT ATT TAT GCT ATA CAT 2013
Ile Ser Thr Leu Lys Lys Lys Thr Asn Lys Phe Ile Tyr Ala Ile His
580                 585                 590                 595

GAT GAA ACA TCT TAT AAA GGA AAA AAA ATA CCG AAA GAC CAA AAA TCA 2061
Asp Glu Thr Ser Tyr Lys Gly Lys Lys Ile Pro Lys Asp Gln Lys Ser
                600                 605                 610

GAA CTA ATT AAC TGT TCA GCC CAG TTT GAA GCA AAT GCT TTT GAA GCA 2109
Glu Leu Ile Asn Cys Ser Ala Gln Phe Glu Ala Asn Ala Phe Glu Ala
            615                 620                 625

CCA CTT ACA TTT GCA AAT GCT GAT TCA GGT TTA TTG CAT TCT TCT GTG 2157
Pro Leu Thr Phe Ala Asn Ala Asp Ser Gly Leu Leu His Ser Ser Val
        630                 635                 640

AAA AGA AGC TGT TCA CAG AAT GAT TCT GAA GAA CCA ACT TTG TCC TTA 2205
Lys Arg Ser Cys Ser Gln Asn Asp Ser Glu Glu Pro Thr Leu Ser Leu
    645                 650                 655

ACT AGC TCT TTT GGG ACA ATT CTG AGG AAA TGT TCT AGA AAT GAA ACA 2253
Thr Ser Ser Phe Gly Thr Ile Leu Arg Lys Cys Ser Arg Asn Glu Thr
660                 665                 670                 675

TGT TCT AAT AAT ACA GTA ATC TCT CAG GAT CTT GAT TAT AAA GAA GCA 2301
Cys Ser Asn Asn Thr Val Ile Ser Gln Asp Leu Asp Tyr Lys Glu Ala
                680                 685                 690

AAA TGT AAT AAG GAA AAA CTA CAG TTA TTT ATT ACC CCA GAA GCT GAT 2349
Lys Cys Asn Lys Glu Lys Leu Gln Leu Phe Ile Thr Pro Glu Ala Asp
            695                 700                 705

TCT CTG TCA TGC CTG CAG GAA GGA CAG TGT GAA AAT GAT CCA AAA AGC 2397
Ser Leu Ser Cys Leu Gln Glu Gly Gln Cys Glu Asn Asp Pro Lys Ser
        710                 715                 720

AAA AAA GTT TCA GAT ATA AAA GAA GAG GTC TTG GCT GCA GCA TGT CAC     2445
Lys Lys Val Ser Asp Ile Lys Glu Glu Val Leu Ala Ala Ala Cys His
    725                 730                 735

CCA GTA CAA CAT TCA AAA GTG GAA TAC AGT GAT ACT GAC TTT CAA TCC     2493
Pro Val Gln His Ser Lys Val Glu Tyr Ser Asp Thr Asp Phe Gln Ser
740                 745                 750                 755

CAG AAA AGT CTT TTA TAT GAT CAT GAA AAT GCC AGC ACT CTT ATT TTA     2541
Gln Lys Ser Leu Leu Tyr Asp His Glu Asn Ala Ser Thr Leu Ile Leu
                760                 765                 770

ACT CCT ACT TCC AAG GAT GTT CTG TCA AAC CTA GTC ATG ATT TCT AGA     2589
Thr Pro Thr Ser Lys Asp Val Leu Ser Asn Leu Val Met Ile Ser Arg
            775                 780                 785

GGC AAA GAA TCA TAC AAA ATG TCA GAC AAG CTC AAA GGT AAC AAT TAT     2637
Gly Lys Glu Ser Tyr Lys Met Ser Asp Lys Leu Lys Gly Asn Asn Tyr
        790                 795                 800

GAA TCT GAT GTT GAA TTA ACC AAA AAT ATT CCC ATG GAA AAG AAT CAA     2685
Glu Ser Asp Val Glu Leu Thr Lys Asn Ile Pro Met Glu Lys Asn Gln
    805                 810                 815

GAT GTA TGT GCT TTA AAT GAA AAT TAT AAA AAC GTT GAG CTG TTG CCA     2733
Asp Val Cys Ala Leu Asn Glu Asn Tyr Lys Asn Val Glu Leu Leu Pro
820                 825                 830                 835

CCT GAA AAA TAC ATG AGA GTA GCA TCA CCT TCA AGA AAG GTA CAA TTC     2781
Pro Glu Lys Tyr Met Arg Val Ala Ser Pro Ser Arg Lys Val Gln Phe
                840                 845                 850

AAC CAA AAC ACA AAT CTA AGA GTA ATC CAA AAA AAT CAA GAA GAA ACT     2829
Asn Gln Asn Thr Asn Leu Arg Val Ile Gln Lys Asn Gln Glu Glu Thr
            855                 860                 865

ACT TCA ATT TCA AAA ATA ACT GTC AAT CCA GAC TCT GAA GAA CTT TTC     2877
Thr Ser Ile Ser Lys Ile Thr Val Asn Pro Asp Ser Glu Glu Leu Phe
        870                 875                 880

TCA GAC AAT GAG AAT AAT TTT GTC TTC CAA GTA GCT AAT GAA AGG AAT     2925
Ser Asp Asn Glu Asn Asn Phe Val Phe Gln Val Ala Asn Glu Arg Asn
    885                 890                 895

AAT CTT GCT TTA GGA AAT ACT AAG GAA CTT CAT GAA ACA GAC TTG ACT     2973
Asn Leu Ala Leu Gly Asn Thr Lys Glu Leu His Glu Thr Asp Leu Thr
900                 905                 910                 915

TGT GTA AAC GAA CCC ATT TTC AAG AAC TCT ACC ATG GTT TTA TAT GGA     3021
Cys Val Asn Glu Pro Ile Phe Lys Asn Ser Thr Met Val Leu Tyr Gly
                920                 925                 930

GAC ACA GGT GAT AAA CAA GCA ACC CAA GTG TCA ATT AAA AAA GAT TTG     3069
Asp Thr Gly Asp Lys Gln Ala Thr Gln Val Ser Ile Lys Lys Asp Leu
            935                 940                 945

GTT TAT GTT CTT GCA GAG GAG AAC AAA AAT AGT GTA AAG CAG CAT ATA     3117
Val Tyr Val Leu Ala Glu Glu Asn Lys Asn Ser Val Lys Gln His Ile
        950                 955                 960

AAA ATG ACT CTA GGT CAA GAT TTA AAA TCG GAC ATC TCC TTG AAT ATA     3165

Lys Met Thr Leu Gly Gln Asp Leu Lys Ser Asp Ile Ser Leu Asn Ile
    965                 970                 975

GAT AAA ATA CCA GAA AAA AAT AAT GAT TAC ATG AAC AAA TGG GCA GGA     3213
Asp Lys Ile Pro Glu Lys Asn Asn Asp Tyr Met Asn Lys Trp Ala Gly
980                 985                 990                 995

CTC TTA GGT CCA ATT TCA AAT CAC AGT TTT GGA GGT AGC TTC AGA ACA     3261
Leu Leu Gly Pro Ile Ser Asn His Ser Phe Gly Gly Ser Phe Arg Thr
                1000                1005                1010

GCT TCA AAT AAG GAA ATC AAG CTC TCT GAA CAT AAC ATT AAG AAG AGC     3309
Ala Ser Asn Lys Glu Ile Lys Leu Ser Glu His Asn Ile Lys Lys Ser
            1015                1020                1025

AAA ATG TTC TTC AAA GAT ATT GAA GAA CAA TAT CCT ACT AGT TTA GCT     3357
Lys Met Phe Phe Lys Asp Ile Glu Glu Gln Tyr Pro Thr Ser Leu Ala
        1030                1035                1040

TGT GTT GAA ATT GTA AAT ACC TTG GCA TTA GAT AAT CAA AAG AAA CTG     3405
Cys Val Glu Ile Val Asn Thr Leu Ala Leu Asp Asn Gln Lys Lys Leu
    1045                1050                1055

AGC AAG CCT CAG TCA ATT AAT ACT GTA TCT GCA CAT TTA CAG AGT AGT     3453
Ser Lys Pro Gln Ser Ile Asn Thr Val Ser Ala His Leu Gln Ser Ser
1060                1065                1070                1075

GTA GTT GTT TCT GAT TGT AAA AAT AGT CAT ATA ACC CCT CAG ATG TTA     3501
Val Val Val Ser Asp Cys Lys Asn Ser His Ile Thr Pro Gln Met Leu
                1080                1085                1090

TTT TCC AAG CAG GAT TTT AAT TCA AAC CAT AAT TTA ACA CCT AGC CAA     3549
Phe Ser Lys Gln Asp Phe Asn Ser Asn His Asn Leu Thr Pro Ser Gln
            1095                1100                1105

AAG GCA GAA ATT ACA GAA CTT TCT ACT ATA TTA GAA GAA TCA GGA AGT     3597
Lys Ala Glu Ile Thr Glu Leu Ser Thr Ile Leu Glu Glu Ser Gly Ser
        1110                1115                1120

CAG TTT GAA TTT ACT CAG TTT AGA AAR CCA AGC TAC ATA TTG CAG AAG     3645
Gln Phe Glu Phe Thr Gln Phe Arg Xaa Pro Ser Tyr Ile Leu Gln Lys
    1125                1130                1135

AGT ACA TTT GAA GTG CCT GAA AAC CAG ATG ACT ATC TTA AAG ACC ACT     3693
Ser Thr Phe Glu Val Pro Glu Asn Gln Met Thr Ile Leu Lys Thr Thr
1140                1145                1150                1155

TCT GAG GAA TGC AGA GAT GCT GAT CTT CAT GTC ATA ATG AAT GCC CCA     3741
Ser Glu Glu Cys Arg Asp Ala Asp Leu His Val Ile Met Asn Ala Pro
                1160                1165                1170

TCG ATT GGT CAG GTA GAC AGC AGC AAG CAA TTT GAA GGT ACA GTT GAA     3789
Ser Ile Gly Gln Val Asp Ser Ser Lys Gln Phe Glu Gly Thr Val Glu
            1175                1180                1185

ATT AAA CGG AAG TTT GCT GGC CTG TTG AAA AAT GAC TGT AAC AAA AGT     3837
Ile Lys Arg Lys Phe Ala Gly Leu Leu Lys Asn Asp Cys Asn Lys Ser
        1190                1195                1200

GCT TCT GGT TAT TTA ACA GAT GAA AAT GAA GTG GGG TTT AGG GGC TTT     3885
Ala Ser Gly Tyr Leu Thr Asp Glu Asn Glu Val Gly Phe Arg Gly Phe

1205                1210                1215

TAT TCT GCT CAT GGC ACA AAA CTG AAT GTT TCT ACT GAA GCT CTG CAA     3933
Tyr Ser Ala His Gly Thr Lys Leu Asn Val Ser Thr Glu Ala Leu Gln
1220                1225                1230                1235

AAA GCT GTG AAA CTG TTT AGT GAT ATT GAG AAT ATT AGT GAG GAA ACT     3981
Lys Ala Val Lys Leu Phe Ser Asp Ile Glu Asn Ile Ser Glu Glu Thr
                1240                1245                1250

TCT GCA GAG GTA CAT CCA ATA AGT TTA TCT TCA AGT AAA TGT CAT GAT     4029
Ser Ala Glu Val His Pro Ile Ser Leu Ser Ser Ser Lys Cys His Asp
            1255                1260                1265

TCT GTC GTT TCA ATG TTT AAG ATA GAA AAT CAT AAT GAT AAA ACT GTA     4077
Ser Val Val Ser Met Phe Lys Ile Glu Asn His Asn Asp Lys Thr Val
        1270                1275                1280

AGT GAA AAA AAT AAT AAA TGC CAA CTG ATA TTA CAA AAT AAT ATT GAA     4125
Ser Glu Lys Asn Asn Lys Cys Gln Leu Ile Leu Gln Asn Asn Ile Glu
    1285                1290                1295

ATG ACT ACT GGC ACT TTT GTT GAA GAA ATT ACT GAA AAT TAC AAG AGA     4173
Met Thr Thr Gly Thr Phe Val Glu Glu Ile Thr Glu Asn Tyr Lys Arg
1300                1305                1310                1315

AAT ACT GAA AAT GAA GAT AAC AAA TAT ACT GCT GCC AGT AGA AAT TCT     4221
Asn Thr Glu Asn Glu Asp Asn Lys Tyr Thr Ala Ala Ser Arg Asn Ser
                1320                1325                1330

CAT AAC TTA GAA TTT GAT GGC AGT GAT TCA AGT AAA AAT GAT ACT GTT     4269
His Asn Leu Glu Phe Asp Gly Ser Asp Ser Ser Lys Asn Asp Thr Val
            1335                1340                1345

TGT ATT CAT AAA GAT GAA ACG GAC TTG CTA TTT ACT GAT CAG CAC AAC     4317
Cys Ile His Lys Asp Glu Thr Asp Leu Leu Phe Thr Asp Gln His Asn
        1350                1355                1360

ATA TGT CTT AAA TTA TCT GGC CAG TTT ATG AAG GAG GGA AAC ACT CAG     4365
Ile Cys Leu Lys Leu Ser Gly Gln Phe Met Lys Glu Gly Asn Thr Gln
    1365                1370                1375

ATT AAA GAA GAT TTG TCA GAT TTA ACT TTT TTG GAA GTT GCG AAA GCT     4413
Ile Lys Glu Asp Leu Ser Asp Leu Thr Phe Leu Glu Val Ala Lys Ala
1380                1385                1390                1395

CAA GAA GCA TGT CAT GGT AAT ACT TCA AAT AAA GAA CAG TTA ACT GCT     4461
Gln Glu Ala Cys His Gly Asn Thr Ser Asn Lys Glu Gln Leu Thr Ala
                1400                1405                1410

ACT AAA ACG GAG CAA AAT ATA AAA GAT TTT GAG ACT TCT GAT ACA TTT     4509
Thr Lys Thr Glu Gln Asn Ile Lys Asp Phe Glu Thr Ser Asp Thr Phe
            1415                1420                1425

TTT CAG ACT GCA AGT GGG AAA AAT ATT AGT GTC GCC AAA GAG TCA TTT     4557
Phe Gln Thr Ala Ser Gly Lys Asn Ile Ser Val Ala Lys Glu Ser Phe
        1430                1435                1440

AAT AAA ATT GTA AAT TTC TTT GAT CAG AAA CCA GAA GAA TTG CAT AAC     4605
Asn Lys Ile Val Asn Phe Phe Asp Gln Lys Pro Glu Glu Leu His Asn
    1445                1450                1455

TTT TCC TTA AAT TCT GAA TTA CAT TCT GAC ATA AGA AAG AAC AAA ATG     4653
Phe Ser Leu Asn Ser Glu Leu His Ser Asp Ile Arg Lys Asn Lys Met
1460                1465                1470                1475

GAC ATT CTA AGT TAT GAG GAA ACA GAC ATA GTT AAA CAC AAA ATA CTG     4701
Asp Ile Leu Ser Tyr Glu Glu Thr Asp Ile Val Lys His Lys Ile Leu
                1480                1485                1490

AAA GAA AGT GTC CCA GTT GGT ACT GGA AAT CAA CTA GTG ACC TTC CAG     4749
Lys Glu Ser Val Pro Val Gly Thr Gly Asn Gln Leu Val Thr Phe Gln
            1495                1500                1505

GGA CAA CCC GAA CGT GAT GAA AAG ATC AAA GAA CCT ACT CTG TTG GGT     4797
Gly Gln Pro Glu Arg Asp Glu Lys Ile Lys Glu Pro Thr Leu Leu Gly
        1510                1515                1520

TTT CAT ACA GCT AGC GGG AAA AAA GTT AAA ATT GCA AAG GAA TCT TTG     4845
Phe His Thr Ala Ser Gly Lys Lys Val Lys Ile Ala Lys Glu Ser Leu
    1525                1530                1535

GAC AAA GTG AAA AAC CTT TTT GAT GAA AAA GAG CAA GGT ACT AGT GAA     4893
Asp Lys Val Lys Asn Leu Phe Asp Glu Lys Glu Gln Gly Thr Ser Glu
1540                1545                1550                1555

ATC ACC AGT TTT AGC CAT CAA TGG GCA AAG ACC CTA AAG TAC AGA GAG     4941
Ile Thr Ser Phe Ser His Gln Trp Ala Lys Thr Leu Lys Tyr Arg Glu
                1560                1565                1570

GCC TGT AAA GAC CTT GAA TTA GCA TGT GAG ACC ATT GAG ATC ACA GCT     4989
Ala Cys Lys Asp Leu Glu Leu Ala Cys Glu Thr Ile Glu Ile Thr Ala
            1575                1580                1585

GCC CCA AAG TGT AAA GAA ATG CAG AAT TCT CTC AAT AAT GAT AAA AAC     5037
Ala Pro Lys Cys Lys Glu Met Gln Asn Ser Leu Asn Asn Asp Lys Asn
        1590                1595                1600

CTT GTT TCT ATT GAG ACT GTG GTG CCA CCT AAG CTC TTA AGT GAT AAT     5085
Leu Val Ser Ile Glu Thr Val Val Pro Pro Lys Leu Leu Ser Asp Asn
    1605                1610                1615

TTA TGT AGA CAA ACT GAA AAT CTC AAA ACA TCA AAA AGT ATC TTT TTG     5133
Leu Cys Arg Gln Thr Glu Asn Leu Lys Thr Ser Lys Ser Ile Phe Leu
1620                1625                1630                1635

AAA GTT AAA GTA CAT GAA AAT GTA GAA AAA GAA ACA GCA AAA AGT CCT     5181
Lys Val Lys Val His Glu Asn Val Glu Lys Glu Thr Ala Lys Ser Pro
                1640                1645                1650

GCA ACT TGT TAC ACA AAT CAG TCC CCT TAT TCA GTC ATT GAA AAT TCA     5229
Ala Thr Cys Tyr Thr Asn Gln Ser Pro Tyr Ser Val Ile Glu Asn Ser
            1655                1660                1665

GCC TTA GCT TTT TAC ACA AGT TGT AGT AGA AAA ACT TCT GTG AGT CAG     5277
Ala Leu Ala Phe Tyr Thr Ser Cys Ser Arg Lys Thr Ser Val Ser Gln
        1670                1675                1680

ACT TCA TTA CTT GAA GCA AAA AAA TGG CTT AGA GAA GGA ATA TTT GAT     5325
Thr Ser Leu Leu Glu Ala Lys Lys Trp Leu Arg Glu Gly Ile Phe Asp
    1685                1690                1695

GGT CAA CCA GAA AGA ATA AAT ACT GCA GAT TAT GTA GGA AAT TAT TTG 5373
Gly Gln Pro Glu Arg Ile Asn Thr Ala Asp Tyr Val Gly Asn Tyr Leu
1700                1705                1710                1715

TAT GAA AAT AAT TCA AAC AGT ACT ATA GCT GAA AAT GAC AAA AAT CAT 5421
Tyr Glu Asn Asn Ser Asn Ser Thr Ile Ala Glu Asn Asp Lys Asn His
                1720                1725                1730

CTC TCC GAA AAA CAA GAT ACT TAT TTA AGT AAC AGT AGC ATG TCT AAC 5469
Leu Ser Glu Lys Gln Asp Thr Tyr Leu Ser Asn Ser Ser Met Ser Asn
            1735                1740                1745

AGC TAT TCC TAC CAT TCT GAT GAG GTA TAT AAT GAT TCA GGA TAT CTC 5517
Ser Tyr Ser Tyr His Ser Asp Glu Val Tyr Asn Asp Ser Gly Tyr Leu
        1750                1755                1760

TCA AAA AAT AAA CTT GAT TCT GGT ATT GAG CCA GTA TTG AAG AAT GTT 5565
Ser Lys Asn Lys Leu Asp Ser Gly Ile Glu Pro Val Leu Lys Asn Val
    1765                1770                1775

GAA GAT CAA AAA AAC ACT AGT TTT TCC AAA GTA ATA TCC AAT GTA AAA 5613
Glu Asp Gln Lys Asn Thr Ser Phe Ser Lys Val Ile Ser Asn Val Lys
1780                1785                1790                1795

GAT GCA AAT GCA TAC CCA CAA ACT GTA AAT GAA GAT ATT TGC GTT GAG 5661
Asp Ala Asn Ala Tyr Pro Gln Thr Val Asn Glu Asp Ile Cys Val Glu
                1800                1805                1810

GAA CTT GTG ACT AGC TCT TCA CCC TGC AAA AAT AAA AAT GCA GCC ATT 5709
Glu Leu Val Thr Ser Ser Ser Pro Cys Lys Asn Lys Asn Ala Ala Ile
            1815                1820                1825

AAA TTG TCC ATA TCT AAT AGT AAT AAT TTT GAG GTA GGG CCA CCT GCA 5757
Lys Leu Ser Ile Ser Asn Ser Asn Asn Phe Glu Val Gly Pro Pro Ala
        1830                1835                1840

TTT AGG ATA GCC AGT GGT AAA ATC GTT TGT GTT TCA CAT GAA ACA ATT 5805
Phe Arg Ile Ala Ser Gly Lys Ile Val Cys Val Ser His Glu Thr Ile
    1845                1850                1855

AAA AAA GTG AAA GAC ATA TTT ACA GAC AGT TTC AGT AAA GTA ATT AAG 5853
Lys Lys Val Lys Asp Ile Phe Thr Asp Ser Phe Ser Lys Val Ile Lys
1860                1865                1870                1875

GAA AAC AAC GAG AAT AAA TCA AAA ATT TGC CAA ACG AAA ATT ATG GCA 5901
Glu Asn Asn Glu Asn Lys Ser Lys Ile Cys Gln Thr Lys Ile Met Ala
                1880                1885                1890

GGT TGT TAC GAG GCA TTG GAT GAT TCA GAG GAT ATT CTT CAT AAC TCT 5949
Gly Cys Tyr Glu Ala Leu Asp Asp Ser Glu Asp Ile Leu His Asn Ser
            1895                1900                1905

CTA GAT AAT GAT GAA TGT AGC ACG CAT TCA CAT AAG GTT TTT GCT GAC 5997
Leu Asp Asn Asp Glu Cys Ser Thr His Ser His Lys Val Phe Ala Asp
        1910                1915                1920

ATT CAG AGT GAA GAA ATT TTA CAA CAT AAC CAA AAT ATG TCT GGA TTG 6045
Ile Gln Ser Glu Glu Ile Leu Gln His Asn Gln Asn Met Ser Gly Leu
    1925                1930                1935

GAG AAA GTT TCT AAA ATA TCA CCT TGT GAT GTT AGT TTG GAA ACT TCA 6093

Glu Lys Val Ser Lys Ile Ser Pro Cys Asp Val Ser Leu Glu Thr Ser
1940                1945                1950                1955

GAT ATA TGT AAA TGT AGT ATA GGG AAG CTT CAT AAG TCA GTC TCA TCT     6141
Asp Ile Cys Lys Cys Ser Ile Gly Lys Leu His Lys Ser Val Ser Ser
                1960                1965                1970

GCA AAT ACT TGT GGG ATT TTT AGC ACA GCA AGT GGA AAA TCT GTC CAG     6189
Ala Asn Thr Cys Gly Ile Phe Ser Thr Ala Ser Gly Lys Ser Val Gln
            1975                1980                1985

GTA TCA GAT GCT TCA TTA CAA AAC GCA AGA CAA GTG TTT TCT GAA ATA     6237
Val Ser Asp Ala Ser Leu Gln Asn Ala Arg Gln Val Phe Ser Glu Ile
        1990                1995                2000

GAA GAT AGT ACC AAG CAA GTC TTT TCC AAA GTA TTG TTT AAA AGT AAC     6285
Glu Asp Ser Thr Lys Gln Val Phe Ser Lys Val Leu Phe Lys Ser Asn
    2005                2010                2015

GAA CAT TCA GAC CAG CTC ACA AGA GAA GAA AAT ACT GCT ATA CGT ACT     6333
Glu His Ser Asp Gln Leu Thr Arg Glu Glu Asn Thr Ala Ile Arg Thr
2020                2025                2030                2035

CCA GAA CAT TTA ATA TCC CAA AAA GGC TTT TCA TAT AAT GTG GTA AAT     6381
Pro Glu His Leu Ile Ser Gln Lys Gly Phe Ser Tyr Asn Val Val Asn
                2040                2045                2050

TCA TCT GCT TTC TCT GGA TTT AGT ACA GCA AGT GGA AAG CAA GTT TCC     6429
Ser Ser Ala Phe Ser Gly Phe Ser Thr Ala Ser Gly Lys Gln Val Ser
            2055                2060                2065

ATT TTA GAA AGT TCC TTA CAC AAA GTT AAG GGA GTG TTA GAG GAA TTT     6477
Ile Leu Glu Ser Ser Leu His Lys Val Lys Gly Val Leu Glu Glu Phe
        2070                2075                2080

GAT TTA ATC AGA ACT GAG CAT AGT CTT CAC TAT TCA CCT ACG TCT AGA     6525
Asp Leu Ile Arg Thr Glu His Ser Leu His Tyr Ser Pro Thr Ser Arg
    2085                2090                2095

CAA AAT GTA TCA AAA ATA CTT CCT CGT GTT GAT AAG AGA AAC CCA GAG     6573
Gln Asn Val Ser Lys Ile Leu Pro Arg Val Asp Lys Arg Asn Pro Glu
2100                2105                2110                2115

CAC TGT GTA AAC TCA GAA ATG GAA AAA ACC TGC AGT AAA GAA TTT AAA     6621
His Cys Val Asn Ser Glu Met Glu Lys Thr Cys Ser Lys Glu Phe Lys
                2120                2125                2130

TTA TCA AAT AAC TTA AAT GTT GAA GGT GGT TCT TCA GAA AAT AAT CAC     6669
Leu Ser Asn Asn Leu Asn Val Glu Gly Gly Ser Ser Glu Asn Asn His
            2135                2140                2145

TCT ATT AAA GTT TCT CCA TAT CTC TCT CAA TTT CAA CAA GAC AAA CAA     6717
Ser Ile Lys Val Ser Pro Tyr Leu Ser Gln Phe Gln Gln Asp Lys Gln
        2150                2155                2160

CAG TTG GTA TTA GGA ACC AAA GTC TCA CTT GTT GAG AAC ATT CAT GTT     6765
Gln Leu Val Leu Gly Thr Lys Val Ser Leu Val Glu Asn Ile His Val
    2165                2170                2175

TTG GGA AAA GAA CAG GCT TCA CCT AAA AAC GTA AAA ATG GAA ATT GGT     6813
Leu Gly Lys Glu Gln Ala Ser Pro Lys Asn Val Lys Met Glu Ile Gly

2180                2185                2190                2195

AAA ACT GAA ACT TTT TCT GAT GTT CCT GTG AAA ACA AAT ATA GAA GTT     6861
Lys Thr Glu Thr Phe Ser Asp Val Pro Val Lys Thr Asn Ile Glu Val
                    2200                2205                2210

TGT TCT ACT TAC TCC AAA GAT TCA GAA AAC TAC TTT GAA ACA GAA GCA     6909
Cys Ser Thr Tyr Ser Lys Asp Ser Glu Asn Tyr Phe Glu Thr Glu Ala
            2215                2220                2225

GTA GAA ATT GCT AAA GCT TTT ATG GAA GAT GAT GAA CTG ACA GAT TCT     6957
Val Glu Ile Ala Lys Ala Phe Met Glu Asp Asp Glu Leu Thr Asp Ser
        2230                2235                2240

AAA CTG CCA AGT CAT GCC ACA CAT TCT CTT TTT ACA TGT CCC GAA AAT     7005
Lys Leu Pro Ser His Ala Thr His Ser Leu Phe Thr Cys Pro Glu Asn
    2245                2250                2255

GAG GAA ATG GTT TTG TCA AAT TCA AGA ATT GGA AAA AGA AGA GGA GAG     7053
Glu Glu Met Val Leu Ser Asn Ser Arg Ile Gly Lys Arg Arg Gly Glu
2260                2265                2270                2275

CCC CTT ATC TTA GTG GGA GAA CCC TCA ATC AAA AGA AAC TTA TTA AAT     7101
Pro Leu Ile Leu Val Gly Glu Pro Ser Ile Lys Arg Asn Leu Leu Asn
                    2280                2285                2290

GAA TTT GAC AGG ATA ATA GAA AAT CAA GAA AAA TCC TTA AAG GCT TCA     7149
Glu Phe Asp Arg Ile Ile Glu Asn Gln Glu Lys Ser Leu Lys Ala Ser
            2295                2300                2305

AAA AGC ACT CCA GAT GGC ACA ATA AAA GAT CGA AGA TTG TTT ATG CAT     7197
Lys Ser Thr Pro Asp Gly Thr Ile Lys Asp Arg Arg Leu Phe Met His
        2310                2315                2320

CAT GTT TCT TTA GAG CCG ATT ACC TGT GTA CCC TTT CGC ACA ACT AAG     7245
His Val Ser Leu Glu Pro Ile Thr Cys Val Pro Phe Arg Thr Thr Lys
    2325                2330                2335

GAA CGT CAA GAG ATA CAG AAT CCA AAT TTT ACC GCA CCT GGT CAA GAA     7293
Glu Arg Gln Glu Ile Gln Asn Pro Asn Phe Thr Ala Pro Gly Gln Glu
2340                2345                2350                2355

TTT CTG TCT AAA TCT CAT TTG TAT GAA CAT CTG ACT TTG GAA AAA TCT     7341
Phe Leu Ser Lys Ser His Leu Tyr Glu His Leu Thr Leu Glu Lys Ser
                    2360                2365                2370

TCA AGC AAT TTA GCA GTT TCA GGA CAT CCA TTT TAT CAA GTT TCT GCT     7389
Ser Ser Asn Leu Ala Val Ser Gly His Pro Phe Tyr Gln Val Ser Ala
            2375                2380                2385

ACA AGA AAT GAA AAA ATG AGA CAC TTG ATT ACT ACA GGC AGA CCA ACC     7437
Thr Arg Asn Glu Lys Met Arg His Leu Ile Thr Thr Gly Arg Pro Thr
        2390                2395                2400

AAA GTC TTT GTT CCA CCT TTT AAA ACT AAA TCA CAT TTT CAC AGA GTT     7485
Lys Val Phe Val Pro Pro Phe Lys Thr Lys Ser His Phe His Arg Val
    2405                2410                2415

GAA CAG TGT GTT AGG AAT ATT AAC TTG GAG GAA AAC AGA CAA AAG CAA     7533
Glu Gln Cys Val Arg Asn Ile Asn Leu Glu Glu Asn Arg Gln Lys Gln
2420                2425                2430                2435

AAC ATT GAT GGA CAT GGC TCT GAT GAT AGT AAA AAT AAG ATT AAT GAC     7581
Asn Ile Asp Gly His Gly Ser Asp Asp Ser Lys Asn Lys Ile Asn Asp
            2440                2445                2450

AAT GAG ATT CAT CAG TTT AAC AAA AAC AAC TCC AAT CAA GCA GCA GCT     7629
Asn Glu Ile His Gln Phe Asn Lys Asn Asn Ser Asn Gln Ala Ala Ala
        2455                2460                2465

GTA ACT TTC ACA AAG TGT GAA GAA GAA CCT TTA GAT TTA ATT ACA AGT     7677
Val Thr Phe Thr Lys Cys Glu Glu Glu Pro Leu Asp Leu Ile Thr Ser
    2470                2475                2480

CTT CAG AAT GCC AGA GAT ATA CAG GAT ATG CGA ATT AAG AAG AAA CAA     7725
Leu Gln Asn Ala Arg Asp Ile Gln Asp Met Arg Ile Lys Lys Lys Gln
2485                2490                2495

AGG CAA CGC GTC TTT CCA CAG CCA GGC AGT CTG TAT CTT GCA AAA ACA     7773
Arg Gln Arg Val Phe Pro Gln Pro Gly Ser Leu Tyr Leu Ala Lys Thr
2500                2505                2510                2515

TCC ACT CTG CCT CGA ATC TCT CTG AAA GCA GCA GTA GGA GGC CAA GTT     7821
Ser Thr Leu Pro Arg Ile Ser Leu Lys Ala Ala Val Gly Gly Gln Val
            2520                2525                2530

CCC TCT GCG TGT TCT CAT AAA CAG CTG TAT ACG TAT GGC GTT TCT AAA     7869
Pro Ser Ala Cys Ser His Lys Gln Leu Tyr Thr Tyr Gly Val Ser Lys
        2535                2540                2545

CAT TGC ATA AAA ATT AAC AGC AAA AAT GCA GAG TCT TTT CAG TTT CAC     7917
His Cys Ile Lys Ile Asn Ser Lys Asn Ala Glu Ser Phe Gln Phe His
    2550                2555                2560

ACT GAA GAT TAT TTT GGT AAG GAA AGT TTA TGG ACT GGA AAA GGA ATA     7965
Thr Glu Asp Tyr Phe Gly Lys Glu Ser Leu Trp Thr Gly Lys Gly Ile
2565                2570                2575

CAG TTG GCT GAT GGT GGA TGG CTC ATA CCC TCC AAT GAT GGA AAG GCT     8013
Gln Leu Ala Asp Gly Gly Trp Leu Ile Pro Ser Asn Asp Gly Lys Ala
2580                2585                2590                2595

GGA AAA GAA GAA TTT TAT AGG GCT CTG TGT GAC ACT CCA GGT GTG GAT     8061
Gly Lys Glu Glu Phe Tyr Arg Ala Leu Cys Asp Thr Pro Gly Val Asp
            2600                2605                2610

CCA AAG CTT ATT TCT AGA ATT TGG GTT TAT AAT CAC TAT AGA TGG ATC     8109
Pro Lys Leu Ile Ser Arg Ile Trp Val Tyr Asn His Tyr Arg Trp Ile
        2615                2620                2625

ATA TGG AAA CTG GCA GCT ATG GAA TGT GCC TTT CCT AAG GAA TTT GCT     8157
Ile Trp Lys Leu Ala Ala Met Glu Cys Ala Phe Pro Lys Glu Phe Ala
    2630                2635                2640

AAT AGA TGC CTA AGC CCA GAA AGG GTG CTT CTT CAA CTA AAA TAC AGA     8205
Asn Arg Cys Leu Ser Pro Glu Arg Val Leu Leu Gln Leu Lys Tyr Arg
2645                2650                2655

TAT GAT ACG GAA ATT GAT AGA AGC AGA AGA TCG GCT ATA AAA AAG ATA     8253
Tyr Asp Thr Glu Ile Asp Arg Ser Arg Arg Ser Ala Ile Lys Lys Ile
2660                2665                2670                2675

ATG GAA AGG GAT GAC ACA GCT GCA AAA ACA CTT GTT CTC TGT GTT TCT 8301
Met Glu Arg Asp Asp Thr Ala Ala Lys Thr Leu Val Leu Cys Val Ser
                2680                2685                2690

GAC ATA ATT TCA TTG AGC GCA AAT ATA TCT GAA ACT TCT AGC AAT AAA 8349
Asp Ile Ile Ser Leu Ser Ala Asn Ile Ser Glu Thr Ser Ser Asn Lys
            2695                2700                2705

ACT AGT AGT GCA GAT ACC CAA AAA GTG GCC ATT ATT GAA CTT ACA GAT 8397
Thr Ser Ser Ala Asp Thr Gln Lys Val Ala Ile Ile Glu Leu Thr Asp
        2710                2715                2720

GGG TGG TAT GCT GTT AAG GCC CAG TTA GAT CCT CCC CTC TTA GCT GTC 8445
Gly Trp Tyr Ala Val Lys Ala Gln Leu Asp Pro Pro Leu Leu Ala Val
    2725                2730                2735

TTA AAG AAT GGC AGA CTG ACA GTT GGT CAG AAG ATT ATT CTT CAT GGA 8493
Leu Lys Asn Gly Arg Leu Thr Val Gly Gln Lys Ile Ile Leu His Gly
2740                2745                2750                2755

GCA GAA CTG GTG GGC TCT CCT GAT GCC TGT ACA CCT CTT GAA GCC CCA 8541
Ala Glu Leu Val Gly Ser Pro Asp Ala Cys Thr Pro Leu Glu Ala Pro
                2760                2765                2770

GAA TCT CTT ATG TTA AAG ATT TCT GCT AAC AGT ACT CGG CCT GCT CGC 8589
Glu Ser Leu Met Leu Lys Ile Ser Ala Asn Ser Thr Arg Pro Ala Arg
            2775                2780                2785

TGG TAT ACC AAA CTT GGA TTC TTT CCT GAC CCT AGA CCT TTT CCT CTG 8637
Trp Tyr Thr Lys Leu Gly Phe Phe Pro Asp Pro Arg Pro Phe Pro Leu
        2790                2795                2800

CCC TTA TCA TCG CTT TTC AGT GAT GGA GGA AAT GTT GGT TGT GTT GAT 8685
Pro Leu Ser Ser Leu Phe Ser Asp Gly Gly Asn Val Gly Cys Val Asp
    2805                2810                2815

GTA ATT ATT CAA AGA GCA TAC CCT ATA CAG TGG ATG GAG AAG ACA TCA 8733
Val Ile Ile Gln Arg Ala Tyr Pro Ile Gln Trp Met Glu Lys Thr Ser
2820                2825                2830                2835

TCT GGA TTA TAC ATA TTT CGC AAT GAA AGA GAG GAA GAA AAG GAA GCA 8781
Ser Gly Leu Tyr Ile Phe Arg Asn Glu Arg Glu Glu Glu Lys Glu Ala
                2840                2845                2850

GCA AAA TAT GTG GAG GCC CAA CAA AAG AGA CTA GAA GCC TTA TTC ACT 8829
Ala Lys Tyr Val Glu Ala Gln Gln Lys Arg Leu Glu Ala Leu Phe Thr
            2855                2860                2865

AAA ATT CAG GAG GAA TTT GAA GAA CAT GAA GAA AAC ACA ACA AAA CCA 8877
Lys Ile Gln Glu Glu Phe Glu Glu His Glu Glu Asn Thr Thr Lys Pro
        2870                2875                2880

TAT TTA CCA TCA CGT GCA CTA ACA AGA CAG CAA GTT CGT GCT TTG CAA 8925
Tyr Leu Pro Ser Arg Ala Leu Thr Arg Gln Gln Val Arg Ala Leu Gln
    2885                2890                2895

GAT GGT GCA GAG CTT TAT GAA GCA GTG AAG AAT GCA GCA GAC CCA GCT 8973
Asp Gly Ala Glu Leu Tyr Glu Ala Val Lys Asn Ala Ala Asp Pro Ala
2900                2905                2910                2915

TAC CTT GAG GGT TAT TTC AGT GAA GAG CAG TTA AGA GCC TTG AAT AAT 9021

Tyr Leu Glu Gly Tyr Phe Ser Glu Glu Gln Leu Arg Ala Leu Asn Asn
                2920                2925                2930

CAC AGG CAA ATG TTG AAT GAT AAG AAA CAA GCT CAG ATC CAG TTG GAA 9069
His Arg Gln Met Leu Asn Asp Lys Lys Gln Ala Gln Ile Gln Leu Glu
            2935                2940                2945

ATT AGG AAG GCC ATG GAA TCT GCT GAA CAA AAG GAA CAA GGT TTA TCA 9117
Ile Arg Lys Ala Met Glu Ser Ala Glu Gln Lys Glu Gln Gly Leu Ser
        2950                2955                2960

AGG GAT GTC ACA ACC GTG TGG AAG TTG CGT ATT GTA AGC TAT TCA AAA 9165
Arg Asp Val Thr Thr Val Trp Lys Leu Arg Ile Val Ser Tyr Ser Lys
    2965                2970                2975

AAA GAA AAA GAT TCA GTT ATA CTG AGT ATT TGG CGT CCA TCA TCA GAT 9213
Lys Glu Lys Asp Ser Val Ile Leu Ser Ile Trp Arg Pro Ser Ser Asp
2980                2985                2990                2995

TTA TAT TCT CTG TTA ACA GAA GGA AAG AGA TAC AGA ATT TAT CAT CTT 9261
Leu Tyr Ser Leu Leu Thr Glu Gly Lys Arg Tyr Arg Ile Tyr His Leu
                3000                3005                3010

GCA ACT TCA AAA TCT AAA AGT AAA TCT GAA AGA GCT AAC ATA CAG TTA 9309
Ala Thr Ser Lys Ser Lys Ser Lys Ser Glu Arg Ala Asn Ile Gln Leu
            3015                3020                3025

GCA GCG ACA AAA AAA ACT CAG TAT CAA CAA CTA CCG GTT TCA GAT GAA 9357
Ala Ala Thr Lys Lys Thr Gln Tyr Gln Gln Leu Pro Val Ser Asp Glu
        3030                3035                3040

ATT TTA TTT CAG ATT TAC CAG CCA CGG GAG CCC CTT CAC TTC AGC AAA 9405
Ile Leu Phe Gln Ile Tyr Gln Pro Arg Glu Pro Leu His Phe Ser Lys
    3045                3050                3055

TTT TTA GAT CCA GAC TTT CAG CCA TCT TGT TCT GAG GTG GAC CTA ATA 9453
Phe Leu Asp Pro Asp Phe Gln Pro Ser Cys Ser Glu Val Asp Leu Ile
3060                3065                3070                3075

GGA TTT GTC GTT TCT GTT GTG AAA AAA ACA GGA CTT GCC CCT TTC GTC 9501
Gly Phe Val Val Ser Val Val Lys Lys Thr Gly Leu Ala Pro Phe Val
                3080                3085                3090

TAT TTG TCA GAC GAA TGT TAC AAT TTA CTG GCA ATA AAG TTT TGG ATA 9549
Tyr Leu Ser Asp Glu Cys Tyr Asn Leu Leu Ala Ile Lys Phe Trp Ile
            3095                3100                3105

GAC CTT AAT GAG GAC ATT ATT AAG CCT CAT ATG TTA ATT GCT GCA AGC 9597
Asp Leu Asn Glu Asp Ile Ile Lys Pro His Met Leu Ile Ala Ala Ser
        3110                3115                3120

AAC CTC CAG TGG CGA CCA GAA TCC AAA TCA GGC CTT CTT ACT TTA TTT 9645
Asn Leu Gln Trp Arg Pro Glu Ser Lys Ser Gly Leu Leu Thr Leu Phe
    3125                3130                3135

GCT GGA GAT TTT TCT GTG TTT TCT GCT AGT CCA AAA GAG GGC CAC TTT 9693
Ala Gly Asp Phe Ser Val Phe Ser Ala Ser Pro Lys Glu Gly His Phe
3140                3145                3150                3155

CAA GAG ACA TTC AAC AAA ATG AAA AAT ACT GTT GAG AAT ATT GAC ATA 9741
Gln Glu Thr Phe Asn Lys Met Lys Asn Thr Val Glu Asn Ile Asp Ile

3160                3165                3170

CTT TGC AAT GAA GCA GAA AAC AAG CTT ATG CAT ATA CTG CAT GCA AAT     9789
Leu Cys Asn Glu Ala Glu Asn Lys Leu Met His Ile Leu His Ala Asn
            3175                3180                3185

GAT CCC AAG TGG TCC ACC CCA ACT AAA GAC TGT ACT TCA GGG CCG TAC     9837
Asp Pro Lys Trp Ser Thr Pro Thr Lys Asp Cys Thr Ser Gly Pro Tyr
        3190                3195                3200

ACT GCT CAA ATC ATT CCT GGT ACA GGA AAC AAG CTT CTG ATG TCT TCT     9885
Thr Ala Gln Ile Ile Pro Gly Thr Gly Asn Lys Leu Leu Met Ser Ser
    3205                3210                3215

CCT AAT TGT GAG ATA TAT TAT CAA AGT CCT TTA TCA CTT TGT ATG GCC     9933
Pro Asn Cys Glu Ile Tyr Tyr Gln Ser Pro Leu Ser Leu Cys Met Ala
3220                3225                3230                3235

AAA AGG AAG TCT GTT TCC ACA CCT GTC TCA GCC CAG ATG ACT TCA AAG     9981
Lys Arg Lys Ser Val Ser Thr Pro Val Ser Ala Gln Met Thr Ser Lys
                3240                3245                3250

TCT TGT AAA GGG GAG AAA GAG ATT GAT GAC CAA AAG AAC TGC AAA AAG     10029
Ser Cys Lys Gly Glu Lys Glu Ile Asp Asp Gln Lys Asn Cys Lys Lys
            3255                3260                3265

AGA AGA GCC TTG GAT TTC TTG AGT AGA CTG CCT TTA CCT CCA CCT GTT     10077
Arg Arg Ala Leu Asp Phe Leu Ser Arg Leu Pro Leu Pro Pro Pro Val
        3270                3275                3280

AGT CCC ATT TGT ACA TTT GTT TCT CCG GCT GCA CAG AAG GCA TTT CAG     10125
Ser Pro Ile Cys Thr Phe Val Ser Pro Ala Ala Gln Lys Ala Phe Gln
    3285                3290                3295

CCA CCA AGG AGT TGT GGC ACC AAA TAC GAA ACA CCC ATA AAG AAA AAA     10173
Pro Pro Arg Ser Cys Gly Thr Lys Tyr Glu Thr Pro Ile Lys Lys Lys
3300                3305                3310                3315

GAA CTG AAT TCT CCT CAG ATG ACT CCA TTT AAA AAA TTC AAT GAA ATT     10221
Glu Leu Asn Ser Pro Gln Met Thr Pro Phe Lys Lys Phe Asn Glu Ile
                3320                3325                3330

TCT CTT TTG GAA AGT AAT TCA ATA GCT GAC GAA GAA CTT GCA TTG ATA     10269
Ser Leu Leu Glu Ser Asn Ser Ile Ala Asp Glu Glu Leu Ala Leu Ile
            3335                3340                3345

AAT ACC CAA GCT CTT TTG TCT GGT TCA ACA GGA GAA AAA CAA TTT ATA     10317
Asn Thr Gln Ala Leu Leu Ser Gly Ser Thr Gly Glu Lys Gln Phe Ile
        3350                3355                3360

TCT GTC AGT GAA TCC ACT AGG ACT GCT CCC ACC AGT TCA GAA GAT TAT     10365
Ser Val Ser Glu Ser Thr Arg Thr Ala Pro Thr Ser Ser Glu Asp Tyr
    3365                3370                3375

CTC AGA CTG AAA CGA CGT TGT ACT ACA TCT CTG ATC AAA GAA CAG GAG     10413
Leu Arg Leu Lys Arg Arg Cys Thr Thr Ser Leu Ile Lys Glu Gln Glu
3380                3385                3390                3395

AGT TCC CAG GCC AGT ACG GAA GAA TGT GAG AAA AAT AAG CAG GAC ACA     10461
Ser Ser Gln Ala Ser Thr Glu Glu Cys Glu Lys Asn Lys Gln Asp Thr
                3400                3405                3410

ATT ACA ACT AAA AAA TAT ATC TAA                                                    10485
Ile Thr Thr Lys Lys Tyr Ile
            3415

(2) INFORMATION FOR SEQ ID NO:7:

(i) SEQUENCE CHARACTERISTICS:
(A) LENGTH: 3418 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:7:

Met Pro Ile Gly Ser Lys Glu Arg Pro Thr Phe Phe Glu Ile Phe Lys
1               5                   10                  15
Thr Arg Cys Asn Lys Ala Asp Leu Gly Pro Ile Ser Leu Asn Trp Phe
            20                  25                  30
Glu Glu Leu Ser Ser Glu Ala Pro Pro Tyr Asn Ser Glu Pro Ala Glu
        35                  40                  45
Glu Ser Glu His Lys Asn Asn Asn Tyr Glu Pro Asn Leu Phe Lys Thr
    50                  55                  60
Pro Gln Arg Lys Pro Ser Tyr Asn Gln Leu Ala Ser Thr Pro Ile Ile
65                  70                  75                  80
Phe Lys Glu Gln Gly Leu Thr Leu Pro Leu Tyr Gln Ser Pro Val Lys
                85                  90                  95
Glu Leu Asp Lys Phe Lys Leu Asp Leu Gly Arg Asn Val Pro Asn Ser
            100                 105                 110
Arg His Lys Ser Leu Arg Thr Val Lys Thr Lys Met Asp Gln Ala Asp
        115                 120                 125
Asp Val Ser Cys Pro Leu Leu Asn Ser Cys Leu Ser Glu Ser Pro Val
    130                 135                 140
Val Leu Gln Cys Thr His Val Thr Pro Gln Arg Asp Lys Ser Val Val
145                 150                 155                 160
Cys Gly Ser Leu Phe His Thr Pro Lys Phe Val Lys Gly Arg Gln Thr
                165                 170                 175
Pro Lys His Ile Ser Glu Ser Leu Gly Ala Glu Val Asp Pro Asp Met
            180                 185                 190
Ser Trp Ser Ser Ser Leu Ala Thr Pro Pro Thr Leu Ser Ser Thr Val
        195                 200                 205
Leu Ile Val Arg Asn Glu Glu Ala Ser Glu Thr Val Phe Pro His Asp
    210                 215                 220
Thr Thr Ala Asn Val Lys Ser Tyr Phe Ser Asn His Asp Glu Ser Leu
225                 230                 235                 240
Lys Lys Asn Asp Arg Phe Ile Ala Ser Val Thr Asp Ser Glu Asn Thr
                245                 250                 255
Asn Gln Arg Glu Ala Ala Ser His Gly Phe Gly Lys Thr Ser Gly Asn
            260                 265                 270
Ser Phe Lys Val Asn Ser Cys Lys Asp His Ile Gly Lys Ser Met Pro
        275                 280                 285
Asn Val Leu Glu Asp Glu Val Tyr Glu Thr Val Val Asp Thr Ser Glu
    290                 295                 300
Glu Asp Ser Phe Ser Leu Cys Phe Ser Lys Cys Arg Thr Lys Asn Leu
305                 310                 315                 320
Gln Lys Val Arg Thr Ser Lys Thr Arg Lys Lys Ile Phe His Glu Ala
                325                 330                 335

Asn Ala Asp Glu Cys Glu Lys Ser Lys Asn Gln Val Lys Glu Lys Tyr
            340                 345                 350
Ser Phe Val Ser Glu Val Glu Pro Asn Asp Thr Asp Pro Leu Asp Ser
        355                 360                 365
Asn Val Ala His Gln Lys Pro Phe Glu Ser Gly Ser Asp Lys Ile Ser
    370                 375                 380
Lys Glu Val Val Pro Ser Leu Ala Cys Glu Trp Ser Gln Leu Thr Leu
385                 390                 395                 400
Ser Gly Leu Asn Gly Ala Gln Met Glu Lys Ile Pro Leu Leu His Ile
                405                 410                 415
Ser Ser Cys Asp Gln Asn Ile Ser Glu Lys Asp Leu Leu Asp Thr Glu
            420                 425                 430
Asn Lys Arg Lys Lys Asp Phe Leu Thr Ser Glu Asn Ser Leu Pro Arg
        435                 440                 445
Ile Ser Ser Leu Pro Lys Ser Glu Lys Pro Leu Asn Glu Glu Thr Val
    450                 455                 460
Val Asn Lys Arg Asp Glu Glu Gln His Leu Glu Ser His Thr Asp Cys
465                 470                 475                 480
Ile Leu Ala Val Lys Gln Ala Ile Ser Gly Thr Ser Pro Val Ala Ser
                485                 490                 495
Ser Phe Gln Gly Ile Lys Lys Ser Ile Phe Arg Ile Arg Glu Ser Pro
            500                 505                 510
Lys Glu Thr Phe Asn Ala Ser Phe Ser Gly His Met Thr Asp Pro Asn
        515                 520                 525
Phe Lys Lys Glu Thr Glu Ala Ser Glu Ser Gly Leu Glu Ile His Thr
    530                 535                 540
Val Cys Ser Gln Lys Glu Asp Ser Leu Cys Pro Asn Leu Ile Asp Asn
545                 550                 555                 560
Gly Ser Trp Pro Ala Thr Thr Thr Gln Asn Ser Val Ala Leu Lys Asn
                565                 570                 575
Ala Gly Leu Ile Ser Thr Leu Lys Lys Lys Thr Asn Lys Phe Ile Tyr
            580                 585                 590
Ala Ile His Asp Glu Thr Ser Tyr Lys Gly Lys Lys Ile Pro Lys Asp
        595                 600                 605
Gln Lys Ser Glu Leu Ile Asn Cys Ser Ala Gln Phe Glu Ala Asn Ala
    610                 615                 620
Phe Glu Ala Pro Leu Thr Phe Ala Asn Ala Asp Ser Gly Leu Leu His
625                 630                 635                 640
Ser Ser Val Lys Arg Ser Cys Ser Gln Asn Asp Ser Glu Glu Pro Thr
                645                 650                 655
Leu Ser Leu Thr Ser Ser Phe Gly Thr Ile Leu Arg Lys Cys Ser Arg
            660                 665                 670
Asn Glu Thr Cys Ser Asn Asn Thr Val Ile Ser Gln Asp Leu Asp Tyr
        675                 680                 685
Lys Glu Ala Lys Cys Asn Lys Glu Lys Leu Gln Leu Phe Ile Thr Pro
    690                 695                 700
Glu Ala Asp Ser Leu Ser Cys Leu Gln Glu Gly Gln Cys Glu Asn Asp
705                 710                 715                 720
Pro Lys Ser Lys Lys Val Ser Asp Ile Lys Glu Glu Val Leu Ala Ala
                725                 730                 735
Ala Cys His Pro Val Gln His Ser Lys Val Glu Tyr Ser Asp Thr Asp
            740                 745                 750
Phe Gln Ser Gln Lys Ser Leu Leu Tyr Asp His Glu Asn Ala Ser Thr
        755                 760                 765
Leu Ile Leu Thr Pro Thr Ser Lys Asp Val Leu Ser Asn Leu Val Met
    770                 775                 780
Ile Ser Arg Gly Lys Glu Ser Tyr Lys Met Ser Asp Lys Leu Lys Gly
785                 790                 795                 800
Asn Asn Tyr Glu Ser Asp Val Glu Leu Thr Lys Asn Ile Pro Met Glu
                805                 810                 815
Lys Asn Gln Asp Val Cys Ala Leu Asn Glu Asn Tyr Lys Asn Val Glu

            820                 825                 830
Leu Leu Pro Pro Glu Lys Tyr Met Arg Val Ala Ser Pro Ser Arg Lys
        835                 840                 845
Val Gln Phe Asn Gln Asn Thr Asn Leu Arg Val Ile Gln Lys Asn Gln
    850                 855                 860
Glu Glu Thr Thr Ser Ile Ser Lys Ile Thr Val Asn Pro Asp Ser Glu
865                 870                 875                 880
Glu Leu Phe Ser Asp Asn Glu Asn Asn Phe Val Phe Gln Val Ala Asn
                885                 890                 895
Glu Arg Asn Asn Leu Ala Leu Gly Asn Thr Lys Glu Leu His Glu Thr
            900                 905                 910
Asp Leu Thr Cys Val Asn Glu Pro Ile Phe Lys Asn Ser Thr Met Val
        915                 920                 925
Leu Tyr Gly Asp Thr Gly Asp Lys Gln Ala Thr Gln Val Ser Ile Lys
    930                 935                 940
Lys Asp Leu Val Tyr Val Leu Ala Glu Glu Asn Lys Asn Ser Val Lys
945                 950                 955                 960
Gln His Ile Lys Met Thr Leu Gly Gln Asp Leu Lys Ser Asp Ile Ser
                965                 970                 975
Leu Asn Ile Asp Lys Ile Pro Glu Lys Asn Asn Asp Tyr Met Asn Lys
            980                 985                 990
Trp Ala Gly Leu Leu Gly Pro Ile Ser Asn His Ser Phe Gly Gly Ser
        995                 1000                1005
Phe Arg Thr Ala Ser Asn Lys Glu Ile Lys Leu Ser Glu His Asn Ile
    1010                1015                1020
Lys Lys Ser Lys Met Phe Phe Lys Asp Ile Glu Glu Gln Tyr Pro Thr
1025 1030 1035 104
Ser Leu Ala Cys Val Glu Ile Val Asn Thr Leu Ala Leu Asp Asn Gln
                1045                1050                1055
Lys Lys Leu Ser Lys Pro Gln Ser Ile Asn Thr Val Ser Ala His Leu
            1060                1065                1070
Gln Ser Ser Val Val Val Ser Asp Cys Lys Asn Ser His Ile Thr Pro
        1075                1080                1085
Gln Met Leu Phe Ser Lys Gln Asp Phe Asn Ser Asn His Asn Leu Thr
    1090                1095                1100
Pro Ser Gln Lys Ala Glu Ile Thr Glu Leu Ser Thr Ile Leu Glu Glu
1105 1110 1115 112
Ser Gly Ser Gln Phe Glu Phe Thr Gln Phe Arg Xaa Pro Ser Tyr Ile
                1125                1130                1135
Leu Gln Lys Ser Thr Phe Glu Val Pro Glu Asn Gln Met Thr Ile Leu
            1140                1145                1150
Lys Thr Thr Ser Glu Glu Cys Arg Asp Ala Asp Leu His Val Ile Met
        1155                1160                1165
Asn Ala Pro Ser Ile Gly Gln Val Asp Ser Ser Lys Gln Phe Glu Gly
    1170                1175                1180
Thr Val Glu Ile Lys Arg Lys Phe Ala Gly Leu Leu Lys Asn Asp Cys
1185 1190 1195 120
Asn Lys Ser Ala Ser Gly Tyr Leu Thr Asp Glu Asn Glu Val Gly Phe
                1205                1210                1215
Arg Gly Phe Tyr Ser Ala His Gly Thr Lys Leu Asn Val Ser Thr Glu
            1220                1225                1230
Ala Leu Gln Lys Ala Val Lys Leu Phe Ser Asp Ile Glu Asn Ile Ser
        1235                1240                1245
Glu Glu Thr Ser Ala Glu Val His Pro Ile Ser Leu Ser Ser Ser Lys
    1250                1255                1260
Cys His Asp Ser Val Val Ser Met Phe Lys Ile Glu Asn His Asn Asp
1265 1270 1275 128
Lys Thr Val Ser Glu Lys Asn Asn Lys Cys Gln Leu Ile Leu Gln Asn
                1285                1290                1295
Asn Ile Glu Met Thr Thr Gly Thr Phe Val Glu Glu Ile Thr Glu Asn
            1300                1305                1310

Tyr Lys Arg Asn Thr Glu Asn Glu Asp Asn Lys Tyr Thr Ala Ala Ser
        1315                1320                1325
Arg Asn Ser His Asn Leu Glu Phe Asp Gly Ser Asp Ser Ser Lys Asn
    1330                1335                1340
Asp Thr Val Cys Ile His Lys Asp Glu Thr Asp Leu Leu Phe Thr Asp
1345                1350                1355                136
Gln His Asn Ile Cys Leu Lys Leu Ser Gly Gln Phe Met Lys Glu Gly
                1365                1370                1375
Asn Thr Gln Ile Lys Glu Asp Leu Ser Asp Leu Thr Phe Leu Glu Val
            1380                1385                1390
Ala Lys Ala Gln Glu Ala Cys His Gly Asn Thr Ser Asn Lys Glu Gln
        1395                1400                1405
Leu Thr Ala Thr Lys Thr Glu Gln Asn Ile Lys Asp Phe Glu Thr Ser
    1410                1415                1420
Asp Thr Phe Phe Gln Thr Ala Ser Gly Lys Asn Ile Ser Val Ala Lys
1425                1430                1435                144
Glu Ser Phe Asn Lys Ile Val Asn Phe Phe Asp Gln Lys Pro Glu Glu
                1445                1450                1455
Leu His Asn Phe Ser Leu Asn Ser Glu Leu His Ser Asp Ile Arg Lys
            1460                1465                1470
Asn Lys Met Asp Ile Leu Ser Tyr Glu Glu Thr Asp Ile Val Lys His
        1475                1480                1485
Lys Ile Leu Lys Glu Ser Val Pro Val Gly Thr Gly Asn Gln Leu Val
    1490                1495                1500
Thr Phe Gln Gly Gln Pro Glu Arg Asp Glu Lys Ile Lys Glu Pro Thr
1505                1510                1515                152
Leu Leu Gly Phe His Thr Ala Ser Gly Lys Lys Val Lys Ile Ala Lys
                1525                1530                1535
Glu Ser Leu Asp Lys Val Lys Asn Leu Phe Asp Glu Lys Glu Gln Gly
            1540                1545                1550
Thr Ser Glu Ile Thr Ser Phe Ser His Gln Trp Ala Lys Thr Leu Lys
        1555                1560                1565
Tyr Arg Glu Ala Cys Lys Asp Leu Glu Leu Ala Cys Glu Thr Ile Glu
    1570                1575                1580
Ile Thr Ala Ala Pro Lys Cys Lys Glu Met Gln Asn Ser Leu Asn Asn
1585                1590                1595                160
Asp Lys Asn Leu Val Ser Ile Glu Thr Val Val Pro Pro Lys Leu Leu
                1605                1610                1615
Ser Asp Asn Leu Cys Arg Gln Thr Glu Asn Leu Lys Thr Ser Lys Ser
            1620                1625                1630
Ile Phe Leu Lys Val Lys Val His Glu Asn Val Glu Lys Glu Thr Ala
        1635                1640                1645
Lys Ser Pro Ala Thr Cys Tyr Thr Asn Gln Ser Pro Tyr Ser Val Ile
    1650                1655                1660
Glu Asn Ser Ala Leu Ala Phe Tyr Thr Ser Cys Ser Arg Lys Thr Ser
1665                1670                1675                168
Val Ser Gln Thr Ser Leu Leu Glu Ala Lys Lys Trp Leu Arg Glu Gly
                1685                1690                1695
Ile Phe Asp Gly Gln Pro Glu Arg Ile Asn Thr Ala Asp Tyr Val Gly
            1700                1705                1710
Asn Tyr Leu Tyr Glu Asn Asn Ser Asn Ser Thr Ile Ala Glu Asn Asp
        1715                1720                1725
Lys Asn His Leu Ser Glu Lys Gln Asp Thr Tyr Leu Ser Asn Ser Ser
    1730                1735                1740
Met Ser Asn Ser Tyr Ser Tyr His Ser Asp Glu Val Tyr Asn Asp Ser
1745                1750                1755                176
Gly Tyr Leu Ser Lys Asn Lys Leu Asp Ser Gly Ile Glu Pro Val Leu
                1765                1770                1775
Lys Asn Val Glu Asp Gln Lys Asn Thr Ser Phe Ser Lys Val Ile Ser
            1780                1785                1790
Asn Val Lys Asp Ala Asn Ala Tyr Pro Gln Thr Val Asn Glu Asp Ile

1795                1800                1805
Cys Val Glu Glu Leu Val Thr Ser Ser Ser Pro Cys Lys Asn Lys Asn
    1810                1815                1820
Ala Ala Ile Lys Leu Ser Ile Ser Asn Ser Asn Asn Phe Glu Val Gly
1825                1830                1835                184
Pro Pro Ala Phe Arg Ile Ala Ser Gly Lys Ile Val Cys Val Ser His
                1845                1850                1855
Glu Thr Ile Lys Lys Val Lys Asp Ile Phe Thr Asp Ser Phe Ser Lys
            1860                1865                1870
Val Ile Lys Glu Asn Asn Glu Asn Lys Ser Lys Ile Cys Gln Thr Lys
        1875                1880                1885
Ile Met Ala Gly Cys Tyr Glu Ala Leu Asp Asp Ser Glu Asp Ile Leu
    1890                1895                1900
His Asn Ser Leu Asp Asn Asp Glu Cys Ser Thr His Ser His Lys Val
1905                1910                1915                192
Phe Ala Asp Ile Gln Ser Glu Glu Ile Leu Gln His Asn Gln Asn Met
                1925                1930                1935
Ser Gly Leu Glu Lys Val Ser Lys Ile Ser Pro Cys Asp Val Ser Leu
            1940                1945                1950
Glu Thr Ser Asp Ile Cys Lys Cys Ser Ile Gly Lys Leu His Lys Ser
        1955                1960                1965
Val Ser Ser Ala Asn Thr Cys Gly Ile Phe Ser Thr Ala Ser Gly Lys
    1970                1975                1980
Ser Val Gln Val Ser Asp Ala Ser Leu Gln Asn Ala Arg Gln Val Phe
1985                1990                1995                200
Ser Glu Ile Glu Asp Ser Thr Lys Gln Val Phe Ser Lys Val Leu Phe
                2005                2010                2015
Lys Ser Asn Glu His Ser Asp Gln Leu Thr Arg Glu Glu Asn Thr Ala
            2020                2025                2030
Ile Arg Thr Pro Glu His Leu Ile Ser Gln Lys Gly Phe Ser Tyr Asn
        2035                2040                2045
Val Val Asn Ser Ser Ala Phe Ser Gly Phe Ser Thr Ala Ser Gly Lys
    2050                2055                2060
Gln Val Ser Ile Leu Glu Ser Ser Leu His Lys Val Lys Gly Val Leu
2065                2070                2075                208
Glu Glu Phe Asp Leu Ile Arg Thr Glu His Ser Leu His Tyr Ser Pro
                2085                2090                2095
Thr Ser Arg Gln Asn Val Ser Lys Ile Leu Pro Arg Val Asp Lys Arg
            2100                2105                2110
Asn Pro Glu His Cys Val Asn Ser Glu Met Glu Lys Thr Cys Ser Lys
        2115                2120                2125
Glu Phe Lys Leu Ser Asn Asn Leu Asn Val Glu Gly Gly Ser Ser Glu
    2130                2135                2140
Asn Asn His Ser Ile Lys Val Ser Pro Tyr Leu Ser Gln Phe Gln Gln
2145                2150                2155                216
Asp Lys Gln Gln Leu Val Leu Gly Thr Lys Val Ser Leu Val Glu Asn
                2165                2170                2175
Ile His Val Leu Gly Lys Glu Gln Ala Ser Pro Lys Asn Val Lys Met
            2180                2185                2190
Glu Ile Gly Lys Thr Glu Thr Phe Ser Asp Val Pro Val Lys Thr Asn
        2195                2200                2205
Ile Glu Val Cys Ser Thr Tyr Ser Lys Asp Ser Glu Asn Tyr Phe Glu
    2210                2215                2220
Thr Glu Ala Val Glu Ile Ala Lys Ala Phe Met Glu Asp Asp Glu Leu
2225                2230                2235                224
Thr Asp Ser Lys Leu Pro Ser His Ala Thr His Ser Leu Phe Thr Cys
                2245                2250                2255
Pro Glu Asn Glu Glu Met Val Leu Ser Asn Ser Arg Ile Gly Lys Arg
            2260                2265                2270
Arg Gly Glu Pro Leu Ile Leu Val Gly Glu Pro Ser Ile Lys Arg Asn
        2275                2280                2285

Leu Leu Asn Glu Phe Asp Arg Ile Ile Glu Asn Gln Glu Lys Ser Leu
    2290                2295                2300
Lys Ala Ser Lys Ser Thr Pro Asp Gly Thr Ile Lys Asp Arg Arg Leu
2305                2310                2315                232
Phe Met His His Val Ser Leu Glu Pro Ile Thr Cys Val Pro Phe Arg
                2325                2330                2335
Thr Thr Lys Glu Arg Gln Glu Ile Gln Asn Pro Asn Phe Thr Ala Pro
            2340                2345                2350
Gly Gln Glu Phe Leu Ser Lys Ser His Leu Tyr Glu His Leu Thr Leu
        2355                2360                2365
Glu Lys Ser Ser Ser Asn Leu Ala Val Ser Gly His Pro Phe Tyr Gln
    2370                2375                2380
Val Ser Ala Thr Arg Asn Glu Lys Met Arg His Leu Ile Thr Thr Gly
2385                2390                2395                240
Arg Pro Thr Lys Val Phe Val Pro Pro Phe Lys Thr Lys Ser His Phe
                2405                2410                2415
His Arg Val Glu Gln Cys Val Arg Asn Ile Asn Leu Glu Glu Asn Arg
            2420                2425                2430
Gln Lys Gln Asn Ile Asp Gly His Gly Ser Asp Asp Ser Lys Asn Lys
        2435                2440                2445
Ile Asn Asp Asn Glu Ile His Gln Phe Asn Lys Asn Asn Ser Asn Gln
    2450                2455                2460
Ala Ala Ala Val Thr Phe Thr Lys Cys Glu Glu Glu Pro Leu Asp Leu
2465                2470                2475                248
Ile Thr Ser Leu Gln Asn Ala Arg Asp Ile Gln Asp Met Arg Ile Lys
                2485                2490                2495
Lys Lys Gln Arg Gln Arg Val Phe Pro Gln Pro Gly Ser Leu Tyr Leu
            2500                2505                2510
Ala Lys Thr Ser Thr Leu Pro Arg Ile Ser Leu Lys Ala Ala Val Gly
        2515                2520                2525
Gly Gln Val Pro Ser Ala Cys Ser His Lys Gln Leu Tyr Thr Tyr Gly
    2530                2535                2540
Val Ser Lys His Cys Ile Lys Ile Asn Ser Lys Asn Ala Glu Ser Phe
2545                2550                2555                256
Gln Phe His Thr Glu Asp Tyr Phe Gly Lys Glu Ser Leu Trp Thr Gly
                2565                2570                2575
Lys Gly Ile Gln Leu Ala Asp Gly Gly Trp Leu Ile Pro Ser Asn Asp
            2580                2585                2590
Gly Lys Ala Gly Lys Glu Glu Phe Tyr Arg Ala Leu Cys Asp Thr Pro
        2595                2600                2605
Gly Val Asp Pro Lys Leu Ile Ser Arg Ile Trp Val Tyr Asn His Tyr
    2610                2615                2620
Arg Trp Ile Ile Trp Lys Leu Ala Ala Met Glu Cys Ala Phe Pro Lys
2625                2630                2635                264
Glu Phe Ala Asn Arg Cys Leu Ser Pro Glu Arg Val Leu Leu Gln Leu
                2645                2650                2655
Lys Tyr Arg Tyr Asp Thr Glu Ile Asp Arg Ser Arg Arg Ser Ala Ile
            2660                2665                2670
Lys Lys Ile Met Glu Arg Asp Asp Thr Ala Ala Lys Thr Leu Val Leu
        2675                2680                2685
Cys Val Ser Asp Ile Ile Ser Leu Ser Ala Asn Ile Ser Glu Thr Ser
    2690                2695                2700
Ser Asn Lys Thr Ser Ser Ala Asp Thr Gln Lys Val Ala Ile Ile Glu
2705                2710                2715                272
Leu Thr Asp Gly Trp Tyr Ala Val Lys Ala Gln Leu Asp Pro Pro Leu
                2725                2730                2735
Leu Ala Val Leu Lys Asn Gly Arg Leu Thr Val Gly Gln Lys Ile Ile
            2740                2745                2750
Leu His Gly Ala Glu Leu Val Gly Ser Pro Asp Ala Cys Thr Pro Leu
        2755                2760                2765
Glu Ala Pro Glu Ser Leu Met Leu Lys Ile Ser Ala Asn Ser Thr Arg

2770                    2775                    2780
Pro Ala Arg Trp Tyr Thr Lys Leu Gly Phe Phe Pro Asp Pro Arg Pro
2785                    2790                    2795                    280
Phe Pro Leu Pro Leu Ser Ser Leu Phe Ser Asp Gly Gly Asn Val Gly
                2805                    2810                    2815
Cys Val Asp Val Ile Ile Gln Arg Ala Tyr Pro Ile Gln Trp Met Glu
            2820                    2825                    2830
Lys Thr Ser Ser Gly Leu Tyr Ile Phe Arg Asn Glu Arg Glu Glu Glu
        2835                    2840                    2845
Lys Glu Ala Ala Lys Tyr Val Glu Ala Gln Gln Lys Arg Leu Glu Ala
    2850                    2855                    2860
Leu Phe Thr Lys Ile Gln Glu Glu Phe Glu Glu His Glu Glu Asn Thr
2865                    2870                    2875                    288
Thr Lys Pro Tyr Leu Pro Ser Arg Ala Leu Thr Arg Gln Gln Val Arg
                2885                    2890                    2895
Ala Leu Gln Asp Gly Ala Glu Leu Tyr Glu Ala Val Lys Asn Ala Ala
            2900                    2905                    2910
Asp Pro Ala Tyr Leu Glu Gly Tyr Phe Ser Glu Glu Gln Leu Arg Ala
        2915                    2920                    2925
Leu Asn Asn His Arg Gln Met Leu Asn Asp Lys Lys Gln Ala Gln Ile
    2930                    2935                    2940
Gln Leu Glu Ile Arg Lys Ala Met Glu Ser Ala Glu Gln Lys Glu Gln
2945                    2950                    2955                    296
Gly Leu Ser Arg Asp Val Thr Thr Val Trp Lys Leu Arg Ile Val Ser
                2965                    2970                    2975
Tyr Ser Lys Lys Glu Lys Asp Ser Val Ile Leu Ser Ile Trp Arg Pro
            2980                    2985                    2990
Ser Ser Asp Leu Tyr Ser Leu Leu Thr Glu Gly Lys Arg Tyr Arg Ile
        2995                    3000                    3005
Tyr His Leu Ala Thr Ser Lys Ser Lys Ser Lys Ser Glu Arg Ala Asn
    3010                    3015                    3020
Ile Gln Leu Ala Ala Thr Lys Lys Thr Gln Tyr Gln Gln Leu Pro Val
3025                    3030                    3035                    304
Ser Asp Glu Ile Leu Phe Gln Ile Tyr Gln Pro Arg Glu Pro Leu His
                3045                    3050                    3055
Phe Ser Lys Phe Leu Asp Pro Asp Phe Gln Pro Ser Cys Ser Glu Val
            3060                    3065                    3070
Asp Leu Ile Gly Phe Val Val Ser Val Val Lys Lys Thr Gly Leu Ala
        3075                    3080                    3085
Pro Phe Val Tyr Leu Ser Asp Glu Cys Tyr Asn Leu Leu Ala Ile Lys
    3090                    3095                    3100
Phe Trp Ile Asp Leu Asn Glu Asp Ile Ile Lys Pro His Met Leu Ile
3105                    3110                    3115                    312
Ala Ala Ser Asn Leu Gln Trp Arg Pro Glu Ser Lys Ser Gly Leu Leu
                3125                    3130                    3135
Thr Leu Phe Ala Gly Asp Phe Ser Val Phe Ser Ala Ser Pro Lys Glu
            3140                    3145                    3150
Gly His Phe Gln Glu Thr Phe Asn Lys Met Lys Asn Thr Val Glu Asn
        3155                    3160                    3165
Ile Asp Ile Leu Cys Asn Glu Ala Glu Asn Lys Leu Met His Ile Leu
    3170                    3175                    3180
His Ala Asn Asp Pro Lys Trp Ser Thr Pro Thr Lys Asp Cys Thr Ser
3185                    3190                    3195                    320
Gly Pro Tyr Thr Ala Gln Ile Ile Pro Gly Thr Gly Asn Lys Leu Leu
                3205                    3210                    3215
Met Ser Ser Pro Asn Cys Glu Ile Tyr Tyr Gln Ser Pro Leu Ser Leu
            3220                    3225                    3230
Cys Met Ala Lys Arg Lys Ser Val Ser Thr Pro Val Ser Ala Gln Met
        3235                    3240                    3245
Thr Ser Lys Ser Cys Lys Gly Glu Lys Glu Ile Asp Asp Gln Lys Asn
    3250                    3255                    3260

Cys Lys Lys Arg Arg Ala Leu Asp Phe Leu Ser Arg Leu Pro Leu Pro
3265                3270                3275                328
Pro Pro Val Ser Pro Ile Cys Thr Phe Val Ser Pro Ala Ala Gln Lys
                3285                3290                3295
Ala Phe Gln Pro Pro Arg Ser Cys Gly Thr Lys Tyr Glu Thr Pro Ile
            3300                3305                3310
Lys Lys Lys Glu Leu Asn Ser Pro Gln Met Thr Pro Phe Lys Lys Phe
        3315                3320                3325
Asn Glu Ile Ser Leu Leu Glu Ser Asn Ser Ile Ala Asp Glu Glu Leu
    3330                3335                3340
Ala Leu Ile Asn Thr Gln Ala Leu Leu Ser Gly Ser Thr Gly Glu Lys
3345                3350                3355                336
Gln Phe Ile Ser Val Ser Glu Ser Thr Arg Thr Ala Pro Thr Ser Ser
                3365                3370                3375
Glu Asp Tyr Leu Arg Leu Lys Arg Arg Cys Thr Thr Ser Leu Ile Lys
            3380                3385                3390
Glu Gln Glu Ser Ser Gln Ala Ser Thr Glu Glu Cys Glu Lys Asn Lys
        3395                3400                3405
Gln Asp Thr Ile Thr Thr Lys Lys Tyr Ile
    3410                3415

(2) INFORMATION FOR SEQ ID NO:8:

(i) SEQUENCE CHARACTERISTICS:
(A) LENGTH: 10485 base pairs
(B) TYPE: nucleic acid
(C) STRANDEDNESS: double
(D) TOPOLOGY: linear

(ii) MOLECULE TYPE: cDNA
(ix) FEATURE:

(A) NAME/KEY: Coding Sequence
(B) LOCATION: 229...10482
(D) OTHER INFORMATION: BRCA2 (OMI3)

(xi) SEQUENCE DESCRIPTION: SEQ ID NO:8:

GGTGGCGCGA GCTTCTGAAA CTAGGCGGCA GAGGCGGAGC CGCTGTGGCA CTGCTGCGCC     60
TCTGCTGCGC CTCGGGTGTC TTTTGCGGCG GTGGGTCGCC GCCGGGAGAA GCGTGAGGGG    120
ACAGATTTGT GACCGGCGCG GTTTTTGTCA GCTTACTCCG GCCAAAAAAG AACTGCACCT    180
CTGGAGCGGA CTTATTTACC AAGCATTGGA GGAATATCGT AGGTAAAA ATG CCT ATT    237
                                                     Met Pro Ile
                                                       1

GGA TCC AAA GAG AGG CCA ACA TTT TTT GAA ATT TTT AAG ACA CGC TGC      285
Gly Ser Lys Glu Arg Pro Thr Phe Phe Glu Ile Phe Lys Thr Arg Cys
    5                   10                  15

AAC AAA GCA GAT TTA GGA CCA ATA AGT CTT AAT TGG TTT GAA GAA CTT      333
Asn Lys Ala Asp Leu Gly Pro Ile Ser Leu Asn Trp Phe Glu Glu Leu
20                  25                  30                  35

TCT TCA GAA GCT CCA CCC TAT AAT TCT GAA CCT GCA GAA GAA TCT GAA      381
Ser Ser Glu Ala Pro Pro Tyr Asn Ser Glu Pro Ala Glu Glu Ser Glu
                40                  45                  50

CAT AAA AAC AAC AAT TAC GAA CCA AAC CTA TTT AAA ACT CCA CAA AGG      429
His Lys Asn Asn Asn Tyr Glu Pro Asn Leu Phe Lys Thr Pro Gln Arg
            55                  60                  65

AAA CCA TCT TAT AAT CAG CTG GCT TCA ACT CCA ATA ATA TTC AAA GAG 477
Lys Pro Ser Tyr Asn Gln Leu Ala Ser Thr Pro Ile Ile Phe Lys Glu
        70                  75                  80

CAA GGG CTG ACT CTG CCG CTG TAC CAA TCT CCT GTA AAA GAA TTA GAT 525
Gln Gly Leu Thr Leu Pro Leu Tyr Gln Ser Pro Val Lys Glu Leu Asp
    85                  90                  95

AAA TTC AAA TTA GAC TTA GGA AGG AAT GTT CCC AAT AGT AGA CAT AAA 573
Lys Phe Lys Leu Asp Leu Gly Arg Asn Val Pro Asn Ser Arg His Lys
100                 105                 110                 115

AGT CTT CGC ACA GTG AAA ACT AAA ATG GAT CAA GCA GAT GAT GTT TCC 621
Ser Leu Arg Thr Val Lys Thr Lys Met Asp Gln Ala Asp Asp Val Ser
                120                 125                 130

TGT CCA CTT CTA AAT TCT TGT CTT AGT GAA AGT CCT GTT GTT CTA CAA 669
Cys Pro Leu Leu Asn Ser Cys Leu Ser Glu Ser Pro Val Val Leu Gln
            135                 140                 145

TGT ACA CAT GTA ACA CCA CAA AGA GAT AAG TCA GTG GTA TGT GGG AGT 717
Cys Thr His Val Thr Pro Gln Arg Asp Lys Ser Val Val Cys Gly Ser
        150                 155                 160

TTG TTT CAT ACA CCA AAG TTT GTG AAG GGT CGT CAG ACA CCA AAA CAT 765
Leu Phe His Thr Pro Lys Phe Val Lys Gly Arg Gln Thr Pro Lys His
    165                 170                 175

ATT TCT GAA AGT CTA GGA GCT GAG GTG GAT CCT GAT ATG TCT TGG TCA 813
Ile Ser Glu Ser Leu Gly Ala Glu Val Asp Pro Asp Met Ser Trp Ser
180                 185                 190                 195

AGT TCT TTA GCT ACA CCA CCC ACC CTT AGT TCT ACT GTG CTC ATA GTC 861
Ser Ser Leu Ala Thr Pro Pro Thr Leu Ser Ser Thr Val Leu Ile Val
                200                 205                 210

AGA AAT GAA GAA GCA TCT GAA ACT GTA TTT CCT CAT GAT ACT ACT GCT 909
Arg Asn Glu Glu Ala Ser Glu Thr Val Phe Pro His Asp Thr Thr Ala
            215                 220                 225

AAT GTG AAA AGC TAT TTT TCC AAT CAT GAT GAA AGT CTG AAG AAA AAT 957
Asn Val Lys Ser Tyr Phe Ser Asn His Asp Glu Ser Leu Lys Lys Asn
        230                 235                 240

GAT AGA TTT ATC GCT TCT GTG ACA GAC AGT GAA AAC ACA AAT CAA AGA 1005
Asp Arg Phe Ile Ala Ser Val Thr Asp Ser Glu Asn Thr Asn Gln Arg
    245                 250                 255

GAA GCT GCA AGT CAT GGA TTT GGA AAA ACA TCA GGG AAT TCA TTT AAA 1053
Glu Ala Ala Ser His Gly Phe Gly Lys Thr Ser Gly Asn Ser Phe Lys
260                 265                 270                 275

GTA AAT AGC TGC AAA GAC CAC ATT GGA AAG TCA ATG CCA CAT GTC CTA 1101
Val Asn Ser Cys Lys Asp His Ile Gly Lys Ser Met Pro His Val Leu
                280                 285                 290

GAA GAT GAA GTA TAT GAA ACA GTT GTA GAT ACC TCT GAA GAA GAT AGT 1149
Glu Asp Glu Val Tyr Glu Thr Val Val Asp Thr Ser Glu Glu Asp Ser
            295                 300                 305

TTT TCA TTA TGT TTT TCT AAA TGT AGA ACA AAA AAT CTA CAA AAA GTA 1197

Phe Ser Leu Cys Phe Ser Lys Cys Arg Thr Lys Asn Leu Gln Lys Val
        310                 315                 320

AGA ACT AGC AAG ACT AGG AAA AAA ATT TTC CAT GAA GCA AAC GCT GAT 1245
Arg Thr Ser Lys Thr Arg Lys Lys Ile Phe His Glu Ala Asn Ala Asp
    325                 330                 335

GAA TGT GAA AAA TCT AAA AAC CAA GTG AAA GAA AAA TAC TCA TTT GTA 1293
Glu Cys Glu Lys Ser Lys Asn Gln Val Lys Glu Lys Tyr Ser Phe Val
340                 345                 350                 355

TCT GAA GTG GAA CCA AAT GAT ACT GAT CCA TTA GAT TCA AAT GTA GCA 1341
Ser Glu Val Glu Pro Asn Asp Thr Asp Pro Leu Asp Ser Asn Val Ala
                360                 365                 370

AAT CAG AAG CCC TTT GAG AGT GGA AGT GAC AAA ATC TCC AAG GAA GTT 1389
Asn Gln Lys Pro Phe Glu Ser Gly Ser Asp Lys Ile Ser Lys Glu Val
            375                 380                 385

GTA CCG TCT TTG GCC TGT GAA TGG TCT CAA CTA ACC CTT TCA GGT CTA 1437
Val Pro Ser Leu Ala Cys Glu Trp Ser Gln Leu Thr Leu Ser Gly Leu
        390                 395                 400

AAT GGA GCC CAG ATG GAG AAA ATA CCC CTA TTG CAT ATT TCT TCA TGT 1485
Asn Gly Ala Gln Met Glu Lys Ile Pro Leu Leu His Ile Ser Ser Cys
    405                 410                 415

GAC CAA AAT ATT TCA GAA AAA GAC CTA TTA GAC ACA GAG AAC AAA AGA 1533
Asp Gln Asn Ile Ser Glu Lys Asp Leu Leu Asp Thr Glu Asn Lys Arg
420                 425                 430                 435

AAG AAA GAT TTT CTT ACT TCA GAG AAT TCT TTG CCA CGT ATT TCT AGC 1581
Lys Lys Asp Phe Leu Thr Ser Glu Asn Ser Leu Pro Arg Ile Ser Ser
                440                 445                 450

CTA CCA AAA TCA GAG AAG CCA TTA AAT GAG GAA ACA GTG GTA AAT AAG 1629
Leu Pro Lys Ser Glu Lys Pro Leu Asn Glu Glu Thr Val Val Asn Lys
            455                 460                 465

AGA GAT GAA GAG CAG CAT CTT GAA TCT CAT ACA GAC TGC ATT CTT GCA 1677
Arg Asp Glu Glu Gln His Leu Glu Ser His Thr Asp Cys Ile Leu Ala
        470                 475                 480

GTA AAG CAG GCA ATA TCT GGA ACT TCT CCA GTG GCT TCT TCA TTT CAG 1725
Val Lys Gln Ala Ile Ser Gly Thr Ser Pro Val Ala Ser Ser Phe Gln
    485                 490                 495

GGT ATC AAA AAG TCT ATA TTC AGA ATA AGA GAA TCA CCT AAA GAG ACT 1773
Gly Ile Lys Lys Ser Ile Phe Arg Ile Arg Glu Ser Pro Lys Glu Thr
500                 505                 510                 515

TTC AAT GCA AGT TTT TCA GGT CAT ATG ACT GAT CCA AAC TTT AAA AAA 1821
Phe Asn Ala Ser Phe Ser Gly His Met Thr Asp Pro Asn Phe Lys Lys
                520                 525                 530

GAA ACT GAA GCC TCT GAA AGT GGA CTG GAA ATA CAT ACT GTT TGC TCA 1869
Glu Thr Glu Ala Ser Glu Ser Gly Leu Glu Ile His Thr Val Cys Ser
            535                 540                 545

CAG AAG GAG GAC TCC TTA TGT CCA AAT TTA ATT GAT AAT GGA AGC TGG 1917
Gln Lys Glu Asp Ser Leu Cys Pro Asn Leu Ile Asp Asn Gly Ser Trp

        550                 555                 560

CCA GCC ACC ACC ACA CAG AAT TCT GTA GCT TTG AAG AAT GCA GGT TTA 1965
Pro Ala Thr Thr Thr Gln Asn Ser Val Ala Leu Lys Asn Ala Gly Leu
    565                 570                 575

ATA TCC ACT TTG AAA AAG AAA ACA AAT AAG TTT ATT TAT GCT ATA CAT 2013
Ile Ser Thr Leu Lys Lys Lys Thr Asn Lys Phe Ile Tyr Ala Ile His
580                 585                 590                 595

GAT GAA ACA TCT TAT AAA GGA AAA AAA ATA CCG AAA GAC CAA AAA TCA 2061
Asp Glu Thr Ser Tyr Lys Gly Lys Lys Ile Pro Lys Asp Gln Lys Ser
                600                 605                 610

GAA CTA ATT AAC TGT TCA GCC CAG TTT GAA GCA AAT GCT TTT GAA GCA 2109
Glu Leu Ile Asn Cys Ser Ala Gln Phe Glu Ala Asn Ala Phe Glu Ala
            615                 620                 625

CCA CTT ACA TTT GCA AAT GCT GAT TCA GGT TTA TTG CAT TCT TCT GTG 2157
Pro Leu Thr Phe Ala Asn Ala Asp Ser Gly Leu Leu His Ser Ser Val
        630                 635                 640

AAA AGA AGC TGT TCA CAG AAT GAT TCT GAA GAA CCA ACT TTG TCC TTA 2205
Lys Arg Ser Cys Ser Gln Asn Asp Ser Glu Glu Pro Thr Leu Ser Leu
    645                 650                 655

ACT AGC TCT TTT GGG ACA ATT CTG AGG AAA TGT TCT AGA AAT GAA ACA 2253
Thr Ser Ser Phe Gly Thr Ile Leu Arg Lys Cys Ser Arg Asn Glu Thr
660                 665                 670                 675

TGT TCT AAT AAT ACA GTA ATC TCT CAG GAT CTT GAT TAT AAA GAA GCA 2301
Cys Ser Asn Asn Thr Val Ile Ser Gln Asp Leu Asp Tyr Lys Glu Ala
                680                 685                 690

AAA TGT AAT AAG GAA AAA CTA CAG TTA TTT ATT ACC CCA GAA GCT GAT 2349
Lys Cys Asn Lys Glu Lys Leu Gln Leu Phe Ile Thr Pro Glu Ala Asp
            695                 700                 705

TCT CTG TCA TGC CTG CAG GAA GGA CAG TGT GAA AAT GAT CCA AAA AGC 2397
Ser Leu Ser Cys Leu Gln Glu Gly Gln Cys Glu Asn Asp Pro Lys Ser
        710                 715                 720

AAA AAA GTT TCA GAT ATA AAA GAA GAG GTC TTG GCT GCA GCA TGT CAC 2445
Lys Lys Val Ser Asp Ile Lys Glu Glu Val Leu Ala Ala Ala Cys His
    725                 730                 735

CCA GTA CAA CAC TCA AAA GTG GAA TAC AGT GAT ACT GAC TTT CAA TCC 2493
Pro Val Gln His Ser Lys Val Glu Tyr Ser Asp Thr Asp Phe Gln Ser
740                 745                 750                 755

CAG AAA AGT CTT TTA TAT GAT CAT GAA AAT GCC AGC ACT CTT ATT TTA 2541
Gln Lys Ser Leu Leu Tyr Asp His Glu Asn Ala Ser Thr Leu Ile Leu
                760                 765                 770

ACT CCT ACT TCC AAG GAT GTT CTG TCA AAC CTA GTC ATG ATT TCT AGA 2589
Thr Pro Thr Ser Lys Asp Val Leu Ser Asn Leu Val Met Ile Ser Arg
            775                 780                 785

GGC AAA GAA TCA TAC AAA ATG TCA GAC AAG CTC AAA GGT AAC AAT TAT 2637
Gly Lys Glu Ser Tyr Lys Met Ser Asp Lys Leu Lys Gly Asn Asn Tyr
        790                 795                 800

GAA TCT GAT GTT GAA TTA ACC AAA AAT ATT CCC ATG GAA AAG AAT CAA 2685
Glu Ser Asp Val Glu Leu Thr Lys Asn Ile Pro Met Glu Lys Asn Gln
    805                 810                 815

GAT GTA TGT GCT TTA AAT GAA AAT TAT AAA AAC GTT GAG CTG TTG CCA 2733
Asp Val Cys Ala Leu Asn Glu Asn Tyr Lys Asn Val Glu Leu Leu Pro
820                 825                 830                 835

CCT GAA AAA TAC ATG AGA GTA GCA TCA CCT TCA AGA AAG GTA CAA TTC 2781
Pro Glu Lys Tyr Met Arg Val Ala Ser Pro Ser Arg Lys Val Gln Phe
                840                 845                 850

AAC CAA AAC ACA AAT CTA AGA GTA ATC CAA AAA AAT CAA GAA GAA ACT 2829
Asn Gln Asn Thr Asn Leu Arg Val Ile Gln Lys Asn Gln Glu Glu Thr
            855                 860                 865

ACT TCA ATT TCA AAA ATA ACT GTC AAT CCA GAC TCT GAA GAA CTT TTC 2877
Thr Ser Ile Ser Lys Ile Thr Val Asn Pro Asp Ser Glu Glu Leu Phe
        870                 875                 880

TCA GAC AAT GAG AAT AAT TTT GTC TTC CAA GTA GCT AAT GAA AGG AAT 2925
Ser Asp Asn Glu Asn Asn Phe Val Phe Gln Val Ala Asn Glu Arg Asn
    885                 890                 895

AAT CTT GCT TTA GGA AAT ACT AAG GAA CTT CAT GAA ACA GAC TTG ACT 2973
Asn Leu Ala Leu Gly Asn Thr Lys Glu Leu His Glu Thr Asp Leu Thr
900                 905                 910                 915

TGT GTA AAC GAA CCC ATT TTC AAG AAC TCT ACC ATG GTT TTA TAT GGA 3021
Cys Val Asn Glu Pro Ile Phe Lys Asn Ser Thr Met Val Leu Tyr Gly
                920                 925                 930

GAC ACA GGT GAT AAA CAA GCA ACC CAA GTG TCA ATT AAA AAA GAT TTG 3069
Asp Thr Gly Asp Lys Gln Ala Thr Gln Val Ser Ile Lys Lys Asp Leu
            935                 940                 945

GTT TAT GTT CTT GCA GAG GAG AAC AAA AAT AGT GTA AAG CAG CAT ATA 3117
Val Tyr Val Leu Ala Glu Glu Asn Lys Asn Ser Val Lys Gln His Ile
        950                 955                 960

AAA ATG ACT CTA GGT CAA GAT TTA AAA TCG GAC ATC TCC TTG AAT ATA 3165
Lys Met Thr Leu Gly Gln Asp Leu Lys Ser Asp Ile Ser Leu Asn Ile
    965                 970                 975

GAT AAA ATA CCA GAA AAA AAT AAT GAT TAC ATG GAC AAA TGG GCA GGA 3213
Asp Lys Ile Pro Glu Lys Asn Asn Asp Tyr Met Asp Lys Trp Ala Gly
980                 985                 990                 995

CTC TTA GGT CCA ATT TCA AAT CAC AGT TTT GGA GGT AGC TTC AGA ACA 3261
Leu Leu Gly Pro Ile Ser Asn His Ser Phe Gly Gly Ser Phe Arg Thr
                1000                1005                1010

GCT TCA AAT AAG GAA ATC AAG CTC TCT GAA CAT AAC ATT AAG AAG AGC 3309
Ala Ser Asn Lys Glu Ile Lys Leu Ser Glu His Asn Ile Lys Lys Ser
            1015                1020                1025

AAA ATG TTC TTC AAA GAT ATT GAA GAA CAA TAT CCT ACT AGT TTA GCT 3357
Lys Met Phe Phe Lys Asp Ile Glu Glu Gln Tyr Pro Thr Ser Leu Ala
        1030                1035                1040

TGT GTT GAA ATT GTA AAT ACC TTG GCA TTA GAT AAT CAA AAG AAA CTG     3405
Cys Val Glu Ile Val Asn Thr Leu Ala Leu Asp Asn Gln Lys Lys Leu
    1045                1050                1055

AGC AAG CCT CAG TCA ATT AAT ACT GTA TCT GCA CAT TTA CAG AGT AGT     3453
Ser Lys Pro Gln Ser Ile Asn Thr Val Ser Ala His Leu Gln Ser Ser
1060                1065                1070                1075

GTA GTT GTT TCT GAT TGT AAA AAT AGT CAT ATA ACC CCT CAG ATG TTA     3501
Val Val Val Ser Asp Cys Lys Asn Ser His Ile Thr Pro Gln Met Leu
                1080                1085                1090

TTT TCC AAG CAG GAT TTT AAT TCA AAC CAT AAT TTA ACA CCT AGC CAA     3549
Phe Ser Lys Gln Asp Phe Asn Ser Asn His Asn Leu Thr Pro Ser Gln
            1095                1100                1105

AAG GCA GAA ATT ACA GAA CTT TCT ACT ATA TTA GAA GAA TCA GGA AGT     3597
Lys Ala Glu Ile Thr Glu Leu Ser Thr Ile Leu Glu Glu Ser Gly Ser
        1110                1115                1120

CAG TTT GAA TTT ACT CAG TTT AGA AAG CCA AGC TAC ATA TTG CAG AAG     3645
Gln Phe Glu Phe Thr Gln Phe Arg Lys Pro Ser Tyr Ile Leu Gln Lys
    1125                1130                1135

AGT ACA TTT GAA GTG CCT GAA AAC CAG ATG ACT ATC TTA AAG ACC ACT     3693
Ser Thr Phe Glu Val Pro Glu Asn Gln Met Thr Ile Leu Lys Thr Thr
1140                1145                1150                1155

TCT GAG GAA TGC AGA GAT GCT GAT CTT CAT GTC ATA ATG AAT GCC CCA     3741
Ser Glu Glu Cys Arg Asp Ala Asp Leu His Val Ile Met Asn Ala Pro
                1160                1165                1170

TCG ATT GGT CAG GTA GAC AGC AGC AAG CAA TTT GAA GGT ACA GTT GAA     3789
Ser Ile Gly Gln Val Asp Ser Ser Lys Gln Phe Glu Gly Thr Val Glu
            1175                1180                1185

ATT AAA CGG AAG TTT GCT GGC CTG TTG AAA AAT GAC TGT AAC AAA AGT     3837
Ile Lys Arg Lys Phe Ala Gly Leu Leu Lys Asn Asp Cys Asn Lys Ser
        1190                1195                1200

GCT TCT GGT TAT TTA ACA GAT GAA AAT GAA GTG GGG TTT AGG GGC TTT     3885
Ala Ser Gly Tyr Leu Thr Asp Glu Asn Glu Val Gly Phe Arg Gly Phe
    1205                1210                1215

TAT TCT GCT CAT GGC ACA AAA CTG AAT GTT TCT ACT GAA GCT CTG CAA     3933
Tyr Ser Ala His Gly Thr Lys Leu Asn Val Ser Thr Glu Ala Leu Gln
1220                1225                1230                1235

AAA GCT GTG AAA CTG TTT AGT GAT ATT GAG AAT ATT AGT GAG GAA ACT     3981
Lys Ala Val Lys Leu Phe Ser Asp Ile Glu Asn Ile Ser Glu Glu Thr
                1240                1245                1250

TCT GCA GAG GTA CAT CCA ATA AGT TTA TCT TCA AGT AAA TGT CAT GAT     4029
Ser Ala Glu Val His Pro Ile Ser Leu Ser Ser Ser Lys Cys His Asp
            1255                1260                1265

TCT GTT GTT TCA ATG TTT AAG ATA GAA AAT CAT AAT GAT AAA ACT GTA     4077
Ser Val Val Ser Met Phe Lys Ile Glu Asn His Asn Asp Lys Thr Val
        1270                1275                1280

AGT GAA AAA AAT AAT AAA TGC CAA CTG ATA TTA CAA AAT AAT ATT GAA     4125

Ser Glu Lys Asn Asn Lys Cys Gln Leu Ile Leu Gln Asn Asn Ile Glu
    1285                1290                1295

ATG ACT ACT GGC ACT TTT GTT GAA GAA ATT ACT GAA AAT TAC AAG AGA 4173
Met Thr Thr Gly Thr Phe Val Glu Glu Ile Thr Glu Asn Tyr Lys Arg
1300                1305                1310                1315

AAT ACT GAA AAT GAA GAT AAC AAA TAT ACT GCT GCC AGT AGA AAT TCT 4221
Asn Thr Glu Asn Glu Asp Asn Lys Tyr Thr Ala Ala Ser Arg Asn Ser
                1320                1325                1330

CAT AAC TTA GAA TTT GAT GGC AGT GAT TCA AGT AAA AAT GAT ACT GTT 4269
His Asn Leu Glu Phe Asp Gly Ser Asp Ser Ser Lys Asn Asp Thr Val
            1335                1340                1345

TGT ATT CAT AAA GAT GAA ACG GAC TTG CTA TTT ACT GAT CAG CAC AAC 4317
Cys Ile His Lys Asp Glu Thr Asp Leu Leu Phe Thr Asp Gln His Asn
        1350                1355                1360

ATA TGT CTT AAA TTA TCT GGC CAG TTT ATG AAG GAG GGA AAC ACT CAG 4365
Ile Cys Leu Lys Leu Ser Gly Gln Phe Met Lys Glu Gly Asn Thr Gln
    1365                1370                1375

ATT AAA GAA GAT TTG TCA GAT TTA ACT TTT TTG GAA GTT GCG AAA GCT 4413
Ile Lys Glu Asp Leu Ser Asp Leu Thr Phe Leu Glu Val Ala Lys Ala
1380                1385                1390                1395

CAA GAA GCA TGT CAT GGT AAT ACT TCA AAT AAA GAA CAG TTA ACT GCT 4461
Gln Glu Ala Cys His Gly Asn Thr Ser Asn Lys Glu Gln Leu Thr Ala
                1400                1405                1410

ACT AAA ACG GAG CAA AAT ATA AAA GAT TTT GAG ACT TCT GAT ACA TTT 4509
Thr Lys Thr Glu Gln Asn Ile Lys Asp Phe Glu Thr Ser Asp Thr Phe
            1415                1420                1425

TTT CAG ACT GCA AGT GGG AAA AAT ATT AGT GTC GCC AAA GAG TCA TTT 4557
Phe Gln Thr Ala Ser Gly Lys Asn Ile Ser Val Ala Lys Glu Ser Phe
        1430                1435                1440

AAT AAA ATT GTA AAT TTC TTT GAT CAG AAA CCA GAA GAA TTG CAT AAC 4605
Asn Lys Ile Val Asn Phe Phe Asp Gln Lys Pro Glu Glu Leu His Asn
    1445                1450                1455

TTT TCC TTA AAT TCT GAA TTA CAT TCT GAC ATA AGA AAG AAC AAA ATG 4653
Phe Ser Leu Asn Ser Glu Leu His Ser Asp Ile Arg Lys Asn Lys Met
1460                1465                1470                1475

GAC ATT CTA AGT TAT GAG GAA ACA GAC ATA GTT AAA CAC AAA ATA CTG 4701
Asp Ile Leu Ser Tyr Glu Glu Thr Asp Ile Val Lys His Lys Ile Leu
                1480                1485                1490

AAA GAA AGT GTC CCA GTT GGT ACT GGA AAT CAA CTA GTG ACC TTC CAG 4749
Lys Glu Ser Val Pro Val Gly Thr Gly Asn Gln Leu Val Thr Phe Gln
            1495                1500                1505

GGA CAA CCC GAA CGT GAT GAA AAG ATC AAA GAA CCT ACT CTG TTG GGT 4797
Gly Gln Pro Glu Arg Asp Glu Lys Ile Lys Glu Pro Thr Leu Leu Gly
        1510                1515                1520

TTT CAT ACA GCT AGC GGG AAA AAA GTT AAA ATT GCA AAG GAA TCT TTG 4845
Phe His Thr Ala Ser Gly Lys Lys Val Lys Ile Ala Lys Glu Ser Leu

1525                    1530                    1535

GAC AAA GTG AAA AAC CTT TTT GAT GAA AAA GAG CAA GGT ACT AGT GAA     4893
Asp Lys Val Lys Asn Leu Phe Asp Glu Lys Glu Gln Gly Thr Ser Glu
1540                1545                1550                1555

ATC ACC AGT TTT AGC CAT CAA TGG GCA AAG ACC CTA AAG TAC AGA GAG     4941
Ile Thr Ser Phe Ser His Gln Trp Ala Lys Thr Leu Lys Tyr Arg Glu
                1560                1565                1570

GCC TGT AAA GAC CTT GAA TTA GCA TGT GAG ACC ATT GAG ATC ACA GCT     4989
Ala Cys Lys Asp Leu Glu Leu Ala Cys Glu Thr Ile Glu Ile Thr Ala
            1575                1580                1585

GCC CCA AAG TGT AAA GAA ATG CAG AAT TCT CTC AAT AAT GAT AAA AAC     5037
Ala Pro Lys Cys Lys Glu Met Gln Asn Ser Leu Asn Asn Asp Lys Asn
        1590                1595                1600

CTT GTT TCT ATT GAG ACT GTG GTG CCA CCT AAG CTC TTA AGT GAT AAT     5085
Leu Val Ser Ile Glu Thr Val Val Pro Pro Lys Leu Leu Ser Asp Asn
    1605                1610                1615

TTA TGT AGA CAA ACT GAA AAT CTC AAA ACA TCA AAA AGT ATC TTT TTG     5133
Leu Cys Arg Gln Thr Glu Asn Leu Lys Thr Ser Lys Ser Ile Phe Leu
1620                1625                1630                1635

AAA GTT AAA GTA CAT GAA AAT GTA GAA AAA GAA ACA GCA AAA AGT CCT     5181
Lys Val Lys Val His Glu Asn Val Glu Lys Glu Thr Ala Lys Ser Pro
                1640                1645                1650

GCA ACT TGT TAC ACA AAT CAG TCC CCT TAT TCA GTC ATT GAA AAT TCA     5229
Ala Thr Cys Tyr Thr Asn Gln Ser Pro Tyr Ser Val Ile Glu Asn Ser
            1655                1660                1665

GCC TTA GCT TTT TAC ACA AGT TGT AGT AGA AAA ACT TCT GTG AGT CAG     5277
Ala Leu Ala Phe Tyr Thr Ser Cys Ser Arg Lys Thr Ser Val Ser Gln
        1670                1675                1680

ACT TCA TTA CTT GAA GCA AAA AAA TGG CTT AGA GAA GGA ATA TTT GAT     5325
Thr Ser Leu Leu Glu Ala Lys Lys Trp Leu Arg Glu Gly Ile Phe Asp
    1685                1690                1695

GGT CAA CCA GAA AGA ATA AAT ACT GCA GAT TAT GTA GGA AAT TAT TTG     5373
Gly Gln Pro Glu Arg Ile Asn Thr Ala Asp Tyr Val Gly Asn Tyr Leu
1700                1705                1710                1715

TAT GAA AAT AAT TCA AAC AGT ACT ATA GCT GAA AAT GAC AAA AAT CAT     5421
Tyr Glu Asn Asn Ser Asn Ser Thr Ile Ala Glu Asn Asp Lys Asn His
                1720                1725                1730

CTC TCC GAA AAA CAA GAT ACT TAT TTA AGT AAC AGT AGC ATG TCT AAC     5469
Leu Ser Glu Lys Gln Asp Thr Tyr Leu Ser Asn Ser Ser Met Ser Asn
            1735                1740                1745

AGC TAT TCC TAC CAT TCT GAT GAG GTA TAT AAT GAT TCA GGA TAT CTC     5517
Ser Tyr Ser Tyr His Ser Asp Glu Val Tyr Asn Asp Ser Gly Tyr Leu
        1750                1755                1760

TCA AAA AAT AAA CTT GAT TCT GGT ATT GAG CCA GTA TTG AAG AAT GTT     5565
Ser Lys Asn Lys Leu Asp Ser Gly Ile Glu Pro Val Leu Lys Asn Val
    1765                1770                1775

GAA GAT CAA AAA AAC ACT AGT TTT TCC AAA GTA ATA TCC AAT GTA AAA 5613
Glu Asp Gln Lys Asn Thr Ser Phe Ser Lys Val Ile Ser Asn Val Lys
1780                1785                1790                1795

GAT GCA AAT GCA TAC CCA CAA ACT GTA AAT GAA GAT ATT TGC GTT GAG 5661
Asp Ala Asn Ala Tyr Pro Gln Thr Val Asn Glu Asp Ile Cys Val Glu
                1800                1805                1810

GAA CTT GTG ACT AGC TCT TCA CCC TGC AAA AAT AAA AAT GCA GCC ATT 5709
Glu Leu Val Thr Ser Ser Ser Pro Cys Lys Asn Lys Asn Ala Ala Ile
            1815                1820                1825

AAA TTG TCC ATA TCT AAT AGT AAT AAT TTT GAG GTA GGG CCA CCT GCA 5757
Lys Leu Ser Ile Ser Asn Ser Asn Asn Phe Glu Val Gly Pro Pro Ala
        1830                1835                1840

TTT AGG ATA GCC AGT GGT AAA ATC GTT TGT GTT TCA CAT GAA ACA ATT 5805
Phe Arg Ile Ala Ser Gly Lys Ile Val Cys Val Ser His Glu Thr Ile
    1845                1850                1855

AAA AAA GTG AAA GAC ATA TTT ACA GAC AGT TTC AGT AAA GTA ATT AAG 5853
Lys Lys Val Lys Asp Ile Phe Thr Asp Ser Phe Ser Lys Val Ile Lys
1860                1865                1870                1875

GAA AAC AAC GAG AAT AAA TCA AAA ATT TGC CAA ACG AAA ATT ATG GCA 5901
Glu Asn Asn Glu Asn Lys Ser Lys Ile Cys Gln Thr Lys Ile Met Ala
                1880                1885                1890

GGT TGT TAC GAG GCA TTG GAT GAT TCA GAG GAT ATT CTT CAT AAC TCT 5949
Gly Cys Tyr Glu Ala Leu Asp Asp Ser Glu Asp Ile Leu His Asn Ser
            1895                1900                1905

CTA GAT AAT GAT GAA TGT AGC ACG CAT TCA CAT AAG GTT TTT GCT GAC 5997
Leu Asp Asn Asp Glu Cys Ser Thr His Ser His Lys Val Phe Ala Asp
        1910                1915                1920

ATT CAG AGT GAA GAA ATT TTA CAA CAT AAC CAA AAT ATG TCT GGA TTG 6045
Ile Gln Ser Glu Glu Ile Leu Gln His Asn Gln Asn Met Ser Gly Leu
    1925                1930                1935

GAG AAA GTT TCT AAA ATA TCA CCT TGT GAT GTT AGT TTG GAA ACT TCA 6093
Glu Lys Val Ser Lys Ile Ser Pro Cys Asp Val Ser Leu Glu Thr Ser
1940                1945                1950                1955

GAT ATA TGT AAA TGT AGT ATA GGG AAG CTT CAT AAG TCA GTC TCA TCT 6141
Asp Ile Cys Lys Cys Ser Ile Gly Lys Leu His Lys Ser Val Ser Ser
                1960                1965                1970

GCA AAT ACT TGT GGG ATT TTT AGC ACA GCA AGT GGA AAA TCT GTC CAG 6189
Ala Asn Thr Cys Gly Ile Phe Ser Thr Ala Ser Gly Lys Ser Val Gln
            1975                1980                1985

GTA TCA GAT GCT TCA TTA CAA AAC GCA AGA CAA GTG TTT TCT GAA ATA 6237
Val Ser Asp Ala Ser Leu Gln Asn Ala Arg Gln Val Phe Ser Glu Ile
        1990                1995                2000

GAA GAT AGT ACC AAG CAA GTC TTT TCC AAA GTA TTG TTT AAA AGT AAC 6285
Glu Asp Ser Thr Lys Gln Val Phe Ser Lys Val Leu Phe Lys Ser Asn
    2005                2010                2015

GAA CAT TCA GAC CAG CTC ACA AGA GAA GAA AAT ACT GCT ATA CGT ACT 6333
Glu His Ser Asp Gln Leu Thr Arg Glu Glu Asn Thr Ala Ile Arg Thr
2020                2025                2030                2035

CCA GAA CAT TTA ATA TCC CAA AAA GGC TTT TCA TAT AAT GTG GTA AAT 6381
Pro Glu His Leu Ile Ser Gln Lys Gly Phe Ser Tyr Asn Val Val Asn
                2040                2045                2050

TCA TCT GCT TTC TCT GGA TTT AGT ACA GCA AGT GGA AAG CAA GTT TCC 6429
Ser Ser Ala Phe Ser Gly Phe Ser Thr Ala Ser Gly Lys Gln Val Ser
            2055                2060                2065

ATT TTA GAA AGT TCC TTA CAC AAA GTT AAG GGA GTG TTA GAG GAA TTT 6477
Ile Leu Glu Ser Ser Leu His Lys Val Lys Gly Val Leu Glu Glu Phe
        2070                2075                2080

GAT TTA ATC AGA ACT GAG CAT AGT CTT CAC TAT TCA CCT ACG TCT AGA 6525
Asp Leu Ile Arg Thr Glu His Ser Leu His Tyr Ser Pro Thr Ser Arg
    2085                2090                2095

CAA AAT GTA TCA AAA ATA CTT CCT CGT GTT GAT AAG AGA AAC CCA GAG 6573
Gln Asn Val Ser Lys Ile Leu Pro Arg Val Asp Lys Arg Asn Pro Glu
2100                2105                2110                2115

CAC TGT GTA AAC TCA GAA ATG GAA AAA ACC TGC AGT AAA GAA TTT AAA 6621
His Cys Val Asn Ser Glu Met Glu Lys Thr Cys Ser Lys Glu Phe Lys
                2120                2125                2130

TTA TCA AAT AAC TTA AAT GTT GAA GGT GGT TCT TCA GAA AAT AAT CAC 6669
Leu Ser Asn Asn Leu Asn Val Glu Gly Gly Ser Ser Glu Asn Asn His
            2135                2140                2145

TCT ATT AAA GTT TCT CCA TAT CTC TCT CAA TTT CAA CAA GAC AAA CAA 6717
Ser Ile Lys Val Ser Pro Tyr Leu Ser Gln Phe Gln Gln Asp Lys Gln
        2150                2155                2160

CAG TTG GTA TTA GGA ACC AAA GTC TCA CTT GTT GAG AAC ATT CAT GTT 6765
Gln Leu Val Leu Gly Thr Lys Val Ser Leu Val Glu Asn Ile His Val
    2165                2170                2175

TTG GGA AAA GAA CAG GCT TCA CCT AAA AAC GTA AAA ATG GAA ATT GGT 6813
Leu Gly Lys Glu Gln Ala Ser Pro Lys Asn Val Lys Met Glu Ile Gly
2180                2185                2190                2195

AAA ACT GAA ACT TTT TCT GAT GTT CCT GTG AAA ACA AAT ATA GAA GTT 6861
Lys Thr Glu Thr Phe Ser Asp Val Pro Val Lys Thr Asn Ile Glu Val
                2200                2205                2210

TGT TCT ACT TAC TCC AAA GAT TCA GAA AAC TAC TTT GAA ACA GAA GCA 6909
Cys Ser Thr Tyr Ser Lys Asp Ser Glu Asn Tyr Phe Glu Thr Glu Ala
            2215                2220                2225

GTA GAA ATT GCT AAA GCT TTT ATG GAA GAT GAT GAA CTG ACA GAT TCT 6957
Val Glu Ile Ala Lys Ala Phe Met Glu Asp Asp Glu Leu Thr Asp Ser
        2230                2235                2240

AAA CTG CCA AGT CAT GCC ACA CAT TCT CTT TTT ACA TGT CCC GAA AAT 7005
Lys Leu Pro Ser His Ala Thr His Ser Leu Phe Thr Cys Pro Glu Asn
    2245                2250                2255

GAG GAA ATG GTT TTG TCA AAT TCA AGA ATT GGA AAA AGA AGA GGA GAG 7053

Glu Glu Met Val Leu Ser Asn Ser Arg Ile Gly Lys Arg Arg Gly Glu
2260                2265                2270                2275

CCC CTT ATC TTA GTG GGA GAA CCC TCA ATC AAA AGA AAC TTA TTA AAT     7101
Pro Leu Ile Leu Val Gly Glu Pro Ser Ile Lys Arg Asn Leu Leu Asn
                2280                2285                2290

GAA TTT GAC AGG ATA ATA GAA AAT CAA GAA AAA TCC TTA AAG GCT TCA     7149
Glu Phe Asp Arg Ile Ile Glu Asn Gln Glu Lys Ser Leu Lys Ala Ser
            2295                2300                2305

AAA AGC ACT CCA GAT GGC ACA ATA AAA GAT CGA AGA TTG TTT ATG CAT     7197
Lys Ser Thr Pro Asp Gly Thr Ile Lys Asp Arg Arg Leu Phe Met His
        2310                2315                2320

CAT GTT TCT TTA GAG CCG ATT ACC TGT GTA CCC TTT CGC ACA ACT AAG     7245
His Val Ser Leu Glu Pro Ile Thr Cys Val Pro Phe Arg Thr Thr Lys
    2325                2330                2335

GAA CGT CAA GAG ATA CAG AAT CCA AAT TTT ACC GCA CCT GGT CAA GAA     7293
Glu Arg Gln Glu Ile Gln Asn Pro Asn Phe Thr Ala Pro Gly Gln Glu
2340                2345                2350                2355

TTT CTG TCT AAA TCT CAT TTG TAT GAA CAT CTG ACT TTG GAA AAA TCT     7341
Phe Leu Ser Lys Ser His Leu Tyr Glu His Leu Thr Leu Glu Lys Ser
                2360                2365                2370

TCA AGC AAT TTA GCA GTT TCA GGA CAT CCA TTT TAT CAA GTT TCT GCT     7389
Ser Ser Asn Leu Ala Val Ser Gly His Pro Phe Tyr Gln Val Ser Ala
            2375                2380                2385

ACA AGA AAT GAA AAA ATG AGA CAC TTG ATT ACT ACA GGC AGA CCA ACC     7437
Thr Arg Asn Glu Lys Met Arg His Leu Ile Thr Thr Gly Arg Pro Thr
        2390                2395                2400

AAA GTC TTT GTT CCA CCT TTT AAA ACT AAA TCG CAT TTT CAC AGA GTT     7485
Lys Val Phe Val Pro Pro Phe Lys Thr Lys Ser His Phe His Arg Val
    2405                2410                2415

GAA CAG TGT GTT AGG AAT ATT AAC TTG GAG GAA AAC AGA CAA AAG CAA     7533
Glu Gln Cys Val Arg Asn Ile Asn Leu Glu Glu Asn Arg Gln Lys Gln
2420                2425                2430                2435

AAC ATT GAT GGA CAT GGC TCT GAT GAT AGT AAA AAT AAG ATT AAT GAC     7581
Asn Ile Asp Gly His Gly Ser Asp Asp Ser Lys Asn Lys Ile Asn Asp
                2440                2445                2450

AAT GAG ATT CAT CAG TTT AAC AAA AAC AAC TCC AAT CAA GCA GCA GCT     7629
Asn Glu Ile His Gln Phe Asn Lys Asn Asn Ser Asn Gln Ala Ala Ala
            2455                2460                2465

GTA ACT TTC ACA AAG TGT GAA GAA GAA CCT TTA GAT TTA ATT ACA AGT     7677
Val Thr Phe Thr Lys Cys Glu Glu Glu Pro Leu Asp Leu Ile Thr Ser
        2470                2475                2480

CTT CAG AAT GCC AGA GAT ATA CAG GAT ATG CGA ATT AAG AAG AAA CAA     7725
Leu Gln Asn Ala Arg Asp Ile Gln Asp Met Arg Ile Lys Lys Lys Gln
    2485                2490                2495

AGG CAA CGC GTC TTT CCA CAG CCA GGC AGT CTG TAT CTT GCA AAA ACA     7773
Arg Gln Arg Val Phe Pro Gln Pro Gly Ser Leu Tyr Leu Ala Lys Thr

2500                2505                2510                2515

TCC ACT CTG CCT CGA ATC TCT CTG AAA GCA GCA GTA GGA GGC CAA GTT     7821
Ser Thr Leu Pro Arg Ile Ser Leu Lys Ala Ala Val Gly Gly Gln Val
            2520                2525                2530

CCC TCT GCG TGT TCT CAT AAA CAG CTG TAT ACG TAT GGC GTT TCT AAA     7869
Pro Ser Ala Cys Ser His Lys Gln Leu Tyr Thr Tyr Gly Val Ser Lys
        2535                2540                2545

CAT TGC ATA AAA ATT AAC AGC AAA AAT GCA GAG TCT TTT CAG TTT CAC     7917
His Cys Ile Lys Ile Asn Ser Lys Asn Ala Glu Ser Phe Gln Phe His
    2550                2555                2560

ACT GAA GAT TAT TTT GGT AAG GAA AGT TTA TGG ACT GGA AAA GGA ATA     7965
Thr Glu Asp Tyr Phe Gly Lys Glu Ser Leu Trp Thr Gly Lys Gly Ile
    2565                2570                2575

CAG TTG GCT GAT GGT GGA TGG CTC ATA CCC TCC AAT GAT GGA AAG GCT     8013
Gln Leu Ala Asp Gly Gly Trp Leu Ile Pro Ser Asn Asp Gly Lys Ala
2580                2585                2590                2595

GGA AAA GAA GAA TTT TAT AGG GCT CTG TGT GAC ACT CCA GGT GTG GAT     8061
Gly Lys Glu Glu Phe Tyr Arg Ala Leu Cys Asp Thr Pro Gly Val Asp
            2600                2605                2610

CCA AAG CTT ATT TCT AGA ATT TGG GTT TAT AAT CAC TAT AGA TGG ATC     8109
Pro Lys Leu Ile Ser Arg Ile Trp Val Tyr Asn His Tyr Arg Trp Ile
        2615                2620                2625

ATA TGG AAA CTG GCA GCT ATG GAA TGT GCC TTT CCT AAG GAA TTT GCT     8157
Ile Trp Lys Leu Ala Ala Met Glu Cys Ala Phe Pro Lys Glu Phe Ala
    2630                2635                2640

AAT AGA TGC CTA AGC CCA GAA AGG GTG CTT CTT CAA CTA AAA TAC AGA     8205
Asn Arg Cys Leu Ser Pro Glu Arg Val Leu Leu Gln Leu Lys Tyr Arg
    2645                2650                2655

TAT GAT ACG GAA ATT GAT AGA AGC AGA AGA TCG GCT ATA AAA AAG ATA     8253
Tyr Asp Thr Glu Ile Asp Arg Ser Arg Arg Ser Ala Ile Lys Lys Ile
2660                2665                2670                2675

ATG GAA AGG GAT GAC ACA GCT GCA AAA ACA CTT GTT CTC TGT GTT TCT     8301
Met Glu Arg Asp Asp Thr Ala Ala Lys Thr Leu Val Leu Cys Val Ser
            2680                2685                2690

GAC ATA ATT TCA TTG AGC GCA AAT ATA TCT GAA ACT TCT AGC AAT AAA     8349
Asp Ile Ile Ser Leu Ser Ala Asn Ile Ser Glu Thr Ser Ser Asn Lys
        2695                2700                2705

ACT AGT AGT GCA GAT ACC CAA AAA GTG GCC ATT ATT GAA CTT ACA GAT     8397
Thr Ser Ser Ala Asp Thr Gln Lys Val Ala Ile Ile Glu Leu Thr Asp
    2710                2715                2720

GGG TGG TAT GCT GTT AAG GCC CAG TTA GAT CCT CCC CTC TTA GCT GTC     8445
Gly Trp Tyr Ala Val Lys Ala Gln Leu Asp Pro Pro Leu Leu Ala Val
    2725                2730                2735

TTA AAG AAT GGC AGA CTG ACA GTT GGT CAG AAG ATT ATT CTT CAT GGA     8493
Leu Lys Asn Gly Arg Leu Thr Val Gly Gln Lys Ile Ile Leu His Gly
2740                2745                2750                2755

GCA GAA CTG GTG GGC TCT CCT GAT GCC TGT ACA CCT CTT GAA GCC CCA 8541
Ala Glu Leu Val Gly Ser Pro Asp Ala Cys Thr Pro Leu Glu Ala Pro
                2760                2765                2770

GAA TCT CTT ATG TTA AAG ATT TCT GCT AAC AGT ACT CGG CCT GCT CGC 8589
Glu Ser Leu Met Leu Lys Ile Ser Ala Asn Ser Thr Arg Pro Ala Arg
            2775                2780                2785

TGG TAT ACC AAA CTT GGA TTC TTT CCT GAC CCT AGA CCT TTT CCT CTG 8637
Trp Tyr Thr Lys Leu Gly Phe Phe Pro Asp Pro Arg Pro Phe Pro Leu
        2790                2795                2800

CCC TTA TCA TCG CTT TTC AGT GAT GGA GGA AAT GTT GGT TGT GTT GAT 8685
Pro Leu Ser Ser Leu Phe Ser Asp Gly Gly Asn Val Gly Cys Val Asp
    2805                2810                2815

GTA ATT ATT CAA AGA GCA TAC CCT ATA CAG TGG ATG GAG AAG ACA TCA 8733
Val Ile Ile Gln Arg Ala Tyr Pro Ile Gln Trp Met Glu Lys Thr Ser
2820                2825                2830                2835

TCT GGA TTA TAC ATA TTT CGC AAT GAA AGA GAG GAA GAA AAG GAA GCA 8781
Ser Gly Leu Tyr Ile Phe Arg Asn Glu Arg Glu Glu Glu Lys Glu Ala
                2840                2845                2850

GCA AAA TAT GTG GAG GCC CAA CAA AAG AGA CTA GAA GCC TTA TTC ACT 8829
Ala Lys Tyr Val Glu Ala Gln Gln Lys Arg Leu Glu Ala Leu Phe Thr
            2855                2860                2865

AAA ATT CAG GAG GAA TTT GAA GAA CAT GAA GAA AAC ACA ACA AAA CCA 8877
Lys Ile Gln Glu Glu Phe Glu Glu His Glu Glu Asn Thr Thr Lys Pro
        2870                2875                2880

TAT TTA CCA TCA CGT GCA CTA ACA AGA CAG CAA GTT CGT GCT TTG CAA 8925
Tyr Leu Pro Ser Arg Ala Leu Thr Arg Gln Gln Val Arg Ala Leu Gln
    2885                2890                2895

GAT GGT GCA GAG CTT TAT GAA GCA GTG AAG AAT GCA GCA GAC CCA GCT 8973
Asp Gly Ala Glu Leu Tyr Glu Ala Val Lys Asn Ala Ala Asp Pro Ala
2900                2905                2910                2915

TAC CTT GAG GGT TAT TTC AGT GAA GAG CAG TTA AGA GCC TTG AAT AAT 9021
Tyr Leu Glu Gly Tyr Phe Ser Glu Glu Gln Leu Arg Ala Leu Asn Asn
                2920                2925                2930

CAC AGG CAA ATG TTG AAT GAT AAG AAA CAA GCT CAG ATC CAG TTG GAA 9069
His Arg Gln Met Leu Asn Asp Lys Lys Gln Ala Gln Ile Gln Leu Glu
            2935                2940                2945

ATT AGG AAG GCC ATG GAA TCT GCT GAA CAA AAG GAA CAA GGT TTA TCA 9117
Ile Arg Lys Ala Met Glu Ser Ala Glu Gln Lys Glu Gln Gly Leu Ser
        2950                2955                2960

AGG GAT GTC ACA ACC GTG TGG AAG TTG CGT ATT GTA AGC TAT TCA AAA 9165
Arg Asp Val Thr Thr Val Trp Lys Leu Arg Ile Val Ser Tyr Ser Lys
    2965                2970                2975

AAA GAA AAA GAT TCA GTT ATA CTG AGT ATT TGG CGT CCA TCA TCA GAT 9213
Lys Glu Lys Asp Ser Val Ile Leu Ser Ile Trp Arg Pro Ser Ser Asp
2980                2985                2990                2995

TTA TAT TCT CTG TTA ACA GAA GGA AAG AGA TAC AGA ATT TAT CAT CTT 9261
Leu Tyr Ser Leu Leu Thr Glu Gly Lys Arg Tyr Arg Ile Tyr His Leu
                3000                3005                3010

GCA ACT TCA AAA TCT AAA AGT AAA TCT GAA AGA GCT AAC ATA CAG TTA 9309
Ala Thr Ser Lys Ser Lys Ser Lys Ser Glu Arg Ala Asn Ile Gln Leu
            3015                3020                3025

GCA GCG ACA AAA AAA ACT CAG TAT CAA CAA CTA CCG GTT TCA GAT GAA 9357
Ala Ala Thr Lys Lys Thr Gln Tyr Gln Gln Leu Pro Val Ser Asp Glu
        3030                3035                3040

ATT TTA TTT CAG ATT TAC CAG CCA CGG GAG CCC CTT CAC TTC AGC AAA 9405
Ile Leu Phe Gln Ile Tyr Gln Pro Arg Glu Pro Leu His Phe Ser Lys
    3045                3050                3055

TTT TTA GAT CCA GAC TTT CAG CCA TCT TGT TCT GAG GTG GAC CTA ATA 9453
Phe Leu Asp Pro Asp Phe Gln Pro Ser Cys Ser Glu Val Asp Leu Ile
3060                3065                3070                3075

GGA TTT GTC GTT TCT GTT GTG AAA AAA ACA GGA CTT GCC CCT TTC GTC 9501
Gly Phe Val Val Ser Val Val Lys Lys Thr Gly Leu Ala Pro Phe Val
                3080                3085                3090

TAT TTG TCA GAC GAA TGT TAC AAT TTA CTG GCA ATA AAG TTT TGG ATA 9549
Tyr Leu Ser Asp Glu Cys Tyr Asn Leu Leu Ala Ile Lys Phe Trp Ile
            3095                3100                3105

GAC CTT AAT GAG GAC ATT ATT AAG CCT CAT ATG TTA ATT GCT GCA AGC 9597
Asp Leu Asn Glu Asp Ile Ile Lys Pro His Met Leu Ile Ala Ala Ser
        3110                3115                3120

AAC CTC CAG TGG CGA CCA GAA TCC AAA TCA GGC CTT CTT ACT TTA TTT 9645
Asn Leu Gln Trp Arg Pro Glu Ser Lys Ser Gly Leu Leu Thr Leu Phe
    3125                3130                3135

GCT GGA GAT TTT TCT GTG TTT TCT GCT AGT CCA AAA GAG GGC CAC TTT 9693
Ala Gly Asp Phe Ser Val Phe Ser Ala Ser Pro Lys Glu Gly His Phe
3140                3145                3150                3155

CAA GAG ACA TTC AAC AAA ATG AAA AAT ACT GTT GAG AAT ATT GAC ATA 9741
Gln Glu Thr Phe Asn Lys Met Lys Asn Thr Val Glu Asn Ile Asp Ile
                3160                3165                3170

CTT TGC AAT GAA GCA GAA AAC AAG CTT ATG CAT ATA CTG CAT GCA AAT 9789
Leu Cys Asn Glu Ala Glu Asn Lys Leu Met His Ile Leu His Ala Asn
            3175                3180                3185

GAT CCC AAG TGG TCC ACC CCA ACT AAA GAC TGT ACT TCA GGG CCG TAC 9837
Asp Pro Lys Trp Ser Thr Pro Thr Lys Asp Cys Thr Ser Gly Pro Tyr
        3190                3195                3200

ACT GCT CAA ATC ATT CCT GGT ACA GGA AAC AAG CTT CTG ATG TCT TCT 9885
Thr Ala Gln Ile Ile Pro Gly Thr Gly Asn Lys Leu Leu Met Ser Ser
    3205                3210                3215

CCT AAT TGT GAG ATA TAT TAT CAA AGT CCT TTA TCA CTT TGT ATG GCC 9933
Pro Asn Cys Glu Ile Tyr Tyr Gln Ser Pro Leu Ser Leu Cys Met Ala
3220                3225                3230                3235

AAA AGG AAG TCT GTT TCC ACA CCT GTC TCA GCC CAG ATG ACT TCA AAG 9981

Lys Arg Lys Ser Val Ser Thr Pro Val Ser Ala Gln Met Thr Ser Lys
                3240                3245                3250

TCT TGT AAA GGG GAG AAA GAG ATT GAT GAC CAA AAG AAC TGC AAA AAG 10029
Ser Cys Lys Gly Glu Lys Glu Ile Asp Asp Gln Lys Asn Cys Lys Lys
            3255                3260                3265

AGA AGA GCC TTG GAT TTC TTG AGT AGA CTG CCT TTA CCT CCA CCT GTT 10077
Arg Arg Ala Leu Asp Phe Leu Ser Arg Leu Pro Leu Pro Pro Pro Val
        3270                3275                3280

AGT CCC ATT TGT ACA TTT GTT TCT CCG GCT GCA CAG AAG GCA TTT CAG 10125
Ser Pro Ile Cys Thr Phe Val Ser Pro Ala Ala Gln Lys Ala Phe Gln
    3285                3290                3295

CCA CCA AGG AGT TGT GGC ACC AAA TAC GAA ACA CCC ATA AAG AAA AAA 10173
Pro Pro Arg Ser Cys Gly Thr Lys Tyr Glu Thr Pro Ile Lys Lys Lys
3300                3305                3310                3315

GAA CTG AAT TCT CCT CAG ATG ACT CCA TTT AAA AAA TTC AAT GAA ATT 10221
Glu Leu Asn Ser Pro Gln Met Thr Pro Phe Lys Lys Phe Asn Glu Ile
                3320                3325                3330

TCT CTT TTG GAA AGT AAT TCA ATA GCT GAC GAA GAA CTT GCA TTG ATA 10269
Ser Leu Leu Glu Ser Asn Ser Ile Ala Asp Glu Glu Leu Ala Leu Ile
            3335                3340                3345

AAT ACC CAA GCT CTT TTG TCT GGT TCA ACA GGA GAA AAA CAA TTT ATA 10317
Asn Thr Gln Ala Leu Leu Ser Gly Ser Thr Gly Glu Lys Gln Phe Ile
        3350                3355                3360

TCT GTC AGT GAA TCC ACT AGG ACT GCT CCC ACC AGT TCA GAA GAT TAT 10365
Ser Val Ser Glu Ser Thr Arg Thr Ala Pro Thr Ser Ser Glu Asp Tyr
    3365                3370                3375

CTC AGA CTG AAA CGA CGT TGT ACT ACA TCT CTG ATC AAA GAA CAG GAG 10413
Leu Arg Leu Lys Arg Arg Cys Thr Thr Ser Leu Ile Lys Glu Gln Glu
3380                3385                3390                3395

AGT TCC CAG GCC AGT ACG GAA GAA TGT GAG AAA AAT AAG CAG GAC ACA 10461
Ser Ser Gln Ala Ser Thr Glu Glu Cys Glu Lys Asn Lys Gln Asp Thr
                3400                3405                3410

ATT ACA ACT AAA AAA TAT ATC TAA 10485
Ile Thr Thr Lys Lys Tyr Ile
            3415

(2) INFORMATION FOR SEQ ID NO:9:

(i) SEQUENCE CHARACTERISTICS:
(A) LENGTH: 3418 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:9:

Met Pro Ile Gly Ser Lys Glu Arg Pro Thr Phe Phe Glu Ile Phe Lys
1               5                   10                  15
Thr Arg Cys Asn Lys Ala Asp Leu Gly Pro Ile Ser Leu Asn Trp Phe
            20                  25                  30
Glu Glu Leu Ser Ser Glu Ala Pro Pro Tyr Asn Ser Glu Pro Ala Glu
        35                  40                  45
Glu Ser Glu His Lys Asn Asn Asn Tyr Glu Pro Asn Leu Phe Lys Thr
    50                  55                  60
Pro Gln Arg Lys Pro Ser Tyr Asn Gln Leu Ala Ser Thr Pro Ile Ile
65                  70                  75                  80
Phe Lys Glu Gln Gly Leu Thr Leu Pro Leu Tyr Gln Ser Pro Val Lys
                85                  90                  95
Glu Leu Asp Lys Phe Lys Leu Asp Leu Gly Arg Asn Val Pro Asn Ser
            100                 105                 110
Arg His Lys Ser Leu Arg Thr Val Lys Thr Lys Met Asp Gln Ala Asp
        115                 120                 125
Asp Val Ser Cys Pro Leu Leu Asn Ser Cys Leu Ser Glu Ser Pro Val
    130                 135                 140
Val Leu Gln Cys Thr His Val Thr Pro Gln Arg Asp Lys Ser Val Val
145                 150                 155                 160
Cys Gly Ser Leu Phe His Thr Pro Lys Phe Val Lys Gly Arg Gln Thr
                165                 170                 175
Pro Lys His Ile Ser Glu Ser Leu Gly Ala Glu Val Asp Pro Asp Met
            180                 185                 190
Ser Trp Ser Ser Ser Leu Ala Thr Pro Pro Thr Leu Ser Ser Thr Val
        195                 200                 205
Leu Ile Val Arg Asn Glu Glu Ala Ser Glu Thr Val Phe Pro His Asp
    210                 215                 220
Thr Thr Ala Asn Val Lys Ser Tyr Phe Ser Asn His Asp Glu Ser Leu
225                 230                 235                 240
Lys Lys Asn Asp Arg Phe Ile Ala Ser Val Thr Asp Ser Glu Asn Thr
                245                 250                 255
Asn Gln Arg Glu Ala Ala Ser His Gly Phe Gly Lys Thr Ser Gly Asn
            260                 265                 270
Ser Phe Lys Val Asn Ser Cys Lys Asp His Ile Gly Lys Ser Met Pro
        275                 280                 285
His Val Leu Glu Asp Glu Val Tyr Glu Thr Val Val Asp Thr Ser Glu
    290                 295                 300
Glu Asp Ser Phe Ser Leu Cys Phe Ser Lys Cys Arg Thr Lys Asn Leu
305                 310                 315                 320
Gln Lys Val Arg Thr Ser Lys Thr Arg Lys Lys Ile Phe His Glu Ala
                325                 330                 335
Asn Ala Asp Glu Cys Glu Lys Ser Lys Asn Gln Val Lys Glu Lys Tyr
            340                 345                 350
Ser Phe Val Ser Glu Val Glu Pro Asn Asp Thr Asp Pro Leu Asp Ser
        355                 360                 365
Asn Val Ala Asn Gln Lys Pro Phe Glu Ser Gly Ser Asp Lys Ile Ser
    370                 375                 380
Lys Glu Val Val Pro Ser Leu Ala Cys Glu Trp Ser Gln Leu Thr Leu
385                 390                 395                 400
Ser Gly Leu Asn Gly Ala Gln Met Glu Lys Ile Pro Leu Leu His Ile
                405                 410                 415
Ser Ser Cys Asp Gln Asn Ile Ser Glu Lys Asp Leu Leu Asp Thr Glu
            420                 425                 430
Asn Lys Arg Lys Lys Asp Phe Leu Thr Ser Glu Asn Ser Leu Pro Arg
        435                 440                 445
Ile Ser Ser Leu Pro Lys Ser Glu Lys Pro Leu Asn Glu Glu Thr Val
    450                 455                 460
Val Asn Lys Arg Asp Glu Glu Gln His Leu Glu Ser His Thr Asp Cys
465                 470                 475                 480
Ile Leu Ala Val Lys Gln Ala Ile Ser Gly Thr Ser Pro Val Ala Ser

                485                 490                 495
Ser Phe Gln Gly Ile Lys Lys Ser Ile Phe Arg Ile Arg Glu Ser Pro
            500                 505                 510
Lys Glu Thr Phe Asn Ala Ser Phe Ser Gly His Met Thr Asp Pro Asn
        515                 520                 525
Phe Lys Lys Glu Thr Glu Ala Ser Glu Ser Gly Leu Glu Ile His Thr
    530                 535                 540
Val Cys Ser Gln Lys Glu Asp Ser Leu Cys Pro Asn Leu Ile Asp Asn
545                 550                 555                 560
Gly Ser Trp Pro Ala Thr Thr Thr Gln Asn Ser Val Ala Leu Lys Asn
                565                 570                 575
Ala Gly Leu Ile Ser Thr Leu Lys Lys Lys Thr Asn Lys Phe Ile Tyr
            580                 585                 590
Ala Ile His Asp Glu Thr Ser Tyr Lys Gly Lys Lys Ile Pro Lys Asp
        595                 600                 605
Gln Lys Ser Glu Leu Ile Asn Cys Ser Ala Gln Phe Glu Ala Asn Ala
    610                 615                 620
Phe Glu Ala Pro Leu Thr Phe Ala Asn Ala Asp Ser Gly Leu Leu His
625                 630                 635                 640
Ser Ser Val Lys Arg Ser Cys Ser Gln Asn Asp Ser Glu Glu Pro Thr
                645                 650                 655
Leu Ser Leu Thr Ser Ser Phe Gly Thr Ile Leu Arg Lys Cys Ser Arg
            660                 665                 670
Asn Glu Thr Cys Ser Asn Asn Thr Val Ile Ser Gln Asp Leu Asp Tyr
        675                 680                 685
Lys Glu Ala Lys Cys Asn Lys Glu Lys Leu Gln Leu Phe Ile Thr Pro
    690                 695                 700
Glu Ala Asp Ser Leu Ser Cys Leu Gln Glu Gly Gln Cys Glu Asn Asp
705                 710                 715                 720
Pro Lys Ser Lys Lys Val Ser Asp Ile Lys Glu Glu Val Leu Ala Ala
                725                 730                 735
Ala Cys His Pro Val Gln His Ser Lys Val Glu Tyr Ser Asp Thr Asp
            740                 745                 750
Phe Gln Ser Gln Lys Ser Leu Leu Tyr Asp His Glu Asn Ala Ser Thr
        755                 760                 765
Leu Ile Leu Thr Pro Thr Ser Lys Asp Val Leu Ser Asn Leu Val Met
    770                 775                 780
Ile Ser Arg Gly Lys Glu Ser Tyr Lys Met Ser Asp Lys Leu Lys Gly
785                 790                 795                 800
Asn Asn Tyr Glu Ser Asp Val Glu Leu Thr Lys Asn Ile Pro Met Glu
                805                 810                 815
Lys Asn Gln Asp Val Cys Ala Leu Asn Glu Asn Tyr Lys Asn Val Glu
            820                 825                 830
Leu Leu Pro Pro Glu Lys Tyr Met Arg Val Ala Ser Pro Ser Arg Lys
        835                 840                 845
Val Gln Phe Asn Gln Asn Thr Asn Leu Arg Val Ile Gln Lys Asn Gln
    850                 855                 860
Glu Glu Thr Thr Ser Ile Ser Lys Ile Thr Val Asn Pro Asp Ser Glu
865                 870                 875                 880
Glu Leu Phe Ser Asp Asn Glu Asn Asn Phe Val Phe Gln Val Ala Asn
                885                 890                 895
Glu Arg Asn Asn Leu Ala Leu Gly Asn Thr Lys Glu Leu His Glu Thr
            900                 905                 910
Asp Leu Thr Cys Val Asn Glu Pro Ile Phe Lys Asn Ser Thr Met Val
        915                 920                 925
Leu Tyr Gly Asp Thr Gly Asp Lys Gln Ala Thr Gln Val Ser Ile Lys
    930                 935                 940
Lys Asp Leu Val Tyr Val Leu Ala Glu Glu Asn Lys Asn Ser Val Lys
945                 950                 955                 960
Gln His Ile Lys Met Thr Leu Gly Gln Asp Leu Lys Ser Asp Ile Ser
                965                 970                 975

Leu Asn Ile Asp Lys Ile Pro Glu Lys Asn Asn Asp Tyr Met Asp Lys
            980                 985                 990
Trp Ala Gly Leu Leu Gly Pro Ile Ser Asn His Ser Phe Gly Gly Ser
        995                 1000                1005
Phe Arg Thr Ala Ser Asn Lys Glu Ile Lys Leu Ser Glu His Asn Ile
    1010                1015                1020
Lys Lys Ser Lys Met Phe Phe Lys Asp Ile Glu Glu Gln Tyr Pro Thr
1025                1030                1035                104
Ser Leu Ala Cys Val Glu Ile Val Asn Thr Leu Ala Leu Asp Asn Gln
                1045                1050                1055
Lys Lys Leu Ser Lys Pro Gln Ser Ile Asn Thr Val Ser Ala His Leu
            1060                1065                1070
Gln Ser Ser Val Val Val Ser Asp Cys Lys Asn Ser His Ile Thr Pro
        1075                1080                1085
Gln Met Leu Phe Ser Lys Gln Asp Phe Asn Ser Asn His Asn Leu Thr
    1090                1095                1100
Pro Ser Gln Lys Ala Glu Ile Thr Glu Leu Ser Thr Ile Leu Glu Glu
1105                1110                1115                112
Ser Gly Ser Gln Phe Glu Phe Thr Gln Phe Arg Lys Pro Ser Tyr Ile
                1125                1130                1135
Leu Gln Lys Ser Thr Phe Glu Val Pro Glu Asn Gln Met Thr Ile Leu
            1140                1145                1150
Lys Thr Thr Ser Glu Glu Cys Arg Asp Ala Asp Leu His Val Ile Met
        1155                1160                1165
Asn Ala Pro Ser Ile Gly Gln Val Asp Ser Ser Lys Gln Phe Glu Gly
    1170                1175                1180
Thr Val Glu Ile Lys Arg Lys Phe Ala Gly Leu Leu Lys Asn Asp Cys
1185                1190                1195                120
Asn Lys Ser Ala Ser Gly Tyr Leu Thr Asp Glu Asn Glu Val Gly Phe
                1205                1210                1215
Arg Gly Phe Tyr Ser Ala His Gly Thr Lys Leu Asn Val Ser Thr Glu
            1220                1225                1230
Ala Leu Gln Lys Ala Val Lys Leu Phe Ser Asp Ile Glu Asn Ile Ser
        1235                1240                1245
Glu Glu Thr Ser Ala Glu Val His Pro Ile Ser Leu Ser Ser Ser Lys
    1250                1255                1260
Cys His Asp Ser Val Val Ser Met Phe Lys Ile Glu Asn His Asn Asp
1265                1270                1275                128
Lys Thr Val Ser Glu Lys Asn Asn Lys Cys Gln Leu Ile Leu Gln Asn
                1285                1290                1295
Asn Ile Glu Met Thr Thr Gly Thr Phe Val Glu Glu Ile Thr Glu Asn
            1300                1305                1310
Tyr Lys Arg Asn Thr Glu Asn Glu Asp Asn Lys Tyr Thr Ala Ala Ser
        1315                1320                1325
Arg Asn Ser His Asn Leu Glu Phe Asp Gly Ser Asp Ser Ser Lys Asn
    1330                1335                1340
Asp Thr Val Cys Ile His Lys Asp Glu Thr Asp Leu Leu Phe Thr Asp
1345                1350                1355                136
Gln His Asn Ile Cys Leu Lys Leu Ser Gly Gln Phe Met Lys Glu Gly
                1365                1370                1375
Asn Thr Gln Ile Lys Glu Asp Leu Ser Asp Leu Thr Phe Leu Glu Val
            1380                1385                1390
Ala Lys Ala Gln Glu Ala Cys His Gly Asn Thr Ser Asn Lys Glu Gln
        1395                1400                1405
Leu Thr Ala Thr Lys Thr Glu Gln Asn Ile Lys Asp Phe Glu Thr Ser
    1410                1415                1420
Asp Thr Phe Phe Gln Thr Ala Ser Gly Lys Asn Ile Ser Val Ala Lys
1425                1430                1435                144
Glu Ser Phe Asn Lys Ile Val Asn Phe Phe Asp Gln Lys Pro Glu Glu
                1445                1450                1455
Leu His Asn Phe Ser Leu Asn Ser Glu Leu His Ser Asp Ile Arg Lys

1460                    1465                    1470
Asn Lys Met Asp Ile Leu Ser Tyr Glu Glu Thr Asp Ile Val Lys His
        1475                    1480                    1485
Lys Ile Leu Lys Glu Ser Val Pro Val Gly Thr Gly Asn Gln Leu Val
    1490                    1495                    1500
Thr Phe Gln Gly Gln Pro Glu Arg Asp Glu Lys Ile Lys Glu Pro Thr
1505                    1510                    1515                    152
Leu Leu Gly Phe His Thr Ala Ser Gly Lys Lys Val Lys Ile Ala Lys
                1525                    1530                    1535
Glu Ser Leu Asp Lys Val Lys Asn Leu Phe Asp Glu Lys Glu Gln Gly
            1540                    1545                    1550
Thr Ser Glu Ile Thr Ser Phe Ser His Gln Trp Ala Lys Thr Leu Lys
        1555                    1560                    1565
Tyr Arg Glu Ala Cys Lys Asp Leu Glu Leu Ala Cys Glu Thr Ile Glu
    1570                    1575                    1580
Ile Thr Ala Ala Pro Lys Cys Lys Glu Met Gln Asn Ser Leu Asn Asn
1585                    1590                    1595                    160
Asp Lys Asn Leu Val Ser Ile Glu Thr Val Val Pro Pro Lys Leu Leu
                1605                    1610                    1615
Ser Asp Asn Leu Cys Arg Gln Thr Glu Asn Leu Lys Thr Ser Lys Ser
            1620                    1625                    1630
Ile Phe Leu Lys Val Lys Val His Glu Asn Val Glu Lys Glu Thr Ala
        1635                    1640                    1645
Lys Ser Pro Ala Thr Cys Tyr Thr Asn Gln Ser Pro Tyr Ser Val Ile
    1650                    1655                    1660
Glu Asn Ser Ala Leu Ala Phe Tyr Thr Ser Cys Ser Arg Lys Thr Ser
1665                    1670                    1675                    168
Val Ser Gln Thr Ser Leu Leu Glu Ala Lys Lys Trp Leu Arg Glu Gly
                1685                    1690                    1695
Ile Phe Asp Gly Gln Pro Glu Arg Ile Asn Thr Ala Asp Tyr Val Gly
            1700                    1705                    1710
Asn Tyr Leu Tyr Glu Asn Asn Ser Asn Ser Thr Ile Ala Glu Asn Asp
        1715                    1720                    1725
Lys Asn His Leu Ser Glu Lys Gln Asp Thr Tyr Leu Ser Asn Ser Ser
    1730                    1735                    1740
Met Ser Asn Ser Tyr Ser Tyr His Ser Asp Glu Val Tyr Asn Asp Ser
1745                    1750                    1755                    176
Gly Tyr Leu Ser Lys Asn Lys Leu Asp Ser Gly Ile Glu Pro Val Leu
                1765                    1770                    1775
Lys Asn Val Glu Asp Gln Lys Asn Thr Ser Phe Ser Lys Val Ile Ser
            1780                    1785                    1790
Asn Val Lys Asp Ala Asn Ala Tyr Pro Gln Thr Val Asn Glu Asp Ile
        1795                    1800                    1805
Cys Val Glu Glu Leu Val Thr Ser Ser Ser Pro Cys Lys Asn Lys Asn
    1810                    1815                    1820
Ala Ala Ile Lys Leu Ser Ile Ser Asn Ser Asn Asn Phe Glu Val Gly
1825                    1830                    1835                    184
Pro Pro Ala Phe Arg Ile Ala Ser Gly Lys Ile Val Cys Val Ser His
                1845                    1850                    1855
Glu Thr Ile Lys Lys Val Lys Asp Ile Phe Thr Asp Ser Phe Ser Lys
            1860                    1865                    1870
Val Ile Lys Glu Asn Asn Glu Asn Lys Ser Lys Ile Cys Gln Thr Lys
        1875                    1880                    1885
Ile Met Ala Gly Cys Tyr Glu Ala Leu Asp Asp Ser Glu Asp Ile Leu
    1890                    1895                    1900
His Asn Ser Leu Asp Asn Asp Glu Cys Ser Thr His Ser His Lys Val
1905                    1910                    1915                    192
Phe Ala Asp Ile Gln Ser Glu Glu Ile Leu Gln His Asn Gln Asn Met
                1925                    1930                    1935
Ser Gly Leu Glu Lys Val Ser Lys Ile Ser Pro Cys Asp Val Ser Leu
            1940                    1945                    1950

Glu Thr Ser Asp Ile Cys Lys Cys Ser Ile Gly Lys Leu His Lys Ser
        1955                1960                1965
Val Ser Ser Ala Asn Thr Cys Gly Ile Phe Ser Thr Ala Ser Gly Lys
    1970                1975                1980
Ser Val Gln Val Ser Asp Ala Ser Leu Gln Asn Ala Arg Gln Val Phe
1985                1990                1995                200
Ser Glu Ile Glu Asp Ser Thr Lys Gln Val Phe Ser Lys Val Leu Phe
                2005                2010                2015
Lys Ser Asn Glu His Ser Asp Gln Leu Thr Arg Glu Glu Asn Thr Ala
            2020                2025                2030
Ile Arg Thr Pro Glu His Leu Ile Ser Gln Lys Gly Phe Ser Tyr Asn
        2035                2040                2045
Val Val Asn Ser Ser Ala Phe Ser Gly Phe Ser Thr Ala Ser Gly Lys
    2050                2055                2060
Gln Val Ser Ile Leu Glu Ser Ser Leu His Lys Val Lys Gly Val Leu
2065                2070                2075                208
Glu Glu Phe Asp Leu Ile Arg Thr Glu His Ser Leu His Tyr Ser Pro
                2085                2090                2095
Thr Ser Arg Gln Asn Val Ser Lys Ile Leu Pro Arg Val Asp Lys Arg
            2100                2105                2110
Asn Pro Glu His Cys Val Asn Ser Glu Met Glu Lys Thr Cys Ser Lys
        2115                2120                2125
Glu Phe Lys Leu Ser Asn Asn Leu Asn Val Glu Gly Gly Ser Ser Glu
    2130                2135                2140
Asn Asn His Ser Ile Lys Val Ser Pro Tyr Leu Ser Gln Phe Gln Gln
2145                2150                2155                216
Asp Lys Gln Gln Leu Val Leu Gly Thr Lys Val Ser Leu Val Glu Asn
                2165                2170                2175
Ile His Val Leu Gly Lys Glu Gln Ala Ser Pro Lys Asn Val Lys Met
            2180                2185                2190
Glu Ile Gly Lys Thr Glu Thr Phe Ser Asp Val Pro Val Lys Thr Asn
        2195                2200                2205
Ile Glu Val Cys Ser Thr Tyr Ser Lys Asp Ser Glu Asn Tyr Phe Glu
    2210                2215                2220
Thr Glu Ala Val Glu Ile Ala Lys Ala Phe Met Glu Asp Asp Glu Leu
2225                2230                2235                224
Thr Asp Ser Lys Leu Pro Ser His Ala Thr His Ser Leu Phe Thr Cys
                2245                2250                2255
Pro Glu Asn Glu Glu Met Val Leu Ser Asn Ser Arg Ile Gly Lys Arg
            2260                2265                2270
Arg Gly Glu Pro Leu Ile Leu Val Gly Glu Pro Ser Ile Lys Arg Asn
        2275                2280                2285
Leu Leu Asn Glu Phe Asp Arg Ile Ile Glu Asn Gln Glu Lys Ser Leu
    2290                2295                2300
Lys Ala Ser Lys Ser Thr Pro Asp Gly Thr Ile Lys Asp Arg Arg Leu
2305                2310                2315                232
Phe Met His His Val Ser Leu Glu Pro Ile Thr Cys Val Pro Phe Arg
                2325                2330                2335
Thr Thr Lys Glu Arg Gln Glu Ile Gln Asn Pro Asn Phe Thr Ala Pro
            2340                2345                2350
Gly Gln Glu Phe Leu Ser Lys Ser His Leu Tyr Glu His Leu Thr Leu
        2355                2360                2365
Glu Lys Ser Ser Ser Asn Leu Ala Val Ser Gly His Pro Phe Tyr Gln
    2370                2375                2380
Val Ser Ala Thr Arg Asn Glu Lys Met Arg His Leu Ile Thr Thr Gly
2385                2390                2395                240
Arg Pro Thr Lys Val Phe Val Pro Pro Phe Lys Thr Lys Ser His Phe
                2405                2410                2415
His Arg Val Glu Gln Cys Val Arg Asn Ile Asn Leu Glu Glu Asn Arg
            2420                2425                2430
Gln Lys Gln Asn Ile Asp Gly His Gly Ser Asp Asp Ser Lys Asn Lys

2435                    2440                    2445
Ile Asn Asp Asn Glu Ile His Gln Phe Asn Lys Asn Asn Ser Asn Gln
    2450                    2455                    2460
Ala Ala Ala Val Thr Phe Thr Lys Cys Glu Glu Glu Pro Leu Asp Leu
2465                    2470                    2475                    248
Ile Thr Ser Leu Gln Asn Ala Arg Asp Ile Gln Asp Met Arg Ile Lys
                2485                    2490                    2495
Lys Lys Gln Arg Gln Arg Val Phe Pro Gln Pro Gly Ser Leu Tyr Leu
            2500                    2505                    2510
Ala Lys Thr Ser Thr Leu Pro Arg Ile Ser Leu Lys Ala Ala Val Gly
        2515                    2520                    2525
Gly Gln Val Pro Ser Ala Cys Ser His Lys Gln Leu Tyr Thr Tyr Gly
    2530                    2535                    2540
Val Ser Lys His Cys Ile Lys Ile Asn Ser Lys Asn Ala Glu Ser Phe
2545                    2550                    2555                    256
Gln Phe His Thr Glu Asp Tyr Phe Gly Lys Glu Ser Leu Trp Thr Gly
                2565                    2570                    2575
Lys Gly Ile Gln Leu Ala Asp Gly Gly Trp Leu Ile Pro Ser Asn Asp
            2580                    2585                    2590
Gly Lys Ala Gly Lys Glu Glu Phe Tyr Arg Ala Leu Cys Asp Thr Pro
        2595                    2600                    2605
Gly Val Asp Pro Lys Leu Ile Ser Arg Ile Trp Val Tyr Asn His Tyr
    2610                    2615                    2620
Arg Trp Ile Ile Trp Lys Leu Ala Ala Met Glu Cys Ala Phe Pro Lys
2625                    2630                    2635                    264
Glu Phe Ala Asn Arg Cys Leu Ser Pro Glu Arg Val Leu Leu Gln Leu
                2645                    2650                    2655
Lys Tyr Arg Tyr Asp Thr Glu Ile Asp Arg Ser Arg Arg Ser Ala Ile
            2660                    2665                    2670
Lys Lys Ile Met Glu Arg Asp Asp Thr Ala Ala Lys Thr Leu Val Leu
        2675                    2680                    2685
Cys Val Ser Asp Ile Ile Ser Leu Ser Ala Asn Ile Ser Glu Thr Ser
    2690                    2695                    2700
Ser Asn Lys Thr Ser Ser Ala Asp Thr Gln Lys Val Ala Ile Ile Glu
2705                    2710                    2715                    272
Leu Thr Asp Gly Trp Tyr Ala Val Lys Ala Gln Leu Asp Pro Pro Leu
                2725                    2730                    2735
Leu Ala Val Leu Lys Asn Gly Arg Leu Thr Val Gly Gln Lys Ile Ile
            2740                    2745                    2750
Leu His Gly Ala Glu Leu Val Gly Ser Pro Asp Ala Cys Thr Pro Leu
        2755                    2760                    2765
Glu Ala Pro Glu Ser Leu Met Leu Lys Ile Ser Ala Asn Ser Thr Arg
    2770                    2775                    2780
Pro Ala Arg Trp Tyr Thr Lys Leu Gly Phe Phe Pro Asp Pro Arg Pro
2785                    2790                    2795                    280
Phe Pro Leu Pro Leu Ser Ser Leu Phe Ser Asp Gly Gly Asn Val Gly
                2805                    2810                    2815
Cys Val Asp Val Ile Ile Gln Arg Ala Tyr Pro Ile Gln Trp Met Glu
            2820                    2825                    2830
Lys Thr Ser Ser Gly Leu Tyr Ile Phe Arg Asn Glu Arg Glu Glu Glu
        2835                    2840                    2845
Lys Glu Ala Ala Lys Tyr Val Glu Ala Gln Gln Lys Arg Leu Glu Ala
    2850                    2855                    2860
Leu Phe Thr Lys Ile Gln Glu Glu Phe Glu Glu His Glu Glu Asn Thr
2865                    2870                    2875                    288
Thr Lys Pro Tyr Leu Pro Ser Arg Ala Leu Thr Arg Gln Gln Val Arg
                2885                    2890                    2895
Ala Leu Gln Asp Gly Ala Glu Leu Tyr Glu Ala Val Lys Asn Ala Ala
            2900                    2905                    2910
Asp Pro Ala Tyr Leu Glu Gly Tyr Phe Ser Glu Glu Gln Leu Arg Ala
        2915                    2920                    2925

Leu Asn Asn His Arg Gln Met Leu Asn Asp Lys Lys Gln Ala Gln Ile
    2930                2935                2940
Gln Leu Glu Ile Arg Lys Ala Met Glu Ser Ala Glu Gln Lys Glu Gln
2945                2950                2955                296
Gly Leu Ser Arg Asp Val Thr Thr Val Trp Lys Leu Arg Ile Val Ser
                2965                2970                2975
Tyr Ser Lys Lys Glu Lys Asp Ser Val Ile Leu Ser Ile Trp Arg Pro
            2980                2985                2990
Ser Ser Asp Leu Tyr Ser Leu Leu Thr Glu Gly Lys Arg Tyr Arg Ile
        2995                3000                3005
Tyr His Leu Ala Thr Ser Lys Ser Lys Ser Lys Ser Glu Arg Ala Asn
    3010                3015                3020
Ile Gln Leu Ala Ala Thr Lys Lys Thr Gln Tyr Gln Gln Leu Pro Val
3025                3030                3035                304
Ser Asp Glu Ile Leu Phe Gln Ile Tyr Gln Pro Arg Glu Pro Leu His
                3045                3050                3055
Phe Ser Lys Phe Leu Asp Pro Asp Phe Gln Pro Ser Cys Ser Glu Val
            3060                3065                3070
Asp Leu Ile Gly Phe Val Val Ser Val Val Lys Lys Thr Gly Leu Ala
        3075                3080                3085
Pro Phe Val Tyr Leu Ser Asp Glu Cys Tyr Asn Leu Leu Ala Ile Lys
    3090                3095                3100
Phe Trp Ile Asp Leu Asn Glu Asp Ile Ile Lys Pro His Met Leu Ile
3105                3110                3115                312
Ala Ala Ser Asn Leu Gln Trp Arg Pro Glu Ser Lys Ser Gly Leu Leu
                3125                3130                3135
Thr Leu Phe Ala Gly Asp Phe Ser Val Phe Ser Ala Ser Pro Lys Glu
            3140                3145                3150
Gly His Phe Gln Glu Thr Phe Asn Lys Met Lys Asn Thr Val Glu Asn
        3155                3160                3165
Ile Asp Ile Leu Cys Asn Glu Ala Glu Asn Lys Leu Met His Ile Leu
    3170                3175                3180
His Ala Asn Asp Pro Lys Trp Ser Thr Pro Thr Lys Asp Cys Thr Ser
3185                3190                3195                320
Gly Pro Tyr Thr Ala Gln Ile Ile Pro Gly Thr Gly Asn Lys Leu Leu
                3205                3210                3215
Met Ser Ser Pro Asn Cys Glu Ile Tyr Tyr Gln Ser Pro Leu Ser Leu
            3220                3225                3230
Cys Met Ala Lys Arg Lys Ser Val Ser Thr Pro Val Ser Ala Gln Met
        3235                3240                3245
Thr Ser Lys Ser Cys Lys Gly Glu Lys Glu Ile Asp Asp Gln Lys Asn
    3250                3255                3260
Cys Lys Lys Arg Arg Ala Leu Asp Phe Leu Ser Arg Leu Pro Leu Pro
3265                3270                3275                328
Pro Pro Val Ser Pro Ile Cys Thr Phe Val Ser Pro Ala Ala Gln Lys
                3285                3290                3295
Ala Phe Gln Pro Pro Arg Ser Cys Gly Thr Lys Tyr Glu Thr Pro Ile
            3300                3305                3310
Lys Lys Lys Glu Leu Asn Ser Pro Gln Met Thr Pro Phe Lys Lys Phe
        3315                3320                3325
Asn Glu Ile Ser Leu Leu Glu Ser Asn Ser Ile Ala Asp Glu Glu Leu
    3330                3335                3340
Ala Leu Ile Asn Thr Gln Ala Leu Leu Ser Gly Ser Thr Gly Glu Lys
3345                3350                3355                336
Gln Phe Ile Ser Val Ser Glu Ser Thr Arg Thr Ala Pro Thr Ser Ser
                3365                3370                3375
Glu Asp Tyr Leu Arg Leu Lys Arg Arg Cys Thr Thr Ser Leu Ile Lys
            3380                3385                3390
Glu Gln Glu Ser Ser Gln Ala Ser Thr Glu Glu Cys Glu Lys Asn Lys
        3395                3400                3405
Gln Asp Thr Ile Thr Thr Lys Lys Tyr Ile

3410                3415

(2) INFORMATION FOR SEQ ID NO:10:

(i) SEQUENCE CHARACTERISTICS:
(A) LENGTH: 10485 base pairs
(B) TYPE: nucleic acid
(C) STRANDEDNESS: double
(D) TOPOLOGY: linear

(ii) MOLECULE TYPE: cDNA
(ix) FEATURE:

(A) NAME/KEY: Coding Sequence
(B) LOCATION: 229...10482
(D) OTHER INFORMATION: BRCA2 (OMI4)

(xi) SEQUENCE DESCRIPTION: SEQ ID NO:10:

GGTGGCGCGA GCTTCTGAAA CTAGGCGGCA GAGGCGGAGC CGCTGTGGCA CTGCTGCGCC 60
TCTGCTGCGC CTCGGGTGTC TTTTGCGGCG GTGGGTCGCC GCCGGGAGAA GCGTGAGGGG 120
ACAGATTTGT GACCGGCGCG GTTTTTGTCA GCTTACTCCG GCCAAAAAAG AACTGCACCT 180
CTGGAGCGGA CTTATTTACC AAGCATTGGA GGAATATCGT AGGTAAAA ATG CCT ATT 237

1

GGA TCC AAA GAG AGG CCA ACA TTT TTT GAA ATT TTT AAG ACA CGC TGC 285

    5                   10                  15

AAC AAA GCA GAT TTA GGA CCA ATA AGT CTT AAT TGG TTT GAA GAA CTT 333

20                  25                  30                  35

TCT TCA GAA GCT CCA CCC TAT AAT TCT GAA CCT GCA GAA GAA TCT GAA 381

                40                  45                  50

CAT AAA AAC AAC AAT TAC GAA CCA AAC CTA TTT AAA ACT CCA CAA AGG 429

            55                  60                  65

AAA CCA TCT TAT AAT CAG CTG GCT TCA ACT CCA ATA ATA TTC AAA GAG 477

        70                  75                  80

CAA GGG CTG ACT CTG CCG CTG TAC CAA TCT CCT GTA AAA GAA TTA GAT 525

    85                  90                  95

AAA TTC AAA TTA GAC TTA GGA AGG AAT GTT CCC AAT AGT AGA CAT AAA 573

100                 105                 110                 115

AGT CTT CGC ACA GTG AAA ACT AAA ATG GAT CAA GCA GAT GAT GTT TCC 621

                120                 125                 130

TGT CCA CTT CTA AAT TCT TGT CTT AGT GAA AGT CCT GTT GTT CTA CAA 669

            135                 140                 145

TGT ACA CAT GTA ACA CCA CAA AGA GAT AAG TCA GTG GTA TGT GGG AGT     717

        150                 155                 160

TTG TTT CAT ACA CCA AAG TTT GTG AAG GGT CGT CAG ACA CCA AAA CAT     765

    165                 170                 175

ATT TCT GAA AGT CTA GGA GCT GAG GTG GAT CCT GAT ATG TCT TGG TCA     813

180                 185                 190                 195

AGT TCT TTA GCT ACA CCA CCC ACC CTT AGT TCT ACT GTG CTC ATA GTC     861

                200                 205                 210

AGA AAT GAA GAA GCA TCT GAA ACT GTA TTT CCT CAT GAT ACT ACT GCT     909

            215                 220                 225

AAT GTG AAA AGC TAT TTT TCC AAT CAT GAT GAA AGT CTG AAG AAA AAT     957

        230                 235                 240

GAT AGA TTT ATC GCT TCT GTG ACA GAC AGT GAA AAC ACA AAT CAA AGA     1005

    245                 250                 255

GAA GCT GCA AGT CAT GGA TTT GGA AAA ACA TCA GGG AAT TCA TTT AAA     1053

260                 265                 270                 275

GTA AAT AGC TGC AAA GAC CAC ATT GGA AAG TCA ATG CCA AAT GTC CTA     1101

                280                 285                 290

GAA GAT GAA GTA TAT GAA ACA GTT GTA GAT ACC TCT GAA GAA GAT AGT     1149

            295                 300                 305

TTT TCA TTA TGT TTT TCT AAA TGT AGA ACA AAA AAT CTA CAA AAA GTA     1197

        310                 315                 320

AGA ACT AGC AAG ACT AGG AAA AAA ATT TTC CAT GAA GCA AAC GCT GAT     1245

    325                 330                 335

GAA TGT GAA AAA TCT AAA AAC CAA GTG AAA GAA AAA TAC TCA TTT GTA     1293

340                 345                 350                 355

TCT GAA GTG GAA CCA AAT GAT ACT GAT CCA TTA GAT TCA AAT GTA GCA     1341

                360                 365                 370

CAT CAG AAG CCC TTT GAG AGT GGA AGT GAC AAA ATC TCC AAG GAA GTT     1389

            375                 380                 385

GTA CCG TCT TTG GCC TGT GAA TGG TCT CAA CTA ACC CTT TCA GGT CTA     1437

        390                 395                 400

AAT GGA GCC CAG ATG GAG AAA ATA CCC CTA TTG CAT ATT TCT TCA TGT     1485

    405                 410                 415

GAC CAA AAT ATT TCA GAA AAA GAC CTA TTA GAC ACA GAG AAC AAA AGA     1533

420                 425                 430                 435

AAG AAA GAT TTT CTT ACT TCA GAG AAT TCT TTG CCA CGT ATT TCT AGC     1581

                440                 445                 450

CTA CCA AAA TCA GAG AAG CCA TTA AAT GAG GAA ACA GTG GTA AAT AAG     1629

            455                 460                 465

AGA GAT GAA GAG CAG CAT CTT GAA TCT CAT ACA GAC TGC ATT CTT GCA     1677

        470                 475                 480

GTA AAG CAG GCA ATA TCT GGA ACT TCT CCA GTG GCT TCT TCA TTT CAG     1725

    485                 490                 495

GGT ATC AAA AAG TCT ATA TTC AGA ATA AGA GAA TCA CCT AAA GAG ACT     1773

500                 505                 510                 515

TTC AAT GCA AGT TTT TCA GGT CAT ATG ACT GAT CCA AAC TTT AAA AAA     1821

                520                 525                 530

GAA ACT GAA GCC TCT GAA AGT GGA CTG GAA ATA CAT ACT GTT TGC TCA     1869

            535                 540                 545

CAG AAG GAG GAC TCC TTA TGT CCA AAT TTA ATT GAT AAT GGA AGC TGG     1917

        550                 555                 560

CCA GCC ACC ACC ACA CAG AAT TCT GTA GCT TTG AAG AAT GCA GGT TTA     1965

    565                 570                 575

ATA TCC ACT TTG AAA AAG AAA ACA AAT AAG TTT ATT TAT GCT ATA CAT     2013

580                 585                 590                 595

GAT GAA ACA TCT TAT AAA GGA AAA AAA ATA CCG AAA GAC CAA AAA TCA     2061

                600                 605                 610

GAA CTA ATT AAC TGT TCA GCC CAG TTT GAA GCA AAT GCT TTT GAA GCA     2109

            615                 620                 625

CCA CTT ACA TTT GCA AAT GCT GAT TCA GGT TTA TTG CAT TCT TCT GTG     2157


        630                 635                 640

AAA AGA AGC TGT TCA CAG AAT GAT TCT GAA GAA CCA ACT TTG TCC TTA     2205

    645                 650                 655

ACT AGC TCT TTT GGG ACA ATT CTG AGG AAA TGT TCT AGA AAT GAA ACA     2253

660                 665                 670                 675

TGT TCT AAT AAT ACA GTA ATC TCT CAG GAT CTT GAT TAT AAA GAA GCA     2301

                680                 685                 690

AAA TGT AAT AAG GAA AAA CTA CAG TTA TTT ATT ACC CCA GAA GCT GAT     2349

            695                 700                 705

TCT CTG TCA TGC CTG CAG GAA GGA CAG TGT GAA AAT GAT CCA AAA AGC     2397

        710                 715                 720

AAA AAA GTT TCA GAT ATA AAA GAA GAG GTC TTG GCT GCA GCA TGT CAC     2445

    725                 730                 735

CCA GTA CAA CAT TCA AAA GTG GAA TAC AGT GAT ACT GAC TTT CAA TCC     2493

740                 745                 750                 755

CAG AAA AGT CTT TTA TAT GAT CAT GAA AAT GCC AGC ACT CTT ATT TTA     2541

                760                 765                 770

ACT CCT ACT TCC AAG GAT GTT CTG TCA AAC CTA GTC ATG ATT TCT AGA     2589

            775                 780                 785

GGC AAA GAA TCA TAC AAA ATG TCA GAC AAG CTC AAA GGT AAC AAT TAT     2637

        790                 795                 800

GAA TCT GAT GTT GAA TTA ACC AAA AAT ATT CCC ATG GAA AAG AAT CAA     2685

    805                 810                 815

GAT GTA TGT GCT TTA AAT GAA AAT TAT AAA AAC GTT GAG CTG TTG CCA     2733

820                 825                 830                 835

CCT GAA AAA TAC ATG AGA GTA GCA TCA CCT TCA AGA AAG GTA CAA TTC     2781

                840                 845                 850

AAC CAA AAC ACA AAT CTA AGA GTA ATC CAA AAA AAT CAA GAA GAA ACT     2829

            855                 860                 865

ACT TCA ATT TCA AAA ATA ACT GTC AAT CCA GAC TCT GAA GAA CTT TTC     2877


870                 875                 880

TCA GAC AAT GAG AAT AAT TTT GTC TTC CAA GTA GCT AAT GAA AGG AAT      2925

    885                 890                 895

AAT CTT GCT TTA GGA AAT ACT AAG GAA CTT CAT GAA ACA GAC TTG ACT      2973

900                 905                 910                 915

TGT GTA AAC GAA CCC ATT TTC AAG AAC TCT ACC ATG GTT TTA TAT GGA      3021

                920                 925                 930

GAC ACA GGT GAT AAA CAA GCA ACC CAA GTG TCA ATT AAA AAA GAT TTG      3069

            935                 940                 945

GTT TAT GTT CTT GCA GAG GAG AAC AAA AAT AGT GTA AAG CAG CAT ATA      3117

        950                 955                 960

AAA ATG ACT CTA GGT CAA GAT TTA AAA TCG GAC ATC TCC TTG AAT ATA      3165

    965                 970                 975

GAT AAA ATA CCA GAA AAA AAT AAT GAT TAC ATG AAC AAA TGG GCA GGA      3213

980                 985                 990                 995

CTC TTA GGT CCA ATT TCA AAT CAC AGT TTT GGA GGT AGC TTC AGA ACA      3261

                1000                1005                1010

GCT TCA AAT AAG GAA ATC AAG CTC TCT GAA CAT AAC ATT AAG AAG AGC      3309

            1015                1020                1025

AAA ATG TTC TTC AAA GAT ATT GAA GAA CAA TAT CCT ACT AGT TTA GCT      3357

        1030                1035                1040

TGT GTT GAA ATT GTA AAT ACC TTG GCA TTA GAT AAT CAA AAG AAA CTG      3405

    1045                1050                1055

AGC AAG CCT CAG TCA ATT AAT ACT GTA TCT GCA CAT TTA CAG AGT AGT      3453

1060                1065                1070                1075

GTA GTT GTT TCT GAT TGT AAA AAT AGT CAT ATA ACC CCT CAG ATG TTA      3501

                1080                1085                1090

TTT TCC AAG CAG GAT TTT AAT TCA AAC CAT AAT TTA ACA CCT AGC CAA      3549

            1095                1100                1105

AAG GCA GAA ATT ACA GAA CTT TCT ACT ATA TTA GAA GAA TCA GGA AGT      3597

        1110                1115                1120

72

CAG TTT GAA TTT ACT CAG TTT AGA AAG CCA AGC TAC ATA TTG CAG AAG 3645

    1125                1130                1135

AGT ACA TTT GAA GTG CCT GAA AAC CAG ATG ACT ATC TTA AAG ACC ACT 3693

1140                1145                1150                1155

TCT GAG GAA TGC AGA GAT GCT GAT CTT CAT GTC ATA ATG AAT GCC CCA 3741

                1160                1165                1170

TCG ATT GGT CAG GTA GAC AGC AGC AAG CAA TTT GAA GGT ACA GTT GAA 3789

            1175                1180                1185

ATT AAA CGG AAG TTT GCT GGC CTG TTG AAA AAT GAC TGT AAC AAA AGT 3837

        1190                1195                1200

GCT TCT GGT TAT TTA ACA GAT GAA AAT GAA GTG GGG TTT AGG GGC TTT 3885

    1205                1210                1215

TAT TCT GCT CAT GGC ACA AAA CTG AAT GTT TCT ACT GAA GCT CTG CAA 3933

1220                1225                1230                1235

AAA GCT GTG AAA CTG TTT AGT GAT ATT GAG AAT ATT AGT GAG GAA ACT 3981

                1240                1245                1250

TCT GCA GAG GTA CAT CCA ATA AGT TTA TCT TCA AGT AAA TGT CAT GAT 4029

            1255                1260                1265

TCT GTT GTT TCA ATG TTT AAG ATA GAA AAT CAT AAT GAT AAA ACT GTA 4077

        1270                1275                1280

AGT GAA AAA AAT AAT AAA TGC CAA CTG ATA TTA CAA AAT AAT ATT GAA 4125

    1285                1290                1295

ATG ACT ACT GGC ACT TTT GTT GAA GAA ATT ACT GAA AAT TAC AAG AGA 4173

1300                1305                1310                1315

AAT ACT GAA AAT GAA GAT AAC AAA TAT ACT GCT GCC AGT AGA AAT TCT 4221

                1320                1325                1330

CAT AAC TTA GAA TTT GAT GGC AGT GAT TCA AGT AAA AAT GAT ACT GTT 4269

            1335                1340                1345

TGT ATT CAT AAA GAT GAA ACG GAC TTG CTA TTT ACT GAT CAG CAC AAC 4317

        1350                1355                1360

ATA TGT CTT AAA TTA TCT GGC CAG TTT ATG AAG GAG GGA AAC ACT CAG     4365

    1365                1370                1375

ATT AAA GAA GAT TTG TCA GAT TTA ACT TTT TTG GAA GTT GCG AAA GCT     4413

1380                1385                1390                1395

CAA GAA GCA TGT CAT GGT AAT ACT TCA AAT AAA GAA CAG TTA ACT GCT     4461

                1400                1405                1410

ACT AAA ACG GAG CAA AAT ATA AAA GAT TTT GAG ACT TCT GAT ACA TTT     4509

            1415                1420                1425

TTT CAG ACT GCA AGT GGG AAA AAT ATT AGT GTC GCC AAA GAG TCA TTT     4557

        1430                1435                1440

AAT AAA ATT GTA AAT TTC TTT GAT CAG AAA CCA GAA GAA TTG CAT AAC     4605

    1445                1450                1455

TTT TCC TTA AAT TCT GAA TTA CAT TCT GAC ATA AGA AAG AAC AAA ATG     4653

1460                1465                1470                1475

GAC ATT CTA AGT TAT GAG GAA ACA GAC ATA GTT AAA CAC AAA ATA CTG     4701

                1480                1485                1490

AAA GAA AGT GTC CCA GTT GGT ACT GGA AAT CAA CTA GTG ACC TTC CAG     4749

            1495                1500                1505

GGA CAA CCC GAA CGT GAT GAA AAG ATC AAA GAA CCT ACT CTG TTG GGT     4797

        1510                1515                1520

TTT CAT ACA GCT AGC GGG AAA AAA GTT AAA ATT GCA AAG GAA TCT TTG     4845

    1525                1530                1535

GAC AAA GTG AAA AAC CTT TTT GAT GAA AAA GAG CAA GGT ACT AGT GAA     4893

1540                1545                1550                1555

ATC ACC AGT TTT AGC CAT CAA TGG GCA AAG ACC CTA AAG TAC AGA GAG     4941

                1560                1565                1570

GCC TGT AAA GAC CTT GAA TTA GCA TGT GAG ACC ATT GAG ATC ACA GCT     4989

            1575                1580                1585

GCC CCA AAG TGT AAA GAA ATG CAG AAT TCT CTC AAT AAT GAT AAA AAC     5037

        1590                1595                1600

CTT GTT TCT ATT GAG ACT GTG GTG CCA CCT AAG CTC TTA AGT GAT AAT     5085


    1605                1610                1615

TTA TGT AGA CAA ACT GAA AAT CTC AAA ACA TCA AAA AGT ATC TTT TTG 5133

1620                1625                1630                1635

AAA GTT AAA GTA CAT GAA AAT GTA GAA AAA GAA ACA GCA AAA AGT CCT 5181

                1640                1645                1650

GCA ACT TGT TAC ACA AAT CAG TCC CCT TAT TCA GTC ATT GAA AAT TCA 5229

            1655                1660                1665

GCC TTA GCT TTT TAC ACA AGT TGT AGT AGA AAA ACT TCT GTG AGT CAG 5277

        1670                1675                1680

ACT TCA TTA CTT GAA GCA AAA AAA TGG CTT AGA GAA GGA ATA TTT GAT 5325

    1685                1690                1695

GGT CAA CCA GAA AGA ATA AAT ACT GCA GAT TAT GTA GGA AAT TAT TTG 5373

1700                1705                1710                1715

TAT GAA AAT AAT TCA AAC AGT ACT ATA GCT GAA AAT GAC AAA AAT CAT 5421

                1720                1725                1730

CTC TCC GAA AAA CAA GAT ACT TAT TTA AGT AAC AGT AGC ATG TCT AAC 5469

            1735                1740                1745

AGC TAT TCC TAC CAT TCT GAT GAG GTA TAT AAT GAT TCA GGA TAT CTC 5517

        1750                1755                1760

TCA AAA AAT AAA CTT GAT TCT GGT ATT GAG CCA GTA TTG AAG AAT GTT 5565

    1765                1770                1775

GAA GAT CAA AAA AAC ACT AGT TTT TCC AAA GTA ATA TCC AAT GTA AAA 5613

1780                1785                1790                1795

GAT GCA AAT GCA TAC CCA CAA ACT GTA AAT GAA GAT ATT TGC GTT GAG 5661

                1800                1805                1810

GAA CTT GTG ACT AGC TCT TCA CCC TGC AAA AAT AAA AAT GCA GCC ATT 5709

            1815                1820                1825

AAA TTG TCC ATA TCT AAT AGT AAT AAT TTT GAG GTA GGG CCA CCT GCA 5757

        1830                1835                1840

TTT AGG ATA GCC AGT GGT AAA ATC GTT TGT GTT TCA CAT GAA ACA ATT 5805


1845                1850                1855

AAA AAA GTG AAA GAC ATA TTT ACA GAC AGT TTC AGT AAA GTA ATT AAG     5853

1860                1865                1870                1875

GAA AAC AAC GAG AAT AAA TCA AAA ATT TGC CAA ACG AAA ATT ATG GCA     5901

                1880                1885                1890

GGT TGT TAC GAG GCA TTG GAT GAT TCA GAG GAT ATT CTT CAT AAC TCT     5949

            1895                1900                1905

CTA GAT AAT GAT GAA TGT AGC ACG CAT TCA CAT AAG GTT TTT GCT GAC     5997

        1910                1915                1920

ATT CAG AGT GAA GAA ATT TTA CAA CAT AAC CAA AAT ATG TCT GGA TTG     6045

    1925                1930                1935

GAG AAA GTT TCT AAA ATA TCA CCT TGT GAT GTT AGT TTG GAA ACT TCA     6093

1940                1945                1950                1955

GAT ATA TGT AAA TGT AGT ATA GGG AAG CTT CAT AAG TCA GTC TCA TCT     6141

                1960                1965                1970

GCA AAT ACT TGT GGG ATT TTT AGC ACA GCA AGT GGA AAA TCT GTC CAG     6189

            1975                1980                1985

GTA TCA GAT GCT TCA TTA CAA AAC GCA AGA CAA GTG TTT TCT GAA ATA     6237

        1990                1995                2000

GAA GAT AGT ACC AAG CAA GTC TTT TCC AAA GTA TTG TTT AAA AGT AAC     6285

    2005                2010                2015

GAA CAT TCA GAC CAG CTC ACA AGA GAA GAA AAT ACT GCT ATA CGT ACT     6333

2020                2025                2030                2035

CCA GAA CAT TTA ATA TCC CAA AAA GGC TTT TCA TAT AAT GTG GTA AAT     6381

                2040                2045                2050

TCA TCT GCT TTC TCT GGA TTT AGT ACA GCA AGT GGA AAG CAA GTT TCC     6429

            2055                2060                2065

ATT TTA GAA AGT TCC TTA CAC AAA GTT AAG GGA GTG TTA GAG GAA TTT     6477

        2070                2075                2080

GAT TTA ATC AGA ACT GAG CAT AGT CTT CAC TAT TCA CCT ACG TCT AGA     6525

    2085                2090                2095

CAA AAT GTA TCA AAA ATA CTT CCT CGT GTT GAT AAG AGA AAC CCA GAG 6573

2100                2105                2110                2115

CAC TGT GTA AAC TCA GAA ATG GAA AAA ACC TGC AGT AAA GAA TTT AAA 6621

                2120                2125                2130

TTA TCA AAT AAC TTA AAT GTT GAA GGT GGT TCT TCA GAA AAT AAT CAC 6669

            2135                2140                2145

TCT ATT AAA GTT TCT CCA TAT CTC TCT CAA TTT CAA CAA GAC AAA CAA 6717

        2150                2155                2160

CAG TTG GTA TTA GGA ACC AAA GTC TCA CTT GTT GAG AAC ATT CAT GTT 6765

    2165                2170                2175

TTG GGA AAA GAA CAG GCT TCA CCT AAA AAC GTA AAA ATG GAA ATT GGT 6813

2180                2185                2190                2195

AAA ACT GAA ACT TTT TCT GAT GTT CCT GTG AAA ACA AAT ATA GAA GTT 6861

                2200                2205                2210

TGT TCT ACT TAC TCC AAA GAT TCA GAA AAC TAC TTT GAA ACA GAA GCA 6909

            2215                2220                2225

GTA GAA ATT GCT AAA GCT TTT ATG GAA GAT GAT GAA CTG ACA GAT TCT 6957

        2230                2235                2240

AAA CTG CCA AGT CAT GCC ACA CAT TCT CTT TTT ACA TGT CCC GAA AAT 7005

    2245                2250                2255

GAG GAA ATG GTT TTG TCA AAT TCA AGA ATT GGA AAA AGA AGA GGA GAG 7053

2260                2265                2270                2275

CCC CTT ATC TTA GTG GGA GAA CCC TCA ATC AAA AGA AAC TTA TTA AAT 7101

                2280                2285                2290

GAA TTT GAC AGG ATA ATA GAA AAT CAA GAA AAA TCC TTA AAG GCT TCA 7149

            2295                2300                2305

AAA AGC ACT CCA GAT GGC ACA ATA AAA GAT CGA AGA TTG TTT ATG CAT 7197

        2310                2315                2320

CAT GTT TCT TTA GAG CCG ATT ACC TGT GTA CCC TTT CGC ACA ACT AAG 7245

    2325                2330                2335

GAA CGT CAA GAG ATA CAG AAT CCA AAT TTT ACC GCA CCT GGT CAA GAA     7293

2340                2345                2350                2355

TTT CTG TCT AAA TCT CAT TTG TAT GAA CAT CTG ACT TTG GAA AAA TCT     7341

                2360                2365                2370

TCA AGC AAT TTA GCA GTT TCA GGA CAT CCA TTT TAT CAA GTT TCT GCT     7389

            2375                2380                2385

ACA AGA AAT GAA AAA ATG AGA CAC TTG ATT ACT ACA GGC AGA CCA ACC     7437

        2390                2395                2400

AAA GTC TTT GTT CCA CCT TTT AAA ACT AAA TCG CAT TTT CAC AGA GTT     7485

    2405                2410                2415

GAA CAG TGT GTT AGG AAT ATT AAC TTG GAG GAA AAC AGA CAA AAG CAA     7533

2420                2425                2430                2435

AAC ATT GAT GGA CAT GGC TCT GAT GAT AGT AAA AAT AAG ATT AAT GAC     7581

                2440                2445                2450

AAT GAG ATT CAT CAG TTT AAC AAA AAC AAC TCC AAT CAA GCA GCA GCT     7629

            2455                2460                2465

GTA ACT TTC ACA AAG TGT GAA GAA GAA CCT TTA GAT TTA ATT ACA AGT     7677

        2470                2475                2480

CTT CAG AAT GCC AGA GAT ATA CAG GAT ATG CGA ATT AAG AAG AAA CAA     7725

    2485                2490                2495

AGG CAA CGC GTC TTT CCA CAG CCA GGC AGT CTG TAT CTT GCA AAA ACA     7773

2500                2505                2510                2515

TCC ACT CTG CCT CGA ATC TCT CTG AAA GCA GCA GTA GGA GGC CAA GTT     7821

                2520                2525                2530

CCC TCT GCG TGT TCT CAT AAA CAG CTG TAT ACG TAT GGC GTT TCT AAA     7869

            2535                2540                2545

CAT TGC ATA AAA ATT AAC AGC AAA AAT GCA GAG TCT TTT CAG TTT CAC     7917

        2550                2555                2560

ACT GAA GAT TAT TTT GGT AAG GAA AGT TTA TGG ACT GGA AAA GGA ATA     7965

    2565                2570                2575

CAG TTG GCT GAT GGT GGA TGG CTC ATA CCC TCC AAT GAT GGA AAG GCT     8013


2580                2585                2590                2595

GGA AAA GAA GAA TTT TAT AGG GCT CTG TGT GAC ACT CCA GGT GTG GAT     8061

                2600                2605                2610

CCA AAG CTT ATT TCT AGA ATT TGG GTT TAT AAT CAC TAT AGA TGG ATC     8109

            2615                2620                2625

ATA TGG AAA CTG GCA GCT ATG GAA TGT GCC TTT CCT AAG GAA TTT GCT     8157

        2630                2635                2640

AAT AGA TGC CTA AGC CCA GAA AGG GTG CTT CTT CAA CTA AAA TAC AGA     8205

    2645                2650                2655

TAT GAT ACG GAA ATT GAT AGA AGC AGA AGA TCG GCT ATA AAA AAG ATA     8253

2660                2665                2670                2675

ATG GAA AGG GAT GAC ACA GCT GCA AAA ACA CTT GTT CTC TGT GTT TCT     8301

                2680                2685                2690

GAC ATA ATT TCA TTG AGC GCA AAT ATA TCT GAA ACT TCT AGC AAT AAA     8349

            2695                2700                2705

ACT AGT AGT GCA GAT ACC CAA AAA GTG GCC ATT ATT GAA CTT ACA GAT     8397

        2710                2715                2720

GGG TGG TAT GCT GTT AAG GCC CAG TTA GAT CCT CCC CTC TTA GCT GTC     8445

    2725                2730                2735

TTA AAG AAT GGC AGA CTG ACA GTT GGT CAG AAG ATT ATT CTT CAT GGA     8493

2740                2745                2750                2755

GCA GAA CTG GTG GGC TCT CCT GAT GCC TGT ACA CCT CTT GAA GCC CCA     8541

                2760                2765                2770

GAA TCT CTT ATG TTA AAG ATT TCT GCT AAC AGT ACT CGG CCT GCT CGC     8589

            2775                2780                2785

TGG TAT ACC AAA CTT GGA TTC TTT CCT GAC CCT AGA CCT TTT CCT CTG     8637

        2790                2795                2800

CCC TTA TCA TCG CTT TTC AGT GAT GGA GGA AAT GTT GGT TGT GTT GAT     8685

    2805                2810                2815

GTA ATT ATT CAA AGA GCA TAC CCT ATA CAG TGG ATG GAG AAG ACA TCA     8733


2820                    2825                    2830                    2835

TCT GGA TTA TAC ATA TTT CGC AAT GAA AGA GAG GAA GAA AAG GAA GCA     8781

                2840                    2845                    2850

GCA AAA TAT GTG GAG GCC CAA CAA AAG AGA CTA GAA GCC TTA TTC ACT     8829

            2855                    2860                    2865

AAA ATT CAG GAG GAA TTT GAA GAA CAT GAA GAA AAC ACA ACA AAA CCA     8877

        2870                    2875                    2880

TAT TTA CCA TCA CGT GCA CTA ACA AGA CAG CAA GTT CGT GCT TTG CAA     8925

    2885                    2890                    2895

GAT GGT GCA GAG CTT TAT GAA GCA GTG AAG AAT GCA GCA GAC CCA GCT     8973

2900                    2905                    2910                    2915

TAC CTT GAG GGT TAT TTC AGT GAA GAG CAG TTA AGA GCC TTG AAT AAT     9021

                2920                    2925                    2930

CAC AGG CAA ATG TTG AAT GAT AAG AAA CAA GCT CAG ATC CAG TTG GAA     9069

            2935                    2940                    2945

ATT AGG AAG GCC ATG GAA TCT GCT GAA CAA AAG GAA CAA GGT TTA TCA     9117

        2950                    2955                    2960

AGG GAT GTC ACA ACC GTG TGG AAG TTG CGT ATT GTA AGC TAT TCA AAA     9165

    2965                    2970                    2975

AAA GAA AAA GAT TCA GTT ATA CTG AGT ATT TGG CGT CCA TCA TCA GAT     9213

2980                    2985                    2990                    2995

TTA TAT TCT CTG TTA ACA GAA GGA AAG AGA TAC AGA ATT TAT CAT CTT     9261

                3000                    3005                    3010

GCA ACT TCA AAA TCT AAA AGT AAA TCT GAA AGA GCT AAC ATA CAG TTA     9309

            3015                    3020                    3025

GCA GCG ACA AAA AAA ACT CAG TAT CAA CAA CTA CCG GTT TCA GAT GAA     9357

        3030                    3035                    3040

ATT TTA TTT CAG ATT TAC CAG CCA CGG GAG CCC CTT CAC TTC AGC AAA     9405

    3045                    3050                    3055

TTT TTA GAT CCA GAC TTT CAG CCA TCT TGT TCT GAG GTG GAC CTA ATA     9453

3060                    3065                    3070                    3075

GGA TTT GTC GTT TCT GTT GTG AAA AAA ACA GGA CTT GCC CCT TTC GTC 9501

                3080                3085                3090

TAT TTG TCA GAC GAA TGT TAC AAT TTA CTG GCA ATA AAG TTT TGG ATA 9549

            3095                3100                3105

GAC CTT AAT GAG GAC ATT ATT AAG CCT CAT ATG TTA ATT GCT GCA AGC 9597

        3110                3115                3120

AAC CTC CAG TGG CGA CCA GAA TCC AAA TCA GGC CTT CTT ACT TTA TTT 9645

    3125                3130                3135

GCT GGA GAT TTT TCT GTG TTT TCT GCT AGT CCA AAA GAG GGC CAC TTT 9693

3140                3145                3150                3155

CAA GAG ACA TTC AAC AAA ATG AAA AAT ACT GTT GAG AAT ATT GAC ATA 9741

                3160                3165                3170

CTT TGC AAT GAA GCA GAA AAC AAG CTT ATG CAT ATA CTG CAT GCA AAT 9789

            3175                3180                3185

GAT CCC AAG TGG TCC ACC CCA ACT AAA GAC TGT ACT TCA GGG CCG TAC 9837

        3190                3195                3200

ACT GCT CAA ATC ATT CCT GGT ACA GGA AAC AAG CTT CTG ATG TCT TCT 9885

    3205                3210                3215

CCT AAT TGT GAG ATA TAT TAT CAA AGT CCT TTA TCA CTT TGT ATG GCC 9933

3220                3225                3230                3235

AAA AGG AAG TCT GTT TCC ACA CCT GTC TCA GCC CAG ATG ACT TCA AAG 9981

                3240                3245                3250

TCT TGT AAA GGG GAG AAA GAG ATT GAT GAC CAA AAG AAC TGC AAA AAG 10029

            3255                3260                3265

AGA AGA GCC TTG GAT TTC TTG AGT AGA CTG CCT TTA CCT CCA CCT GTT 10077

        3270                3275                3280

AGT CCC ATT TGT ACA TTT GTT TCT CCG GCT GCA CAG AAG GCA TTT CAG 10125

    3285                3290                3295

CCA CCA AGG AGT TGT GGC ACC AAA TAC GAA ACA CCC ATA AAG AAA AAA 10173

3300                3305                3310                3315

GAA CTG AAT TCT CCT CAG ATG ACT CCA TTT AAA AAA TTC AAT GAA ATT    10221

                3320                3325                3330

TCT CTT TTG GAA AGT AAT TCA ATA GCT GAC GAA GAA CTT GCA TTG ATA    10269

            3335                3340                3345

AAT ACC CAA GCT CTT TTG TCT GGT TCA ACA GGA GAA AAA CAA TTT ATA    10317

        3350                3355                3360

TCT GTC AGT GAA TCC ACT AGG ACT GCT CCC ACC AGT TCA GAA GAT TAT    10365

    3365                3370                3375

CTC AGA CTG AAA CGA CGT TGT ACT ACA TCT CTG ATC AAA GAA CAG GAG    10413

3380                3385                3390                3395

AGT TCC CAG GCC AGT ACG GAA GAA TGT GAG AAA AAT AAG CAG GAC ACA    10461

                3400                3405                3410

ATT ACA ACT AAA AAA TAT ATC TAA                                    10485

            3415

(2) INFORMATION FOR SEQ ID NO:11:

(i) SEQUENCE CHARACTERISTICS:
(A) LENGTH: 3418 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:11:

(2) INFORMATION FOR SEQ ID NO:12:

(i) SEQUENCE CHARACTERISTICS:
(A) LENGTH: 10485 base pairs
(B) TYPE: nucleic acid
(C) STRANDEDNESS: double
(D) TOPOLOGY: linear

(ii) MOLECULE TYPE: cDNA
(ix) FEATURE:

(A) NAME/KEY: Coding Sequence
(B) LOCATION: 229...10482
(D) OTHER INFORMATION: BRCA2 (OMI5)

(xi) SEQUENCE DESCRIPTION: SEQ ID NO:12:

GGTGGCGCGA GCTTCTGAAA CTAGGCGGCA GAGGCGGAGC CGCTGTGGCA CTGCTGCGCC 60
TCTGCTGCGC CTCGGGTGTC TTTTGCGGCG GTGGGTCGCC GCCGGGAGAA GCGTGAGGGG 120
ACAGATTTGT GACCGGCGCG GTTTTTGTCA GCTTACTCCG GCCAAAAAAG AACTGCACCT 180
CTGGAGCGGA CTTATTTACC AAGCATTGGA GGAATATCGT AGGTAAAA ATG CCT ATT 237
Met Pro Ile
1

GGA TCC AAA GAG AGG CCA ACA TTT TTT GAA ATT TTT AAG ACA CGC TGC 285
Gly Ser Lys Glu Arg Pro Thr Phe Phe Glu Ile Phe Lys Thr Arg Cys
    5                   10                  15

AAC AAA GCA GAT TTA GGA CCA ATA AGT CTT AAT TGG TTT GAA GAA CTT 333
Asn Lys Ala Asp Leu Gly Pro Ile Ser Leu Asn Trp Phe Glu Glu Leu
20                  25                  30                  35

TCT TCA GAA GCT CCA CCC TAT AAT TCT GAA CCT GCA GAA GAA TCT GAA 381
Ser Ser Glu Ala Pro Pro Tyr Asn Ser Glu Pro Ala Glu Glu Ser Glu
                40                  45                  50

CAT AAA AAC AAC AAT TAC GAA CCA AAC CTA TTT AAA ACT CCA CAA AGG 429
His Lys Asn Asn Asn Tyr Glu Pro Asn Leu Phe Lys Thr Pro Gln Arg
            55                  60                  65

AAA CCA TCT TAT AAT CAG CTG GCT TCA ACT CCA ATA ATA TTC AAA GAG 477
Lys Pro Ser Tyr Asn Gln Leu Ala Ser Thr Pro Ile Ile Phe Lys Glu
        70                  75                  80

CAA GGG CTG ACT CTG CCG CTG TAC CAA TCT CCT GTA AAA GAA TTA GAT 525
Gln Gly Leu Thr Leu Pro Leu Tyr Gln Ser Pro Val Lys Glu Leu Asp
    85                  90                  95

AAA TTC AAA TTA GAC TTA GGA AGG AAT GTT CCC AAT AGT AGA CAT AAA 573
Lys Phe Lys Leu Asp Leu Gly Arg Asn Val Pro Asn Ser Arg His Lys
100                 105                 110                 115

AGT CTT CGC ACA GTG AAA ACT AAA ATG GAT CAA GCA GAT GAT GTT TCC 621
Ser Leu Arg Thr Val Lys Thr Lys Met Asp Gln Ala Asp Asp Val Ser
                120                 125                 130

TGT CCA CTT CTA AAT TCT TGT CTT AGT GAA AGT CCT GTT GTT CTA CAA 669
Cys Pro Leu Leu Asn Ser Cys Leu Ser Glu Ser Pro Val Val Leu Gln
            135                 140                 145

TGT ACA CAT GTA ACA CCA CAA AGA GAT AAG TCA GTG GTA TGT GGG AGT 717
Cys Thr His Val Thr Pro Gln Arg Asp Lys Ser Val Val Cys Gly Ser
        150                 155                 160

TTG TTT CAT ACA CCA AAG TTT GTG AAG GGT CGT CAG ACA CCA AAA CAT 765
Leu Phe His Thr Pro Lys Phe Val Lys Gly Arg Gln Thr Pro Lys His
    165                 170                 175

ATT TCT GAA AGT CTA GGA GCT GAG GTG GAT CCT GAT ATG TCT TGG TCA 813
Ile Ser Glu Ser Leu Gly Ala Glu Val Asp Pro Asp Met Ser Trp Ser
180                 185                 190                 195

AGT TCT TTA GCT ACA CCA CCC ACC CTT AGT TCT ACT GTG CTC ATA GTC 861
Ser Ser Leu Ala Thr Pro Pro Thr Leu Ser Ser Thr Val Leu Ile Val
                200                 205                 210

AGA AAT GAA GAA GCA TCT GAA ACT GTA TTT CCT CAT GAT ACT ACT GCT 909
Arg Asn Glu Glu Ala Ser Glu Thr Val Phe Pro His Asp Thr Thr Ala

            215                 220                 225

AAT GTG AAA AGC TAT TTT TCC AAT CAT GAT GAA AGT CTG AAG AAA AAT 957
Asn Val Lys Ser Tyr Phe Ser Asn His Asp Glu Ser Leu Lys Lys Asn
        230                 235                 240

GAT AGA TTT ATC GCT TCT GTG ACA GAC AGT GAA AAC ACA AAT CAA AGA 1005
Asp Arg Phe Ile Ala Ser Val Thr Asp Ser Glu Asn Thr Asn Gln Arg
    245                 250                 255

GAA GCT GCA AGT CAT GGA TTT GGA AAA ACA TCA GGG AAT TCA TTT AAA 1053
Glu Ala Ala Ser His Gly Phe Gly Lys Thr Ser Gly Asn Ser Phe Lys
260                 265                 270                 275

GTA AAT AGC TGC AAA GAC CAC ATT GGA AAG TCA ATG CCA CAT GTC CTA 1101
Val Asn Ser Cys Lys Asp His Ile Gly Lys Ser Met Pro His Val Leu
                280                 285                 290

GAA GAT GAA GTA TAT GAA ACA GTT GTA GAT ACC TCT GAA GAA GAT AGT 1149
Glu Asp Glu Val Tyr Glu Thr Val Val Asp Thr Ser Glu Glu Asp Ser
            295                 300                 305

TTT TCA TTA TGT TTT TCT AAA TGT AGA ACA AAA AAT CTA CAA AAA GTA 1197
Phe Ser Leu Cys Phe Ser Lys Cys Arg Thr Lys Asn Leu Gln Lys Val
        310                 315                 320

AGA ACT AGC AAG ACT AGG AAA AAA ATT TTC CAT GAA GCA AAC GCT GAT 1245
Arg Thr Ser Lys Thr Arg Lys Lys Ile Phe His Glu Ala Asn Ala Asp
    325                 330                 335

GAA TGT GAA AAA TCT AAA AAC CAA GTG AAA GAA AAA TAC TCA TTT GTA 1293
Glu Cys Glu Lys Ser Lys Asn Gln Val Lys Glu Lys Tyr Ser Phe Val
340                 345                 350                 355

TCT GAA GTG GAA CCA AAT GAT ACT GAT CCA TTA GAT TCA AAT GTA GCA 1341
Ser Glu Val Glu Pro Asn Asp Thr Asp Pro Leu Asp Ser Asn Val Ala
                360                 365                 370

CAT CAG AAG CCC TTT GAG AGT GGA AGT GAC AAA ATC TCC AAG GAA GTT 1389
His Gln Lys Pro Phe Glu Ser Gly Ser Asp Lys Ile Ser Lys Glu Val
            375                 380                 385

GTA CCG TCT TTG GCC TGT GAA TGG TCT CAA CTA ACC CTT TCA GGT CTA 1437
Val Pro Ser Leu Ala Cys Glu Trp Ser Gln Leu Thr Leu Ser Gly Leu
        390                 395                 400

AAT GGA GCC CAG ATG GAG AAA ATA CCC CTA TTG CAT ATT TCT TCA TGT 1485
Asn Gly Ala Gln Met Glu Lys Ile Pro Leu Leu His Ile Ser Ser Cys
    405                 410                 415

GAC CAA AAT ATT TCA GAA AAA GAC CTA TTA GAC ACA GAG AAC AAA AGA 1533
Asp Gln Asn Ile Ser Glu Lys Asp Leu Leu Asp Thr Glu Asn Lys Arg
420                 425                 430                 435

AAG AAA GAT TTT CTT ACT TCA GAG AAT TCT TTG CCA CGT ATT TCT AGC 1581
Lys Lys Asp Phe Leu Thr Ser Glu Asn Ser Leu Pro Arg Ile Ser Ser
                440                 445                 450

CTA CCA AAA TCG GAG AAG CCA TTA AAT GAG GAA ACA GTG GTA AAT AAG 1629
Leu Pro Lys Ser Glu Lys Pro Leu Asn Glu Glu Thr Val Val Asn Lys
            455                 460                 465

AGA GAT GAA GAG CAG CAT CTT GAA TCT CAT ACA GAC TGC ATT CTT GCA     1677
Arg Asp Glu Glu Gln His Leu Glu Ser His Thr Asp Cys Ile Leu Ala
        470                 475                 480

GTA AAG CAG GCA ATA TCT GGA ACT TCT CCA GTG GCT TCT TCA TTT CAG     1725
Val Lys Gln Ala Ile Ser Gly Thr Ser Pro Val Ala Ser Ser Phe Gln
    485                 490                 495

GGT ATC AAA AAG TCT ATA TTC AGA ATA AGA GAA TCA CCT AAA GAG ACT     1773
Gly Ile Lys Lys Ser Ile Phe Arg Ile Arg Glu Ser Pro Lys Glu Thr
500                 505                 510                 515

TTC AAT GCA AGT TTT TCA GGT CAT ATG ACT GAT CCA AAC TTT AAA AAA     1821
Phe Asn Ala Ser Phe Ser Gly His Met Thr Asp Pro Asn Phe Lys Lys
                520                 525                 530

GAA ACT GAA GCC TCT GAA AGT GGA CTG GAA ATA CAT ACT GTT TGC TCA     1869
Glu Thr Glu Ala Ser Glu Ser Gly Leu Glu Ile His Thr Val Cys Ser
            535                 540                 545

CAG AAG GAG GAC TCC TTA TGT CCA AAT TTA ATT GAT AAT GGA AGC TGG     1917
Gln Lys Glu Asp Ser Leu Cys Pro Asn Leu Ile Asp Asn Gly Ser Trp
        550                 555                 560

CCA GCC ACC ACC ACA CAG AAT TCT GTA GCT TTG AAG AAT GCA GGT TTA     1965
Pro Ala Thr Thr Thr Gln Asn Ser Val Ala Leu Lys Asn Ala Gly Leu
    565                 570                 575

ATA TCC ACT TTG AAA AAG AAA ACA AAT AAG TTT ATT TAT GCT ATA CAT     2013
Ile Ser Thr Leu Lys Lys Lys Thr Asn Lys Phe Ile Tyr Ala Ile His
580                 585                 590                 595

GAT GAA ACA TCT TAT AAA GGA AAA AAA ATA CCG AAA GAC CAA AAA TCA     2061
Asp Glu Thr Ser Tyr Lys Gly Lys Lys Ile Pro Lys Asp Gln Lys Ser
                600                 605                 610

GAA CTA ATT AAC TGT TCA GCC CAG TTT GAA GCA AAT GCT TTT GAA GCA     2109
Glu Leu Ile Asn Cys Ser Ala Gln Phe Glu Ala Asn Ala Phe Glu Ala
            615                 620                 625

CCA CTT ACA TTT GCA AAT GCT GAT TCA GGT TTA TTG CAT TCT TCT GTG     2157
Pro Leu Thr Phe Ala Asn Ala Asp Ser Gly Leu Leu His Ser Ser Val
        630                 635                 640

AAA AGA AGC TGT TCA CAG AAT GAT TCT GAA GAA CCA ACT TTG TCC TTA     2205
Lys Arg Ser Cys Ser Gln Asn Asp Ser Glu Glu Pro Thr Leu Ser Leu
    645                 650                 655

ACT AGC TCT TTT GGG ACA ATT CTG AGG AAA TGT TCT AGA AAT GAA ACA     2253
Thr Ser Ser Phe Gly Thr Ile Leu Arg Lys Cys Ser Arg Asn Glu Thr
660                 665                 670                 675

TGT TCT AAT AAT ACA GTA ATC TCT CAG GAT CTT GAT TAT AAA GAA GCA     2301
Cys Ser Asn Asn Thr Val Ile Ser Gln Asp Leu Asp Tyr Lys Glu Ala
                680                 685                 690

AAA TGT AAT AAG GAA AAA CTA CAG TTA TTT ATT ACC CCA GAA GCT GAT     2349
Lys Cys Asn Lys Glu Lys Leu Gln Leu Phe Ile Thr Pro Glu Ala Asp
            695                 700                 705

TCT CTG TCA TGC CTG CAG GAA GGA CAG TGT GAA AAT GAT CCA AAA AGC     2397
Ser Leu Ser Cys Leu Gln Glu Gly Gln Cys Glu Asn Asp Pro Lys Ser
        710                 715                 720

AAA AAA GTT TCA GAT ATA AAA GAA GAG GTC TTG GCT GCA GCA TGT CAC     2445
Lys Lys Val Ser Asp Ile Lys Glu Glu Val Leu Ala Ala Ala Cys His
    725                 730                 735

CCA GTA CAA CAC TCA AAA GTG GAA TAC AGT GAT ACT GAC TTT CAA TCC     2493
Pro Val Gln His Ser Lys Val Glu Tyr Ser Asp Thr Asp Phe Gln Ser
740                 745                 750                 755

CAG AAA AGT CTT TTA TAT GAT CAT GAA AAT GCC AGC ACT CTT ATT TTA     2541
Gln Lys Ser Leu Leu Tyr Asp His Glu Asn Ala Ser Thr Leu Ile Leu
                760                 765                 770

ACT CCT ACT TCC AAG GAT GTT CTG TCA AAC CTA GTC ATG ATT TCT AGA     2589
Thr Pro Thr Ser Lys Asp Val Leu Ser Asn Leu Val Met Ile Ser Arg
            775                 780                 785

GGC AAA GAA TCA TAC AAA ATG TCA GAC AAG CTC AAA GGT AAC AAT TAT     2637
Gly Lys Glu Ser Tyr Lys Met Ser Asp Lys Leu Lys Gly Asn Asn Tyr
        790                 795                 800

GAA TCT GAT GTT GAA TTA ACC AAA AAT ATT CCC ATG GAA AAG AAT CAA     2685
Glu Ser Asp Val Glu Leu Thr Lys Asn Ile Pro Met Glu Lys Asn Gln
    805                 810                 815

GAT GTA TGT GCT TTA AAT GAA AAT TAT AAA AAC GTT GAG CTG TTG CCA     2733
Asp Val Cys Ala Leu Asn Glu Asn Tyr Lys Asn Val Glu Leu Leu Pro
820                 825                 830                 835

CCT GAA AAA TAC ATG AGA GTA GCA TCA CCT TCA AGA AAG GTA CAA TTC     2781
Pro Glu Lys Tyr Met Arg Val Ala Ser Pro Ser Arg Lys Val Gln Phe
                840                 845                 850

AAC CAA AAC ACA AAT CTA AGA GTA ATC CAA AAA AAT CAA GAA GAA ACT     2829
Asn Gln Asn Thr Asn Leu Arg Val Ile Gln Lys Asn Gln Glu Glu Thr
            855                 860                 865

ACT TCA ATT TCA AAA ATA ACT GTC AAT CCA GAC TCT GAA GAA CTT TTC     2877
Thr Ser Ile Ser Lys Ile Thr Val Asn Pro Asp Ser Glu Glu Leu Phe
        870                 875                 880

TCA GAC AAT GAG AAT AAT TTT GTC TTC CAA ATA GCT AAT GAA AGG AAT     2925
Ser Asp Asn Glu Asn Asn Phe Val Phe Gln Ile Ala Asn Glu Arg Asn
    885                 890                 895

AAT CTT GCT TTA GGA AAT ACT AAG GAA CTT CAT GAA ACA GAC TTG ACT     2973
Asn Leu Ala Leu Gly Asn Thr Lys Glu Leu His Glu Thr Asp Leu Thr
900                 905                 910                 915

TGT GTA AAC GAA CCC ATT TTC AAG AAC TCT ACC ATG GTT TTA TAT GGA     3021
Cys Val Asn Glu Pro Ile Phe Lys Asn Ser Thr Met Val Leu Tyr Gly
                920                 925                 930

GAC ACA GGT GAT AAA CAA GCA ACC CAA GTG TCA ATT AAA AAA GAT TTG     3069
Asp Thr Gly Asp Lys Gln Ala Thr Gln Val Ser Ile Lys Lys Asp Leu
            935                 940                 945

GTT TAT GTT CTT GCA GAG GAG AAC AAA AAT AGT GTA AAG CAG CAT ATA     3117

Val Tyr Val Leu Ala Glu Glu Asn Lys Asn Ser Val Lys Gln His Ile
        950                 955                 960

AAA ATG ACT CTA GGT CAA GAT TTA AAA TCG GAC ATC TCC TTG AAT ATA     3165
Lys Met Thr Leu Gly Gln Asp Leu Lys Ser Asp Ile Ser Leu Asn Ile
    965                 970                 975

GAT AAA ATA CCA GAA AAA AAT AAT GAT TAC ATG GAC AAA TGG GCA GGA     3213
Asp Lys Ile Pro Glu Lys Asn Asn Asp Tyr Met Asp Lys Trp Ala Gly
980                 985                 990                 995

CTC TTA GGT CCA ATT TCA AAT CAC AGT TTT GGA GGT AGC TTC AGA ACA     3261
Leu Leu Gly Pro Ile Ser Asn His Ser Phe Gly Gly Ser Phe Arg Thr
                1000                1005                1010

GCT TCA AAT AAG GAA ATC AAG CTC TCT GAA CAT AAC ATT AAG AAG AGC     3309
Ala Ser Asn Lys Glu Ile Lys Leu Ser Glu His Asn Ile Lys Lys Ser
            1015                1020                1025

AAA ATG TTC TTC AAA GAT ATT GAA GAA CAA TAT CCT ACT AGT TTA GCT     3357
Lys Met Phe Phe Lys Asp Ile Glu Glu Gln Tyr Pro Thr Ser Leu Ala
        1030                1035                1040

TGT GTT GAA ATT GTA AAT ACC TTG GCA TTA GAT AAT CAA AAG AAA CTG     3405
Cys Val Glu Ile Val Asn Thr Leu Ala Leu Asp Asn Gln Lys Lys Leu
    1045                1050                1055

AGC AAG CCT CAG TCA ATT AAT ACT GTA TCT GCA CAT TTA CAG AGT AGT     3453
Ser Lys Pro Gln Ser Ile Asn Thr Val Ser Ala His Leu Gln Ser Ser
1060                1065                1070                1075

GTA GTT GTT TCT GAT TGT AAA AAT AGT CAT ATA ACC CCT CAG ATG TTA     3501
Val Val Val Ser Asp Cys Lys Asn Ser His Ile Thr Pro Gln Met Leu
                1080                1085                1090

TTT TCC AAG CAG GAT TTT AAT TCA AAC CAT AAT TTA ACA CCT AGC CAA     3549
Phe Ser Lys Gln Asp Phe Asn Ser Asn His Asn Leu Thr Pro Ser Gln
            1095                1100                1105

AAG GCA GAA ATT ACA GAA CTT TCT ACT ATA TTA GAA GAA TCA GGA AGT     3597
Lys Ala Glu Ile Thr Glu Leu Ser Thr Ile Leu Glu Glu Ser Gly Ser
        1110                1115                1120

CAG TTT GAA TTT ACT CAG TTT AGA AAA CCA AGC TAC ATA TTG CAG AAG     3645
Gln Phe Glu Phe Thr Gln Phe Arg Lys Pro Ser Tyr Ile Leu Gln Lys
    1125                1130                1135

AGT ACA TTT GAA GTG CCT GAA AAC CAG ATG ACT ATC TTA AAG ACC ACT     3693
Ser Thr Phe Glu Val Pro Glu Asn Gln Met Thr Ile Leu Lys Thr Thr
1140                1145                1150                1155

TCT GAG GAA TGC AGA GAT GCT GAT CTT CAT GTC ATA ATG AAT GCC CCA     3741
Ser Glu Glu Cys Arg Asp Ala Asp Leu His Val Ile Met Asn Ala Pro
                1160                1165                1170

TCG ATT GGT CAG GTA GAC AGC AGC AAG CAA TTT GAA GGT ACA GTT GAA     3789
Ser Ile Gly Gln Val Asp Ser Ser Lys Gln Phe Glu Gly Thr Val Glu
            1175                1180                1185

ATT AAA CGG AAG TTT GCT GGC CTG TTG AAA AAT GAC TGT AAC AAA AGT     3837
Ile Lys Arg Lys Phe Ala Gly Leu Leu Lys Asn Asp Cys Asn Lys Ser

1190                1195                1200

GCT TCT GGT TAT TTA ACA GAT GAA AAT GAA GTG GGG TTT AGG GGC TTT     3885
Ala Ser Gly Tyr Leu Thr Asp Glu Asn Glu Val Gly Phe Arg Gly Phe
    1205                1210                1215

TAT TCT GCT CAT GGC ACA AAA CTG AAT GTT TCT ACT GAA GCT CTG CAA     3933
Tyr Ser Ala His Gly Thr Lys Leu Asn Val Ser Thr Glu Ala Leu Gln
1220                1225                1230                1235

AAA GCT GTG AAA CTG TTT AGT GAT ATT GAG AAT ATT AGT GAG GAA ACT     3981
Lys Ala Val Lys Leu Phe Ser Asp Ile Glu Asn Ile Ser Glu Glu Thr
                1240                1245                1250

TCT GCA GAG GTA CAT CCA ATA AGT TTA TCT TCA AGT AAA TGT CAT GAT     4029
Ser Ala Glu Val His Pro Ile Ser Leu Ser Ser Ser Lys Cys His Asp
            1255                1260                1265

TCT GTT GTT TCA ATG TTT AAG ATA GAA AAT CAT AAT GAT AAA ACT GTA     4077
Ser Val Val Ser Met Phe Lys Ile Glu Asn His Asn Asp Lys Thr Val
        1270                1275                1280

AGT GAA AAA AAT AAT AAA TGC CAA CTG ATA TTA CAA AAT AAT ATT GAA     4125
Ser Glu Lys Asn Asn Lys Cys Gln Leu Ile Leu Gln Asn Asn Ile Glu
    1285                1290                1295

ATG ACT ACT GGC ACT TTT GTT GAA GAA ATT ACT GAA AAT TAC AAG AGA     4173
Met Thr Thr Gly Thr Phe Val Glu Glu Ile Thr Glu Asn Tyr Lys Arg
1300                1305                1310                1315

AAT ACT GAA AAT GAA GAT AAC AAA TAT ACT GCT GCC AGT AGA AAT TCT     4221
Asn Thr Glu Asn Glu Asp Asn Lys Tyr Thr Ala Ala Ser Arg Asn Ser
                1320                1325                1330

CAT AAC TTA GAA TTT GAT GGC AGT GAT TCA AGT AAA AAT GAT ACT GTT     4269
His Asn Leu Glu Phe Asp Gly Ser Asp Ser Ser Lys Asn Asp Thr Val
            1335                1340                1345

TGT ATT CAT AAA GAT GAA ACG GAC TTG CTA TTT ACT GAT CAG CAC AAC     4317
Cys Ile His Lys Asp Glu Thr Asp Leu Leu Phe Thr Asp Gln His Asn
        1350                1355                1360

ATA TGT CTT AAA TTA TCT GGC CAG TTT ATG AAG GAG GGA AAC ACT CAG     4365
Ile Cys Leu Lys Leu Ser Gly Gln Phe Met Lys Glu Gly Asn Thr Gln
    1365                1370                1375

ATT AAA GAA GAT TTG TCA GAT TTA ACT TTT TTG GAA GTT GCG AAA GCT     4413
Ile Lys Glu Asp Leu Ser Asp Leu Thr Phe Leu Glu Val Ala Lys Ala
1380                1385                1390                1395

CAA GAA GCA TGT CAT GGT AAT ACT TCA AAT AAA GAA CAG TTA ACT GCT     4461
Gln Glu Ala Cys His Gly Asn Thr Ser Asn Lys Glu Gln Leu Thr Ala
                1400                1405                1410

ACT AAA ACG GAG CAA AAT ATA AAA GAT TTT GAG ACT TCT GAT ACA TTT     4509
Thr Lys Thr Glu Gln Asn Ile Lys Asp Phe Glu Thr Ser Asp Thr Phe
            1415                1420                1425

TTT CAG ACT GCA AGT GGG AAA AAT ATT AGT GTC GCC AAA GAG TCA TTT     4557
Phe Gln Thr Ala Ser Gly Lys Asn Ile Ser Val Ala Lys Glu Ser Phe
        1430                1435                1440

AAT AAA ATT GTA AAT TTC TTT GAT CAG AAA CCA GAA GAA TTG CAT AAC     4605
Asn Lys Ile Val Asn Phe Phe Asp Gln Lys Pro Glu Glu Leu His Asn
    1445                1450                1455

TTT TCC TTA AAT TCT GAA TTA CAT TCT GAC ATA AGA AAG AAC AAA ATG     4653
Phe Ser Leu Asn Ser Glu Leu His Ser Asp Ile Arg Lys Asn Lys Met
1460                1465                1470                1475

GAC ATT CTA AGT TAT GAG GAA ACA GAC ATA GTT AAA CAC AAA ATA CTG     4701
Asp Ile Leu Ser Tyr Glu Glu Thr Asp Ile Val Lys His Lys Ile Leu
                1480                1485                1490

AAA GAA AGT GTC CCA GTT GGT ACT GGA AAT CAA CTA GTG ACC TTC CAG     4749
Lys Glu Ser Val Pro Val Gly Thr Gly Asn Gln Leu Val Thr Phe Gln
            1495                1500                1505

GGA CAA CCC GAA CGT GAT GAA AAG ATC AAA GAA CCT ACT CTG TTG GGT     4797
Gly Gln Pro Glu Arg Asp Glu Lys Ile Lys Glu Pro Thr Leu Leu Gly
        1510                1515                1520

TTT CAT ACA GCT AGC GGG AAA AAA GTT AAA ATT GCA AAG GAA TCT TTG     4845
Phe His Thr Ala Ser Gly Lys Lys Val Lys Ile Ala Lys Glu Ser Leu
    1525                1530                1535

GAC AAA GTG AAA AAC CTT TTT GAT GAA AAA GAG CAA GGT ACT AGT GAA     4893
Asp Lys Val Lys Asn Leu Phe Asp Glu Lys Glu Gln Gly Thr Ser Glu
1540                1545                1550                1555

ATC ACC AGT TTT AGC CAT CAA TGG GCA AAG ACC CTA AAG TAC AGA GAG     4941
Ile Thr Ser Phe Ser His Gln Trp Ala Lys Thr Leu Lys Tyr Arg Glu
                1560                1565                1570

GCC TGT AAA GAC CTT GAA TTA GCA TGT GAG ACC ATT GAG ATC ACA GCT     4989
Ala Cys Lys Asp Leu Glu Leu Ala Cys Glu Thr Ile Glu Ile Thr Ala
            1575                1580                1585

GCC CCA AAG TGT AAA GAA ATG CAG AAT TCT CTC AAT AAT GAT AAA AAC     5037
Ala Pro Lys Cys Lys Glu Met Gln Asn Ser Leu Asn Asn Asp Lys Asn
        1590                1595                1600

CTT GTT TCT ATT GAG ACT GTG GTG CCA CCT AAG CTC TTA AGT GAT AAT     5085
Leu Val Ser Ile Glu Thr Val Val Pro Pro Lys Leu Leu Ser Asp Asn
    1605                1610                1615

TTA TGT AGA CAA ACT GAA AAT CTC AAA ACA TCA AAA AGT ATC TTT TTG     5133
Leu Cys Arg Gln Thr Glu Asn Leu Lys Thr Ser Lys Ser Ile Phe Leu
1620                1625                1630                1635

AAA GTT AAA GTA CAT GAA AAT GTA GAA AAA GAA ACA GCA AAA AGT CCT     5181
Lys Val Lys Val His Glu Asn Val Glu Lys Glu Thr Ala Lys Ser Pro
                1640                1645                1650

GCA ACT TGT TAC ACA AAT CAG TCC CCT TAT TCA GTC ATT GAA AAT TCA     5229
Ala Thr Cys Tyr Thr Asn Gln Ser Pro Tyr Ser Val Ile Glu Asn Ser
            1655                1660                1665

GCC TTA GCT TTT TAC ACA AGT TGT AGT AGA AAA ACT TCT GTG AGT CAG     5277
Ala Leu Ala Phe Tyr Thr Ser Cys Ser Arg Lys Thr Ser Val Ser Gln
        1670                1675                1680

ACT TCA TTA CTT GAA GCA AAA AAA TGG CTT AGA GAA GGA ATA TTT GAT 5325
Thr Ser Leu Leu Glu Ala Lys Lys Trp Leu Arg Glu Gly Ile Phe Asp
    1685                1690                1695

GGT CAA CCA GAA AGA ATA AAT ACT GCA GAT TAT GTA GGA AAT TAT TTG 5373
Gly Gln Pro Glu Arg Ile Asn Thr Ala Asp Tyr Val Gly Asn Tyr Leu
1700                1705                1710                1715

TAT GAA AAT AAT TCA AAC AGT ACT ATA GCT GAA AAT GAC AAA AAT CAT 5421
Tyr Glu Asn Asn Ser Asn Ser Thr Ile Ala Glu Asn Asp Lys Asn His
                1720                1725                1730

CTC TCC GAA AAA CAA GAT ACT TAT TTA AGT AAC AGT AGC ATG TCT AAC 5469
Leu Ser Glu Lys Gln Asp Thr Tyr Leu Ser Asn Ser Ser Met Ser Asn
            1735                1740                1745

AGC TAT TCC TAC CAT TCT GAT GAG GTA TAT AAT GAT TCA GGA TAT CTC 5517
Ser Tyr Ser Tyr His Ser Asp Glu Val Tyr Asn Asp Ser Gly Tyr Leu
        1750                1755                1760

TCA AAA AAT AAA CTT GAT TCT GGT ATT GAG CCA GTA TTG AAG AAT GTT 5565
Ser Lys Asn Lys Leu Asp Ser Gly Ile Glu Pro Val Leu Lys Asn Val
    1765                1770                1775

GAA GAT CAA AAA AAC ACT AGT TTT TCC AAA GTA ATA TCC AAT GTA AAA 5613
Glu Asp Gln Lys Asn Thr Ser Phe Ser Lys Val Ile Ser Asn Val Lys
1780                1785                1790                1795

GAT GCA AAT GCA TAC CCA CAA ACT GTA AAT GAA GAT ATT TGC GTT GAG 5661
Asp Ala Asn Ala Tyr Pro Gln Thr Val Asn Glu Asp Ile Cys Val Glu
                1800                1805                1810

GAA CTT GTG ACT AGC TCT TCA CCC TGC AAA AAT AAA AAT GCA GCC ATT 5709
Glu Leu Val Thr Ser Ser Ser Pro Cys Lys Asn Lys Asn Ala Ala Ile
            1815                1820                1825

AAA TTG TCC ATA TCT AAT AGT AAT AAT TTT GAG GTA GGG CCA CCT GCA 5757
Lys Leu Ser Ile Ser Asn Ser Asn Asn Phe Glu Val Gly Pro Pro Ala
        1830                1835                1840

TTT AGG ATA GCC AGT GGT AAA ATC GTT TGT GTT TCA CAT GAA ACA ATT 5805
Phe Arg Ile Ala Ser Gly Lys Ile Val Cys Val Ser His Glu Thr Ile
    1845                1850                1855

AAA AAA GTG AAA GAC ATA TTT ACA GAC AGT TTC AGT AAA GTA ATT AAG 5853
Lys Lys Val Lys Asp Ile Phe Thr Asp Ser Phe Ser Lys Val Ile Lys
1860                1865                1870                1875

GAA AAC AAC GAG AAT AAA TCA AAA ATT TGC CAA ACG AAA ATT ATG GCA 5901
Glu Asn Asn Glu Asn Lys Ser Lys Ile Cys Gln Thr Lys Ile Met Ala
                1880                1885                1890

GGT TGT TAC GAG GCA TTG GAT GAT TCA GAG GAT ATT CTT CAT AAC TCT 5949
Gly Cys Tyr Glu Ala Leu Asp Asp Ser Glu Asp Ile Leu His Asn Ser
            1895                1900                1905

CTA GAT AAT GAT GAA TGT AGC ACG CAT TCA CAT AAG GTT TTT GCT GAC 5997
Leu Asp Asn Asp Glu Cys Ser Thr His Ser His Lys Val Phe Ala Asp
        1910                1915                1920

ATT CAG AGT GAA GAA ATT TTA CAA CAT AAC CAA AAT ATG TCT GGA TTG 6045

Ile Gln Ser Glu Glu Ile Leu Gln His Asn Gln Asn Met Ser Gly Leu
    1925                1930                1935

GAG AAA GTT TCT AAA ATA TCA CCT TGT GAT GTT AGT TTG GAA ACT TCA 6093
Glu Lys Val Ser Lys Ile Ser Pro Cys Asp Val Ser Leu Glu Thr Ser
1940                1945                1950                1955

GAT ATA TGT AAA TGT AGT ATA GGG AAG CTT CAT AAG TCA GTC TCA TCT 6141
Asp Ile Cys Lys Cys Ser Ile Gly Lys Leu His Lys Ser Val Ser Ser
                1960                1965                1970

GCA AAT ACT TGT GGG ATT TTT AGC ACA GCA AGT GGA AAA TCT GTC CAG 6189
Ala Asn Thr Cys Gly Ile Phe Ser Thr Ala Ser Gly Lys Ser Val Gln
            1975                1980                1985

GTA TCA GAT GCT TCA TTA CAA AAC GCA AGA CAA GTG TTT TCT GAA ATA 6237
Val Ser Asp Ala Ser Leu Gln Asn Ala Arg Gln Val Phe Ser Glu Ile
        1990                1995                2000

GAA GAT AGT ACC AAG CAA GTC TTT TCC AAA GTA TTG TTT AAA AGT AAC 6285
Glu Asp Ser Thr Lys Gln Val Phe Ser Lys Val Leu Phe Lys Ser Asn
    2005                2010                2015

GAA CAT TCA GAC CAG CTC ACA AGA GAA GAA AAT ACT GCT ATA CGT ACT 6333
Glu His Ser Asp Gln Leu Thr Arg Glu Glu Asn Thr Ala Ile Arg Thr
2020                2025                2030                2035

CCA GAA CAT TTA ATA TCC CAA AAA GGC TTT TCA TAT AAT GTG GTA AAT 6381
Pro Glu His Leu Ile Ser Gln Lys Gly Phe Ser Tyr Asn Val Val Asn
                2040                2045                2050

TCA TCT GCT TTC TCT GGA TTT AGT ACA GCA AGT GGA AAG CAA GTT TCC 6429
Ser Ser Ala Phe Ser Gly Phe Ser Thr Ala Ser Gly Lys Gln Val Ser
            2055                2060                2065

ATT TTA GAA AGT TCC TTA CAC AAA GTT AAG GGA GTG TTA GAG GAA TTT 6477
Ile Leu Glu Ser Ser Leu His Lys Val Lys Gly Val Leu Glu Glu Phe
        2070                2075                2080

GAT TTA ATC AGA ACT GAG CAT AGT CTT CAC TAT TCA CCT ACG TCT AGA 6525
Asp Leu Ile Arg Thr Glu His Ser Leu His Tyr Ser Pro Thr Ser Arg
    2085                2090                2095

CAA AAT GTA TCA AAA ATA CTT CCT CGT GTT GAT AAG AGA AAC CCA GAG 6573
Gln Asn Val Ser Lys Ile Leu Pro Arg Val Asp Lys Arg Asn Pro Glu
2100                2105                2110                2115

CAC TGT GTA AAC TCA GAA ATG GAA AAA ACC TGC AGT AAA GAA TTT AAA 6621
His Cys Val Asn Ser Glu Met Glu Lys Thr Cys Ser Lys Glu Phe Lys
                2120                2125                2130

TTA TCA AAT AAC TTA AAT GTT GAA GGT GGT TCT TCA GAA AAT AAT CAC 6669
Leu Ser Asn Asn Leu Asn Val Glu Gly Gly Ser Ser Glu Asn Asn His
            2135                2140                2145

TCT ATT AAA GTT TCT CCA TAT CTC TCT CAA TTT CAA CAA GAC AAA CAA 6717
Ser Ile Lys Val Ser Pro Tyr Leu Ser Gln Phe Gln Gln Asp Lys Gln
        2150                2155                2160

CAG TTG GTA TTA GGA ACC AAA GTC TCA CTT GTT GAG AAC ATT CAT GTT 6765
Gln Leu Val Leu Gly Thr Lys Val Ser Leu Val Glu Asn Ile His Val

2165                2170                2175

TTG GGA AAA GAA CAG GCT TCA CCT AAA AAC GTA AAA ATG GAA ATT GGT     6813
Leu Gly Lys Glu Gln Ala Ser Pro Lys Asn Val Lys Met Glu Ile Gly
2180                2185                2190                2195

AAA ACT GAA ACT TTT TCT GAT GTT CCT GTG AAA ACA AAT ATA GAA GTT     6861
Lys Thr Glu Thr Phe Ser Asp Val Pro Val Lys Thr Asn Ile Glu Val
                2200                2205                2210

TGT TCT ACT TAC TCC AAA GAT TCA GAA AAC TAC TTT GAA ACA GAA GCA     6909
Cys Ser Thr Tyr Ser Lys Asp Ser Glu Asn Tyr Phe Glu Thr Glu Ala
            2215                2220                2225

GTA GAA ATT GCT AAA GCT TTT ATG GAA GAT GAT GAA CTG ACA GAT TCT     6957
Val Glu Ile Ala Lys Ala Phe Met Glu Asp Asp Glu Leu Thr Asp Ser
        2230                2235                2240

AAA CTG CCA AGT CAT GCC ACA CAT TCT CTT TTT ACA TGT CCC GAA AAT     7005
Lys Leu Pro Ser His Ala Thr His Ser Leu Phe Thr Cys Pro Glu Asn
    2245                2250                2255

GAG GAA ATG GTT TTG TCA AAT TCA AGA ATT GGA AAA AGA AGA GGA GAG     7053
Glu Glu Met Val Leu Ser Asn Ser Arg Ile Gly Lys Arg Arg Gly Glu
2260                2265                2270                2275

CCC CTT ATC TTA GTG GGA GAA CCC TCA ATC AAA AGA AAC TTA TTA AAT     7101
Pro Leu Ile Leu Val Gly Glu Pro Ser Ile Lys Arg Asn Leu Leu Asn
                2280                2285                2290

GAA TTT GAC AGG ATA ATA GAA AAT CAA GAA AAA TCC TTA AAG GCT TCA     7149
Glu Phe Asp Arg Ile Ile Glu Asn Gln Glu Lys Ser Leu Lys Ala Ser
            2295                2300                2305

AAA AGC ACT CCA GAT GGC ACA ATA AAA GAT CGA AGA TTG TTT ATG CAT     7197
Lys Ser Thr Pro Asp Gly Thr Ile Lys Asp Arg Arg Leu Phe Met His
        2310                2315                2320

CAT GTT TCT TTA GAG CCG ATT ACC TGT GTA CCC TTT CGC ACA ACT AAG     7245
His Val Ser Leu Glu Pro Ile Thr Cys Val Pro Phe Arg Thr Thr Lys
    2325                2330                2335

GAA CGT CAA GAG ATA CAG AAT CCA AAT TTT ACC GCA CCT GGT CAA GAA     7293
Glu Arg Gln Glu Ile Gln Asn Pro Asn Phe Thr Ala Pro Gly Gln Glu
2340                2345                2350                2355

TTT CTG TCT AAA TCT CAT TTG TAT GAA CAT CTG ACT TTG GAA AAA TCT     7341
Phe Leu Ser Lys Ser His Leu Tyr Glu His Leu Thr Leu Glu Lys Ser
                2360                2365                2370

TCA AGC AAT TTA GCA GTT TCA GGA CAT CCA TTT TAT CAA GTT TCT GCT     7389
Ser Ser Asn Leu Ala Val Ser Gly His Pro Phe Tyr Gln Val Ser Ala
            2375                2380                2385

ACA AGA AAT GAA AAA ATG AGA CAC TTG ATT ACT ACA GGC AGA CCA ACC     7437
Thr Arg Asn Glu Lys Met Arg His Leu Ile Thr Thr Gly Arg Pro Thr
        2390                2395                2400

AAA GTC TTT GTT CCA CCT TTT AAA ACT AAA TCA CAT TTT CAC AGA GTT     7485
Lys Val Phe Val Pro Pro Phe Lys Thr Lys Ser His Phe His Arg Val
    2405                2410                2415

GAA CAG TGT GTT AGG AAT ATT AAC TTG GAG GAA AAC AGA CAA AAG CAA 7533
Glu Gln Cys Val Arg Asn Ile Asn Leu Glu Glu Asn Arg Gln Lys Gln
2420                2425                2430                2435

AAC ATT GAT GGA CAT GGC TCT GAT GAT AGT AAA AAT AAG ATT AAT GAC 7581
Asn Ile Asp Gly His Gly Ser Asp Asp Ser Lys Asn Lys Ile Asn Asp
                2440                2445                2450

AAT GAG ATT CAT CAG TTT AAC AAA AAC AAC TCC AAT CAA GCA GCA GCT 7629
Asn Glu Ile His Gln Phe Asn Lys Asn Asn Ser Asn Gln Ala Ala Ala
            2455                2460                2465

GTA ACT TTC ACA AAG TGT GAA GAA GAA CCT TTA GAT TTA ATT ACA AGT 7677
Val Thr Phe Thr Lys Cys Glu Glu Glu Pro Leu Asp Leu Ile Thr Ser
        2470                2475                2480

CTT CAG AAT GCC AGA GAT ATA CAG GAT ATG CGA ATT AAG AAG AAA CAA 7725
Leu Gln Asn Ala Arg Asp Ile Gln Asp Met Arg Ile Lys Lys Lys Gln
    2485                2490                2495

AGG CAA CGC GTC TTT CCA CAG CCA GGC AGT CTG TAT CTT GCA AAA ACA 7773
Arg Gln Arg Val Phe Pro Gln Pro Gly Ser Leu Tyr Leu Ala Lys Thr
2500                2505                2510                2515

TCC ACT CTG CCT CGA ATC TCT CTG AAA GCA GCA GTA GGA GGC CAA GTT 7821
Ser Thr Leu Pro Arg Ile Ser Leu Lys Ala Ala Val Gly Gly Gln Val
                2520                2525                2530

CCC TCT GCG TGT TCT CAT AAA CAG CTG TAT ACG TAT GGC GTT TCT AAA 7869
Pro Ser Ala Cys Ser His Lys Gln Leu Tyr Thr Tyr Gly Val Ser Lys
            2535                2540                2545

CAT TGC ATA AAA ATT AAC AGC AAA AAT GCA GAG TCT TTT CAG TTT CAC 7917
His Cys Ile Lys Ile Asn Ser Lys Asn Ala Glu Ser Phe Gln Phe His
        2550                2555                2560

ACT GAA GAT TAT TTT GGT AAG GAA AGT TTA TGG ACT GGA AAA GGA ATA 7965
Thr Glu Asp Tyr Phe Gly Lys Glu Ser Leu Trp Thr Gly Lys Gly Ile
    2565                2570                2575

CAG TTG GCT GAT GGT GGA TGG CTC ATA CCC TCC AAT GAT GGA AAG GCT 8013
Gln Leu Ala Asp Gly Gly Trp Leu Ile Pro Ser Asn Asp Gly Lys Ala
2580                2585                2590                2595

GGA AAA GAA GAA TTT TAT AGG GCT CTG TGT GAC ACT CCA GGT GTG GAT 8061
Gly Lys Glu Glu Phe Tyr Arg Ala Leu Cys Asp Thr Pro Gly Val Asp
                2600                2605                2610

CCA AAG CTT ATT TCT AGA ATT TGG GTT TAT AAT CAC TAT AGA TGG ATC 8109
Pro Lys Leu Ile Ser Arg Ile Trp Val Tyr Asn His Tyr Arg Trp Ile
            2615                2620                2625

ATA TGG AAA CTG GCA GCT ATG GAA TGT GCC TTT CCT AAG GAA TTT GCT 8157
Ile Trp Lys Leu Ala Ala Met Glu Cys Ala Phe Pro Lys Glu Phe Ala
        2630                2635                2640

AAT AGA TGC CTA AGC CCA GAA AGG GTG CTT CTT CAA CTA AAA TAC AGA 8205
Asn Arg Cys Leu Ser Pro Glu Arg Val Leu Leu Gln Leu Lys Tyr Arg
    2645                2650                2655

TAT GAT ACG GAA ATT GAT AGA AGC AGA AGA TCG GCT ATA AAA AAG ATA 8253
Tyr Asp Thr Glu Ile Asp Arg Ser Arg Arg Ser Ala Ile Lys Lys Ile
2660                2665                2670                2675

ATG GAA AGG GAT GAC ACA GCT GCA AAA ACA CTT GTT CTC TGT GTT TCT 8301
Met Glu Arg Asp Asp Thr Ala Ala Lys Thr Leu Val Leu Cys Val Ser
                2680                2685                2690

GAC ATA ATT TCA TTG AGC GCA AAT ATA TCT GAA ACT TCT AGC AAT AAA 8349
Asp Ile Ile Ser Leu Ser Ala Asn Ile Ser Glu Thr Ser Ser Asn Lys
            2695                2700                2705

ACT AGT AGT GCA GAT ACC CAA AAA GTG GCC ATT ATT GAA CTT ACA GAT 8397
Thr Ser Ser Ala Asp Thr Gln Lys Val Ala Ile Ile Glu Leu Thr Asp
        2710                2715                2720

GGG TGG TAT GCT GTT AAG GCC CAG TTA GAT CCT CCC CTC TTA GCT GTC 8445
Gly Trp Tyr Ala Val Lys Ala Gln Leu Asp Pro Pro Leu Leu Ala Val
    2725                2730                2735

TTA AAG AAT GGC AGA CTG ACA GTT GGT CAG AAG ATT ATT CTT CAT GGA 8493
Leu Lys Asn Gly Arg Leu Thr Val Gly Gln Lys Ile Ile Leu His Gly
2740                2745                2750                2755

GCA GAA CTG GTG GGC TCT CCT GAT GCC TGT ACA CCT CTT GAA GCC CCA 8541
Ala Glu Leu Val Gly Ser Pro Asp Ala Cys Thr Pro Leu Glu Ala Pro
                2760                2765                2770

GAA TCT CTT ATG TTA AAG ATT TCT GCT AAC AGT ACT CGG CCT GCT CGC 8589
Glu Ser Leu Met Leu Lys Ile Ser Ala Asn Ser Thr Arg Pro Ala Arg
            2775                2780                2785

TGG TAT ACC AAA CTT GGA TTC TTT CCT GAC CCT AGA CCT TTT CCT CTG 8637
Trp Tyr Thr Lys Leu Gly Phe Phe Pro Asp Pro Arg Pro Phe Pro Leu
        2790                2795                2800

CCC TTA TCA TCG CTT TTC AGT GAT GGA GGA AAT GTT GGT TGT GTT GAT 8685
Pro Leu Ser Ser Leu Phe Ser Asp Gly Gly Asn Val Gly Cys Val Asp
    2805                2810                2815

GTA ATT ATT CAA AGA GCA TAC CCT ATA CAG TGG ATG GAG AAG ACA TCA 8733
Val Ile Ile Gln Arg Ala Tyr Pro Ile Gln Trp Met Glu Lys Thr Ser
2820                2825                2830                2835

TCT GGA TTA TAC ATA TTT CGC AAT GAA AGA GAG GAA GAA AAG GAA GCA 8781
Ser Gly Leu Tyr Ile Phe Arg Asn Glu Arg Glu Glu Glu Lys Glu Ala
                2840                2845                2850

GCA AAA TAT GTG GAG GCC CAA CAA AAG AGA CTA GAA GCC TTA TTC ACT 8829
Ala Lys Tyr Val Glu Ala Gln Gln Lys Arg Leu Glu Ala Leu Phe Thr
            2855                2860                2865

AAA ATT CAG GAG GAA TTT GAA GAA CAT GAA GAA AAC ACA ACA AAA CCA 8877
Lys Ile Gln Glu Glu Phe Glu Glu His Glu Glu Asn Thr Thr Lys Pro
        2870                2875                2880

TAT TTA CCA TCA CGT GCA CTA ACA AGA CAG CAA GTT CGT GCT TTG CAA 8925
Tyr Leu Pro Ser Arg Ala Leu Thr Arg Gln Gln Val Arg Ala Leu Gln
    2885                2890                2895

GAT GGT GCA GAG CTT TAT GAA GCA GTG AAG AAT GCA GCA GAC CCA GCT 8973

Asp Gly Ala Glu Leu Tyr Glu Ala Val Lys Asn Ala Ala Asp Pro Ala
2900                2905                2910                2915

TAC CTT GAG GGT TAT TTC AGT GAA GAG CAG TTA AGA GCC TTG AAT AAT     9021
Tyr Leu Glu Gly Tyr Phe Ser Glu Glu Gln Leu Arg Ala Leu Asn Asn
                2920                2925                2930

CAC AGG CAA ATG TTG AAT GAT AAG AAA CAA GCT CAG ATC CAG TTG GAA     9069
His Arg Gln Met Leu Asn Asp Lys Lys Gln Ala Gln Ile Gln Leu Glu
            2935                2940                2945

ATT AGG AAG ACC ATG GAA TCT GCT GAA CAA AAG GAA CAA GGT TTA TCA     9117
Ile Arg Lys Thr Met Glu Ser Ala Glu Gln Lys Glu Gln Gly Leu Ser
        2950                2955                2960

AGG GAT GTC ACA ACC GTG TGG AAG TTG CGT ATT GTA AGC TAT TCA AAA     9165
Arg Asp Val Thr Thr Val Trp Lys Leu Arg Ile Val Ser Tyr Ser Lys
    2965                2970                2975

AAA GAA AAA GAT TCA GTT ATA CTG AGT ATT TGG CGT CCA TCA TCA GAT     9213
Lys Glu Lys Asp Ser Val Ile Leu Ser Ile Trp Arg Pro Ser Ser Asp
2980                2985                2990                2995

TTA TAT TCT CTG TTA ACA GAA GGA AAG AGA TAC AGA ATT TAT CAT CTT     9261
Leu Tyr Ser Leu Leu Thr Glu Gly Lys Arg Tyr Arg Ile Tyr His Leu
                3000                3005                3010

GCA ACT TCA AAA TCT AAA AGT AAA TCT GAA AGA GCT AAC ATA CAG TTA     9309
Ala Thr Ser Lys Ser Lys Ser Lys Ser Glu Arg Ala Asn Ile Gln Leu
            3015                3020                3025

GCA GCG ACA AAA AAA ACT CAG TAT CAA CAA CTA CCG GTT TCA GAT GAA     9357
Ala Ala Thr Lys Lys Thr Gln Tyr Gln Gln Leu Pro Val Ser Asp Glu
        3030                3035                3040

ATT TTA TTT CAG ATT TAC CAG CCA CGG GAG CCC CTT CAC TTC AGC AAA     9405
Ile Leu Phe Gln Ile Tyr Gln Pro Arg Glu Pro Leu His Phe Ser Lys
    3045                3050                3055

TTT TTA GAT CCA GAC TTT CAG CCA TCT TGT TCT GAG GTG GAC CTA ATA     9453
Phe Leu Asp Pro Asp Phe Gln Pro Ser Cys Ser Glu Val Asp Leu Ile
3060                3065                3070                3075

GGA TTT GTC GTT TCT GTT GTG AAA AAA ACA GGA CTT GCC CCT TTC GTC     9501
Gly Phe Val Val Ser Val Val Lys Lys Thr Gly Leu Ala Pro Phe Val
                3080                3085                3090

TAT TTG TCA GAC GAA TGT TAC AAT TTA CTG GCA ATA AAG TTT TGG ATA     9549
Tyr Leu Ser Asp Glu Cys Tyr Asn Leu Leu Ala Ile Lys Phe Trp Ile
            3095                3100                3105

GAC CTT AAT GAG GAC ATT ATT AAG CCT CAT ATG TTA ATT GCT GCA AGC     9597
Asp Leu Asn Glu Asp Ile Ile Lys Pro His Met Leu Ile Ala Ala Ser
        3110                3115                3120

AAC CTC CAG TGG CGA CCA GAA TCC AAA TCA GGC CTT CTT ACT TTA TTT     9645
Asn Leu Gln Trp Arg Pro Glu Ser Lys Ser Gly Leu Leu Thr Leu Phe
    3125                3130                3135

GCT GGA GAT TTT TCT GTG TTT TCT GCT AGT CCA AAA GAG GGC CAC TTT     9693
Ala Gly Asp Phe Ser Val Phe Ser Ala Ser Pro Lys Glu Gly His Phe

3140                3145                3150                3155

CAA GAG ACA TTC AAC AAA ATG AAA AAT ACT GTT GAG AAT ATT GAC ATA    9741
Gln Glu Thr Phe Asn Lys Met Lys Asn Thr Val Glu Asn Ile Asp Ile
            3160                3165                3170

CTT TGC AAT GAA GCA GAA AAC AAG CTT ATG CAT ATA CTG CAT GCA AAT    9789
Leu Cys Asn Glu Ala Glu Asn Lys Leu Met His Ile Leu His Ala Asn
        3175                3180                3185

GAT CCC AAG TGG TCC ACC CCA ACT AAA GAC TGT ACT TCA GGG CCG TAC    9837
Asp Pro Lys Trp Ser Thr Pro Thr Lys Asp Cys Thr Ser Gly Pro Tyr
    3190                3195                3200

ACT GCT CAA ATC ATT CCT GGT ACA GGA AAC AAG CTT CTG ATG TCT TCT    9885
Thr Ala Gln Ile Ile Pro Gly Thr Gly Asn Lys Leu Leu Met Ser Ser
    3205                3210                3215

CCT AAT TGT GAG ATA TAT TAT CAA AGT CCT TTA TCA CTT TGT ATG GCC    9933
Pro Asn Cys Glu Ile Tyr Tyr Gln Ser Pro Leu Ser Leu Cys Met Ala
3220                3225                3230                3235

AAA AGG AAG TCT GTT TCC ACA CCT GTC TCA GCC CAG ATG ACT TCA AAG    9981
Lys Arg Lys Ser Val Ser Thr Pro Val Ser Ala Gln Met Thr Ser Lys
            3240                3245                3250

TCT TGT AAA GGG GAG AAA GAG ATT GAT GAC CAA AAG AAC TGC AAA AAG    10029
Ser Cys Lys Gly Glu Lys Glu Ile Asp Asp Gln Lys Asn Cys Lys Lys
        3255                3260                3265

AGA AGA GCC TTG GAT TTC TTG AGT AGA CTG CCT TTA CCT CCA CCT GTT    10077
Arg Arg Ala Leu Asp Phe Leu Ser Arg Leu Pro Leu Pro Pro Pro Val
    3270                3275                3280

AGT CCC ATT TGT ACA TTT GTT TCT CCG GCT GCA CAG AAG GCA TTT CAG    10125
Ser Pro Ile Cys Thr Phe Val Ser Pro Ala Ala Gln Lys Ala Phe Gln
    3285                3290                3295

CCA CCA AGG AGT TGT GGC ACC AAA TAC GAA ACA CCC ATA AAG AAA AAA    10173
Pro Pro Arg Ser Cys Gly Thr Lys Tyr Glu Thr Pro Ile Lys Lys Lys
3300                3305                3310                3315

GAA CTG AAT TCT CCT CAG ATG ACT CCA TTT AAA AAA TTC AAT GAA ATT    10221
Glu Leu Asn Ser Pro Gln Met Thr Pro Phe Lys Lys Phe Asn Glu Ile
            3320                3325                3330

TCT CTT TTG GAA AGT AAT TCA ATA GCT GAC GAA GAA CTT GCA TTG ATA    10269
Ser Leu Leu Glu Ser Asn Ser Ile Ala Asp Glu Glu Leu Ala Leu Ile
        3335                3340                3345

AAT ACC CAA GCT CTT TTG TCT GGT TCA ACA GGA GAA AAA CAA TTT ATA    10317
Asn Thr Gln Ala Leu Leu Ser Gly Ser Thr Gly Glu Lys Gln Phe Ile
    3350                3355                3360

TCT GTC AGT GAA TCC ACT AGG ACT GCT CCC ACC AGT TCA GAA GAT TAT    10365
Ser Val Ser Glu Ser Thr Arg Thr Ala Pro Thr Ser Ser Glu Asp Tyr
    3365                3370                3375

CTC AGA CTG AAA CGA CGT TGT ACT ACA TCT CTG ATC AAA GAA CAG GAG    10413
Leu Arg Leu Lys Arg Arg Cys Thr Thr Ser Leu Ile Lys Glu Gln Glu
3380                3385                3390                3395

AGT TCC CAG GCC AGT ACG GAA GAA TGT GAG AAA AAT AAG CAG GAC ACA    10461
Ser Ser Gln Ala Ser Thr Glu Glu Cys Glu Lys Asn Lys Gln Asp Thr
            3400                3405                3410

ATT ACA ACT AAA AAA TAT ATC TAA                                    10485
Ile Thr Thr Lys Lys Tyr Ile
        3415

(2) INFORMATION FOR SEQ ID NO:13:

(i) SEQUENCE CHARACTERISTICS:
(A) LENGTH: 3418 amino acids
(B) TYPE: amino acid
(C) STRANDEDNESS: single
(D) TOPOLOGY: linear

(ii) MOLECULE TYPE: protein
(v) FRAGMENT TYPE: internal

(xi) SEQUENCE DESCRIPTION: SEQ ID NO:13:

Met Pro Ile Gly Ser Lys Glu Arg Pro Thr Phe Phe Glu Ile Phe Lys
 1               5                  10                  15
Thr Arg Cys Asn Lys Ala Asp Leu Gly Pro Ile Ser Leu Asn Trp Phe
            20                  25                  30
Glu Glu Leu Ser Ser Glu Ala Pro Pro Tyr Asn Ser Glu Pro Ala Glu
        35                  40                  45
Glu Ser Glu His Lys Asn Asn Asn Tyr Glu Pro Asn Leu Phe Lys Thr
    50                  55                  60
Pro Gln Arg Lys Pro Ser Tyr Asn Gln Leu Ala Ser Thr Pro Ile Ile
65                  70                  75                  80
Phe Lys Glu Gln Gly Leu Thr Leu Pro Leu Tyr Gln Ser Pro Val Lys
                85                  90                  95
Glu Leu Asp Lys Phe Lys Leu Asp Leu Gly Arg Asn Val Pro Asn Ser
            100                 105                 110
Arg His Lys Ser Leu Arg Thr Val Lys Thr Lys Met Asp Gln Ala Asp
        115                 120                 125
Asp Val Ser Cys Pro Leu Leu Asn Ser Cys Leu Ser Glu Ser Pro Val
    130                 135                 140
Val Leu Gln Cys Thr His Val Thr Pro Gln Arg Asp Lys Ser Val Val
145                 150                 155                 160
Cys Gly Ser Leu Phe His Thr Pro Lys Phe Val Lys Gly Arg Gln Thr
                165                 170                 175
Pro Lys His Ile Ser Glu Ser Leu Gly Ala Glu Val Asp Pro Asp Met
            180                 185                 190
Ser Trp Ser Ser Ser Leu Ala Thr Pro Pro Thr Leu Ser Ser Thr Val
        195                 200                 205
Leu Ile Val Arg Asn Glu Glu Ala Ser Glu Thr Val Phe Pro His Asp
    210                 215                 220
Thr Thr Ala Asn Val Lys Ser Tyr Phe Ser Asn His Asp Glu Ser Leu
225                 230                 235                 240
Lys Lys Asn Asp Arg Phe Ile Ala Ser Val Thr Asp Ser Glu Asn Thr
                245                 250                 255
Asn Gln Arg Glu Ala Ala Ser His Gly Phe Gly Lys Thr Ser Gly Asn
            260                 265                 270
Ser Phe Lys Val Asn Ser Cys Lys Asp His Ile Gly Lys Ser Met Pro
        275                 280                 285
His Val Leu Glu Asp Glu Val Tyr Glu Thr Val Val Asp Thr Ser Glu
    290                 295                 300

Glu Asp Ser Phe Ser Leu Cys Phe Ser Lys Cys Arg Thr Lys Asn Leu
305                 310                 315                 320
Gln Lys Val Arg Thr Ser Lys Thr Arg Lys Lys Ile Phe His Glu Ala
                325                 330                 335
Asn Ala Asp Glu Cys Glu Lys Ser Lys Asn Gln Val Lys Glu Lys Tyr
            340                 345                 350
Ser Phe Val Ser Glu Val Glu Pro Asn Asp Thr Asp Pro Leu Asp Ser
        355                 360                 365
Asn Val Ala His Gln Lys Pro Phe Glu Ser Gly Ser Asp Lys Ile Ser
    370                 375                 380
Lys Glu Val Val Pro Ser Leu Ala Cys Glu Trp Ser Gln Leu Thr Leu
385                 390                 395                 400
Ser Gly Leu Asn Gly Ala Gln Met Glu Lys Ile Pro Leu Leu His Ile
                405                 410                 415
Ser Ser Cys Asp Gln Asn Ile Ser Glu Lys Asp Leu Leu Asp Thr Glu
            420                 425                 430
Asn Lys Arg Lys Lys Asp Phe Leu Thr Ser Glu Asn Ser Leu Pro Arg
        435                 440                 445
Ile Ser Ser Leu Pro Lys Ser Glu Lys Pro Leu Asn Glu Glu Thr Val
    450                 455                 460
Val Asn Lys Arg Asp Glu Glu Gln His Leu Glu Ser His Thr Asp Cys
465                 470                 475                 480
Ile Leu Ala Val Lys Gln Ala Ile Ser Gly Thr Ser Pro Val Ala Ser
                485                 490                 495
Ser Phe Gln Gly Ile Lys Lys Ser Ile Phe Arg Ile Arg Glu Ser Pro
            500                 505                 510
Lys Glu Thr Phe Asn Ala Ser Phe Ser Gly His Met Thr Asp Pro Asn
        515                 520                 525
Phe Lys Lys Glu Thr Glu Ala Ser Glu Ser Gly Leu Glu Ile His Thr
    530                 535                 540
Val Cys Ser Gln Lys Glu Asp Ser Leu Cys Pro Asn Leu Ile Asp Asn
545                 550                 555                 560
Gly Ser Trp Pro Ala Thr Thr Thr Gln Asn Ser Val Ala Leu Lys Asn
                565                 570                 575
Ala Gly Leu Ile Ser Thr Leu Lys Lys Lys Thr Asn Lys Phe Ile Tyr
            580                 585                 590
Ala Ile His Asp Glu Thr Ser Tyr Lys Gly Lys Lys Ile Pro Lys Asp
        595                 600                 605
Gln Lys Ser Glu Leu Ile Asn Cys Ser Ala Gln Phe Glu Ala Asn Ala
    610                 615                 620
Phe Glu Ala Pro Leu Thr Phe Ala Asn Ala Asp Ser Gly Leu Leu His
625                 630                 635                 640
Ser Ser Val Lys Arg Ser Cys Ser Gln Asn Asp Ser Glu Glu Pro Thr
                645                 650                 655
Leu Ser Leu Thr Ser Ser Phe Gly Thr Ile Leu Arg Lys Cys Ser Arg
            660                 665                 670
Asn Glu Thr Cys Ser Asn Asn Thr Val Ile Ser Gln Asp Leu Asp Tyr
        675                 680                 685
Lys Glu Ala Lys Cys Asn Lys Glu Lys Leu Gln Leu Phe Ile Thr Pro
    690                 695                 700
Glu Ala Asp Ser Leu Ser Cys Leu Gln Glu Gly Gln Cys Glu Asn Asp
705                 710                 715                 720
Pro Lys Ser Lys Lys Val Ser Asp Ile Lys Glu Glu Val Leu Ala Ala
                725                 730                 735
Ala Cys His Pro Val Gln His Ser Lys Val Glu Tyr Ser Asp Thr Asp
            740                 745                 750
Phe Gln Ser Gln Lys Ser Leu Leu Tyr Asp His Glu Asn Ala Ser Thr
        755                 760                 765
Leu Ile Leu Thr Pro Thr Ser Lys Asp Val Leu Ser Asn Leu Val Met
    770                 775                 780
Ile Ser Arg Gly Lys Glu Ser Tyr Lys Met Ser Asp Lys Leu Lys Gly

785                 790                 795                 800
Asn Asn Tyr Glu Ser Asp Val Glu Leu Thr Lys Asn Ile Pro Met Glu
                805                 810                 815
Lys Asn Gln Asp Val Cys Ala Leu Asn Glu Asn Tyr Lys Asn Val Glu
            820                 825                 830
Leu Leu Pro Pro Glu Lys Tyr Met Arg Val Ala Ser Pro Ser Arg Lys
        835                 840                 845
Val Gln Phe Asn Gln Asn Thr Asn Leu Arg Val Ile Gln Lys Asn Gln
    850                 855                 860
Glu Glu Thr Thr Ser Ile Ser Lys Ile Thr Val Asn Pro Asp Ser Glu
865                 870                 875                 880
Glu Leu Phe Ser Asp Asn Glu Asn Asn Phe Val Phe Gln Ile Ala Asn
                885                 890                 895
Glu Arg Asn Asn Leu Ala Leu Gly Asn Thr Lys Glu Leu His Glu Thr
            900                 905                 910
Asp Leu Thr Cys Val Asn Glu Pro Ile Phe Lys Asn Ser Thr Met Val
        915                 920                 925
Leu Tyr Gly Asp Thr Gly Asp Lys Gln Ala Thr Gln Val Ser Ile Lys
    930                 935                 940
Lys Asp Leu Val Tyr Val Leu Ala Glu Glu Asn Lys Asn Ser Val Lys
945                 950                 955                 960
Gln His Ile Lys Met Thr Leu Gly Gln Asp Leu Lys Ser Asp Ile Ser
                965                 970                 975
Leu Asn Ile Asp Lys Ile Pro Glu Lys Asn Asn Asp Tyr Met Asp Lys
            980                 985                 990
Trp Ala Gly Leu Leu Gly Pro Ile Ser Asn His Ser Phe Gly Gly Ser
        995                 1000                1005
Phe Arg Thr Ala Ser Asn Lys Glu Ile Lys Leu Ser Glu His Asn Ile
    1010                1015                1020
Lys Lys Ser Lys Met Phe Phe Lys Asp Ile Glu Glu Gln Tyr Pro Thr
1025                1030                1035                104
Ser Leu Ala Cys Val Glu Ile Val Asn Thr Leu Ala Leu Asp Asn Gln
                1045                1050                1055
Lys Lys Leu Ser Lys Pro Gln Ser Ile Asn Thr Val Ser Ala His Leu
            1060                1065                1070
Gln Ser Ser Val Val Val Ser Asp Cys Lys Asn Ser His Ile Thr Pro
        1075                1080                1085
Gln Met Leu Phe Ser Lys Gln Asp Phe Asn Ser Asn His Asn Leu Thr
    1090                1095                1100
Pro Ser Gln Lys Ala Glu Ile Thr Glu Leu Ser Thr Ile Leu Glu Glu
1105                1110                1115                112
Ser Gly Ser Gln Phe Glu Phe Thr Gln Phe Arg Lys Pro Ser Tyr Ile
                1125                1130                1135
Leu Gln Lys Ser Thr Phe Glu Val Pro Glu Asn Gln Met Thr Ile Leu
            1140                1145                1150
Lys Thr Thr Ser Glu Glu Cys Arg Asp Ala Asp Leu His Val Ile Met
        1155                1160                1165
Asn Ala Pro Ser Ile Gly Gln Val Asp Ser Ser Lys Gln Phe Glu Gly
    1170                1175                1180
Thr Val Glu Ile Lys Arg Lys Phe Ala Gly Leu Leu Lys Asn Asp Cys
1185                1190                1195                120
Asn Lys Ser Ala Ser Gly Tyr Leu Thr Asp Glu Asn Glu Val Gly Phe
                1205                1210                1215
Arg Gly Phe Tyr Ser Ala His Gly Thr Lys Leu Asn Val Ser Thr Glu
            1220                1225                1230
Ala Leu Gln Lys Ala Val Lys Leu Phe Ser Asp Ile Glu Asn Ile Ser
        1235                1240                1245
Glu Glu Thr Ser Ala Glu Val His Pro Ile Ser Leu Ser Ser Ser Lys
    1250                1255                1260
Cys His Asp Ser Val Val Ser Met Phe Lys Ile Glu Asn His Asn Asp
1265                1270                1275                128

Lys Thr Val Ser Glu Lys Asn Asn Lys Cys Gln Leu Ile Leu Gln Asn
                1285                1290                1295
Asn Ile Glu Met Thr Thr Gly Thr Phe Val Glu Glu Ile Thr Glu Asn
            1300                1305                1310
Tyr Lys Arg Asn Thr Glu Asn Glu Asp Asn Lys Tyr Thr Ala Ala Ser
        1315                1320                1325
Arg Asn Ser His Asn Leu Glu Phe Asp Gly Ser Asp Ser Ser Lys Asn
    1330                1335                1340
Asp Thr Val Cys Ile His Lys Asp Glu Thr Asp Leu Leu Phe Thr Asp
1345                1350                1355                136
Gln His Asn Ile Cys Leu Lys Leu Ser Gly Gln Phe Met Lys Glu Gly
                1365                1370                1375
Asn Thr Gln Ile Lys Glu Asp Leu Ser Asp Leu Thr Phe Leu Glu Val
            1380                1385                1390
Ala Lys Ala Gln Glu Ala Cys His Gly Asn Thr Ser Asn Lys Glu Gln
        1395                1400                1405
Leu Thr Ala Thr Lys Thr Glu Gln Asn Ile Lys Asp Phe Glu Thr Ser
    1410                1415                1420
Asp Thr Phe Phe Gln Thr Ala Ser Gly Lys Asn Ile Ser Val Ala Lys
1425                1430                1435                144
Glu Ser Phe Asn Lys Ile Val Asn Phe Phe Asp Gln Lys Pro Glu Glu
                1445                1450                1455
Leu His Asn Phe Ser Leu Asn Ser Glu Leu His Ser Asp Ile Arg Lys
            1460                1465                1470
Asn Lys Met Asp Ile Leu Ser Tyr Glu Glu Thr Asp Ile Val Lys His
        1475                1480                1485
Lys Ile Leu Lys Glu Ser Val Pro Val Gly Thr Gly Asn Gln Leu Val
    1490                1495                1500
Thr Phe Gln Gly Gln Pro Glu Arg Asp Glu Lys Ile Lys Glu Pro Thr
1505                1510                1515                152
Leu Leu Gly Phe His Thr Ala Ser Gly Lys Lys Val Lys Ile Ala Lys
                1525                1530                1535
Glu Ser Leu Asp Lys Val Lys Asn Leu Phe Asp Glu Lys Glu Gln Gly
            1540                1545                1550
Thr Ser Glu Ile Thr Ser Phe Ser His Gln Trp Ala Lys Thr Leu Lys
        1555                1560                1565
Tyr Arg Glu Ala Cys Lys Asp Leu Glu Leu Ala Cys Glu Thr Ile Glu
    1570                1575                1580
Ile Thr Ala Ala Pro Lys Cys Lys Glu Met Gln Asn Ser Leu Asn Asn
1585                1590                1595                160
Asp Lys Asn Leu Val Ser Ile Glu Thr Val Val Pro Pro Lys Leu Leu
                1605                1610                1615
Ser Asp Asn Leu Cys Arg Gln Thr Glu Asn Leu Lys Thr Ser Lys Ser
            1620                1625                1630
Ile Phe Leu Lys Val Lys Val His Glu Asn Val Glu Lys Glu Thr Ala
        1635                1640                1645
Lys Ser Pro Ala Thr Cys Tyr Thr Asn Gln Ser Pro Tyr Ser Val Ile
    1650                1655                1660
Glu Asn Ser Ala Leu Ala Phe Tyr Thr Ser Cys Ser Arg Lys Thr Ser
1665                1670                1675                168
Val Ser Gln Thr Ser Leu Leu Glu Ala Lys Lys Trp Leu Arg Glu Gly
                1685                1690                1695
Ile Phe Asp Gly Gln Pro Glu Arg Ile Asn Thr Ala Asp Tyr Val Gly
            1700                1705                1710
Asn Tyr Leu Tyr Glu Asn Asn Ser Asn Ser Thr Ile Ala Glu Asn Asp
        1715                1720                1725
Lys Asn His Leu Ser Glu Lys Gln Asp Thr Tyr Leu Ser Asn Ser Ser
    1730                1735                1740
Met Ser Asn Ser Tyr Ser Tyr His Ser Asp Glu Val Tyr Asn Asp Ser
1745                1750                1755                176
Gly Tyr Leu Ser Lys Asn Lys Leu Asp Ser Gly Ile Glu Pro Val Leu

1765                    1770                    1775
Lys Asn Val Glu Asp Gln Lys Asn Thr Ser Phe Ser Lys Val Ile Ser
            1780                    1785                    1790
Asn Val Lys Asp Ala Asn Ala Tyr Pro Gln Thr Val Asn Glu Asp Ile
        1795                    1800                    1805
Cys Val Glu Glu Leu Val Thr Ser Ser Ser Pro Cys Lys Asn Lys Asn
    1810                    1815                    1820
Ala Ala Ile Lys Leu Ser Ile Ser Asn Ser Asn Asn Phe Glu Val Gly
1825                    1830                    1835                    184
Pro Pro Ala Phe Arg Ile Ala Ser Gly Lys Ile Val Cys Val Ser His
                1845                    1850                    1855
Glu Thr Ile Lys Lys Val Lys Asp Ile Phe Thr Asp Ser Phe Ser Lys
            1860                    1865                    1870
Val Ile Lys Glu Asn Asn Glu Asn Lys Ser Lys Ile Cys Gln Thr Lys
        1875                    1880                    1885
Ile Met Ala Gly Cys Tyr Glu Ala Leu Asp Asp Ser Glu Asp Ile Leu
    1890                    1895                    1900
His Asn Ser Leu Asp Asn Asp Glu Cys Ser Thr His Ser His Lys Val
1905                    1910                    1915                    192
Phe Ala Asp Ile Gln Ser Glu Glu Ile Leu Gln His Asn Gln Asn Met
                1925                    1930                    1935
Ser Gly Leu Glu Lys Val Ser Lys Ile Ser Pro Cys Asp Val Ser Leu
            1940                    1945                    1950
Glu Thr Ser Asp Ile Cys Lys Cys Ser Ile Gly Lys Leu His Lys Ser
        1955                    1960                    1965
Val Ser Ser Ala Asn Thr Cys Gly Ile Phe Ser Thr Ala Ser Gly Lys
    1970                    1975                    1980
Ser Val Gln Val Ser Asp Ala Ser Leu Gln Asn Ala Arg Gln Val Phe
1985                    1990                    1995                    200
Ser Glu Ile Glu Asp Ser Thr Lys Gln Val Phe Ser Lys Val Leu Phe
                2005                    2010                    2015
Lys Ser Asn Glu His Ser Asp Gln Leu Thr Arg Glu Glu Asn Thr Ala
            2020                    2025                    2030
Ile Arg Thr Pro Glu His Leu Ile Ser Gln Lys Gly Phe Ser Tyr Asn
        2035                    2040                    2045
Val Val Asn Ser Ser Ala Phe Ser Gly Phe Ser Thr Ala Ser Gly Lys
    2050                    2055                    2060
Gln Val Ser Ile Leu Glu Ser Ser Leu His Lys Val Lys Gly Val Leu
2065                    2070                    2075                    208
Glu Glu Phe Asp Leu Ile Arg Thr Glu His Ser Leu His Tyr Ser Pro
                2085                    2090                    2095
Thr Ser Arg Gln Asn Val Ser Lys Ile Leu Pro Arg Val Asp Lys Arg
            2100                    2105                    2110
Asn Pro Glu His Cys Val Asn Ser Glu Met Glu Lys Thr Cys Ser Lys
        2115                    2120                    2125
Glu Phe Lys Leu Ser Asn Asn Leu Asn Val Glu Gly Gly Ser Ser Glu
    2130                    2135                    2140
Asn Asn His Ser Ile Lys Val Ser Pro Tyr Leu Ser Gln Phe Gln Gln
2145                    2150                    2155                    216
Asp Lys Gln Gln Leu Val Leu Gly Thr Lys Val Ser Leu Val Glu Asn
                2165                    2170                    2175
Ile His Val Leu Gly Lys Glu Gln Ala Ser Pro Lys Asn Val Lys Met
            2180                    2185                    2190
Glu Ile Gly Lys Thr Glu Thr Phe Ser Asp Val Pro Val Lys Thr Asn
        2195                    2200                    2205
Ile Glu Val Cys Ser Thr Tyr Ser Lys Asp Ser Glu Asn Tyr Phe Glu
    2210                    2215                    2220
Thr Glu Ala Val Glu Ile Ala Lys Ala Phe Met Glu Asp Asp Glu Leu
2225                    2230                    2235                    224
Thr Asp Ser Lys Leu Pro Ser His Ala Thr His Ser Leu Phe Thr Cys
                2245                    2250                    2255

Pro Glu Asn Glu Glu Met Val Leu Ser Asn Ser Arg Ile Gly Lys Arg
            2260                2265                2270
Arg Gly Glu Pro Leu Ile Leu Val Gly Glu Pro Ser Ile Lys Arg Asn
        2275                2280                2285
Leu Leu Asn Glu Phe Asp Arg Ile Ile Glu Asn Gln Glu Lys Ser Leu
    2290                2295                2300
Lys Ala Ser Lys Ser Thr Pro Asp Gly Thr Ile Lys Asp Arg Arg Leu
2305                2310                2315                232
Phe Met His His Val Ser Leu Glu Pro Ile Thr Cys Val Pro Phe Arg
                2325                2330                2335
Thr Thr Lys Glu Arg Gln Glu Ile Gln Asn Pro Asn Phe Thr Ala Pro
            2340                2345                2350
Gly Gln Glu Phe Leu Ser Lys Ser His Leu Tyr Glu His Leu Thr Leu
        2355                2360                2365
Glu Lys Ser Ser Ser Asn Leu Ala Val Ser Gly His Pro Phe Tyr Gln
    2370                2375                2380
Val Ser Ala Thr Arg Asn Glu Lys Met Arg His Leu Ile Thr Thr Gly
2385                2390                2395                240
Arg Pro Thr Lys Val Phe Val Pro Pro Phe Lys Thr Lys Ser His Phe
                2405                2410                2415
His Arg Val Glu Gln Cys Val Arg Asn Ile Asn Leu Glu Glu Asn Arg
            2420                2425                2430
Gln Lys Gln Asn Ile Asp Gly His Gly Ser Asp Asp Ser Lys Asn Lys
        2435                2440                2445
Ile Asn Asp Asn Glu Ile His Gln Phe Asn Lys Asn Asn Ser Asn Gln
    2450                2455                2460
Ala Ala Ala Val Thr Phe Thr Lys Cys Glu Glu Glu Pro Leu Asp Leu
2465                2470                2475                248
Ile Thr Ser Leu Gln Asn Ala Arg Asp Ile Gln Asp Met Arg Ile Lys
                2485                2490                2495
Lys Lys Gln Arg Gln Arg Val Phe Pro Gln Pro Gly Ser Leu Tyr Leu
            2500                2505                2510
Ala Lys Thr Ser Thr Leu Pro Arg Ile Ser Leu Lys Ala Ala Val Gly
        2515                2520                2525
Gly Gln Val Pro Ser Ala Cys Ser His Lys Gln Leu Tyr Thr Tyr Gly
    2530                2535                2540
Val Ser Lys His Cys Ile Lys Ile Asn Ser Lys Asn Ala Glu Ser Phe
2545                2550                2555                256
Gln Phe His Thr Glu Asp Tyr Phe Gly Lys Glu Ser Leu Trp Thr Gly
                2565                2570                2575
Lys Gly Ile Gln Leu Ala Asp Gly Gly Trp Leu Ile Pro Ser Asn Asp
            2580                2585                2590
Gly Lys Ala Gly Lys Glu Glu Phe Tyr Arg Ala Leu Cys Asp Thr Pro
        2595                2600                2605
Gly Val Asp Pro Lys Leu Ile Ser Arg Ile Trp Val Tyr Asn His Tyr
    2610                2615                2620
Arg Trp Ile Ile Trp Lys Leu Ala Ala Met Glu Cys Ala Phe Pro Lys
2625                2630                2635                264
Glu Phe Ala Asn Arg Cys Leu Ser Pro Glu Arg Val Leu Leu Gln Leu
                2645                2650                2655
Lys Tyr Arg Tyr Asp Thr Glu Ile Asp Arg Ser Arg Arg Ser Ala Ile
            2660                2665                2670
Lys Lys Ile Met Glu Arg Asp Asp Thr Ala Ala Lys Thr Leu Val Leu
        2675                2680                2685
Cys Val Ser Asp Ile Ile Ser Leu Ser Ala Asn Ile Ser Glu Thr Ser
    2690                2695                2700
Ser Asn Lys Thr Ser Ser Ala Asp Thr Gln Lys Val Ala Ile Ile Glu
2705                2710                2715                272
Leu Thr Asp Gly Trp Tyr Ala Val Lys Ala Gln Leu Asp Pro Pro Leu
                2725                2730                2735
Leu Ala Val Leu Lys Asn Gly Arg Leu Thr Val Gly Gln Lys Ile Ile

2740                    2745                    2750
Leu His Gly Ala Glu Leu Val Gly Ser Pro Asp Ala Cys Thr Pro Leu
        2755                    2760                    2765
Glu Ala Pro Glu Ser Leu Met Leu Lys Ile Ser Ala Asn Ser Thr Arg
    2770                    2775                    2780
Pro Ala Arg Trp Tyr Thr Lys Leu Gly Phe Phe Pro Asp Pro Arg Pro
2785                    2790                    2795                    280
Phe Pro Leu Pro Leu Ser Ser Leu Phe Ser Asp Gly Gly Asn Val Gly
                2805                    2810                    2815
Cys Val Asp Val Ile Ile Gln Arg Ala Tyr Pro Ile Gln Trp Met Glu
            2820                    2825                    2830
Lys Thr Ser Ser Gly Leu Tyr Ile Phe Arg Asn Glu Arg Glu Glu Glu
        2835                    2840                    2845
Lys Glu Ala Ala Lys Tyr Val Glu Ala Gln Gln Lys Arg Leu Glu Ala
    2850                    2855                    2860
Leu Phe Thr Lys Ile Gln Glu Glu Phe Glu Glu His Glu Glu Asn Thr
2865                    2870                    2875                    288
Thr Lys Pro Tyr Leu Pro Ser Arg Ala Leu Thr Arg Gln Gln Val Arg
                2885                    2890                    2895
Ala Leu Gln Asp Gly Ala Glu Leu Tyr Glu Ala Val Lys Asn Ala Ala
            2900                    2905                    2910
Asp Pro Ala Tyr Leu Glu Gly Tyr Phe Ser Glu Glu Gln Leu Arg Ala
        2915                    2920                    2925
Leu Asn Asn His Arg Gln Met Leu Asn Asp Lys Lys Gln Ala Gln Ile
    2930                    2935                    2940
Gln Leu Glu Ile Arg Lys Thr Met Glu Ser Ala Glu Gln Lys Glu Gln
2945                    2950                    2955                    296
Gly Leu Ser Arg Asp Val Thr Thr Val Trp Lys Leu Arg Ile Val Ser
                2965                    2970                    2975
Tyr Ser Lys Lys Glu Lys Asp Ser Val Ile Leu Ser Ile Trp Arg Pro
            2980                    2985                    2990
Ser Ser Asp Leu Tyr Ser Leu Leu Thr Glu Gly Lys Arg Tyr Arg Ile
        2995                    3000                    3005
Tyr His Leu Ala Thr Ser Lys Ser Lys Ser Lys Ser Glu Arg Ala Asn
    3010                    3015                    3020
Ile Gln Leu Ala Ala Thr Lys Lys Thr Gln Tyr Gln Gln Leu Pro Val
3025                    3030                    3035                    304
Ser Asp Glu Ile Leu Phe Gln Ile Tyr Gln Pro Arg Glu Pro Leu His
                3045                    3050                    3055
Phe Ser Lys Phe Leu Asp Pro Asp Phe Gln Pro Ser Cys Ser Glu Val
            3060                    3065                    3070
Asp Leu Ile Gly Phe Val Val Ser Val Val Lys Lys Thr Gly Leu Ala
        3075                    3080                    3085
Pro Phe Val Tyr Leu Ser Asp Glu Cys Tyr Asn Leu Leu Ala Ile Lys
    3090                    3095                    3100
Phe Trp Ile Asp Leu Asn Glu Asp Ile Ile Lys Pro His Met Leu Ile
3105                    3110                    3115                    312
Ala Ala Ser Asn Leu Gln Trp Arg Pro Glu Ser Lys Ser Gly Leu Leu
                3125                    3130                    3135
Thr Leu Phe Ala Gly Asp Phe Ser Val Phe Ser Ala Ser Pro Lys Glu
            3140                    3145                    3150
Gly His Phe Gln Glu Thr Phe Asn Lys Met Lys Asn Thr Val Glu Asn
        3155                    3160                    3165
Ile Asp Ile Leu Cys Asn Glu Ala Glu Asn Lys Leu Met His Ile Leu
    3170                    3175                    3180
His Ala Asn Asp Pro Lys Trp Ser Thr Pro Thr Lys Asp Cys Thr Ser
3185                    3190                    3195                    320
Gly Pro Tyr Thr Ala Gln Ile Ile Pro Gly Thr Gly Asn Lys Leu Leu
                3205                    3210                    3215
Met Ser Ser Pro Asn Cys Glu Ile Tyr Tyr Gln Ser Pro Leu Ser Leu
            3220                    3225                    3230

Cys Met Ala Lys Arg Lys Ser Val Ser Thr Pro Val Ser Ala Gln Met
        3235                3240                3245
Thr Ser Lys Ser Cys Lys Gly Glu Lys Glu Ile Asp Asp Gln Lys Asn
    3250                3255                3260
Cys Lys Lys Arg Arg Ala Leu Asp Phe Leu Ser Arg Leu Pro Leu Pro
3265                3270                3275                328
Pro Pro Val Ser Pro Ile Cys Thr Phe Val Ser Pro Ala Ala Gln Lys
                3285                3290                3295
Ala Phe Gln Pro Pro Arg Ser Cys Gly Thr Lys Tyr Glu Thr Pro Ile
            3300                3305                3310
Lys Lys Lys Glu Leu Asn Ser Pro Gln Met Thr Pro Phe Lys Lys Phe
        3315                3320                3325
Asn Glu Ile Ser Leu Leu Glu Ser Asn Ser Ile Ala Asp Glu Glu Leu
    3330                3335                3340
Ala Leu Ile Asn Thr Gln Ala Leu Leu Ser Gly Ser Thr Gly Glu Lys
3345                3350                3355                336
Gln Phe Ile Ser Val Ser Glu Ser Thr Arg Thr Ala Pro Thr Ser Ser
                3365                3370                3375
Glu Asp Tyr Leu Arg Leu Lys Arg Arg Cys Thr Thr Ser Leu Ile Lys
            3380                3385                3390
Glu Gln Glu Ser Ser Gln Ala Ser Thr Glu Glu Cys Glu Lys Asn Lys
        3395                3400                3405
Gln Asp Thr Ile Thr Thr Lys Lys Tyr Ile
    3410                3415

(2) INFORMATION FOR SEQ ID NO:14:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 2F primer

(xi) SEQUENCE DESCRIPTION: SEQ ID NO:14:

TGAGTTTTAC CTCAGTCACA                                                20

(2) INFORMATION FOR SEQ ID NO:16:

(i) SEQUENCE CHARACTERISTICS:
(A) LENGTH: 41 base pairs
(B) TYPE: nucleic acid
(C) STRANDEDNESS: single
(D) TOPOLOGY: linear

(xi) SEQUENCE DESCRIPTION: SEQ ID NO:16:

CAGGAAACAG CTATGACCCT GTGACGTACT GGGTTTTTAG C                        41

(2) INFORMATION FOR SEQ ID NO:17:

(i) SEQUENCE CHARACTERISTICS:
(A) LENGTH: 24 base pairs
(B) TYPE: nucleic acid
(C) STRANDEDNESS: single

(D) TOPOLOGY: linear

(A) NAME/KEY:
(B) LOCATION:
(D) OTHER INFORMATION: 3FII primer

(xi) SEQUENCE DESCRIPTION: SEQ ID NO:17:

GATCTTTAAC TGTTCTGGGT CACA 24

(2) INFORMATION FOR SEQ ID NO:18:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 3RII primer

(xi) SEQUENCE DESCRIPTION: SEQ ID NO:18:

CCCAGCATGA CACAATTAAT GA 22

(2) INFORMATION FOR SEQ ID NO:19:

(i) SEQUENCE CHARACTERISTICS:
(A) LENGTH: 44 base pairs
(B) TYPE: nucleic acid
(C) STRANDEDNESS: single
(D) TOPOLOGY: linear

(A) NAME/KEY:
(B) LOCATION:
(D) OTHER INFORMATION: 4F/M 13F primer

(xi) SEQUENCE DESCRIPTION: SEQ ID NO:19:

TGTAAAACGA CGGCCAGTAG AATGCAAATT TATAATCCAG AGTA 44

(2) INFORMATION FOR SEQ ID NO:20:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 4R-1A primer

(xi) SEQUENCE DESCRIPTION: SEQ ID NO:20:

ATCAGATTCA TCTTTATAGA AC 22

(2) INFORMATION FOR SEQ ID NO:21:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 5+6F/M13F primer

(xi) SEQUENCE DESCRIPTION: SEQ ID NO:21:

TGTAAAACGA CGGCCAGTTG TGTTGGCATT TTAAACATCA 40

(2) INFORMATION FOR SEQ ID NO:22:

(i) SEQUENCE CHARACTERISTICS:
(A) LENGTH: 38 base pairs
(B) TYPE: nucleic acid
(C) STRANDEDNESS: single
(D) TOPOLOGY: linear

(A) NAME/KEY:
(B) LOCATION:
(D) OTHER INFORMATION: 5+6R/M13R primer

(xi) SEQUENCE DESCRIPTION: SEQ ID NO:22:

CAGGAAACAG CTATGACCCA GGGCAAAGGT ATAACGCT 38

(2) INFORMATION FOR SEQ ID NO:23:

(i) SEQUENCE CHARACTERISTICS:
(A) LENGTH: 38 base pairs
(B) TYPE: nucleic acid
(C) STRANDEDNESS: single
(D) TOPOLOGY: linear

(A) NAME/KEY:
(B) LOCATION:
(D) OTHER INFORMATION: 7F/M13F primer

(xi) SEQUENCE DESCRIPTION: SEQ ID NO:23:

TGTAAAACGA CGGCCAGTTA AGTGAAATAA AGAGTGAA 38

(2) INFORMATION FOR SEQ ID NO:24:

(i) SEQUENCE CHARACTERISTICS:
(A) LENGTH: 36 base pairs
(B) TYPE: nucleic acid
(C) STRANDEDNESS: single
(D) TOPOLOGY: linear

(A) NAME/KEY:
(B) LOCATION:
(D) OTHER INFORMATION: 7R/M13R primer

(xi) SEQUENCE DESCRIPTION: SEQ ID NO:24:

CAGGAAACAG CTATGACCAG AAGTATTAGA GATGAC 36

(2) INFORMATION FOR SEQ ID NO:25:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 8F/M13F primer

(xi) SEQUENCE DESCRIPTION: SEQ ID NO:25:

TGTAAAACGA CGGCCAGTGC CATATCTTAC CACCTTGTGA 40

(2) INFORMATION FOR SEQ ID NO:26:

(i) SEQUENCE CHARACTERISTICS:
(A) LENGTH: 22 base pairs
(B) TYPE: nucleic acid
(C) STRANDEDNESS: single
(D) TOPOLOGY: linear

(ix) FEATURE:

(A) NAME/KEY:
(B) LOCATION:
(D) OTHER INFORMATION: 8FIA primer

(xi) SEQUENCE DESCRIPTION: SEQ ID NO:26:

TTGCATTCTA GTGATAATAT AC 22

(2) INFORMATION FOR SEQ ID NO:27:

(i) SEQUENCE CHARACTERISTICS:
(A) LENGTH: 19 base pairs
(B) TYPE: nucleic acid
(C) STRANDEDNESS: single
(D) TOPOLOGY: linear

(A) NAME/KEY:
(B) LOCATION:
(D) OTHER INFORMATION: 8RIA primer

(xi) SEQUENCE DESCRIPTION: SEQ ID NO:27:

AATTGTTAGC AATTTCAAC 19

(2) INFORMATION FOR SEQ ID NO:28:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 9F/M13F primer

(xi) SEQUENCE DESCRIPTION: SEQ ID NO:28:

TGTAAAACGA CGGCCAGTTG GACCTAGGTT GATTGCAGAT 40

(2) INFORMATION FOR SEQ ID NO:29:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 9R/M13R primer

(xi) SEQUENCE DESCRIPTION: SEQ ID NO:29:

CAGGAAACAG CTATGACCTA AACTGAGATC ACGGGTGACA 40

(2) INFORMATION FOR SEQ ID NO:30:

(i) SEQUENCE CHARACTERISTICS:
(A) LENGTH: 24 base pairs
(B) TYPE: nucleic acid
(C) STRANDEDNESS: single
(D) TOPOLOGY: linear

(A) NAME/KEY:
(B) LOCATION:
(D) OTHER INFORMATION: 10AF primer

(xi) SEQUENCE DESCRIPTION: SEQ ID NO:30:

GAATAATATA AATTATATGG CTTA 24

(2) INFORMATION FOR SEQ ID NO:31:

(i) SEQUENCE CHARACTERISTICS:
(A) LENGTH: 37 base pairs
(B) TYPE: nucleic acid
(C) STRANDEDNESS: single
(D) TOPOLOGY: linear

(A) NAME/KEY:
(B) LOCATION:

(D) OTHER INFORMATION: 10AR/M13R primer

(xi) SEQUENCE DESCRIPTION: SEQ ID NO:31:

CAGGAAACAG CTATGACCCC TAGTCTTGCT AGTTCTT 37

(2) INFORMATION FOR SEQ ID NO:32:

(i) SEQUENCE CHARACTERISTICS:
(A) LENGTH: 42 base pairs
(B) TYPE: nucleic acid
(C) STRANDEDNESS: single
(D) TOPOLOGY: linear

(A) NAME/KEY:
(B) LOCATION:
(D) OTHER INFORMATION: 10BF/M13F primer

(xi) SEQUENCE DESCRIPTION: SEQ ID NO:32:

TGTAAAACGA CGGCCAGTAR CTGAAGTGGA ACCAAATGAT AC 42

(2) INFORMATION FOR SEQ ID NO:33:

(i) SEQUENCE CHARACTERISTICS:
(A) LENGTH: 44 base pairs
(B) TYPE: nucleic acid
(C) STRANDEDNESS: single
(D) TOPOLOGY: linear

(A) NAME/KEY:
(B) LOCATION:
(D) OTHER INFORMATION: 10BR/M13R primer

(xi) SEQUENCE DESCRIPTION: SEQ ID NO:33:

CAGGAAACAG CTATGACCAC GTGGCAAAGA ATTCTCTGAA GTAA 44

(2) INFORMATION FOR SEQ ID NO:34:

(i) SEQUENCE CHARACTERISTICS:
(A) LENGTH: 40 base pairs
(B) TYPE: nucleic acid
(C) STRANDEDNESS: single
(D) TOPOLOGY: linear

(ix) FEATURE:

(A) NAME/KEY:
(B) LOCATION:
(D) OTHER INFORMATION: 10CF/M13F primer

(xi) SEQUENCE DESCRIPTION: SEQ ID NO:34:

TGTAAAACGA CGGCCAGTCA GCATCTTGAA TCTCATACAG 40

(2) INFORMATION FOR SEQ ID NO:35:

(i) SEQUENCE CHARACTERISTICS:

(A) LENGTH: 19 base pairs
(B) TYPE: nucleic acid
(C) STRANDEDNESS: single
(D) TOPOLOGY: linear

(A) NAME/KEY:
(B) LOCATION:
(D) OTHER INFORMATION: 10CRII primer

(xi) SEQUENCE DESCRIPTION: SEQ ID NO:35:

AGACAGAGGT ACCTGAATC 19

(2) INFORMATION FOR SEQ ID NO:36:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 11AF-M13 primer

(xi) SEQUENCE DESCRIPTION: SEQ ID NO:36:

TGTAAAACGA CGGCCAGTTG GTACTTTAAT TTTGTCACTT 40

(2) INFORMATION FOR SEQ ID NO:37:

(i) SEQUENCE CHARACTERISTICS:
(A) LENGTH: 37 base pairs
(B) TYPE: nucleic acid
(C) STRANDEDNESS: single
(D) TOPOLOGY: linear

(A) NAME/KEY:
(B) LOCATION:
(D) OTHER INFORMATION: 11AR-M13 primer

(xi) SEQUENCE DESCRIPTION: SEQ ID NO:37:

CAGGAAACAG CTATGACCTG CAGGCATGAC AGAGAAT 37

(2) INFORMATION FOR SEQ ID NO:38:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 11BF primer

(xi) SEQUENCE DESCRIPTION: SEQ ID NO:38:

AAGAAGCAAA ATGTAATAAG GA 22

(2) INFORMATION FOR SEQ ID NO:39:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 11BR primer

(xi) SEQUENCE DESCRIPTION: SEQ ID NO:39:

CATTTAAAGC ACATACATCT TG 22

(2) INFORMATION FOR SEQ ID NO:40:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11CF primer

(xi) SEQUENCE DESCRIPTION: SEQ ID NO:40:

TCTAGAGGCA AAGAATCATA C 21

(2) INFORMATION FOR SEQ ID NO:41:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 11CR primer

(xi) SEQUENCE DESCRIPTION: SEQ ID NO:41:

CAAGATTATT CCTTTCATTA GC 22

(2) INFORMATION FOR SEQ ID NO:42:

(i) SEQUENCE CHARACTERISTICS:
(A) LENGTH: 22 base pairs
(B) TYPE: nucleic acid
(C) STRANDEDNESS: single

(D) TOPOLOGY: linear

(A) NAME/KEY:
(B) LOCATION:
(D) OTHER INFORMATION: 11DF primer

(xi) SEQUENCE DESCRIPTION: SEQ ID NO:42:

AACCAAAACA CAAATCTAAG AG 22

(2) INFORMATION FOR SEQ ID NO:43:

(i) SEQUENCE CHARACTERISTICS:
(A) LENGTH: 23 base pairs
(B) TYPE: nucleic acid
(C) STRANDEDNESS: single
(D) TOPOLOGY: linear

(A) NAME/KEY:
(B) LOCATION:
(D) OTHER INFORMATION: 11DR primer

(xi) SEQUENCE DESCRIPTION: SEQ ID NO:43:

GTCATTTTTA TATGCTGCTT TAC 23

(2) INFORMATION FOR SEQ ID NO:44:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11EF primer

(xi) SEQUENCE DESCRIPTION: SEQ ID NO:44:

GGTTTTATAT GGAGACACAG G 21

(2) INFORMATION FOR SEQ ID NO:45:

(i) SEQUENCE CHARACTERISTICS:
(A) LENGTH: 23 base pairs
(B) TYPE: nucleic acid
(C) STRANDEDNESS: single
(D) TOPOLOGY: linear

(A) NAME/KEY:
(B) LOCATION:
(D) OTHER INFORMATION: 11ER primer

(xi) SEQUENCE DESCRIPTION: SEQ ID NO:45:

GTATTTACAA TTTCAACACA AGC 23

(2) INFORMATION FOR SEQ ID NO:46:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 11FF primer

(xi) SEQUENCE DESCRIPTION: SEQ ID NO:46:

ATCACAGTTT TGGAGGTAGC 20

(2) INFORMATION FOR SEQ ID NO:47:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11FR primer

(xi) SEQUENCE DESCRIPTION: SEQ ID NO:47:

CTGACTTCCT GATTCTTCTA A 21

(2) INFORMATION FOR SEQ ID NO:48:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11GF primer

(xi) SEQUENCE DESCRIPTION: SEQ ID NO:48:

CTCAGATGTT ATTTTCCAAG C 21

(2) INFORMATION FOR SEQ ID NO:49:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11GR primer

(xi) SEQUENCE DESCRIPTION: SEQ ID NO:49:

CTGTTAAATA ACCAGAAGCA C 21

(2) INFORMATION FOR SEQ ID NO:50:

(i) SEQUENCE CHARACTERISTICS:
(A) LENGTH: 18 base pairs
(B) TYPE: nucleic acid
(C) STRANDEDNESS: single
(D) TOPOLOGY: linear

(A) NAME/KEY:
(B) LOCATION:
(D) OTHER INFORMATION: 11HF primer

(xi) SEQUENCE DESCRIPTION: SEQ ID NO:50:

AGGTAGACAG CAGCAAGC 18

(2) INFORMATION FOR SEQ ID NO:51:

(i) SEQUENCE CHARACTERISTICS:
(A) LENGTH: 22 base pairs
(B) TYPE: nucleic acid
(C) STRANDEDNESS: single
(D) TOPOLOGY: linear

(ix) FEATURE:

(A) NAME/KEY: None
(B) LOCATION:
(D) OTHER INFORMATION: 11HR primer

(xi) SEQUENCE DESCRIPTION: SEQ ID NO:51:

GTAATATCAG TTGGCATTTA TT 22

(2) INFORMATION FOR SEQ ID NO:52:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11IF primer

(xi) SEQUENCE DESCRIPTION: SEQ ID NO:52:

TGCAGAGGTA CATCCAATAA G 21

(2) INFORMATION FOR SEQ ID NO:53:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11IR primer

(xi) SEQUENCE DESCRIPTION: SEQ ID NO:53:

GATCAGTAAA TAGCAAGTCC G 21

(2) INFORMATION FOR SEQ ID NO:54:

(i) SEQUENCE CHARACTERISTICS:
(A) LENGTH: 23 base pairs
(B) TYPE: nucleic acid
(C) STRANDEDNESS: single
(D) TOPOLOGY: linear

(A) NAME/KEY:
(B) LOCATION:
(D) OTHER INFORMATION: 11JF primer

(xi) SEQUENCE DESCRIPTION: SEQ ID NO:54:

TACTGAAAAT GAAGATAACA AAT 23

(2) INFORMATION FOR SEQ ID NO:55:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: !!JR primer

(xi) SEQUENCE DESCRIPTION: SEQ ID NO:55:

ATTTTGTTCT TTCTTATGTC AG 22

(2) INFORMATION FOR SEQ ID NO:56:

(i) SEQUENCE CHARACTERISTICS:
(A) LENGTH: 35 base pairs
(B) TYPE: nucleic acid
(C) STRANDEDNESS: single
(D) TOPOLOGY: linear

(A) NAME/KEY:
(B) LOCATION:

(D) OTHER INFORMATION: 11KF-M13 primer

(xi) SEQUENCE DESCRIPTION: SEQ ID NO:56:

TGTAAAACGA CGGCCAGTCT ACTAAAACGG AGCAA 35

(2) INFORMATION FOR SEQ ID NO:57:

(i) SEQUENCE CHARACTERISTICS:
(A) LENGTH: 35 base pairs
(B) TYPE: nucleic acid
(C) STRANDEDNESS: single
(D) TOPOLOGY: linear

(A) NAME/KEY:
(B) LOCATION:
(D) OTHER INFORMATION: 11KR-M13 primer

(xi) SEQUENCE DESCRIPTION: SEQ ID NO:57:

CAGGAAACAG CTATGACCGT ATGAAAACCC AACAG 35

(2) INFORMATION FOR SEQ ID NO:58:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 11LF primer

(xi) SEQUENCE DESCRIPTION: SEQ ID NO:58:

CACAAAATAC TGAAAGAAAG TG 22

(2) INFORMATION FOR SEQ ID NO:59:

(i) SEQUENCE CHARACTERISTICS:
(A) LENGTH: 19 base pairs
(B) TYPE: nucleic acid
(C) STRANDEDNESS: single
(D) TOPOLOGY: linear

(A) NAME/KEY:
(B) LOCATION:
(D) OTHER INFORMATION: 11LR primer

(xi) SEQUENCE DESCRIPTION: SEQ ID NO:59:

GGCACCACAG TCTCAATAG 19

(2) INFORMATION FOR SEQ ID NO:60:

(i) SEQUENCE CHARACTERISTICS:
(A) LENGTH: 20 base pairs

(B) TYPE: nucleic acid
(C) STRANDEDNESS: single
(D) TOPOLOGY: linear

(A) NAME/KEY:
(B) LOCATION:
(D) OTHER INFORMATION: 11MF primer

(xi) SEQUENCE DESCRIPTION: SEQ ID NO:60:

GCAAAGACCC TAAAGTACAG 20

(2) INFORMATION FOR SEQ ID NO:61:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 11MR primer

(xi) SEQUENCE DESCRIPTION: SEQ ID NO:61:

CATCAAATAT TCCTTCTCTA AG 22

(2) INFORMATION FOR SEQ ID NO:62:

(i) SEQUENCE CHARACTERISTICS:
(A) LENGTH: 35 base pairs
(B) TYPE: nucleic acid
(C) STRANDEDNESS: single
(D) TOPOLOGY: linear

(A) NAME/KEY:
(B) LOCATION:
(D) OTHER INFORMATION: 11NF-M13 primer

(xi) SEQUENCE DESCRIPTION: SEQ ID NO:62:

TGTAAAACGA CGGCCAGTGA AAATTCAGCC TTAGC 35

(2) INFORMATION FOR SEQ ID NO:63:

(i) SEQUENCE CHARACTERISTICS:
(A) LENGTH: 35 base pairs
(B) TYPE: nucleic acid
(C) STRANDEDNESS: single
(D) TOPOLOGY: linear

(A) NAME/KEY:
(B) LOCATION:
(D) OTHER INFORMATION: 11NR-M13 primer

(xi) SEQUENCE DESCRIPTION: SEQ ID NO:63:

CAGGAAACAG CTATGACCAT CAGAATGGTA GGAAT 35

(2) INFORMATION FOR SEQ ID NO:64:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 110F primer

(xi) SEQUENCE DESCRIPTION: SEQ ID NO:64:

GTACTATAGC TGAAAATGAC AA 22

(2) INFORMATION FOR SEQ ID NO:65:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 110R primer

(xi) SEQUENCE DESCRIPTION: SEQ ID NO:65:

ACCACTGGCT ATCCTAAATG 20

(2) INFORMATION FOR SEQ ID NO:66:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 11PF primer

(xi) SEQUENCE DESCRIPTION: SEQ ID NO:66:

TGAAGATATT TGCGTTGAGG 20

(2) INFORMATION FOR SEQ ID NO:67:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 11PR primer

(xi) SEQUENCE DESCRIPTION: SEQ ID NO:67:

GTCAGCAAAA ACCTTATGTG 20

(2) INFORMATION FOR SEQ ID NO:68:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11QF primer

(xi) SEQUENCE DESCRIPTION: SEQ ID NO:68:

ACGAAAATTA TGGCAGGTTG T 21

(2) INFORMATION FOR SEQ ID NO:69:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11QR primer

(xi) SEQUENCE DESCRIPTION: SEQ ID NO:69:

CTTGTCTTGC GTTTTGTAAT G 21

(2) INFORMATION FOR SEQ ID NO:70:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 11RF primer

(xi) SEQUENCE DESCRIPTION: SEQ ID NO:70:

GCTTCATAAG TCAGTCTCAT 20

(2) INFORMATION FOR SEQ ID NO:71:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 11RR primer

(xi) SEQUENCE DESCRIPTION: SEQ ID NO:71:

TCAAATTCCT CTAACACTCC 20

(2) INFORMATION FOR SEQ ID NO:72:

(i) SEQUENCE CHARACTERISTICS:
(A) LENGTH: 35 base pairs
(B) TYPE: nucleic acid
(C) STRANDEDNESS: single
(D) TOPOLOGY: linear

(A) NAME/KEY:
(B) LOCATION:
(D) OTHER INFORMATION: 11SF-M13 primer

(xi) SEQUENCE DESCRIPTION: SEQ ID NO:72:

TGTAAAACGA CGGCCAGTTA CAGCAAGTGG AAAGC 35

(2) INFORMATION FOR SEQ ID NO:73:

(i) SEQUENCE CHARACTERISTICS:
(A) LENGTH: 37 base pairs
(B) TYPE: nucleic acid
(C) STRANDEDNESS: single
(D) TOPOLOGY: linear

(A) NAME/KEY:
(B) LOCATION:
(D) OTHER INFORMATION: 11SR-M13 primer

(xi) SEQUENCE DESCRIPTION: SEQ ID NO:73:

CAGGAAACAG CTATGACCAA GTTTCAGTTT TACCAAT 37

(2) INFORMATION FOR SEQ ID NO:74:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:

(B) LOCATION:
(D) OTHER INFORMATION: 11TF primer

(xi) SEQUENCE DESCRIPTION: SEQ ID NO:74:

GTTCTTCAGA AAATAATCAC TC                                    22

(2) INFORMATION FOR SEQ ID NO:75:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 11TR primer

(xi) SEQUENCE DESCRIPTION: SEQ ID NO:75:

TGTAAAAAGA GAATGTGTGG C                                    21

(2) INFORMATION FOR SEQ ID NO:76:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 11UF-M13 primer

(xi) SEQUENCE DESCRIPTION: SEQ ID NO:76:

TGTAAAACGA CGGCCAGTAC TTTTTCTGAT GTTCCTGTG                                    39

(2) INFORMATION FOR SEQ ID NO:77:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 11UR-M13 primer

(xi) SEQUENCE DESCRIPTION: SEQ ID NO:77:

CAGGAAACAG CTATGACCTA AAAATAGTGA TTGGCAACA                                    39

(2) INFORMATION FOR SEQ ID NO:78:

(i) SEQUENCE CHARACTERISTICS:

(A) LENGTH: 42 base pairs
(B) TYPE: nucleic acid
(C) STRANDEDNESS: single
(D) TOPOLOGY: linear

(A) NAME/KEY:
(B) LOCATION:
(D) OTHER INFORMATION: 12F/M13F primer

(xi) SEQUENCE DESCRIPTION: SEQ ID NO:78:

TGTAAAACGA CGGCCAGTAG TGGTGTTTTA AAGTGGTCAA AA 42

(2) INFORMATION FOR SEQ ID NO:79:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 12R/M13R primer

(xi) SEQUENCE DESCRIPTION: SEQ ID NO:79:

CAGGAAACAG CTATGACCGG ATCCACCTGA GGTCAGAATA 40

(2) INFORMATION FOR SEQ ID NO:80:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 13-2F primer

(xi) SEQUENCE DESCRIPTION: SEQ ID NO:80:

TAACATTTAA GCATCCGTTA C 21

(2) INFORMATION FOR SEQ ID NO:81:

(i) SEQUENCE CHARACTERISTICS:
(A) LENGTH: 28 base pairs
(B) TYPE: nucleic acid
(C) STRANDEDNESS: single
(D) TOPOLOGY: linear

(A) NAME/KEY:
(B) LOCATION:
(D) OTHER INFORMATION: 13-2R primer

(xi) SEQUENCE DESCRIPTION: SEQ ID NO:81:

AAACGAGACT TTTCTCATAC TGTATTAG 28

(2) INFORMATION FOR SEQ ID NO:82:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 14F primer

(xi) SEQUENCE DESCRIPTION: SEQ ID NO:82:

ACCATGTAGC AAATGAGGGT CT 22

(2) INFORMATION FOR SEQ ID NO:83:

(i) SEQUENCE CHARACTERISTICS:
(A) LENGTH: 22 base pairs
(B) TYPE: nucleic acid
(C) STRANDEDNESS: single
(D) TOPOLOGY: linear

(A) NAME/KEY:
(B) LOCATION:
(D) OTHER INFORMATION: 14AR primer

(xi) SEQUENCE DESCRIPTION: SEQ ID NO:83:

GCTTTTGTCT GTTTTCCTCC AA 22

(2) INFORMATION FOR SEQ ID NO:84:

(i) SEQUENCE CHARACTERISTICS:
(A) LENGTH: 21 base pairs
(B) TYPE: nucleic acid
(C) STRANDEDNESS: single
(D) TOPOLOGY: linear

(A) NAME/KEY:
(B) LOCATION:
(D) OTHER INFORMATION: 15-2F primer

(xi) SEQUENCE DESCRIPTION: SEQ ID NO:84:

CCAGGGGTTG TGCTTTTTAA A 21

(2) INFORMATION FOR SEQ ID NO:85:

(i) SEQUENCE CHARACTERISTICS:
(A) LENGTH: 38 base pairs
(B) TYPE: nucleic acid
(C) STRANDEDNESS: single

(D) TOPOLOGY: linear

(A) NAME/KEY:
(B) LOCATION:
(D) OTHER INFORMATION: 15FUT/M13-R primer

(xi) SEQUENCE DESCRIPTION: SEQ ID NO:85:

CAGGAAACAG CTATGACCAC TCTGTCATAA AAGCCATC 38

(2) INFORMATION FOR SEQ ID NO:86:

(i) SEQUENCE CHARACTERISTICS:
(A) LENGTH: 24 base pairs
(B) TYPE: nucleic acid
(C) STRANDEDNESS: single
(D) TOPOLOGY: linear

(A) NAME/KEY:
(B) LOCATION:
(D) OTHER INFORMATION: 16AF primer

(xi) SEQUENCE DESCRIPTION: SEQ ID NO:86:

TTTGGTTTGT TATAATTGTT TTTA 24

(2) INFORMATION FOR SEQ ID NO:87:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 16AR primer

(xi) SEQUENCE DESCRIPTION: SEQ ID NO:87:

CCAACTTTTT AGTTCGAGAG 20

(2) INFORMATION FOR SEQ ID NO:88:

(i) SEQUENCE CHARACTERISTICS:
(A) LENGTH: 19 base pairs
(B) TYPE: nucleic acid
(C) STRANDEDNESS: single
(D) TOPOLOGY: linear

(A) NAME/KEY:
(B) LOCATION:
(D) OTHER INFORMATION: 17F primer

(xi) SEQUENCE DESCRIPTION: SEQ ID NO:88:

TTCAGTATCA TCCTATGTG 19

(2) INFORMATION FOR SEQ ID NO:89:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 17AR primer

(xi) SEQUENCE DESCRIPTION: SEQ ID NO:89:

AGAAACCTTA ACCCATACTG 20

(2) INFORMATION FOR SEQ ID NO:90:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 18FUT/M13-AF primer

(xi) SEQUENCE DESCRIPTION: SEQ ID NO:90:

TGTAAAACGA CGGCCAGTGA ATTCTAGAGT CACACTTCC 39

(2) INFORMATION FOR SEQ ID NO:91:

(i) SEQUENCE CHARACTERISTICS:
(A) LENGTH: 38 base pairs
(B) TYPE: nucleic acid
(C) STRANDEDNESS: single
(D) TOPOLOGY: linear

(A) NAME/KEY:
(B) LOCATION:
(D) OTHER INFORMATION: 18R/M13R primer

(xi) SEQUENCE DESCRIPTION: SEQ ID NO:91:

CAGGAAACAG CTATGACCTT TAACTGAATC AATGACTG 38

(2) INFORMATION FOR SEQ ID NO:92:

(i) SEQUENCE CHARACTERISTICS:
(A) LENGTH: 41 base pairs
(B) TYPE: nucleic acid
(C) STRANDEDNESS: single
(D) TOPOLOGY: linear

(A) NAME/KEY:
(B) LOCATION:
(D) OTHER INFORMATION: 19F/M13F primer

(xi) SEQUENCE DESCRIPTION: SEQ ID NO:92:

TGTAAAACGA CGGCCAGTAA GTGAATATTT TTAAGGCAGT T 41

(2) INFORMATION FOR SEQ ID NO:93:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 19FUT/M13-R primer

(xi) SEQUENCE DESCRIPTION: SEQ ID NO:93:

CAGGAAACAG CTATGACCAA GAGACCGAAA CTCCATCTC 39

(2) INFORMATION FOR SEQ ID NO:94:

(i) SEQUENCE CHARACTERISTICS:
(A) LENGTH: 38 base pairs
(B) TYPE: nucleic acid
(C) STRANDEDNESS: single
(D) TOPOLOGY: linear

(A) NAME/KEY:
(B) LOCATION:
(D) OTHER INFORMATION: 20F/M13F primer

(xi) SEQUENCE DESCRIPTION: SEQ ID NO:94:

TGTAAAACGA CGGCCAGTCA CTGTCCCTGG CCTGATAC 38

(2) INFORMATION FOR SEQ ID NO:95:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 20R/M13R primer

(xi) SEQUENCE DESCRIPTION: SEQ ID NO:95:

CAGGAAACAG CTATGACCAT GTTAAATTCA AAGTCTCTA 39

(2) INFORMATION FOR SEQ ID NO:96:

(i) SEQUENCE CHARACTERISTICS:
(A) LENGTH: 39 base pairs
(B) TYPE: nucleic acid
(C) STRANDEDNESS: single
(D) TOPOLOGY: linear

(A) NAME/KEY:
(B) LOCATION:
(D) OTHER INFORMATION: 21F/M13F primer

(xi) SEQUENCE DESCRIPTION: SEQ ID NO:96:

TGTAAAACGA CGGCCAGTGG GTGTTTTATG CTTGGTTCT 39

(2) INFORMATION FOR SEQ ID NO:97:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 21R/M13R primer

(xi) SEQUENCE DESCRIPTION: SEQ ID NO:97:

CAGGAAACAG CTATGACCCA TTTCAACATA TTCCTTCCTG 40

(2) INFORMATION FOR SEQ ID NO:98:

(i) SEQUENCE CHARACTERISTICS:
(A) LENGTH: 19 base pairs
(B) TYPE: nucleic acid
(C) STRANDEDNESS: single
(D) TOPOLOGY: linear

(A) NAME/KEY:
(B) LOCATION:
(D) OTHER INFORMATION: 22F-1A primer

(xi) SEQUENCE DESCRIPTION: SEQ ID NO:98:

AACCACACCC TTAAGATGA 19

(2) INFORMATION FOR SEQ ID NO:99:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 22R-1A primer

(xi) SEQUENCE DESCRIPTION: SEQ ID NO:99:

GCATTAGTAG TGGATTTTGC 20

(2) INFORMATION FOR SEQ ID NO:100:

(i) SEQUENCE CHARACTERISTICS:
(A) LENGTH: 16 base pairs
(B) TYPE: nucleic acid
(C) STRANDEDNESS: single
(D) TOPOLOGY: linear

(A) NAME/KEY:
(B) LOCATION:
(D) OTHER INFORMATION: 23FII primer

(xi) SEQUENCE DESCRIPTION: SEQ ID NO:100:

TCACTTCCAT TGCATC 16

(2) INFORMATION FOR SEQ ID NO:101:

(i) SEQUENCE CHARACTERISTICS:
(A) LENGTH: 17 base pairs
(B) TYPE: nucleic acid
(C) STRANDEDNESS: single
(D) TOPOLOGY: linear

(A) NAME/KEY:
(B) LOCATION:
(D) OTHER INFORMATION: 23RII primer

(xi) SEQUENCE DESCRIPTION: SEQ ID NO:101:

TGCCAACTGG TAGCTCC 17

(2) INFORMATION FOR SEQ ID NO:102:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 24 2F primer

(xi) SEQUENCE DESCRIPTION: SEQ ID NO:102:

TACAGTTAGC AGCGACAAAA 20

(2) INFORMATION FOR SEQ ID NO:103:

(i) SEQUENCE CHARACTERISTICS:
(A) LENGTH: 38 base pairs
(B) TYPE: nucleic acid

(C) STRANDEDNESS: single
(D) TOPOLOGY: linear

(A) NAME/KEY:
(B) LOCATION:
(D) OTHER INFORMATION: 24R/M13R primer

(xi) SEQUENCE DESCRIPTION: SEQ ID NO:103:

CAGGAAACAG CTATGACCAT TTGCCAACTG GTAGCTCC 38

(2) INFORMATION FOR SEQ ID NO:104:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 25F-7/23 primer

(xi) SEQUENCE DESCRIPTION: SEQ ID NO:104:

GCTTTCGCCA AATTCAGCTA 20

(2) INFORMATION FOR SEQ ID NO:105:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 25R-7/23 primer

(xi) SEQUENCE DESCRIPTION: SEQ ID NO:105:

TACCAAAATG TGTGGTGATG 20

(2) INFORMATION FOR SEQ ID NO:106:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 26-2F primer

(xi) SEQUENCE DESCRIPTION: SEQ ID NO:106:

AATCACTGAT ACTGGTTTTG 20

(2) INFORMATION FOR SEQ ID NO:107:

(i) SEQUENCE CHARACTERISTICS:
(A) LENGTH: 20 base pairs
(B) TYPE: nucleic acid
(C) STRANDEDNESS: single
(D) TOPOLOGY: linear

(A) NAME/KEY:
(B) LOCATION:
(D) OTHER INFORMATION: 26-2R primer

(xi) SEQUENCE DESCRIPTION: SEQ ID NO:107:

TATACTTACA GGAGCCACAT 20

(2) INFORMATION FOR SEQ ID NO:108:

(i) SEQUENCE CHARACTERISTICS:
(A) LENGTH: 18 base pairs
(B) TYPE: nucleic acid
(C) STRANDEDNESS: single
(D) TOPOLOGY: linear

(A) NAME/KEY:
(B) LOCATION:
(D) OTHER INFORMATION: 27AF-1A primer

(xi) SEQUENCE DESCRIPTION: SEQ ID NO:108:

CTGTGTGTAA TATTTGCG 18

(2) INFORMATION FOR SEQ ID NO:109:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 27AR/M13R primer

(xi) SEQUENCE DESCRIPTION: SEQ ID NO:109:

CAGGAAACAG CTATGACGGC AAGTTCTTCG TCAGCTATTG 40

(2) INFORMATION FOR SEQ ID NO:110:

(i) SEQUENCE CHARACTERISTICS:
(A) LENGTH: 40 base pairs
(B) TYPE: nucleic acid
(C) STRANDEDNESS: single
(D) TOPOLOGY: linear

(A) NAME/KEY:
(B) LOCATION:
(D) OTHER INFORMATION: 27BF/M13F primer

(xi) SEQUENCE DESCRIPTION: SEQ ID NO:110:

TGTAAAACGA CGGCCAGTGA ATTCTCCTCA GATGACTCCA 40

(2) INFORMATION FOR SEQ ID NO:111:

(i) SEQUENCE CHARACTERISTICS:
(A) LENGTH: 38 base pairs
(B) TYPE: nucleic acid
(C) STRANDEDNESS: single
(D) TOPOLOGY: linear

(A) NAME/KEY:
(B) LOCATION:
(D) OTHER INFORMATION: 27BR/M13R primer

(xi) SEQUENCE DESCRIPTION: SEQ ID NO:111:

CAGGAAACAG CTATGACCTC TTTGCTCATT GTGCAACA 38